(19) Europäisches Patentamt
European Patent Office
Office européen des brevets

(11) **EP 3 974 533 A1**

(12) **EUROPEAN PATENT APPLICATION**
published in accordance with Art. 153(4) EPC


(43) Date of publication:
**30.03.2022 Bulletin 2022/13**

(21) Application number: **20810635.1**

(22) Date of filing: **21.05.2020**

(51) International Patent Classification (IPC):
***C12N 15/113*** *(2010.01)* ***C07D 211/58*** *(2006.01)*
***C07C 229/16*** *(2006.01)* ***C07D 211/28*** *(2006.01)*
***C07C 229/26*** *(2006.01)* ***C07C 237/06*** *(2006.01)*
***C07C 229/24*** *(2006.01)* ***A61K 31/7088*** *(2006.01)*
***A61K 47/54*** *(2017.01)* ***A61P 19/06*** *(2006.01)*

(52) Cooperative Patent Classification (CPC):
**A61K 31/7088; A61K 47/54; A61P 19/06; C07C 229/16; C07C 229/24; C07C 229/26; C07C 237/06; C07D 211/28; C07D 211/58; C12N 15/113**

(86) International application number:
**PCT/CN2020/091485**

(87) International publication number:
**WO 2020/233651 (26.11.2020 Gazette 2020/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.05.2019 CN 201910430586**



(71) Applicant: **Suzhou Ribo Life Science Co., Ltd.
Kunshan, Jiangsu 215300 (CN)**

(72) Inventors:
- **ZHANG, Hongyan
Kunshan, Jiangsu 215300 (CN)**
- **GAO, Shan
Kunshan, Jiangsu 215300 (CN)**
- **KANG, Daiwu
Kunshan, Jiangsu 215300 (CN)**
- **LI, Haitao
Kunshan, Jiangsu 215300 (CN)**

(74) Representative: **Ladendorf, Oliver
Kraus & Weisert
Patentanwälte PartGmbB
Thomas-Wimmer-Ring 15
80539 München (DE)**


(54) **NUCLEIC ACID, PHARMACEUTICAL COMPOSITION, CONJUGATE, PREPARATION METHOD, AND USE**


(57) Provided are an siRNA which inhibits purine nucleoside phosphorylase (PNP) gene expression, a pharmaceutical composition comprising the siRNA, and an siRNA conjugate, capable of effectively treating and/or preventing abnormal uric acid metabolism, or diseases or physiological conditions caused by abnormal uric acid metabolism. Each nucleotide in the siRNA is independently a modified or unmodified nucleotide.


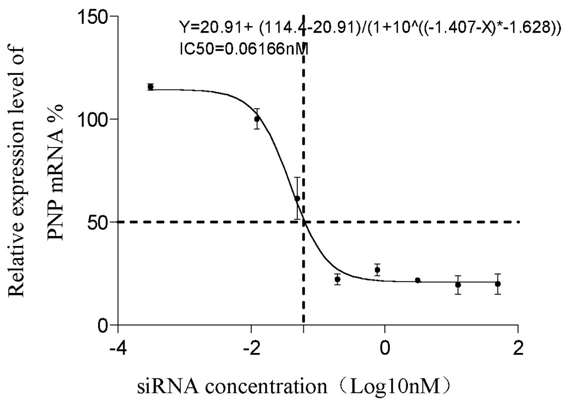


Figure 1D

EP 3 974 533 A1

Sequence Listing

<110> SU ZHOU RIBO LIFE SCIENCE CO.,LTD

<120> NUCLEIC ACID, PHARMACEUTICAL COMPOSITION, CONJUGATE, PREPARATION METHOD, AND USE

<130> FP1200356P

<150> CN 2019104305868
<151> 2019-5-22

<160> 280

<210> 1
<211> 19
<212> RNA
<213> Artificial Sequence

<220>
<221> misc_feature
<222> (19)..(19)
<223> n is Z1, Z1 is A

<220>
<223> siRNA

<400> 1
ccuaugaaga uuauaagan 19

<210> 2
<211> 19
<212> RNA
<213> Artificial Sequence

<220>
<221> misc_feature
<222> (1)..(1)
<223> n is Z2, Z2 is U

<220>
<223> siRNA

<400> 2
nucuuauaau cuucauagg 19

<210> 3
<211> 19
<212> RNA
<213> Artificial Sequence

<220>
<221> misc_feature
<222> (19)..(19)
<223> n is Z3, Z3 is A, U, G or C

<220>
<223> siRNA

<400> 3
ccuaugaaga uuauaagan 19

<210> 4

<211> 19
<212> RNA
<213> Artificial Sequence

<220>
<221> misc_feature
<222> (1)..(1)
<223> n is Z4, Z4 is a nucleotide complementary to Z3, Z3 is selected from A, U, G or C

<220>
<223> siRNA

<400> 4
nucuuauaau cuucauagg 19

<210> 5
<211> 19
<212> RNA
<213> Artificial Sequence

<220>
<221> misc_feature
<222> (19)..(19)
<223> n is Z3, Z3 is A, U, G or C

<220>
<223> siRNA

<400> 5
ccuaugaaga uuauaagan 19

<210> 6
<211> 21
<212> RNA
<213> Artificial Sequence

<220>
<221> misc_feature
<222> (1)..(1)
<223> n is Z4, Z4 is a nucleotide complementary to Z3, Z3 is selected from A, U, G or C

<220>
<223> siRNA

<400> 6
nucuuauaau cuucauaggu g 21

<210> 7
<211> 21
<212> RNA
<213> Artificial Sequence

<220>
<221> misc_feature
<222> (21)..(21)
<223> n is Z3, Z3 is A, U, G or C

<220>
<223> siRNA

<400> 7

caccuaugaa gauuauaaga n 21

<210> 8
<211> 23
<212> RNA
<213> Artificial Sequence

<220>
<221> misc_feature
<222> (1)..(1)
<223> n is Z4, Z4 is a nucleotide complementary to Z3,
Z3 is selected from A, U, G or C

<220>
<223> siRNA

<400> 8
nucuuauaau cuucauaggu gua 23

<210> 9
<211> 19
<212> RNA
<213> Artificial Sequence

<220>
<223> siRNA

<400> 9
ccuaugaaga uuauaagaa 19

<210> 10
<211> 21
<212> RNA
<213> Artificial Sequence

<220>
<223> siRNA

<400> 10
uucuuauaau cuucauaggu g 21

<210> 11
<211> 21
<212> RNA
<213> Artificial Sequence

<220>
<223> siRNA

<400> 11
caccuaugaa gauuauaaga a 21

<210> 12
<211> 23
<212> RNA
<213> Artificial Sequence

<220>
<223> siRNA

<400> 12
uucuuauaau cuucauaggu gua 23

<210> 13
<211> 19
<212> RNA
<213> Artificial Sequence

<220>
<223> siRNA

<400> 13
ccuaugaaga uuauaagaa 19

<210> 14
<211> 21
<212> RNA
<213> Artificial Sequence

<220>
<223> siRNA

<400> 14
uucuuauaau cuucauaggu g 21

<210> 15
<211> 21
<212> RNA
<213> Artificial Sequence

<220>
<223> siRNA

<400> 15
caccuaugaa gauuauaaga a 21

<210> 16
<211> 23
<212> RNA
<213> Artificial Sequence

<220>
<223> siRNA

<400> 16
uucuuauaau cuucauaggu gug 23

<210> 17
<211> 19
<212> RNA
<213> Artificial Sequence

<220>
<223> siRNA

<400> 17
ccuaugaaga uuauaagaa 19

<210> 18
<211> 21
<212> RNA
<213> Artificial Sequence

<220>
<223> siRNA

<400> 18
uucuuauaau cuucauaggu g 21

<210> 19
<211> 21
<212> RNA
<213> Artificial Sequence

<220>
<223> siRNA

<400> 19
caccuaugaa gauuauaaga a 21

<210> 20
<211> 23
<212> RNA
<213> Artificial Sequence

<220>
<223> siRNA

<400> 20
uucuuauaau cuucauaggu gug 23

<210> 21
<211> 19
<212> RNA
<213> Artificial Sequence

<220>
<223> siRNA

<400> 21
ccuaugaaga uuauaagaa 19

<210> 22
<211> 21
<212> RNA
<213> Artificial Sequence

<220>
<223> siRNA

<400> 22
uucuuauaau cuucauaggu g 21

<210> 23
<211> 21
<212> RNA
<213> Artificial Sequence

<220>
<223> siRNA

<400> 23
caccuaugaa gauuauaaga a 21

<210> 24
<211> 23
<212> RNA
<213> Artificial Sequence

<220>
<223> siRNA

<400> 24
uucuuauaau cuucauaggu gug 23

<210> 25
<211> 19
<212> RNA
<213> Artificial Sequence

<220>
<223> siRNA

<400> 25
ccuaugaaga uuauaagaa 19

<210> 26
<211> 21
<212> RNA
<213> Artificial Sequence

<220>
<223> siRNA

<400> 26
uucuuauaau cuucauaggu g 21

<210> 27
<211> 21
<212> RNA
<213> Artificial Sequence

<220>
<223> siRNA

<400> 27
caccuaugaa gauuauaaga a 21

<210> 28
<211> 23
<212> RNA
<213> Artificial Sequence

<220>
<223> siRNA

<400> 28
uucuuauaau cuucauaggu gug 23

<210> 29
<211> 19
<212> RNA
<213> Artificial Sequence

<220>
<223> siRNA

<400> 29
ccuaugaaga uuauaagaa 19

<210> 30
<211> 21

<212> RNA
<213> Artificial Sequence

<220>
<223> siRNA

<400> 30
uucuuauaau cuucauaggu g 21

<210> 31
<211> 21
<212> RNA
<213> Artificial Sequence

<220>
<223> siRNA

<400> 31
caccuaugaa gauuauaaga a 21

<210> 32
<211> 23
<212> RNA
<213> Artificial Sequence

<220>
<223> siRNA

<400> 32
uucuuauaau cuucauaggu gug 23

<210> 33
<211> 19
<212> RNA
<213> Artificial Sequence

<220>
<223> siRNA

<400> 33
ccuaugaaga uuauaagaa 19

<210> 34
<211> 21
<212> RNA
<213> Artificial Sequence

<220>
<223> siRNA

<400> 34
uucuuauaau cuucauaggu g 21

<210> 35
<211> 21
<212> RNA
<213> Artificial Sequence

<220>
<223> siRNA

<400> 35
caccuaugaa gauuauaaga a 21

<210> 36
<211> 23
<212> RNA
<213> Artificial Sequence

<220>
<223> siRNA

<400> 36
uucuuauaau cuucauaggu gug 23

<210> 37
<211> 19
<212> RNA
<213> Artificial Sequence

<220>
<223> siRNA

<400> 37
ccuaugaaga uuauaagaa 19

<210> 38
<211> 21
<212> RNA
<213> Artificial Sequence

<220>
<223> siRNA

<400> 38
uucuuauaau cuucauaggu g 21

<210> 39
<211> 21
<212> RNA
<213> Artificial Sequence

<220>
<223> siRNA

<400> 39
caccuaugaa gauuauaaga a 21

<210> 40
<211> 23
<212> RNA
<213> Artificial Sequence

<220>
<223> siRNA

<400> 40
uucuuauaau cuucauaggu gug 23

<210> 41
<211> 19
<212> RNA
<213> Artificial Sequence

<220>
<223> siRNA

<400> 41
ccuaugaaga uuauaagaa 19

<210> 42
<211> 21
<212> RNA
<213> Artificial Sequence

<220>
<223> siRNA

<400> 42
uucuuauaau cuucauaggu g 21

<210> 43
<211> 21
<212> RNA
<213> Artificial Sequence

<220>
<223> siRNA

<400> 43
caccuaugaa gauuauaaga a 21

<210> 44
<211> 23
<212> RNA
<213> Artificial Sequence

<220>
<223> siRNA

<400> 44
uucuuauaau cuucauaggu gug 23

<210> 45
<211> 19
<212> RNA
<213> Artificial Sequence

<220>
<223> siRNA

<400> 45
ccuaugaaga uuauaagaa 19

<210> 46
<211> 21
<212> RNA
<213> Artificial Sequence

<220>
<223> siRNA

<400> 46
uucuuauaau cuucauaggu g 21

<210> 47
<211> 21
<212> RNA
<213> Artificial Sequence

<220>
<223> siRNA

<400> 47
caccuaugaa gauuauaaga a 21

<210> 48
<211> 23
<212> RNA
<213> Artificial Sequence

<220>
<223> siRNA

<400> 48
uucuuauaau cuucauaggu gug 23

<210> 49
<211> 19
<212> RNA
<213> Artificial Sequence

<220>
<223> siRNA

<400> 49
ccuaugaaga uuauaagaa 19

<210> 50
<211> 21
<212> RNA
<213> Artificial Sequence

<220>
<223> siRNA

<400> 50
uucuuauaau cuucauaggu g 21

<210> 51
<211> 21
<212> RNA
<213> Artificial Sequence

<220>
<223> siRNA

<400> 51
caccuaugaa gauuauaaga a 21

<210> 52
<211> 23
<212> RNA
<213> Artificial Sequence

<220>
<223> siRNA

<400> 52
uucuuauaau cuucauaggu gug 23

<210> 53

<211> 19
<212> RNA
<213> Artificial Sequence

<220>
<223> siRNA

<400> 53
ccuaugaaga uuauaagaa 19

<210> 54
<211> 21
<212> RNA
<213> Artificial Sequence

<220>
<223> siRNA

<400> 54
uucuuauaau cuucauaggu g 21

<210> 55
<211> 21
<212> RNA
<213> Artificial Sequence

<220>
<223> siRNA

<400> 55
caccuaugaa gauuauaaga a 21

<210> 56
<211> 23
<212> RNA
<213> Artificial Sequence

<220>
<223> siRNA

<400> 56
uucuuauaau cuucauaggu gug 23

<210> 57
<211> 19
<212> RNA
<213> Artificial Sequence

<220>
<223> siRNA

<400> 57
ccuaugaaga uuauaagaa 19

<210> 58
<211> 21
<212> RNA
<213> Artificial Sequence

<220>
<223> siRNA

<400> 58

uucuuauaau cuucauaggu g 21

<210> 59
<211> 21
<212> RNA
<213> Artificial Sequence

<220>
<223> siRNA

<400> 59
caccuaugaa gauuauaaga a 21

<210> 60
<211> 23
<212> RNA
<213> Artificial Sequence

<220>
<223> siRNA

<400> 60
uucuuauaau cuucauaggu gug 23

<210> 61
<211> 19
<212> RNA
<213> Artificial Sequence

<220>
<221> misc_feature
<222> (19)..(19)
<223> n is Z5, Z5 is U

<220>
<223> siRNA

<400> 61
guacaguacc agaaguuan 19

<210> 62
<211> 19
<212> RNA
<213> Artificial Sequence

<220>
<221> misc_feature
<222> (1)..(1)
<223> n is Z6, Z6 is A

<220>
<223> siRNA

<400> 62
nuaacuucug guacuguac 19

<210> 63
<211> 19
<212> RNA
<213> Artificial Sequence

<220>
<221> misc_feature

<222> (19)..(19)
<223> n is Z7, Z7 is A, U, G or C

<220>
<223> siRNA

<400> 63
guacaguacc agaaguuan 19

<210> 64
<211> 19
<212> RNA
<213> Artificial Sequence

<220>
<221> misc_feature
<222> (1)..(1)
<223> n is Z8, Z8 is a nucleotide complementary to Z7,
Z3 is selected from A, U, G or C

<220>
<223> siRNA

<400> 64
nuaacuucug guacuguac 19

<210> 65
<211> 19
<212> RNA
<213> Artificial Sequence

<220>
<221> misc_feature
<222> (19)..(19)
<223> n is Z7, Z7 is A, U, G or C

<220>
<223> siRNA

<400> 65
guacaguacc agaaguuan 19

<210> 66
<211> 21
<212> RNA
<213> Artificial Sequence

<220>
<221> misc_feature
<222> (1)..(1)
<223> n is Z8, Z8 is a nucleotide complementary to Z7,
Z3 is selected from A, U, G or C

<220>
<223> siRNA

<400> 66
nuaacuucug guacuguacu c 21

<210> 67
<211> 21
<212> RNA
<213> Artificial Sequence

<220>
<221> misc_feature
<222> (21)..(21)
<223> n is Z7, Z7 is A, U, G or C

<220>
<223> siRNA

<400> 67
gaguacagua ccagaaguua n 21

<210> 68
<211> 23
<212> RNA
<213> Artificial Sequence

<220>
<221> misc_feature
<222> (1)..(1)
<223> n is Z8, Z8 is a nucleotide complementary to Z7,
Z3 is selected from A, U, G or C

<220>
<223> siRNA

<400> 68
nuaacuucug guacuguacu cau 23

<210> 69
<211> 19
<212> RNA
<213> Artificial Sequence

<220>
<223> siRNA

<400> 69
guacaguacc agaaguuau 19

<210> 70
<211> 21
<212> RNA
<213> Artificial Sequence

<220>
<223> siRNA

<400> 70
auaacuucug guacuguacuc 21

<210> 71
<211> 21
<212> RNA
<213> Artificial Sequence

<220>
<223> siRNA

<400> 71
gaguacagua ccagaaguua u 21

<210> 72

<211> 23
<212> RNA
<213> Artificial Sequence

<220>
<223> siRNA

<400> 72
auaacuucug guacuguacu cau 23

<210> 73
<211> 19
<212> RNA
<213> Artificial Sequence

<220>
<223> siRNA

<400> 73
guacaguacc agaaguuau 19

<210> 74
<211> 21
<212> RNA
<213> Artificial Sequence

<220>
<223> siRNA

<400> 74
auaacuucug guacuguacu c 21

<210> 75
<211> 19
<212> RNA
<213> Artificial Sequence

<220>
<223> siRNA

<400> 75
gaguacagua ccagaaguua u 19

<210> 76
<211> 23
<212> RNA
<213> Artificial Sequence

<220>
<223> siRNA

<400> 76
auaacuucug guacuguacu cau 23

<210> 77
<211> 19
<212> RNA
<213> Artificial Sequence

<220>
<223> siRNA

<400> 77

guacaguacc agaaguuau 19

<210> 78
<211> 21
<212> RNA
<213> Artificial Sequence

<220>
<223> siRNA

<400> 78
auaacuucug guacuguacu c 21

<210> 79
<211> 21
<212> RNA
<213> Artificial Sequence

<220>
<223> siRNA

<400> 79
gaguacagua ccagaaguua u 21

<210> 80
<211> 23
<212> RNA
<213> Artificial Sequence

<220>
<223> siRNA

<400> 80
auaacuucug guacuguacu cau 23

<210> 81
<211> 19
<212> RNA
<213> Artificial Sequence

<220>
<223> siRNA

<400> 81
guacaguacc agaaguuau 19

<210> 82
<211> 21
<212> RNA
<213> Artificial Sequence

<220>
<223> siRNA

<400> 82
auaacuucug guacuguacu c 21

<210> 83
<211> 21
<212> RNA
<213> Artificial Sequence

<220>

<223> siRNA

<400> 83
gaguacagua ccagaaguua u 21

<210> 84
<211> 23
<212> RNA
<213> Artificial Sequence

<220>
<223> siRNA

<400> 84
auaacuucug guacuguacu cau 23

<210> 85
<211> 19
<212> RNA
<213> Artificial Sequence

<220>
<223> siRNA

<400> 85
guacaguacc agaaguuau 19

<210> 86
<211> 21
<212> RNA
<213> Artificial Sequence

<220>
<223> siRNA

<400> 86
auaacuucug guacuguacu c 21

<210> 87
<211> 21
<212> RNA
<213> Artificial Sequence

<220>
<223> siRNA

<400> 87
gaguacagua ccagaaguua u 21

<210> 88
<211> 23
<212> RNA
<213> Artificial Sequence

<220>
<223> siRNA

<400> 88
auaacuucug guacuguacu cau 23

<210> 89
<211> 19
<212> RNA

<213>  Artificial Sequence

<220>
<223>  siRNA

<400>  89
guacaguacc agaaguuau                                                    19

<210>  90
<211>  21
<212>  RNA
<213>  Artificial Sequence

<220>
<223>  siRNA

<400>  90
auaacuucug guacuguacu c                                                 21

<210>  91
<211>  21
<212>  RNA
<213>  Artificial Sequence

<220>
<223>  siRNA

<400>  91
gaguacagua ccagaaguua u                                                 21

<210>  92
<211>  23
<212>  RNA
<213>  Artificial Sequence

<220>
<223>  siRNA

<400>  92
auaacuucug guacuguacu cau                                               23

<210>  93
<211>  19
<212>  RNA
<213>  Artificial Sequence

<220>
<223>  siRNA

<400>  93
guacaguacc agaaguuau                                                    19

<210>  94
<211>  21
<212>  RNA
<213>  Artificial Sequence

<220>
<223>  siRNA

<400>  94
auaacuucug guacuguacu c                                                 21

<210> 95
<211> 21
<212> RNA
<213> Artificial Sequence

<220>
<223> siRNA

<400> 95
gaguacagua ccagaaguua u 21

<210> 96
<211> 23
<212> RNA
<213> Artificial Sequence

<220>
<223> siRNA

<400> 96
auaacuucug guacuguacu cau 23

<210> 97
<211> 19
<212> RNA
<213> Artificial Sequence

<220>
<223> siRNA

<400> 97
guacaguacc agaaguuau 19

<210> 98
<211> 21
<212> RNA
<213> Artificial Sequence

<220>
<223> siRNA

<400> 98
auaacuucug guacuguacu c 21

<210> 99
<211> 21
<212> RNA
<213> Artificial Sequence

<220>
<223> siRNA

<400> 99
gaguacagua ccagaaguua u 21

<210> 100
<211> 23
<212> RNA
<213> Artificial Sequence

<220>
<223> siRNA

<400> 100
auaacuucug guacuguacu cau 23

<210> 101
<211> 19
<212> RNA
<213> Artificial Sequence

<220>
<223> siRNA

<400> 101
guacaguacc agaaguuau 19

<210> 102
<211> 21
<212> RNA
<213> Artificial Sequence

<220>
<223> siRNA

<400> 102
auaacuucug guacuguacu c 21

<210> 103
<211> 21
<212> RNA
<213> Artificial Sequence

<220>
<223> siRNA

<400> 103
gaguacagua ccagaaguua u 21

<210> 104
<211> 23
<212> RNA
<213> Artificial Sequence

<220>
<223> siRNA

<400> 104
auaacuucug guacuguacu cau 23

<210> 105
<211> 19
<212> RNA
<213> Artificial Sequence

<220>
<223> siRNA

<400> 105
guacaguacc agaaguuau 19

<210> 106
<211> 21
<212> RNA
<213> Artificial Sequence

<220>
<223> siRNA

<400> 106
auaacuucug guacuguacu c 21

<210> 107
<211> 21
<212> RNA
<213> Artificial Sequence

<220>
<223> siRNA

<400> 107
gaguacagua ccagaaguua u 21

<210> 108
<211> 23
<212> RNA
<213> Artificial Sequence

<220>
<223> siRNA

<400> 108
auaacuucug guacuguacu cau 23

<210> 109
<211> 19
<212> RNA
<213> Artificial Sequence

<220>
<223> siRNA

<400> 109
guacaguacc agaaguuau 19

<210> 110
<211> 21
<212> RNA
<213> Artificial Sequence

<220>
<223> siRNA

<400> 110
auaacuucug guacuguacu c 21

<210> 111
<211> 21
<212> RNA
<213> Artificial Sequence

<220>
<223> siRNA

<400> 111
gaguacagua ccagaaguua u 21

<210> 112
<211> 23

<212> RNA
<213> Artificial Sequence

<220>
<223> siRNA

<400> 112
auaacuucug guacuguacu cau 23

<210> 113
<211> 19
<212> RNA
<213> Artificial Sequence

<220>
<223> siRNA

<400> 113
guacaguacc agaaguuau 19

<210> 114
<211> 21
<212> RNA
<213> Artificial Sequence

<220>
<223> siRNA

<400> 114
auaacuucug guacuguacu c 21

<210> 115
<211> 21
<212> RNA
<213> Artificial Sequence

<220>
<223> siRNA

<400> 115
gaguacagua ccagaaguua u 21

<210> 116
<211> 23
<212> RNA
<213> Artificial Sequence

<220>
<223> siRNA

<400> 116
auaacuucug guacuguacu cau 23

<210> 117
<211> 19
<212> RNA
<213> Artificial Sequence

<220>
<223> siRNA

<400> 117
guacaguacc agaaguuau 19

<210> 118
<211> 21
<212> RNA
<213> Artificial Sequence

<220>
<223> siRNA

<400> 118
auaacuucug guacuguacu c 21

<210> 119
<211> 21
<212> RNA
<213> Artificial Sequence

<220>
<223> siRNA

<400> 119
gaguacagua ccagaaguua u 21

<210> 120
<211> 23
<212> RNA
<213> Artificial Sequence

<220>
<223> siRNA

<400> 120
auaacuucug guacuguacu cau 23

<210> 121
<211> 19
<212> RNA
<213> Artificial Sequence

<220>
<221> misc_feature
<222> (19)..(19)
<223> n is Z9, Z9 is A

<220>
<223> siRNA

<400> 121
caaacaagcu gcacagaan 19

<210> 122
<211> 19
<212> RNA
<213> Artificial Sequence

<220>
<221> misc_feature
<222> (1)..(1)
<223> n is Z10, Z10 is U

<220>
<223> siRNA

<400> 122
nuucugugca gcuuguuug 19

<210> 123
<211> 19
<212> RNA
<213> Artificial Sequence

<220>
<221> misc_feature
<222> (19)..(19)
<223> n is Z11, Z11 is A, U, G or C

<220>
<223> siRNA

<400> 123
caaacaagcu gcacagaan 19

<210> 124
<211> 19
<212> RNA
<213> Artificial Sequence

<220>
<221> misc_feature
<222> (1)..(1)
<223> n is Z12, Z12 is a nucleotide complementary to Z11,
Z11 is selected from A, U, G or C

<220>
<223> siRNA

<400> 124
nuucugugca gcuuguuug 19

<210> 125
<211> 19
<212> RNA
<213> Artificial Sequence

<220>
<221> misc_feature
<222> (19)..(19)
<223> n is Z11, Z11 is A, U, G or C

<220>
<223> siRNA

<400> 125
caaacaagcu gcacagaan 19

<210> 126
<211> 21
<212> RNA
<213> Artificial Sequence

<220>
<221> misc_feature
<222> (1)..(1)
<223> n is Z12, Z12 is a nucleotide complementary to Z11,
Z11 is selected from A, U, G or C

<220>
<223> siRNA

<400> 126
nuucugugca gcuuguuugc c 21

<210> 127
<211> 21
<212> RNA
<213> Artificial Sequence

<220>
<221> misc_feature
<222> (21)..(21)
<223> n is Z11, Z11 is A, U, G or C

<220>
<223> siRNA

<400> 127
ggcaaacaag cugcacagaa n 21

<210> 128
<211> 23
<212> RNA
<213> Artificial Sequence

<220>
<221> misc_feature
<222> (1)..(1)
<223> n is Z12, Z12 is a nucleotide complementary to Z11,
Z11 is selected from A, U, G or C

<220>
<223> siRNA

<400> 128
nuucugugca gcuuguuugc cag 23

<210> 129
<211> 19
<212> RNA
<213> Artificial Sequence

<220>
<223> siRNA

<400> 129
caaacaagcu gcacagaaa 19

<210> 130
<211> 21
<212> RNA
<213> Artificial Sequence

<220>
<223> siRNA

<400> 130
uuucugugca gcuuguuugc c 21

<210> 131
<211> 21

<212> RNA
<213> Artificial Sequence

<220>
<223> siRNA

<400> 131
ggcaaacaag cugcacagaa a 21

<210> 132
<211> 23
<212> RNA
<213> Artificial Sequence

<220>
<223> siRNA

<400> 132
uuucugugca gcuuguuugc cag 23

<210> 133
<211> 19
<212> RNA
<213> Artificial Sequence

<220>
<223> siRNA

<400> 133
caaacaagcu gcacagaaa 19

<210> 134
<211> 21
<212> RNA
<213> Artificial Sequence

<220>
<223> siRNA

<400> 134
uuucugugca gcuuguuugc c 21

<210> 135
<211> 21
<212> RNA
<213> Artificial Sequence

<220>
<223> siRNA

<400> 135
ggcaaacaag cugcacagaa a 21

<210> 136
<211> 23
<212> RNA
<213> Artificial Sequence

<220>
<223> siRNA

<400> 136
uuucugugca gcuuguuugc cag 23

<210> 137
<211> 19
<212> RNA
<213> Artificial Sequence

<220>
<223> siRNA

<400> 137
caaacaagcu gcacagaaa 19

<210> 138
<211> 21
<212> RNA
<213> Artificial Sequence

<220>
<223> siRNA

<400> 138
uuucugugca gcuuguuugc c 21

<210> 139
<211> 21
<212> RNA
<213> Artificial Sequence

<220>
<223> siRNA

<400> 139
ggcaaacaag cugcacagaa a 21

<210> 140
<211> 23
<212> RNA
<213> Artificial Sequence

<220>
<223> siRNA

<400> 140
uuucugugca gcuuguuugc cag 23

<210> 141
<211> 19
<212> RNA
<213> Artificial Sequence

<220>
<223> siRNA

<400> 141
caaacaagcu gcacagaaa 19

<210> 142
<211> 21
<212> RNA
<213> Artificial Sequence

<220>
<223> siRNA

<400> 142
uuucugugca gcuuguuugc c 21

<210> 143
<211> 21
<212> RNA
<213> Artificial Sequence

<220>
<223> siRNA

<400> 143
ggcaaacaag cugcacagaa a 21

<210> 144
<211> 23
<212> RNA
<213> Artificial Sequence

<220>
<223> siRNA

<400> 144
uuucugugca gcuuguuugc cag 23

<210> 145
<211> 19
<212> RNA
<213> Artificial Sequence

<220>
<223> siRNA

<400> 145
caaacaagcu gcacagaaa 19

<210> 146
<211> 21
<212> RNA
<213> Artificial Sequence

<220>
<223> siRNA

<400> 146
uuucugugca gcuuguuugc c 21

<210> 147
<211> 19
<212> RNA
<213> Artificial Sequence

<220>
<223> siRNA

<400> 147
ggcaaacaag cugcacagaa a 19

<210> 148
<211> 23
<212> RNA
<213> Artificial Sequence

<220>
<223> siRNA

<400> 148
uuucugugca gcuuguuugc cag 23

<210> 149
<211> 19
<212> RNA
<213> Artificial Sequence

<220>
<223> siRNA

<400> 149
caaacaagcu gcacagaaa 19

<210> 150
<211> 21
<212> RNA
<213> Artificial Sequence

<220>
<223> siRNA

<400> 150
uuucugugca gcuuguuugc c 21

<210> 151
<211> 21
<212> RNA
<213> Artificial Sequence

<220>
<223> siRNA

<400> 151
ggcaaacaag cugcacagaa a 21

<210> 152
<211> 23
<212> RNA
<213> Artificial Sequence

<220>
<223> siRNA

<400> 152
uuucugugca gcuuguuugc cag 23

<210> 153
<211> 19
<212> RNA
<213> Artificial Sequence

<220>
<223> siRNA

<400> 153
caaacaagcu gcacagaaa 19

<210> 154

<211> 21
<212> RNA
<213> Artificial Sequence

<220>
<223> siRNA

<400> 154
uuucugugca gcuuguuugc c 21

<210> 155
<211> 21
<212> RNA
<213> Artificial Sequence

<220>
<223> siRNA

<400> 155
ggcaaacaag cugcacagaa a 21

<210> 156
<211> 23
<212> RNA
<213> Artificial Sequence

<220>
<223> siRNA

<400> 156
uuucugugca gcuuguuugc cag 23

<210> 157
<211> 19
<212> RNA
<213> Artificial Sequence

<220>
<223> siRNA

<400> 157
caaacaagcu gcacagaaa 19

<210> 158
<211> 21
<212> RNA
<213> Artificial Sequence

<220>
<223> siRNA

<400> 158
uuucugugca gcuuguuugc c 21

<210> 159
<211> 19
<212> RNA
<213> Artificial Sequence

<220>
<223> siRNA

<400> 159

ggcaaacaag cugcacagaa a 19

<210> 160
<211> 23
<212> RNA
<213> Artificial Sequence

<220>
<223> siRNA

<400> 160
uuucugugca gcuuguuugc cag 23

<210> 161
<211> 19
<212> RNA
<213> Artificial Sequence

<220>
<223> siRNA

<400> 161
caaacaagcu gcacagaaa 19

<210> 162
<211> 21
<212> RNA
<213> Artificial Sequence

<220>
<223> siRNA

<400> 162
uuucugugca gcuuguuugc c 21

<210> 163
<211> 21
<212> RNA
<213> Artificial Sequence

<220>
<223> siRNA

<400> 163
ggcaaacaag cugcacagaa a 21

<210> 164
<211> 23
<212> RNA
<213> Artificial Sequence

<220>
<223> siRNA

<400> 164
uuucugugca gcuuguuugc cag 23

<210> 165
<211> 19
<212> RNA
<213> Artificial Sequence

<220>

<223> siRNA

<400> 165
caaacaagcu gcacagaaa 19

<210> 166
<211> 21
<212> RNA
<213> Artificial Sequence

<220>
<223> siRNA

<400> 166
uuucugugca gcuuguuugc c 21

<210> 167
<211> 21
<212> RNA
<213> Artificial Sequence

<220>
<223> siRNA

<400> 167
ggcaaacaag cugcacagaa a 21

<210> 168
<211> 23
<212> RNA
<213> Artificial Sequence

<220>
<223> siRNA

<400> 168
uuucugugca gcuuguuugc cag 23

<210> 169
<211> 19
<212> RNA
<213> Artificial Sequence

<220>
<223> siRNA

<400> 169
caaacaagcu gcacagaaa 19

<210> 170
<211> 21
<212> RNA
<213> Artificial Sequence

<220>
<223> siRNA

<400> 170
uuucugugca gcuuguuugc c 21

<210> 171
<211> 21
<212> RNA

<213> Artificial Sequence

<220>
<223> siRNA

<400> 171
ggcaaacaag cugcacagaa a 21

<210> 172
<211> 23
<212> RNA
<213> Artificial Sequence

<220>
<223> siRNA

<400> 172
uuucugugca gcuuguuugc cag 23

<210> 173
<211> 19
<212> RNA
<213> Artificial Sequence

<220>
<223> siRNA

<400> 173
caaacaagcu gcacagaaa 19

<210> 174
<211> 21
<212> RNA
<213> Artificial Sequence

<220>
<223> siRNA

<400> 174
uuucugugca gcuuguuugc c 21

<210> 175
<211> 21
<212> RNA
<213> Artificial Sequence

<220>
<223> siRNA

<400> 175
ggcaaacaag cugcacagaa a 21

<210> 176
<211> 23
<212> RNA
<213> Artificial Sequence

<220>
<223> siRNA

<400> 176
uuucugugca gcuuguuugc cag 23

<210> 177
<211> 19
<212> RNA
<213> Artificial Sequence

<220>
<223> siRNA

<400> 177
caaacaagcu gcacagaaa 19

<210> 178
<211> 21
<212> RNA
<213> Artificial Sequence

<220>
<223> siRNA

<400> 178
uuucugugca gcuuguuugc c 21

<210> 179
<211> 21
<212> RNA
<213> Artificial Sequence

<220>
<223> siRNA

<400> 179
ggcaaacaag cugcacagaa a 21

<210> 180
<211> 23
<212> RNA
<213> Artificial Sequence

<220>
<223> siRNA

<400> 180
uuucugugca gcuuguuugc cag 23

<210> 181
<211> 19
<212> RNA
<213> Artificial Sequence

<220>
<221> misc_feature
<222> (19)..(19)
<223> n is Z13, Z13 is A

<220>
<223> siRNA

<400> 181
caaacaagga cuaauccan 19

<210> 182
<211> 19
<212> RNA

<213> Artificial Sequence

<220>
<221> misc_feature
<222> (1)..(1)
<223> n is Z14, Z14 is U

<220>
<223> siRNA

<400> 182
nuggauuagu ccuuguuug 19

<210> 183
<211> 19
<212> RNA
<213> Artificial Sequence

<220>
<221> misc_feature
<222> (19)..(19)
<223> n is Z15, Z15 is A, U, G or C

<220>
<223> siRNA

<400> 183
caaacaagga cuaauccan 19

<210> 184
<211> 19
<212> RNA
<213> Artificial Sequence

<220>
<221> misc_feature
<222> (1)..(1)
<223> n is Z16, Z16 is a nucleotide complementary to Z15, Z15 is selected from A, U, G or C

<220>
<223> siRNA

<400> 184
nuggauuagu ccuuguuug 19

<210> 185
<211> 19
<212> RNA
<213> Artificial Sequence

<220>
<221> misc_feature
<222> (19)..(19)
<223> n is Z15, Z15 is A, U, G or C

<220>
<223> siRNA

<400> 185
caaacaagga cuaauccan 19

<210> 186

<211> 21
<212> RNA
<213> Artificial Sequence

<220>
<221> misc_feature
<222> (1)..(1)
<223> n is Z16, Z16 is a nucleotide complementary to Z15,
Z15 is selected from A, U, G or C

<220>
<223> siRNA

<400> 186
nuggauuagu ccuuguuugg u 21

<210> 187
<211> 21
<212> RNA
<213> Artificial Sequence

<220>
<221> misc_feature
<222> (21)..(21)
<223> n is Z15, Z15 is A, U, G or C

<220>
<223> siRNA

<400> 187
accaaacaag gacuaaucca n 21

<210> 188
<211> 23
<212> RNA
<213> Artificial Sequence

<220>
<221> misc_feature
<222> (1)..(1)
<223> n is Z16, Z16 is a nucleotide complementary to Z15,
Z15 is selected from A, U, G or C

<220>
<223> siRNA

<400> 188
nuggauuagu ccuuguuugg ucu 23

<210> 189
<211> 19
<212> RNA
<213> Artificial Sequence

<220>
<223> siRNA

<400> 189
caaacaagga cuaauccaa 19

<210> 190
<211> 21
<212> RNA

<213> Artificial Sequence

<220>
<223> siRNA

<400> 190
uuggauuagu ccuuguuugg u 21

<210> 191
<211> 21
<212> RNA
<213> Artificial Sequence

<220>
<223> siRNA

<400> 191
accaaacaag gacuaaucca a 21

<210> 192
<211> 23
<212> RNA
<213> Artificial Sequence

<220>
<223> siRNA

<400> 192
uuggauuagu ccuuguuugg ucu 23

<210> 193
<211> 19
<212> RNA
<213> Artificial Sequence

<220>
<223> siRNA

<400> 193
caaacaagga cuaauccaa 19

<210> 194
<211> 21
<212> RNA
<213> Artificial Sequence

<220>
<223> siRNA

<400> 194
uuggauuagu ccuuguuugg u 21

<210> 195
<211> 21
<212> RNA
<213> Artificial Sequence

<220>
<223> siRNA

<400> 195
accaaacaag gacuaaucca a 21

<210> 196
<211> 23
<212> RNA
<213> Artificial Sequence

<220>
<223> siRNA

<400> 196
uuggauuagu ccuuguuugg ucu 23

<210> 197
<211> 19
<212> RNA
<213> Artificial Sequence

<220>
<223> siRNA

<400> 197
caaacaagga cuaauccaa 19

<210> 198
<211> 21
<212> RNA
<213> Artificial Sequence

<220>
<223> siRNA

<400> 198
uuggauuagu ccuuguuugg u 21

<210> 199
<211> 21
<212> RNA
<213> Artificial Sequence

<220>
<223> siRNA

<400> 199
accaaacaag gacuaaucca a 21

<210> 200
<211> 23
<212> RNA
<213> Artificial Sequence

<220>
<223> siRNA

<400> 200
uuggauuagu ccuuguuugg ucu 23

<210> 201
<211> 19
<212> RNA
<213> Artificial Sequence

<220>
<223> siRNA

<400> 201
caaacaagga cuaauccaa 19

<210> 202
<211> 21
<212> RNA
<213> Artificial Sequence

<220>
<223> siRNA

<400> 202
uuggauuagu ccuuguuugg u 21

<210> 203
<211> 21
<212> RNA
<213> Artificial Sequence

<220>
<223> siRNA

<400> 203
accaaacaag gacuaaucca a 21

<210> 204
<211> 23
<212> RNA
<213> Artificial Sequence

<220>
<223> siRNA

<400> 204
uuggauuagu ccuuguuugg ucu 23

<210> 205
<211> 19
<212> RNA
<213> Artificial Sequence

<220>
<223> siRNA

<400> 205
caaacaagga cuaauccaa 19

<210> 206
<211> 21
<212> RNA
<213> Artificial Sequence

<220>
<223> siRNA

<400> 206
uuggauuagu ccuuguuugg u 21

<210> 207
<211> 21
<212> RNA
<213> Artificial Sequence

<220>
<223> siRNA

<400> 207
accaaacaag gacuaaucca a 21

<210> 208
<211> 23
<212> RNA
<213> Artificial Sequence

<220>
<223> siRNA

<400> 208
uuggauuagu ccuuguuugg ucu 23

<210> 209
<211> 19
<212> RNA
<213> Artificial Sequence

<220>
<223> siRNA

<400> 209
caaacaagga cuaauccaa 19

<210> 210
<211> 21
<212> RNA
<213> Artificial Sequence

<220>
<223> siRNA

<400> 210
uuggauuagu ccuuguuugg u 21

<210> 211
<211> 21
<212> RNA
<213> Artificial Sequence

<220>
<223> siRNA

<400> 211
accaaacaag gacuaaucca a 21

<210> 212
<211> 23
<212> RNA
<213> Artificial Sequence

<220>
<223> siRNA

<400> 212
uuggauuagu ccuuguuugg ucu 23

<210> 213
<211> 19

<212> RNA
<213> Artificial Sequence

<220>
<223> siRNA

<400> 213
caaacaagga cuaauccaa 19

<210> 214
<211> 21
<212> RNA
<213> Artificial Sequence

<220>
<223> siRNA

<400> 214
uuggauuagu ccuuguuugg u 21

<210> 215
<211> 21
<212> RNA
<213> Artificial Sequence

<220>
<223> siRNA

<400> 215
accaaacaag gacuaaucca a 21

<210> 216
<211> 23
<212> RNA
<213> Artificial Sequence

<220>
<223> siRNA

<400> 216
uuggauuagu ccuuguuugg ucu 23

<210> 217
<211> 19
<212> RNA
<213> Artificial Sequence

<220>
<223> siRNA

<400> 217
caaacaagga cuaauccaa 19

<210> 218
<211> 21
<212> RNA
<213> Artificial Sequence

<220>
<223> siRNA

<400> 218
uuggauuagu ccuuguuugg u 21

<210> 219
<211> 21
<212> RNA
<213> Artificial Sequence

<220>
<223> siRNA

<400> 219
accaaacaag gacuaaucca a 21

<210> 220
<211> 23
<212> RNA
<213> Artificial Sequence

<220>
<223> siRNA

<400> 220
uuggauuagu ccuuguuugg ucu 23

<210> 221
<211> 19
<212> RNA
<213> Artificial Sequence

<220>
<223> siRNA

<400> 221
caaacaagga cuaauccaa 19

<210> 222
<211> 21
<212> RNA
<213> Artificial Sequence

<220>
<223> siRNA

<400> 222
uuggauuagu ccuuguuugg u 21

<210> 223
<211> 21
<212> RNA
<213> Artificial Sequence

<220>
<223> siRNA

<400> 223
accaaacaag gacuaaucca a 21

<210> 224
<211> 23
<212> RNA
<213> Artificial Sequence

<220>
<223> siRNA

<400> 224
uuggauuagu ccuuguuugg ucu 23

<210> 225
<211> 19
<212> RNA
<213> Artificial Sequence

<220>
<223> siRNA

<400> 225
caaacaagga cuaauccaa 19

<210> 226
<211> 21
<212> RNA
<213> Artificial Sequence

<220>
<223> siRNA

<400> 226
uuggauuagu ccuuguuugg u 21

<210> 227
<211> 21
<212> RNA
<213> Artificial Sequence

<220>
<223> siRNA

<400> 227
accaaacaag gacuaaucca a 21

<210> 228
<211> 23
<212> RNA
<213> Artificial Sequence

<220>
<223> siRNA

<400> 228
uuggauuagu ccuuguuugg ucu 23

<210> 229
<211> 19
<212> RNA
<213> Artificial Sequence

<220>
<223> siRNA

<400> 229
caaacaagga cuaauccaa 19

<210> 230
<211> 21
<212> RNA
<213> Artificial Sequence

<220>
<223> siRNA

<400> 230
uuggauuagu ccuuguuugg u 21

<210> 231
<211> 21
<212> RNA
<213> Artificial Sequence

<220>
<223> siRNA

<400> 231
accaaacaag gacuaaucca a 21

<210> 232
<211> 23
<212> RNA
<213> Artificial Sequence

<220>
<223> siRNA

<400> 232
uuggauuagu ccuuguuugg ucu 23

<210> 233
<211> 19
<212> RNA
<213> Artificial Sequence

<220>
<223> siRNA

<400> 233
caaacaagga cuaauccaa 19

<210> 234
<211> 21
<212> RNA
<213> Artificial Sequence

<220>
<223> siRNA

<400> 234
uuggauuagu ccuuguuugg u 21

<210> 235
<211> 21
<212> RNA
<213> Artificial Sequence

<220>
<223> siRNA

<400> 235
accaaacaag gacuaaucca a 21

<210> 236

<211> 23
<212> RNA
<213> Artificial Sequence

<220>
<223> siRNA

<400> 236
uuggauuagu ccuuguuugg ucu 23

<210> 237
<211> 19
<212> RNA
<213> Artificial Sequence

<220>
<223> siRNA

<400> 237
caaacaagga cuaauccaa 19

<210> 238
<211> 21
<212> RNA
<213> Artificial Sequence

<220>
<223> siRNA

<400> 238
uuggauuagu ccuuguuugg u 21

<210> 239
<211> 21
<212> RNA
<213> Artificial Sequence

<220>
<223> siRNA

<400> 239
accaaacaag gacuaaucca a 21

<210> 240
<211> 23
<212> RNA
<213> Artificial Sequence

<220>
<223> siRNA

<400> 240
uuggauuagu ccuuguuugg ucu 23

<210> 241
<211> 19
<212> RNA
<213> Artificial Sequence

<220>
<223> siRNA

<400> 241

ccuaugaaga uuauaagaa 19

<210> 242
<211> 21
<212> RNA
<213> Artificial Sequence

<220>
<223> siRNA

<400> 242
uucuuauaau cuucauaggu g 21

<210> 243
<211> 19
<212> RNA
<213> Artificial Sequence

<220>
<223> siRNA

<400> 243
guacaguacc agaaguuau 19

<210> 244
<211> 21
<212> RNA
<213> Artificial Sequence

<220>
<223> siRNA

<400> 244
auaacuucug guacuguacu c 21

<210> 245
<211> 19
<212> RNA
<213> Artificial Sequence

<220>
<223> siRNA

<400> 245
caaacaagcu gcacagaaa 19

<210> 246
<211> 21
<212> RNA
<213> Artificial Sequence

<220>
<223> siRNA

<400> 246
uuucugugca gcuuguuugc c 21

<210> 247
<211> 19
<212> RNA
<213> Artificial Sequence

<220>

<223> siRNA

<400> 247
ccuaugaaga uuauaagaa 19

<210> 248
<211> 21
<212> RNA
<213> Artificial Sequence

<220>
<223> siRNA

<400> 248
uucuuauaau cuucauaggu g 21

<210> 249
<211> 19
<212> RNA
<213> Artificial Sequence

<220>
<223> siRNA

<400> 249
guacaguacc agaaguuau 19

<210> 250
<211> 21
<212> RNA
<213> Artificial Sequence

<220>
<223> siRNA

<400> 250
auaacuucug guacuguacu c 21

<210> 251
<211> 19
<212> RNA
<213> Artificial Sequence

<220>
<223> siRNA

<400> 251
caaacaagcu gcacagaaa 19

<210> 252
<211> 21
<212> RNA
<213> Artificial Sequence

<220>
<223> siRNA

<400> 252
uuucugugca gcuuguuugc c 21

<210> 253
<211> 19
<212> RNA

<213> Artificial Sequence

<220>
<223> siRNA

<400> 253
caaacaagga cuaauccaa 19

<210> 254
<211> 21
<212> RNA
<213> Artificial Sequence

<220>
<223> siRNA

<400> 254
uuggauuagu ccuuguuugg u 21

<210> 255
<211> 19
<212> RNA
<213> Artificial Sequence

<220>
<223> siRNA

<400> 255
caaacaagga cuaauccaa 19

<210> 256
<211> 21
<212> RNA
<213> Artificial Sequence

<220>
<223> siRNA

<400> 256
uuggauuagu ccuuguuugg u 21

<210> 257
<211> 19
<212> RNA
<213> Artificial Sequence

<220>
<223> siRNA

<400> 257
guacaguacc agaaguuaa 19

<210> 258
<211> 21
<212> RNA
<213> Artificial Sequence

<220>
<223> siRNA

<400> 258
uuaacuucug guacuguacu c 21

<210> 259
<211> 19
<212> RNA
<213> Artificial Sequence

<220>
<223> siRNA

<400> 259
guacaguacc agaaguuaa 19

<210> 260
<211> 21
<212> RNA
<213> Artificial Sequence

<220>
<223> siRNA

<400> 260
uuaacuucug guacuguacu u 21

<210> 261
<211> 19
<212> RNA
<213> Artificial Sequence

<220>
<223> siRNA

<400> 261
caaacaagcu gcacagaaa 19

<210> 262
<211> 21
<212> RNA
<213> Artificial Sequence

<220>
<223> siRNA

<400> 262
uuucugugca gcuuguuugu u 21

<210> 263
<211> 19
<212> RNA
<213> Artificial Sequence

<220>
<223> siRNA

<400> 263
caaacaagga cuaauccaa 19

<210> 264
<211> 21
<212> RNA
<213> Artificial Sequence

<220>
<223> siRNA

<400> 264
uuggauuagu ccuuguuugu u 21

<210> 265
<211> 19
<212> RNA
<213> Artificial Sequence

<220>
<223> siRNA

<400> 265
gcacaguccc agaaguuau 19

<210> 266
<211> 21
<212> RNA
<213> Artificial Sequence

<220>
<223> siRNA

<400> 266
auaacuucug ggacugugcu c 21

<210> 267
<211> 19
<212> RNA
<213> Artificial Sequence

<220>
<223> siRNA

<400> 267
ccuaugaaga uuauaagaa 19

<210> 268
<211> 21
<212> RNA
<213> Artificial Sequence

<220>
<223> siRNA

<400> 268
uucuuauaau cuucauaggu g 21

<210> 269
<211> 19
<212> RNA
<213> Artificial Sequence

<220>
<223> siRNA

<400> 269
guacaguacc agaaguuau 19

<210> 270
<211> 21
<212> RNA
<213> Artificial Sequence

<220>
<223> siRNA

<400> 270
auaacuucug guacuguacu c 21

<210> 271
<211> 19
<212> RNA
<213> Artificial Sequence

<220>
<223> siRNA

<400> 271
caaacaagcu gcacagaaa 19

<210> 272
<211> 21
<212> RNA
<213> Artificial Sequence

<220>
<223> siRNA

<400> 272
uuucugugca gcuuguuugc c 21

<210> 273
<211> 19
<212> RNA
<213> Artificial Sequence

<220>
<223> siRNA

<400> 273
caaacaagga cuaauccaa 19

<210> 274
<211> 21
<212> RNA
<213> Artificial Sequence

<220>
<223> siRNA

<400> 274
uuggauuagu ccuuguuugg u 21

<210> 275
<211> 21
<212> RNA
<213> Artificial Sequence

<220>
<223> siRNA

<400> 275
uucuccgaac gugucacgut t 21

<210> 276
<211> 21

<212> RNA
<213> Artificial Sequence

<220>
<223> siRNA

<400> 276
acgugacacg uucggagaat t 21

<210> 277
<211> 20
<212> DNA
<213> Artificial Sequence

<220>
<223> primer

<400> 277
actggtgttt gggttcctga 20

<210> 278
<211> 20
<212> DNA
<213> Artificial Sequence

<220>
<223> primer

<400> 278
tcctgctgca ttggtgacta 20

<210> 279
<211> 19
<212> DNA
<213> Artificial Sequence

<220>
<223> primer

<400> 279
ggtcggagtc aacggattt 19

<210> 280
<211> 19
<212> DNA
<213> Artificial Sequence

<220>
<223> primer

<400> 280
ccagcatcgc cccacttga 19

## Description

### FIELD OF THE INVENTION

The present disclosure relates to a nucleic acid capable of inhibiting purine nucleoside phosphorylase (PNP) gene expression and a pharmaceutical composition and an siRNA conjugate containing the nucleic acid. The present disclosure also relates to a preparation method and use of the nucleic acid, pharmaceutical composition and siRNA conjugate.

### BACKGROUND OF THE INVENTION

Gout is a disease directly associated with hyperuricemia caused by purine metabolic disorders and/or reduced uric acid excretion. Gout has been a common disease in developed countries such as Europe and America since ancient times, after the Second World War, with the economic development of various countries, the prevalence of gout has been increasing year by year in the world, and it has a trend of younger people. There are currently 12 million gout patients in China.

Purine Nucleotide Phosphorylase (PNP) is one of the key targets for the treatment of gout. By inhibiting the expression of PNP, the production of hypoxanthine and guanine can be effectively inhibited, so that the production of uric acid is reduced, and the purposes of relieving the progression of gout disease and reversing the disease condition are achieved. By inhibiting the expression of PNP gene, diseases caused by abnormal uric acid metabolism, especially hyperuricemia and gout, can be prevented and treated at the cellular level. Small interfering RNA (siRNA) can inhibit or block the expression of any target gene of interest in a sequence-specific manner based on RNA interference (RNAi) mechanism, thereby achieving the purpose of treating diseases.

One of the keys to the development of siRNA drugs that inhibit PNP gene expression and treat diseases caused by abnormal uric acid metabolism is to find suitable siRNA and its modification as well as an effective delivery system.

### SUMMARY OF THE INVENTION

The inventors of the present disclosure have unexpectedly found that the following siRNA and its modification sequence provided by the present disclosure can specifically inhibit the expression of PNP gene, the pharmaceutical composition or the siRNA conjugate can specifically target the liver, so that the expression of PNP gene in the liver can be inhibited, and the treatment or prevention of diseases caused by abnormal uric acid metabolism, especially hyperuricemia and gout, is achieved, thereby completing the present invention.

In some embodiments, the present disclosure provides a first siRNA capable of inhibiting expression of PNP gene, which comprises a sense strand and an antisense strand, each nucleotide in the siRNA being independently a modified or unmodified nucleotide; wherein the sense strand comprises a nucleotide sequence I, and the antisense strand comprises a nucleotide sequence II; the nucleotide sequence I and the nucleotide sequence II are at least partly reverse complementary to form a double-stranded region; wherein the nucleotide sequence I has the same length as the nucleotide sequence represented by SEQ ID NO: 1 with no more than 3 nucleotide differences; and the nucleotide sequence II has the same length as the nucleotide sequence represented by SEQ ID NO: 2 with no more than 3 nucleotide differences:

5'-CCUAUGAAGAUUAUAAGA$Z_1$-3' (SEQ ID NO: 1);
5'-$Z_2$UCUUAUAAUCUUCAUAGG-3' (SEQ ID NO: 2),

wherein $Z_1$ is A, and $Z_2$ is U; the nucleotide sequence I comprises a nucleotide $Z_3$ at the position corresponding to $Z_1$; the nucleotide sequence II comprises a nucleotide $Z_4$ at the position corresponding to $Z_2$; the $Z_4$ is the first nucleotide at 5' terminal of the antisense strand.

In some embodiments, the present disclosure provides a second siRNA capable of inhibiting expression of PNP gene, which comprises a sense strand and an antisense strand, each nucleotide in the siRNA being independently a modified or unmodified nucleotide; wherein the sense strand comprises a nucleotide sequence I, and the antisense strand comprises a nucleotide sequence II; the nucleotide sequence I and the nucleotide sequence II are at least partly reverse complementary to form a double-stranded region; wherein the nucleotide sequence I has the same length as the nucleotide sequence represented by SEQ ID NO: 61 with no more than 3 nucleotide differences; and the nucleotide sequence II has the same length as the nucleotide sequence represented by SEQ ID NO: 62 with no more than 3 nucleotide differences:

5'-GUACAGUACCAGAAGUUA$Z_5$-3' (SEQ ID NO: 61);

5'-$Z_6$UAACUUCUGGUACUGUAC-3' (SEQ ID NO: 62),

wherein $Z_5$ is U, and $Z_6$ is A; the nucleotide sequence I comprises a nucleotide $Z_7$ at the position corresponding to $Z_5$; the nucleotide sequence II comprises a nucleotide $Z_8$ at the position corresponding to $Z_6$; the $Z_8$ is the first nucleotide at 5' terminal of the antisense strand.

In some embodiments, the present disclosure provides a third siRNA capable of inhibiting expression of PNP gene, which comprises a sense strand and an antisense strand, each nucleotide in the siRNA being independently a modified or unmodified nucleotide; wherein the sense strand comprises a nucleotide sequence I, and the antisense strand comprises a nucleotide sequence II; the nucleotide sequence I and the nucleotide sequence II are at least partly reverse complementary to form a double-stranded region; wherein the nucleotide sequence I has the same length as the nucleotide sequence represented by SEQ ID NO: 121 with no more than 3 nucleotide differences; and the nucleotide sequence II has the same length as the nucleotide sequence represented by SEQ ID NO: 122 with no more than 3 nucleotide differences:

5'-CAAACAAGCUGCACAGAA$Z_9$-3' (SEQ ID NO: 121);
5'-$Z_{10}$UUCUGUGCAGCUUGUUUG-3' (SEQ ID NO: 122),

wherein $Z_9$ is A, and $Z_{10}$ is U; the nucleotide sequence I comprises a nucleotide $Z_{11}$ at the position corresponding to $Z_9$; the nucleotide sequence II comprises a nucleotide $Z_{12}$ at the position corresponding to $Z_{10}$; the $Z_{12}$ is the first nucleotide at 5' terminal of the antisense strand.

In some embodiments, the present disclosure provides a fourth siRNA capable of inhibiting expression of PNP gene, which comprises a sense strand and an antisense strand, each nucleotide in the siRNA being independently a modified or unmodified nucleotide; wherein the sense strand comprises a nucleotide sequence I, and the antisense strand comprises a nucleotide sequence II; the nucleotide sequence I and the nucleotide sequence II are at least partly reverse complementary to form a double-stranded region; wherein the nucleotide sequence I has the same length as the nucleotide sequence represented by SEQ ID NO: 181 with no more than 3 nucleotide differences; and the nucleotide sequence II has the same length as the nucleotide sequence represented by SEQ ID NO: 182 with no more than 3 nucleotide differences:

5'- CAAACAAGGACUAAUCCA$Z_{13}$-3' (SEQ ID NO: 181);
5'-$Z_{14}$UGGAUUAGUCCUUGUUUG-3' (SEQ ID NO: 182),

wherein $Z_{13}$ is A, and $Z_{14}$ is U; the nucleotide sequence I comprises a nucleotide $Z_{15}$ at the position corresponding to $Z_{13}$; the nucleotide sequence II comprises a nucleotide $Z_{16}$ at the position corresponding to $Z_{14}$; the $Z_{16}$ is the first nucleotide at 5' terminal of the antisense strand.

In some embodiments, the present disclosure provides a pharmaceutical composition comprising the siRNA of the present disclosure and a pharmaceutically acceptable carrier.

In some embodiments, the present disclosure provides an siRNA conjugate comprising an siRNA provided by the present disclosure and a conjugating group conjugated to the siRNA.

In some embodiments, the present disclosure provides a use of the siRNA and/or pharmaceutical composition and/or siRNA conjugate of the present disclosure in the manufacture of a medicament for treating and/or preventing abnormal uric acid metabolism or diseases or physiological conditions caused by abnormal uric acid metabolism.

In some embodiments, the present disclosure provides a method for treating and/or preventing abnormal uric acid metabolism or diseases or physiological conditions caused by abnormal uric acid metabolism, comprising administering an effective amount of the siRNA and/or pharmaceutical composition and/or siRNA conjugate of the present disclosure to a subject in need thereof.

In some embodiments, the present disclosure provides a method for inhibiting expression of PNP gene in hepatocytes, comprising contacting an effective amount of the siRNA and/or pharmaceutical composition and/or siRNA conjugate of the present disclosure with the hepatocytes.

In some embodiments, the present disclosure provides a kit comprising the siRNA and/or pharmaceutical composition and/or siRNA conjugate of the present disclosure.

## BENEFICIAL EFFECTS

The siRNA, pharmaceutical composition and siRNA conjugate provided by the present disclosure have good stability, higher PNP mRNA inhibitory activity, lower off-target effects, and/or could remarkably treat or relieve gout symptoms.

In some embodiments, the siRNA, pharmaceutical composition, or siRNA conjugate provided by the present

disclosure exhibits excellent target mRNA inhibitory activity in *in vitro* cellular experiments. In some embodiments, the siRNA, siRNA composition, or siRNA conjugate provided by the present disclosure exhibits a target mRNA inhibition rate of at least 20%, 30%, 40%, 50%, 60%, 70%, 80%, 90%, or 95% in hepatocytes. In some embodiments, the siRNA provided by the present disclosure exhibits a PNP mRNA inhibition rate of 61.43% to 74.83% in SMMC-7721 cells at a concentration of 50 nM. In some embodiments, the siRNA provided by the present disclosure exhibits up to PNP mRNA inhibition rate of 89.15% in Huh7 cells at a concentration of 50 nM. In some embodiments, the siRNA provided by the present disclosure exhibits a PNP mRNA inhibition rate of 57.35% to 65.37% in HepG2 cells at a concentration of 50 nM. In some embodiments, the siRNA provided by the present disclosure exhibits a higher PNP mRNA inhibition rate in SMMC-7721 cells at various concentrations, up to 81.23% at a concentration of 50 nM. In some embodiments, the siRNA provided by the present disclosure exhibits a higher PNP mRNA inhibition rate in HepG2 cells at different concentrations. In some embodiments, the siRNA conjugate provided by the present disclosure exhibits a higher PNP mRNA inhibition rate in SMMC-7721 cells with an $IC_{50}$ value between 0.017-0.113 nM. In some embodiments, the siRNA conjugate provided by the present disclosure exhibits a higher PNP mRNA inhibition rate in HepG2 cells at various concentrations, up to 68.0% at a concentration of 50 nM. In some embodiments, the siRNA conjugate provided by the present disclosure exhibits a PNP mRNA inhibition rate of up to 88.93% in SMMC-7721 cells at a concentration of 50 nM. In some embodiments, the siRNA conjugate provided by the present disclosure exhibits a PNP mRNA inhibition rate of up to 77.89% in Huh7 cells at a concentration of 50 nM.

In some embodiments, the siRNA, pharmaceutical composition, or siRNA conjugate provided by the present disclosure may have greater stability and/or greater activity *in vivo.* In some embodiments, the siRNA, siRNA composition, or siRNA conjugate provided by the present disclosure exhibits a target gene expression inhibition rate of at least 20%, 30%, 40%, 50%, 60%, 70%, 80%, 90%, or 95% *in vivo.* In some embodiments, the siRNA, siRNA composition, or siRNA conjugate provided by the present disclosure exhibits a PNP gene expression inhibition rate of at least 20%, 30%, 40%, 50%, 60%, 70%, 80%, 90%, or 95% *in vivo.* In some embodiments, the siRNA, siRNA composition, or siRNA conjugate provided by the present disclosure exhibits a PNP gene expression inhibition rate in liver of at least 20%, 30%, 40%, 50%, 60%, 70%, 80%, 90%, or 95% *in vivo.* In some embodiments, the siRNA, siRNA composition, or siRNA conjugate provided by the present disclosure exhibits a PNP gene expression inhibition rate in liver in an animal model of at least 20%, 30%, 40%, 50%, 60%, 70%, 80%, 90%, or 95% *in vivo.* In some embodiments, the siRNA, siRNA composition, or siRNA conjugate provided by the present disclosure exhibits a PNP gene expression inhibition rate in liver in a human subject of at least 20%, 30%, 40%, 50%, 60%, 70%, 80%, 90%, or 95% *in vivo.* In some embodiments, the siRNA conjugate of the present disclosure exhibits a significant effect of inhibiting blood uric acid levels in acute hyperuric acid mouse models. In some embodiments, the siRNA, pharmaceutical composition, or siRNA conjugate provided by the present disclosure exhibits no significant off-target effect. An off-target effect may be, for example, the inhibition on normal expression of a gene which is not the target gene. It is considered that the off-target effect is not significant if the binding/inhibition of off-target gene expression is at a level of lower than 50%, 40%, 30%, 20%, or 10% of the on-target effect.

This shows that the siRNA, pharmaceutical composition and siRNA conjugate provided by the disclosure could inhibit the expression of PNP gene, effectively treat and/or prevent abnormal uric acid metabolism or diseases or physiological conditions caused by abnormal uric acid metabolism, in particular hyperuricemia and/or gout symptoms, and thus have good application prospect.

Additional features and advantages of the present disclosure will be illustrated in detail in the following part of detailed description of the invention.

## BRIEF DESCRIPTION OF THE DRAWINGS

**Figs. 1A-1D** are, in sequence, dose-effect curves fitting the relative expression levels of PNP mRNA in SMMC-7721 cells after transfection with different concentrations of conjugates 4-7 and the $IC_{50}$ values calculated therefrom.

**Fig. 2A** shows a dose-effect curve fitting the relative expression levels of PNP mRNA in SMMC-7721 cells *in vitro* after transfection with different concentrations of Conjugate 2 and the $IC_{50}$ values calculated therefrom.

**Fig. 2B** shows a dose-effect curve fitting the relative expression levels of PNP mRNA in SMMC-7721 cells *in vitro* after transfection with different concentrations of Conjugate 8 and the $IC_{50}$ values calculated therefrom.

**Fig. 3** shows a line chart of the mean value of relative uric acid contents in serum in a blank control mouse, an acute hyperuricemia mouse model, and an acute hyperuric acid mouse model after administration of Conjugate 13.

## DETAILED DESCRIPTION OF THE INVENTION

The specific embodiments of the present disclosure are described in detail as below. It should be understood that the specific embodiments described herein are only used to illustrate and explain the present disclosure and are not intended to limit the disclosure in any respect.

In the present disclosure, PNP mRNA refers to mRNA having a sequence shown in Genbank Accession No. NM_000270.3. Further, unless otherwise specified, as used in the present disclosure, the term "target gene" refers to a gene that transcribes the above PNP mRNA, and the term "target mRNA" refers to the above PNP mRNA.

Definitions

In the context of the present disclosure, unless otherwise specified, C, G, U, and A represent the base composition of the nucleotides; m represents that the nucleotide adjacent to the left side of the letter m is a methoxy modified nucleotide; f represents that the nucleotide adjacent to the left side of the letter f is a fluoro modified nucleotide; s represents that the two nucleotides adjacent to both sides of the letter s are linked by a phosphorothioate linkage; Pl represents that the nucleotide adjacent to the right side of Pl is a 5'-phosphate nucleotide or a 5'-phosphate analogue modified nucleotide, and P represents that the nucleotide adjacent to the right side of the letter P is a 5'- phosphate nucleotide.

In the context of the present disclosure, the "fluoro modified nucleotide" refers to a nucleotide formed by substituting the 2'-hydroxy of the ribose group thereof with a fluorine atom. The "non-fluoro modified nucleotide" refers to a nucleotide formed by substituting the 2'-hydroxy of the ribose group thereof with a non-fluoro group, or a nucleotide analogue. The "nucleotide analogue" refers to a group that can replace a nucleotide in a nucleic acid, while structurally differs from an adenine ribonucleotide, a guanine ribonucleotide, a cytosine ribonucleotide, a uracil ribonucleotide or thymine deoxyribonucleotide, such as an isonucleotide, a bridged nucleic acid (BNA) nucleotide or an acyclic nucleotide. The "methoxy modified nucleotide" refers to a nucleotide formed by substituting the 2'-hydroxy of the ribose group with a methoxy group.

In the context of the present disclosure, expressions "complementary" and "reverse complementary" are interchangeably used herein, and have the meaning well-known in the art, namely, bases in one strand are each paired complementarily with those in another strand in a double-stranded nucleic acid molecule. In DNAs, a purine base adenine (A) is always paired with a pyrimidine base thymine (T) (or a uracil (U) in RNAs); and a purine base guanine (C) is always paired with a pyrimidine base cytosine (G). Each base pair comprises a purine and a pyrimidine. While adenines in one strand are always paired with thymines (or uracils) in another strand, and guanines are always paired with cytosines, these two strands are considered as being complementary each other; and the sequence of a strand may be deduced from the sequence of its complementary strand. Correspondingly, a "mispairing" in the art means that the bases at corresponding positions are not present in a manner of being complementary paired in a double-stranded nucleic acid.

In the context of the present disclosure, unless otherwise specified, "basically reverse complementary" means that there is no more than 3 base mispairings between two nucleotide sequences. "Substantially reverse complementary" means that there is no more than 1 base mispairing between two nucleotide sequences. "Completely reverse complementary" means that there is no base mispairing between two nucleotide sequences.

In the context of the present disclosure, a "nucleotide difference" between a nucleotide sequence and another nucleotide sequence refers to a change in the type of the nucleotide base at the same position therebetween. For example, in the case that a nucleotide base in the later sequence is A while the nucleotide base at the same position in the former sequence is U, C, G or T, it is considered that a nucleotide difference is located in this position between these two nucleotide sequences. In some embodiments, while a nucleotide at a position is replaced with an abasic nucleotide or a nucleotide analogue, it is also considered that there is a nucleotide difference at the position.

In the context of the present disclosure, particularly in the description of the method for preparing the siRNA, the pharmaceutical composition, or the siRNA conjugate of the present disclosure, unless otherwise specified, the "nucleoside monomer" refers to, according to the type and sequence of the nucleotides in the siRNA or siRNA conjugate to be prepared, unmodified or modified RNA phosphoramidites used in the phosphoramidite solid phase synthesis (sometimes the RNA phosphoramidites are referred to as Nucleoside phosphoramidites). Phosphoramidite solid phase synthesis is a method well known by those skilled in the art to be used in the synthesis of RNA. Nucleoside monomers used in the present disclosure are all commercially available.

In the context of the present disclosure, unless otherwise specified, "conjugating" refers to two or more chemical moieties each having specific function being covalently linked with each other. Correspondingly, "conjugate" refers to a compound formed by covalent linkage of individual chemical moieties. Further, "siRNA conjugate" represents a compound formed by covalently linking one or more chemical moieties having specific functions to siRNA. In the following text, the siRNA conjugate of the present disclosure is sometimes abbreviated as "conjugate". According to the context of the present disclosure, the siRNA conjugate should be understood as the general term of multiple siRNA conjugates, or an

siRNA conjugate represented by a certain chemical Formula. In the context of the present disclosure, "conjugating molecule" should be interpreted as a specific compound capable of being conjugated to an siRNA via reactions, thus finally forming the siRNA conjugate of the present disclosure.

As used herein, "optional" or "optionally" is meant that the subsequently described event or circumstance may or may not occur, and that the description includes instances wherein the event or circumstance occurs and instances in which it does not. For example, "optionally substituted alkyl" encompasses both "alkyl" and "substituted alkyl" as defined below. It will be understood by those skilled in the art, with respect to any group containing one or more substituents, that such groups are not intended to introduce any substitution or substitution patterns that are sterically impractical, synthetically infeasible and/or inherently unstable.

As used herein, "alkyl" refers to straight chain and branched chain having the indicated number of carbon atoms, usually from 1 to 20 carbon atoms, for example 1 to 10 carbon atoms, such as 1 to 8 or 1 to 6 carbon atoms. For example $C_1$-$C_6$ alkyl encompasses both straight and branched chain alkyl of from 1 to 6 carbon atoms. When referring to an alkyl residue having a specific number of carbons, all branched and straight chain forms having that number of carbons are intended to be encompassed; thus, for example, "butyl" is meant to include n-butyl, sec-butyl, isobutyl and t-butyl; "propyl" includes n-propyl and isopropyl. Alkylene is a subset of alkyl, referring to the same residues as alkyl, but having two attachment points.

As used herein, "alkenyl" refers to an unsaturated branched or straight-chain alkyl group having at least one carbon-carbon double bond obtained by removing one hydrogen molecule from two adjacent carbon atoms of the parent alkyl. The group may be in either the cis or trans configuration of the double bond(s). Typical alkenyl groups include, but are not limited to, ethenyl; propenyls such as prop-1-en-1-yl, prop-1-en-2-yl, prop-2-en-1-yl (allyl), prop-2-en-2-yl; butenyls such as but-1-en-1-yl, but-1-en-2-yl, 2-methyl-prop-1-en-1-yl, but-2-en-1-yl, but-2-en-2-yl, buta-1,3-dien-1-yl, buta-1,3-dien-2-yl; and the like. In certain embodiments, an alkenyl group has from 2 to 20 carbon atoms and in other embodiments, from 2 to 10, 2 to 8, or 2 to 6 carbon atoms. Alkenylene is a subset of alkenyl, referring to the same residues as alkenyl, but having two attachment points.

As used herein, "alkynyl" refers to an unsaturated branched or straight-chain alkyl group having at least one carbon-carbon triple bond obtained by removing two hydrogen molecules from two adjacent carbon atoms of the parent alkyl. Typical alkynyl groups include, but are not limited to, ethynyl; propynyls such as prop-1-yn-1-yl, prop-2-yn-1-yl; butynyls such as but-1-yn-1-yl, but-1-yn-3-yl, but-3-yn-1-yl; and the like. In certain embodiments, an alkynyl group has from 2 to 20 carbon atoms and in other embodiments, from 2 to 10, 2 to 8, or 2 to 6 carbon atoms. Alkynylene is a subset of alkynyl, referring to the same residues as alkynyl, but having two attachment points.

As used herein, "alkoxy" refers to an alkyl group of the indicated number of carbon atoms linked through an oxygen bridge such as, for example, methoxy, ethoxy, propoxy, isopropoxy, n-butoxy, sec-butoxy, tert-butoxy, pentyloxy, 2-pentyloxy, isopentyloxy, neopentyloxy, hexyloxy, 2-hexyloxy, 3-hexyloxy, 3-methylpentyloxy, and the like. Alkoxy groups will usually have from 1 to 10, 1 to 8, 1 to 6, or 1 to 4 carbon atoms linked through oxygen bridge.

As used herein, "aryl" refers to a radical derived from an aromatic monocyclic or multicyclic hydrocarbon ring system by removing a hydrogen atom from a ring carbon atom. The aromatic monocyclic or multicyclic hydrocarbon ring system contains only hydrogen and carbon, including from 6 to 18 carbon atoms, where at least one ring in the ring system is fully unsaturated, i.e., it contains a cyclic, delocalized $(4n+2)\pi$-electron system in accordance with the Hückel theory. Aryl groups include, but are not limited to, groups such as phenyl, fluorenyl, and naphthyl. Arylene is a subset of aryl, referring to the same residues as aryl, but having two attachment points.

As used herein, "halo substituent" or "halo" refers to fluoro, chloro, bromo, or iodo, and the term "halogen" includes fluorine, chlorine, bromine, or iodine.

As used herein, "haloalkyl" refers to alkyl as defined above having the specified number of carbon atoms, substituted with 1 or more halogen atoms, up to the maximum allowable number of halogen atoms. Examples of haloalkyl include, but are not limited to, trifluoromethyl, difluoromethyl, 2-fluoroethyl, or pentafluoroethyl.

"Heterocyclyl" refers to a stable 3 to 18 membered non-aromatic ring radical that comprises 2 to 12 carbon atoms and 1 to 6 heteroatoms selected from nitrogen, oxygen or sulfur. Unless stated otherwise specifically in the specification, the heterocyclyl is a monocyclic, bicyclic, tricyclic or tetracyclic system, which may include fused or bridged ring system(s). The heteroatom(s) in the heterocyclyl may be optionally oxidized. One or more nitrogen atoms, if present, are optionally quaternized. The heterocyclyl is partially or fully saturated. The heterocyclyl may be linked to the rest of the molecule through any atom of the ring(s). Examples of such heterocyclyl include, but are not limited to, dioxolanyl, thienyl[1,3]dithianyl, decahydroisoquinolyl, imidazolinyl, imidazolidinyl, isothiazolidinyl, isoxazolidinyl, morpholinyl, octahydroindolyl, octahydroisoindolyl, 2-oxapiperazinyl, 2-oxapiperidinyl, 2-oxapyrrolidinyl, oxazolidinyl, piperidinyl, piperazinyl, 4-piperidonyl, pyrrolidinyl, pyrazolidinyl, quinuclidinyl, thiazolidinyl, tetrahydrofuryl, trithianyl, tetrahydropyranyl, thiomorpholinyl, thiamorpholinyl, 1-oxo-thiomorpholinyl, and 1,1-dioxo-thiomorpholinyl.

"Heteroaryl" refers to a radical derived from a 3 to 18 membered aromatic ring radical that comprises 2 to 17 carbon atoms and 1 to 6 heteroatoms selected from nitrogen, oxygen and sulfur. As used herein, the heteroaryl radical may be a monocyclic, bicyclic, tricyclic or tetracyclic ring system, wherein at least one ring in the ring system is fully

unsaturated, i.e., it contains a cyclic, delocalized (4n+2)π-electron system in accordance with the Hückel theory. Heteroaryl includes fused or bridged ring system(s). The heteroatom(s) in the heteroaryl radical is optionally oxidized. One or more nitrogen atoms, if present, are optionally quaternized. The heteroaryl is linked to the rest of the molecule through any atom of the ring(s). Examples of heteroaryl include, but are not limited to, azepinyl, acridinyl, benzimidazolyl, benzindolyl, 1,3-benzodioxazolyl, benzofuranyl, benzoxazolyl, benzo[d]thiazolyl, benzothiadiazolyl, benzo[b][1,4]dioxepinyl, benzo[b][1,4]oxazinyl, 1,4 benzodioxanyl, benzonaphthofuranyl, benzoxazolyl, benzodioxolyl, benzodioxinyl, benzopyranyl, benzopyranonyl, benzofuranyl, benzofuranonyl, benzothienyl, benzothieno[3,2-d]pyrimidinyl, benzotriazolyl, benzo[4,6]imidazo[1,2-a]pyridinyl, carbazolyl, cinnolinyl, cyclopentano[d]pyrimidinyl, 6,7-dihydro 5H-cyclopenta[4,5]thieno[2,3-d]pyrimidinyl, 5,6-dihydrobenzo[h]quinazolinyl, 5,6 dihydrobenzo[h]cinnolinyl, 6,7-dihydro-5H-benzo[6,7]cyclohepta[1,2-c]pyridazinyl, dibenzofuranyl, dibenzothiophenyl, furanyl, furanonyl, furo[3,2-c]pyridinyl, 5,6,7,8,9,10 hexahydrocyclooctano[d]pyrimidinyl, 5,6,7,8,9,10-hexahydrocycloocta[d]pyridazinyl, 5,6,7,8,9,10-hexahydrocycloocta[d]pyridinyl, isothiazolyl, imidazolyl, indazolyl, indolyl, isoindolyl, indolinyl, isoindolinyl, isoquinolyl, indolizinyl, isoxazolyl, 5,8-methano-5,6,7,8-tetrahydroquinazolinyl, naphthyridinyl, 1,6-naphthyridinonyl, oxadiazolyl, 2-oxoazepinyl, oxazolyl, oxiranyl, 5,6,6a,7,8,9,10,10a-octahydrobenzo[h]quinazolinyl, 1-phenyl-1*H*-pyrrolyl, phenazinyl, phenothiazinyl, phenoxazinyl, phthalazinyl, pteridinyl, purinyl, pyrrolyl, pyrazolyl, pyrazolo[3,4-d]pyrimidinyl, pyridinyl, pyrido[3,2-d]pyrimidinyl, pyrido[3,4-d]pyrimidinyl, pyrazinyl, pyrimidinyl, pyridazinyl, quinazolinyl, quinoxalinyl, quinolinyl, tetrahydroquinolinyl, 5,6,7,8-tetrahydroquinazolinyl, 5,6,7,8-tetrahydrobenzo[4,5]thieno[2,3-d]pyrimidinyl, 6,7,8,9-tetrahydro-5H-cyclohepta [4,5]thieno[2,3-d]pyrimidinyl, 5,6,7,8-tetrahydropyrido[4,5-c]pyridazinyl, thiazolyl, thiadiazolyl, triazolyl, tetrazolyl, triazinyl, thieno[2,3-d]pyrimidinyl, thieno[3,2-d]pyrimidinyl, thieno[2,3-c]pyridinyl and thiophenyl/thienyl.

Various hydroxyl protecting groups may be used in the present disclosure. In general, protecting groups render chemical functional groups inert to specific reaction conditions, and may be appended to and removed from such functional groups in a molecule without substantially damaging the rest of the molecule. Representative hydroxyl protecting groups are disclosed in Beaucage, et al., Tetrahedron 1992, 48, 2223-2311, and also in Greene and Wuts, Protective Groups in Organic Synthesis, Chapter 2, 2d ed, John Wiley & Sons, New York, 1991, each of which is hereby incorporated by reference in their entirety. In some embodiments, the protecting group is stable under basic conditions but may be removed under acidic conditions. In some embodiments, non-exclusive examples of the hydroxyl protecting groups that may be used herein include dimethoxytrityl (DMT), monomethoxytrityl, 9-phenylxanthen-9-yl (Pixyl) and 9-(p-methoxyphenyl)xanthen-9-yl (Mox). In some embodiments, non-exclusive examples of the hydroxyl protecting groups that may be used herein comprises Tr (trityl), MMTr (4-methoxytrityl), DMTr (4,4'-dimethoxytrityl), and TMTr (4,4',4"-trimethoxytrityl).

The term "subject", as used herein, refers to any animal, e.g., a mammal or marsupial. Subject of the present disclosure includes but are not limited to human, non-human primate (e.g., rhesus or other types of macaques), mouse, pig, horse, donkey, cow, sheep, rat or any kind of poultry.

As used herein, "treatment" refers to an approach for obtaining beneficial or desired results including but not limited to therapeutic benefit. By "therapeutic benefit" is meant eradication or amelioration of the underlying disorder being treated. Also, a therapeutic benefit is achieved by eradicating or ameliorating one or more of the physiological symptoms associated with the underlying disorder such that amelioration is observed in the subject, notwithstanding that the subject may still be afflicted with the underlying disorder.

As used herein, "prevention" refers to an approach for obtaining beneficial or desired results including but not limited to a prophylactic benefit. For "prophylactic benefit", the siRNA conjugates or pharmaceutical compositions may be administered to a subject at risk of developing a particular disease, or to a subject reporting one or more of the physiological symptoms of a disease, even though a diagnosis of this disease may not have been made.

In one aspect, the present disclosure provides the first to fourth siRNAs capable of inhibiting expression of PNP gene. Which in turn are described in detail below.

The siRNA of the present disclosure contains a nucleotide group as a basic structural unit, and those skilled in the art know that the nucleotide group contains a phosphate group, a ribose group and a base, which is not described in detail herein.

First siRNA

According to the present disclosure, the siRNA may be a first siRNA.

The first siRNA comprises a sense strand and an antisense strand, each nucleotide in the first siRNA being independently a modified or unmodified nucleotide; wherein the sense strand comprises a nucleotide sequence I, and the antisense strand comprises a nucleotide sequence II; the nucleotide sequence I and the nucleotide sequence II are at least partly reverse complementary to form a double-stranded region; wherein the nucleotide sequence I has the same length as the nucleotide sequence represented by SEQ ID NO: 1 with no more than 3 nucleotide differences; and the nucleotide sequence II has the same length as the nucleotide sequence represented by SEQ ID NO: 2 with no more than 3 nucleotide differences:

5'-CCUAUGAAGAUUAUAAGA$Z_1$-3' (SEQ ID NO: 1);
5'-$Z_2$UCUUAUAAUCUUCAUAGG-3' (SEQ ID NO: 2),

where $Z_1$ is A, and $Z_2$ is U;
the nucleotide sequence I comprises a nucleotide $Z_3$ at the position corresponding to $Z_1$; the nucleotide sequence II comprises a nucleotide $Z_4$ at the position corresponding to $Z_2$; the $Z_4$ is the first nucleotide at 5' terminal of the antisense strand.

In the context of the present disclosure, "corresponding position" refers to the same position in the nucleotide sequence by counting from the same terminal of the nucleotide sequence. For example, the first nucleotide at the 3' terminal of nucleotide sequence I is a nucleotide at the corresponding position of the first nucleotide at the 3' terminal of SEQ ID NO: 1.

In some embodiments, the sense strand comprises only nucleotide sequence I, and the antisense strand comprises only nucleotide sequence II.

In some embodiments, there is no more than 1 nucleotide difference between the nucleotide sequence I and the nucleotide sequence represented by SEQ ID NO: 1, and/or there is no more than 1 nucleotide difference between the nucleotide sequence II and the nucleotide sequence represented by SEQ ID NO: 2.

In some embodiments, the nucleotide difference between the nucleotide sequence II and the nucleotide sequence represented by SEQ ID NO: 2 includes a difference at the position of $Z_4$, wherein $Z_4$ is selected from A, C or G. In some embodiments, the nucleotide difference is a difference at the position of $Z_4$, wherein $Z_4$ is selected from A, C or G. In some embodiments, $Z_3$ is a nucleotide complementary to $Z_4$. The siRNAs with the above nucleotide differences have higher target mRNA inhibition ability, and these siRNAs containing the nucleotide differences are also within the protection scope of the present disclosure.

In some embodiments, the nucleotide sequence I and the nucleotide sequence II are basically reverse complementary, substantially reverse complementary, or completely reverse complementary to each otherl. "Basically reverse complementary" means that there is no more than 3 base mispairings between two nucleotide sequences. "Substantially reverse complementary" means that there is no more than 1 base mispairing between two nucleotide sequences. "Completely reverse complementary" means that there is no base mispairing between two nucleotide sequences.

In some embodiments, the nucleotide sequence I is a nucleotide sequence represented by SEQ ID NO: 3, and the nucleotide sequence II is a nucleotide sequence represented by SEQ ID NO: 4:

5'-CCUAUGAAGAUUAUAAGA$Z_3$-3' (SEQ ID NO: 3);
5'- $Z_4$UCUUAUAAUCUUCAUAGG-3' (SEQ ID NO: 4),

wherein, the $Z_4$ is the first nucleotide at the 5' terminal of the antisense strand, $Z_3$ is selected from A, U, G or C, and $Z_4$ is a nucleotide complementary to $Z_3$. In some embodiments, $Z_3$ is A, and $Z_4$ is U.

In some embodiment, the sense strand further comprises a nucleotide sequence III, the antisense strand further comprise a nucleotide sequence IV, and the nucleotide sequence III and the nucleotide sequence IV each have a length of 1 to 4 nucleotides; the nucleotide sequence III and the nucleotide sequence IV have the same length, and are substantially reverse complementary or completely reverse complementary; the nucleotide sequence III is linked to the 5' terminal of the nucleotide sequence I, and the nucleotide sequence IV is linked to the 3' terminal of the nucleotide sequence II. In some embodiments, the nucleotide sequence IV is substantially reverse complementary or completely reverse complementary to a second nucleotide sequence, which refers to a nucleotide sequence adjacent to the 5' terminal of the nucleotide sequence represented by SEQ ID NO: 1 in a target mRNA and having the same length as the nucleotide sequence IV.

In some embodiments, the nucleotide sequence III and the nucleotide sequence IV both have a length of one nucleotide, and the base of the nucleotide sequence III is A, and the base of the nucleotide sequence IV is U; in this case, the length ratio of the sense strand and the antisense strand thereof is 20/20; or, the nucleotide sequence III and the nucleotide sequence IV both have a length of 2 nucleotides, and in the direction from 5' terminal to 3' terminal, the the base composition of nucleotide sequence III is CA, and the base composition of the nucleotide sequence IV is UG; in this case, the length ratio of the sense strand and the antisense strand thereof is 21/21; or, the nucleotide sequence III and the nucleotide sequence IV both have a length of 3 nucleotides, and in the direction from 5' terminal to 3' terminal, the base composition of the nucleotide sequence III is ACA, and the base composition of the nucleotide sequence IV is UGU; in this case, the length ratio of the sense strand and the antisense strand thereof is 22/22; or, the nucleotide sequence III and the nucleotide sequence IV both have a length of 4 nucleotides, and in the direction from 5' terminal to 3' terminal, the base composition of the nucleotide sequence III is UACA, and the base composition of the nucleotide sequence IV is UGUA; in this case, the length ratio of the sense strand and the antisense strand thereof is 23/23. In some embodiments, the nucleotide sequence III and the nucleotide sequence IV have a length of 2 nucleotides, and in the direction from 5' terminal to 3' terminal, the base composition of the nucleotide sequence III is CA, and the base

composition of the nucleotide sequence IV is UG; in this case, the length ratio of the sense strand and the antisense strand thereof is 21/21.

In some embodiments, the nucleotide sequence III and the nucleotide sequence IV are completely reverse complementary. Hence, where the base(s) of nucleotide sequence III is(are) provided, the base(s) of nucleotide sequence IV is(are) determined.

Second siRNA

According to the present disclosure, the siRNA may be a second siRNA.

The second siRNA comprises a sense strand and an antisense strand, each nucleotide in the second siRNA being independently a modified or unmodified nucleotide; wherein the sense strand comprises a nucleotide sequence I, and the antisense strand comprises a nucleotide sequence II; the nucleotide sequence I and the nucleotide sequence II are at least partly reverse complementary to form a double-stranded region; wherein the nucleotide sequence I has the same length as the nucleotide sequence represented by SEQ ID NO: 61 with no more than 3 nucleotide differences; and the nucleotide sequence II has the same length as the nucleotide sequence represented by SEQ ID NO: 62 with no more than 3 nucleotide differences:

5'-GUACAGUACCAGAAGUUA$Z_5$-3' (SEQ ID NO: 61);
5'-$Z_6$UAACUUCUGGUACUGUAC-3' (SEQ ID NO: 62),

wherein $Z_5$ is A, and $Z_6$ is U;
and the nucleotide sequence I comprises a nucleotide $Z_7$ at the position corresponding to $Z_5$; the nucleotide sequence II comprises a nucleotide $Z_8$ at the position corresponding to $Z_6$; the $Z_8$ is the first nucleotide at 5' terminal of the antisense strand.

In some embodiments, the sense strand comprises only nucleotide sequence I, and the antisense strand comprises only nucleotide sequence II.

In some embodiments, there is no more than 1 nucleotide difference between the nucleotide sequence I and the nucleotide sequence represented by SEQ ID NO: 61, and/or there is no more than 1 nucleotide difference between the nucleotide sequence II and the nucleotide sequence represented by SEQ ID NO: 62.

In some embodiments, the nucleotide difference between the nucleotide sequence II and the nucleotide sequence represented by SEQ ID NO: 62 includes a difference at the position of $Z_8$, wherein $Z_8$ is selected from A, C or G. In some embodiments, the nucleotide difference is a difference at the position of $Z_8$, wherein $Z_8$ is selected from A, C or G. In some embodiments, $Z_7$ is a nucleotide complementary to $Z_8$. The siRNAs with the above nucleotide differences have higher target mRNA inhibition ability, and these siRNAs containing the nucleotide differences are also within the protection scope of the present disclosure.

In some embodiments, the nucleotide sequence I and the nucleotide sequence II are basically reverse complementary, substantially reverse complementary, or completely reverse complementary to each other.

In some embodiments, the nucleotide sequence I is a nucleotide sequence represented by SEQ ID NO: 63, and the nucleotide sequence II is a nucleotide sequence represented by SEQ ID NO: 64:

5'-GUACAGUACCAGAAGUUA$Z_7$-3' (SEQ ID NO: 63);
5'-$Z_8$UAACUUCUGGUACUGUAC-3' (SEQ ID NO: 64),

wherein, the $Z_8$ is the first nucleotide at the 5' terminal of the antisense strand, $Z_7$ is selected from A, U, G or C, and $Z_8$ is a nucleotide complementary to $Z_7$. In some embodiments, $Z_7$ is U, and $Z_8$ is A.

In some embodiment, the sense strand further comprises a nucleotide sequence III, the antisense strand further comprise a nucleotide sequence IV, and the nucleotide sequence III and the nucleotide sequence IV each have a length of 1 to 4 nucleotides; the nucleotide sequence III and the nucleotide sequence IV have the same length, and are substantially reverse complementary or completely reverse complementary; the nucleotide sequence III is linked to the 5' terminal of the nucleotide sequence I, and the nucleotide sequence IV is linked to the 3' terminal of the nucleotide sequence II. The nucleotide sequence IV is substantially reverse complementary or completely reverse complementary to a second nucleotide sequence, which refers to a nucleotide sequence adjacent to the 5' terminal of the nucleotide sequence represented by SEQ ID NO: 61 in a target mRNA and having the same length as the nucleotide sequence IV.

In some embodiments, the nucleotide sequence III and the nucleotide sequence IV both have a length of one nucleotide, and the base of the nucleotide sequence III is A, and the base of the nucleotide sequence IV is U; in this case, the length ratio of the sense strand and the antisense strand thereof is 20/20; or, the nucleotide sequence III and the nucleotide sequence IV both have a length of 2 nucleotides, and in the direction from 5' terminal to 3' terminal, the base composition of the nucleotide sequence III is GA, and the base composition of the nucleotide sequence IV is UC;

in this case, the length ratio of the sense strand and the antisense strand thereof is 21/21; or, the nucleotide sequence III and the nucleotide sequence IV both have a length of 3 nucleotides, and in the direction from 5' terminal to 3' terminal, the base composition of the nucleotide sequence III is UGA, and the base composition of the nucleotide sequence IV is UCA; in this case, the length ratio of the sense strand and the antisense strand thereof is 22/22; or, the nucleotide sequence III and the nucleotide sequence IV both have a length of 4 nucleotides, and in the direction from 5' terminal to 3' terminal, the base composition of the nucleotide sequence III is AUGA, and the base composition of the nucleotide sequence IV is UCAU; in this case, the length ratio of the sense strand and the antisense strand thereof is 23/23. In some embodiments, the nucleotide sequence III and the nucleotide sequence IV have a length of 2 nucleotides, and in the direction from 5' terminal to 3' terminal, the base composition of the nucleotide sequence III is GA, and the base composition of the nucleotide sequence IV is UC; in this case, the length ratio of the sense strand and the antisense strand thereof is 21/21.

In some embodiments, the nucleotide sequence III and the nucleotide sequence IV are completely reverse complementary. Hence, where the base(s) of nucleotide sequence III is(are) provided, the base(s) of nucleotide sequence IV is(are) determined.

Third siRNA

According to the present disclosure, the siRNA may be a third siRNA.

The third siRNA comprises a sense strand and an antisense strand, each nucleotide in the third siRNA being independently a modified or unmodified nucleotide; wherein the sense strand comprises a nucleotide sequence I, and the antisense strand comprises a nucleotide sequence II; the nucleotide sequence I and the nucleotide sequence II are at least partly reverse complementary to form a double-stranded region; wherein the nucleotide sequence I has the same length as the nucleotide sequence represented by SEQ ID NO: 121 with no more than 3 nucleotide differences; and the nucleotide sequence II has the same length as the nucleotide sequence represented by SEQ ID NO: 122 with no more than 3 nucleotide differences:

5'- CAAACAAGCUGCACAGAA$Z_9$-3' (SEQ ID NO: 121);
5'-$Z_{10}$UUCUGUGCAGCUUGUUUG-3' (SEQ ID NO: 122),

wherein $Z_9$ is A, and $Z_{10}$ is U;
the nucleotide sequence I comprises a nucleotide $Z_{11}$ at the position corresponding to $Z_9$; the nucleotide sequence II comprises a nucleotide $Z_{12}$ at the position corresponding to $Z_{10}$; the $Z_{12}$ is the first nucleotide at 5' terminal of the antisense strand.

In some embodiments, the sense strand comprises only nucleotide sequence I, and the antisense strand comprises only nucleotide sequence II.

In some embodiments, there is no more than 1 nucleotide difference between the nucleotide sequence I and the nucleotide sequence represented by SEQ ID NO: 121, and/or there is no more than 1 nucleotide difference between the nucleotide sequence II and the nucleotide sequence represented by SEQ ID NO: 122.

In some embodiments, the nucleotide difference between the nucleotide sequence II and the nucleotide sequence represented by SEQ ID NO: 122 includes a difference at the position of $Z_{12}$, wherein $Z_{12}$ is selected from A, C or G. In some embodiments, the nucleotide difference is a difference at the position of $Z_{12}$, wherein $Z_{12}$ is selected from A, C or G. In some embodiments, $Z_{11}$ is a nucleotide complementary to $Z_{12}$. The siRNAs with the above nucleotide differences have higher target mRNA inhibition ability, and these siRNAs containing the nucleotide differences are also within the protection scope of the present disclosure.

In some embodiments, the nucleotide sequence I and the nucleotide sequence II are basically reverse complementary, substantially reverse complementary, or completely reverse complementary to each other.

In some embodiments, the nucleotide sequence I is a nucleotide sequence represented by SEQ ID NO: 123, and the nucleotide sequence II is a nucleotide sequence represented by SEQ ID NO: 124:

5'- CAAACAAGCUGCACAGAA$Z_{11}$-3' (SEQ ID NO: 123);
5'-$Z_{12}$UUCUGUGCAGCUUGUUUG-3' (SEQ ID NO: 124),

wherein, the $Z_{12}$ is the first nucleotide at the 5' terminal of the antisense strand, $Z_{11}$ is selected from A, U, G or C, and $Z_{12}$ is a nucleotide complementary to $Z_{11}$. In some embodiments, $Z_{11}$ is A, and $Z_{12}$ is U.

In some embodiment, the sense strand further comprises a nucleotide sequence III, the antisense strand further comprise a nucleotide sequence IV, and the nucleotide sequence III and the nucleotide sequence IV each have a length of 1 to 4 nucleotides; the nucleotide sequence III and the nucleotide sequence IV have the same length, and are substantially reverse complementary or completely reverse complementary; the nucleotide sequence III is linked to the

5' terminal of the nucleotide sequence I, and the nucleotide sequence IV is linked to the 3' terminal of the nucleotide sequence II. The nucleotide sequence IV is substantially reverse complementary or completely reverse complementary to a second nucleotide sequence, which refers to a nucleotide sequence adjacent to the 5' terminal of the nucleotide sequence represented by SEQ ID NO: 121 in a target mRNA and having the same length as the nucleotide sequence IV.

In some embodiments, the nucleotide sequence III and the nucleotide sequence IV both have a length of one nucleotide, and the base of the nucleotide sequence III is G, and the base of the nucleotide sequence IV is C; in this case, the length ratio of the sense strand and the antisense strand thereof is 20/20; or, the nucleotide sequence III and the nucleotide sequence IV both have a length of 2 nucleotides, and in the direction from 5' terminal to 3' terminal, the base composition of the nucleotide sequence III is GG, and the base composition of the nucleotide sequence IV is CC; in this case, the length ratio of the sense strand and the antisense strand thereof is 21/21; or, the nucleotide sequence III and the nucleotide sequence IV both have a length of 3 nucleotides, and in the direction from 5' terminal to 3' terminal, the base composition of the nucleotide sequence III is UGG, and the base composition of the nucleotide sequence IV is CCA; in this case, the length ratio of the sense strand and the antisense strand thereof is 22/22; or, the nucleotide sequence III and the nucleotide sequence IV both have a length of 4 nucleotides, and in the direction from 5' terminal to 3' terminal, the base composition of the nucleotide sequence III is CUGG, and the base composition of the nucleotide sequence IV is CCAG; in this case, the length ratio of the sense strand and the antisense strand thereof is 23/23. In some embodiments, the nucleotide sequence III and the nucleotide sequence IV have a length of 2 nucleotides, and in the direction from 5' terminal to 3' terminal, the base composition of the nucleotide sequence III is GG, and the base composition of the nucleotide sequence IV is CC; in this case, the length ratio of the sense strand and the antisense strand thereof is 21/21.

In some embodiments, the nucleotide sequence III and the nucleotide sequence IV are completely reverse complementary. Hence, where the base(s) of nucleotide sequence III is(are) provided, the base(s) of nucleotide sequence IV is(are) determined.

In some embodiments, the sense strand of the siRNA comprises a nucleotide sequence represented by SEQ ID NO: 125, and the antisense strand of the siRNA comprises a nucleotide sequence represented by SEQ ID NO: 126:

5'- CAAACAAGCUGCACAGAA$Z_{11}$-3' (SEQ ID NO: 125);
5'-$Z_{12}$UUCUGUGCAGCUUGUUUGCC-3' (SEQ ID NO: 126),

or, the sense strand of the siRNA comprises a nucleotide sequence represented by SEQ ID NO: 127, and the antisense strand of the siRNA comprises a nucleotide sequence represented by SEQ ID NO: 128:

5'- GGCAAACAAGCUGCACAGAA$Z_{11}$-3' (SEQ ID NO: 127);
5'-$Z_{12}$UUCUGUGCAGCUUGUUUGCCAG-3' (SEQ ID NO: 128),

wherein, the $Z_{12}$ is the first nucleotide at the 5' terminal of the antisense strand, $Z_{11}$ is selected from A, U, G or C, and $Z_{12}$ is a nucleotide complementary to $Z_{11}$.

<u>Fourth siRNA</u>

According to the present disclosure, the siRNA may be a fourth siRNA.

The fourth siRNA comprises a sense strand and an antisense strand, each nucleotide in the fourth siRNA being independently a modified or unmodified nucleotide; wherein the sense strand comprises a nucleotide sequence I, and the antisense strand comprises a nucleotide sequence II; the nucleotide sequence I and the nucleotide sequence II are at least partly reverse complementary to form a double-stranded region; wherein the nucleotide sequence I has the same length as the nucleotide sequence represented by SEQ ID NO: 181 with no more than 3 nucleotide differences; and the nucleotide sequence II has the same length as the nucleotide sequence represented by SEQ ID NO: 182 with no more than 3 nucleotide differences:

5'- CAAACAAGGACUAAUCCA$Z_{13}$-3' (SEQ ID NO: 181);
5'-$Z_{14}$UGGAUUAGUCCUUGUUUG-3' (SEQ ID NO: 182),

wherein $Z_{13}$ is A, and $Z_{14}$ is U;
the nucleotide sequence I comprises a nucleotide $Z_{15}$ at the position corresponding to $Z_{13}$; the nucleotide sequence II comprises a nucleotide $Z_{16}$ at the position corresponding to $Z_{14}$; the $Z_{16}$ is the first nucleotide at 5' terminal of the antisense strand.

In some embodiments, the sense strand comprises only nucleotide sequence I, and the antisense strand comprises only nucleotide sequence II.

In some embodiments, there is no more than 1 nucleotide difference between the nucleotide sequence I and the nucleotide sequence represented by SEQ ID NO: 181, and/or there is no more than 1 nucleotide difference between the nucleotide sequence II and the nucleotide sequence represented by SEQ ID NO: 182.

In some embodiments, the nucleotide difference between the nucleotide sequence II and the nucleotide sequence represented by SEQ ID NO: 182 includes a difference at the position of $Z_{16}$, wherein $Z_{16}$ is selected from A, C or G. In some embodiments, the nucleotide difference is a difference at the position of $Z_{16}$, wherein $Z_{16}$ is selected from A, C or G. In some embodiments, $Z_{15}$ is a nucleotide complementary to $Z_{16}$. The siRNAs with the above nucleotide differences have higher target mRNA inhibition ability, and these siRNAs containing the nucleotide differences are also within the protection scope of the present disclosure.

In some embodiments, the nucleotide sequence I and the nucleotide sequence II are basically reverse complementary, substantially reverse complementary, or completely reverse complementary to each other.

In some embodiments, the nucleotide sequence I is a nucleotide sequence represented by SEQ ID NO: 183, and the nucleotide sequence II is a nucleotide sequence represented by SEQ ID NO: 184:

5'- CAAACAAGGACUAAUCCA$Z_{15}$-3' (SEQ ID NO: 183);
5'-$Z_{16}$UGGAUUAGUCCUUGUUUG-3' (SEQ ID NO: 184),

wherein, the $Z_{16}$ is the first nucleotide at the 5' terminal of the antisense strand, $Z_{15}$ is selected from A, U, G or C, and $Z_{16}$ is a nucleotide complementary to $Z_{15}$. In some embodiments, $Z_{15}$ is A, and $Z_{16}$ is U.

In some embodiment, the sense strand further comprises a nucleotide sequence III, the antisense strand further comprise a nucleotide sequence IV, and the nucleotide sequence III and the nucleotide sequence IV each have a length of 1 to 4 nucleotides; the nucleotide sequence III and the nucleotide sequence IV have the same length, and are substantially reverse complementary or completely reverse complementary; the nucleotide sequence III is linked to the 5' terminal of the nucleotide sequence I, and the nucleotide sequence IV is linked to the 3' terminal of the nucleotide sequence II. The nucleotide sequence IV is substantially reverse complementary or completely reverse complementary to a second nucleotide sequence, which refers to a nucleotide sequence adjacent to the 5' terminal of the nucleotide sequence represented by SEQ ID NO: 181 in a target mRNA and having the same length as the nucleotide sequence IV.

In some embodiments, the nucleotide sequence III and the nucleotide sequence IV both have a length of one nucleotide, and the base of the nucleotide sequence III is C, and the base of the nucleotide sequence IV is G; in this case, the length ratio of the sense strand and the antisense strand thereof is 20/20; or, the nucleotide sequence III and the nucleotide sequence IV both have a length of 2 nucleotides, and in the direction from 5' terminal to 3' terminal, the base composition of the nucleotide sequence III is AC, and the base composition of the nucleotide sequence IV is GU; in this case, the length ratio of the sense strand and the antisense strand thereof is 21/21; or, the nucleotide sequence III and the nucleotide sequence IV both have a length of 3 nucleotides, and in the direction from 5' terminal to 3' terminal, the base composition of the nucleotide sequence III is GAC, and the base composition of the nucleotide sequence IV is GUC; in this case, the length ratio of the sense strand and the antisense strand thereof is 22/22; or, the nucleotide sequence III and the nucleotide sequence IV both have a length of 4 nucleotides, and in the direction from 5' terminal to 3' terminal, the base composition of the nucleotide sequence III is AGAC, and the base composition of the nucleotide sequence IV is GUCU; in this case, the length ratio of the sense strand and the antisense strand thereof is 23/23. In some embodiments, the nucleotide sequence III and the nucleotide sequence IV have a length of 2 nucleotides, and in the direction from 5' terminal to 3' terminal, the base composition of the nucleotide sequence III is AC, and the base composition of the nucleotide sequence IV is GU; in this case, the length ratio of the sense strand and the antisense strand thereof is 21/21.

In some embodiments, the nucleotide sequence III and the nucleotide sequence IV are completely reverse complementary. Hence, where the base(s) of nucleotide sequence III is(are) provided, the base(s) of nucleotide sequence IV is(are) determined.

Hereinafter, the descriptions of nucleotide sequence V, nucleic acid sequence, nucleotide modification and modification sequence in the siRNA, are applied to any one of the above-mentioned first to fourth siRNAs. That is, unless otherwise specified, the following description of siRNAs is to be taken as describing the first siRNA, the second siRNA, the third siRNA, and the fourth siRNA one by one. For example, if a specific siRNA is not specifically indicated, "The siRNA further comprises a nucleotide sequence V" means "The first siRNA, the second siRNA, the third siRNA or the fourth siRNA further comprises a nucleotide sequence V".

In some embodiments, the sense strand and the antisense strand have different lengths. The antisense strand futher comprises nucleotide sequence V. The nucleotide sequence V has a length of 1 to 3 nucleotides and is linked to the 3' terminal of the antisense strand, thereby forming a 3' overhang terminal of the antisense strand. In this case, the length ratio of the sense strand and the antisense strand of the siRNA of provided by the present disclosure may be 19/20, 19/21, 19/22, 20/21, 20/22, 20/23, 21/22, 21/23, 21/24, 22/23, 22/24, 22/25, 23/24, 23/25 or 23/26. In some embodiments, the nucleotide sequence V has a length of two nucleotides. In this case, the length ratio of the sense

strand and the antisense strand of the siRNA of provided by the present disclosure may be 19/21, 21/23 or 23/25.

Each nucleotide in the nucleotide sequence V may be any nucleotide. In order to facilitate the synthesis and to save synthesis cost, the nucleotide sequence V is 2 continuous thymine deoxyribonucleotides (dTdT) or 2 continuous uridine ribonucleotides (UU); or, in order to enhance the affinity of the antisense strand of the siRNA to the target mRNA, the nucleotide sequence V is complementary to the nucleotide(s) at the corresponding position(s) of the target mRNA. Thus, in some embodiments, the length ratio of the sense strand and the antisense strand of the siRNA of the present disclosure is 19/21 or 21/23. In this case, the siRNA of the present disclosure has better activity for silencing the target mRNA.

The nucleotide at the corresponding position of the target mRNA refers to the nucleotide or nucleotide sequence adjacent to the 5' terminal of the third nucleotide sequence of the target mRNA, and the third nucleotide sequence is the nucleotide sequence substantially reverse complementary or completely reverse complementary to nucleotide sequence II, or substantially reverse complementary or completely reverse complementary to the nucleotide sequence consisting of nucleotide sequence II and nucleotide sequence IV.

In some embodiments, for the first siRNA, the sense strand of the siRNA comprises the nucleotide sequence represented by SEQ ID NO: 5, and the antisense strand of the siRNA comprises the nucleotide sequence represented by SEQ ID NO: 6:

5'- CCUAUGAAGAUUAUAAGA$Z_3$-3' (SEQ ID NO: 5);
5'- $Z_4$UCUUAUAAUCUUCAUAGGUG-3' (SEQ ID NO: 6);

or, the sense strand of the siRNA comprises the nucleotide sequence represented by SEQ ID NO: 7, and the antisense strand of the siRNA comprises the nucleotide sequence represented by SEQ ID NO: 8:

5'- CACCUAUGAAGAUUAUAAGA$Z_3$-3' (SEQ ID NO: 7);
5'- $Z_4$UCUUAUAAUCUUCAUAGGUGUA-3' (SEQ ID NO: 8);

wherein, the $Z_4$ is the first nucleotide at the 5' terminal of the antisense strand, $Z_3$ is selected from of A, U, G or C, and $Z_4$ is a nucleotide complementary to $Z_3$.

In some embodiments, for the second siRNA, the sense strand of the siRNA comprises the nucleotide sequence represented by SEQ ID NO: 65, and the antisense strand of the siRNA comprises the nucleotide sequence represented by SEQ ID NO: 66:

5'- GUACAGUACCAGAAGUUA$Z_7$-3' (SEQ ID NO: 65);
5'- $Z_8$UAACUUCUGGUACUGUACUC-3' (SEQ ID NO: 66),

or, the sense strand of the siRNA comprises the nucleotide sequence represented by SEQ ID NO: 67, and the antisense strand of the siRNA comprises the nucleotide sequence represented by SEQ ID NO: 68:

5'- GAGUACAGUACCAGAAGUUA$Z_7$-3' (SEQ ID NO: 67);
5'- $Z_8$UAACUUCUGGUACUGUACUCAU-3' (SEQ ID NO: 68),

wherein, the $Z_8$ is the first nucleotide at the 5' terminal of the antisense strand, $Z_7$ is selected from of A, U, G or C, and $Z_8$ is a nucleotide complementary to $Z_7$.

In some embodiments, for the third siRNA, the sense strand of the siRNA comprises the nucleotide sequence represented by SEQ ID NO: 125, and the antisense strand of the siRNA comprises the nucleotide sequence represented by SEQ ID NO: 126:

5'- CAAACAAGCUGCACAGAA$Z_{11}$-3' (SEQ ID NO: 125);
5'-$Z_{12}$UUCUGUGCAGCUUGUUUGCC-3' (SEQ ID NO: 126),

or, the sense strand of the siRNA comprises the nucleotide sequence represented by SEQ ID NO: 127, and the antisense strand of the siRNA comprises the nucleotide sequence represented by SEQ ID NO: 128:

5'- GGCAAACAAGCUGCACAGAA$Z_{11}$-3' (SEQ ID NO: 127);
5'-$Z_{12}$UUCUGUGCAGCUUGUUUGCCAG-3' (SEQ ID NO: 128),

wherein, the $Z_{12}$ is the first nucleotide at the 5' terminal of the antisense strand, $Z_{11}$ is selected from of A, U, G or C, and $Z_{12}$ is a nucleotide complementary to $Z_{11}$. In some embodiments, for the fourth siRNA, the sense strand of the

siRNA comprises the nucleotide sequence represented by SEQ ID NO: 185, and the antisense strand of the siRNA comprises the nucleotide sequence represented by SEQ ID NO: 186:

5'- CAAACAAGGACUAAUCCA$Z_{15}$-3' (SEQ ID NO: 185);
5'-$Z_{16}$UGGAUUAGUCCUUGUUUGGU-3' (SEQ ID NO: 186),

or, the sense strand of the siRNA comprises the nucleotide sequence represented by SEQ ID NO: 187, and the antisense strand of the siRNA comprises the nucleotide sequence represented by SEQ ID NO: 188:

5'- ACCAAACAAGGACUAAUCCA$Z_{15}$-3' (SEQ ID NO: 187);
5'-$Z_{16}$UGGAUUAGUCCUUGUUUGGUCU-3' (SEQ ID NO: 188),

wherein, the $Z_{16}$ is the first nucleotide at the 5' terminal of the antisense strand, $Z_{15}$ is selected from of A, U, G or C, and $Z_{16}$ is a nucleotide complementary to $Z_{15}$.

In some embodiment, the siRNA described in the present disclosure are siPNa1, siPNa2, siPNb1, siPNb2, siPNcl, siPNc2, siPNd1, or siPNd2 listed in Tables 1a-1d.

As mentioned above, the nucleotides in the siRNA of the present disclosure are each independently modified or unmodified nucleotides. In some embodiments, the nucleotides in the siRNA of the present disclosure are unmodified nucleotides; in some embodiments, partial or all nucleotides in the siRNA of the present disclosure are modified nucleotides. These modifications on the nucleotide groups would not lead to significant impair or loss of functions of the siRNA of the present disclosure in inhibiting the expression of PNP gene.

In some embodiments, the siRNA of the present disclosure comprises at least one modified nucleotide. In the context of the present disclosure, the used term "modified nucleotide" refers to a nucleotide formed by substituting the 2'-hydroxy of the ribose group thereof with other groups, or a nucleotide analogue, or a nucleotide with a modified base. The modified nucleotide would not lead to significant impair or loss of functions of the siRNA in inhibiting gene expression. For example, modified nucleotides disclosed in J.K. Watts, G.F. Deleavey, and M.J. Damha, Chemically Modified siRNA: tools and applications. Drug Discov Today, 2008, 13(19-20): p.842-55 may be selected.

In some embodiments, at least one nucleotide in the sense strand or the antisense strand of the siRNA provided by the present disclosure is a modified nucleotide, and/or at least one phosphate group is a phosphate group with modified group(s). In other words, at least a portion of the phosphate goups and/or the ribose groups in phosphate-ribose backbone of at least one single strand in the sense strand and the antisense strand are a phosphate and/or ribose group with modified group(s).

In some embodiments, all nucleotides in the sense strand and/or the antisense strand are modified nucleotides. In some embodiments, each nucleotide in the sense strand and the antisense strand of the siRNA provided by the present disclosure is independently a fluoro modified nucleotide or a non-fluoro modified nucleotide.

The inventors of the present disclosure have surprisingly found that the siRNA of the present disclosure achieves a high degree of balance between stability in plasma and gene silencing efficiency in the animal experiment.

In some embodiments, the fluoro modified nucleotide is present in the nucleotide sequence I and nucleotide sequence II. Moreover, in the direction from 5' terminal to 3' terminal, at least the nucleotides at positions 7, 8, 9 of the nucleotide sequence I are fluoro modified nucleotides; in the direction from 5' terminal to 3' terminal, at least the nucleotides at positions 2, 6, 14 and 16 of the nucleotide sequence II are fluoro modified nucleotides.

In some embodiments, the fluoro modified nucleotide is present in the nucleotide sequence I and nucleotide sequence II. There is no more than 5 fluoro modified nucleotides present in the nucleotide sequence I. Moreover, in the direction from 5' terminal to 3' terminal, at least the nucleotides at positions 7, 8 and 9 of the nucleotide sequence I are fluoro modified nucleotides; there is no more than 7 fluoro modified nucleotides present in the nucleotide sequence II. Moreover, at least the nucleotides at the positions 2, 6, 14 and 16 of the nucleotide sequence II are fluoro modified nucleotides.

In some embodiments, in the direction from 5' terminal to 3' terminal, the nucleotides at positions 7, 8 and 9 or at positions 5, 7, 8 and 9 of the nucleotide sequence I in the sense strand are fluoro modified nucleotides, and the nucleotides at the other positions in the sense strand are non-fluoro modified nucleotides; in the direction from 5' terminal to 3' terminal, the nucleotides at positions 2, 6, 14 and 16 or at positions 2, 6, 8, 9, 14 and 16 of the nucleotide sequence II in the antisense strand are fluoro modified nucleotides, and the nucleotides at the other positions in the antisense strand are non-fluoro modified nucleotides.

In the context of the present disclosure, "fluoro modified nucleotide" refers to a nucleotide formed by substituting the 2'-hydroxy of the ribose group thereof with fluoro, and has a structures represented by the following Formula (7). "Non-fluoro modified nucleotide" refers to a nucleotide formed by substituting the 2'-hydroxy of the ribose group thereof with a non-fluoro group, or a nucleotide analogue. In some embodiments, each non-fluoro modified nucleotide is independently selected from a nucleotide formed by substituting the 2'-hydroxy of the ribose group thereof with a non-fluoro

group, or a nucleotide analogue.

A nucleotide formed by substituting the 2'-hydroxy of the ribose group with a non-fluoro group is well-known to those skilled in the art, such as one selected from the group consisting of 2'-alkoxy modified nucleotides, 2'-substituted alkoxy modified nucleotides, 2'-alkyl modified nucleotides, 2'-substituted alkyl modified nucleotides, 2'-amino modified nucleotides, 2'-substituted amino modified nucleotides and 2'-deoxy nucleotides.

In some embodiments, the 2'-alkoxy modified nucleotide is a 2'-methoxy (2'-OMe) modified nucleotide, represented by Formula (8). In some embodiments, the 2'-substituted alkoxy modified nucleotide is for example a 2'-methoxyethyl (2'-MOE) modified nucleotide, represented by Formula (9). In some embodiments, the 2'-amino (2'-$NH_2$) modified nucleotide is represented by Formula (10). In some embodiments, the 2'-deoxy nucleotide (DNA) is represented by Formula (11):

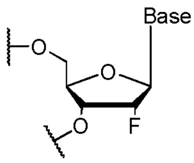 , 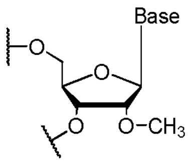 , 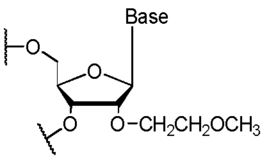 , 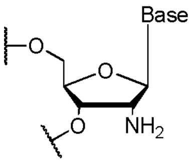 , 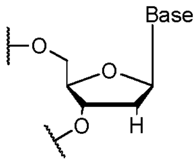 .

Formula (7) Formula (8) Formula (9) Formula (10) Formula (11)

A nucleotide analogue refers to a group that can replace a nucleotide in the nucleic acid, while differs from an adenine ribonucleotide, a guanine ribonucleotide, a cytosine ribonucleotide, a uracil ribonucleotide or thymine deoxyribonucleotide in terms of structure. In some embodiments, the nucleotide analogue may be an isonucleotide, a bridged nucleic acid or an acyclic nucleotide.

Bridged nucleic acid (BNA) refers to a constrained or inaccessible nucleotide. BNA can contain a 5-, 6-membered or a 7-membered ring bridged structure with a "fixed" C3'-endo sugar puckering. The bridge is typically incorporated at the 2'- and 4'-position of the ribose to afford a 2',4'-BNA nucleotide. In some embodiments, BNA may be LNA, ENA and cET BNA which are represented by Formulae (12), (13) and (14), respectively:

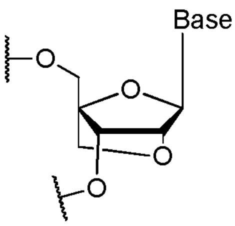 , 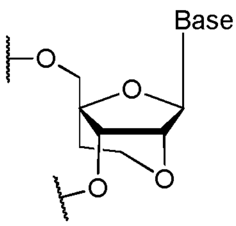 , 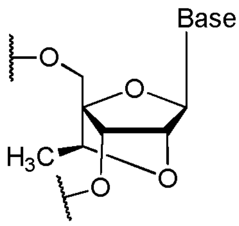 .

Formula (12) Formula (13) Formula (14)

An acyclic nucleotide is a category of nucleotides in which the ribose ring is opened. In some embodiments, the acrylic nucleotide may be an unlocked nucleic acid (UNA) and a glycerol nucleic acid (GNA), which are represented by Formulae (15) and (16), respectively:

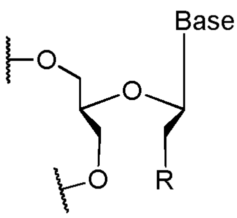 , 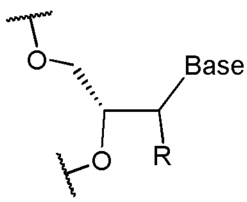 .

Formula (15) Formula (16)

In the above Formulae (15) and (16), R is selected from H, OH or alkoxy (O-alkyl).

An isonucleotide refers to a compound in which the position of the base on the ribose ring in the nucleotide is changed. In some embodiments, the isonucleotide may be a compound formed by transposing the base from position-1' to position-2' or position-3' on the ribose ring, as represented by Formula (17) or (18), respectively.

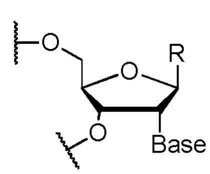 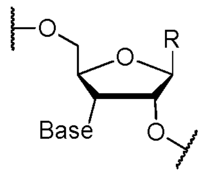

Formula (17) Formula (18)

In the compounds represented by Formulae (17)-(18) above, Base represents a nucleic acid base, such as A, U, G, C or T; R is selected from H, OH, F or a non-fluoro group as described above.

In some embodiments, a nucleotide analogue is one selected from isonucleotide, LNA, ENA, cET, UNA and GNA. In some embodiments, each non-fluoro modified nucleotide is a methoxy modified nucleotide. In the context of the present disclosure, the methoxy modified nucleotide refers to a nucleotide formed by substituting the 2'-hydroxy of the ribose group with a methoxy group.

In the context of the disclosure, a "fluoro modified nucleotide", a "2'-fluoro modified nucleotide", a "nucleotide in which 2'-hydroxy of a ribose group is replaced with fluoro" and a "nucleotide having 2'-fluororibosyl" have the same meaning, referring to a compound in which 2'-hydroxy of the nucleotide is replaced with fluoro, having a structure represented by Formula (7). A "methoxy modified nucleotide", a "2'-methoxy modified nucleotide", a "nucleotide in which 2'-hydroxy of a ribose group is replaced with methoxy" and a "nucleotide having 2'-methoxyribosyl" have the same meaning, referring to a compound in which 2'-hydroxy of the ribose group in the nucleotide is replaced with methoxy, having a structure represented by Formula (8).

In some embodiments, the siRNA of the present disclosure is an siRNA with the following modifications: in the direction from 5' terminal to 3' terminal, the nucleotides at positions 7, 8 and 9 or at positions 5, 7, 8 and 9 of the nucleotide sequence I in the sense strand are fluoro modified nucleotides, and the nucleotides at the other positions in the sense strand are methoxy modified nucleotides; the nucleotides at positions 2, 6, 14 and 16 or at positions 2, 6, 8, 9, 14 and 16 of the nucleotide sequence II in the antisense strand are fluoro modified nucleotides, and the nucleotides at the other positions in the antisense strand are methoxy modified nucleotides.

In some embodiments, the siRNA of the present disclosure is an siRNA with the following modifications: in the direction from 5' terminal to 3' terminal, the nucleotides at positions 5, 7, 8 and 9 of the nucleotide sequence I in the sense strand of the siRNA are fluoro modified nucleotides, and the nucleotides at the other positions of the sense strand of the siRNA are methoxy modified nucleotides; and in the direction from 5' terminal to 3' terminal, the nucleotides at positions 2, 6, 8, 9, 14 and 16 of the nucleotide sequence II in the antisense strand of the siRNA are fluoro modified nucleotides, and the nucleotides at the other positions in the antisense strand of the siRNA are methoxy modified nucleotides;

or, in the direction from 5' terminal to 3' terminal, the nucleotides at positions 5, 7, 8 and 9 of the nucleotide sequence I in the sense strand of the siRNA are fluoro modified nucleotides, and the nucleotides at the other positions in the sense strand of the siRNA are methoxy modified nucleotides; and in the direction from 5' terminal to 3' terminal, the nucleotides at positions 2, 6, 14 and 16 of the nucleotide sequence II in the antisense strand of the siRNA are fluoro modified nucleotides, and the nucleotides at the other positions in the antisense strand of the siRNA are methoxy modified nucleotides;

or, in the direction from 5' terminal to 3' terminal, the nucleotides at positions 7, 8 and 9 of the nucleotide sequence I in the sense strand of the siRNA are fluoro modified nucleotides, and the nucleotides at the other positions in the sense strand of the siRNA are methoxy modified nucleotides; and in the direction from 5' terminal to 3' terminal, the nucleotides at positions 2, 6, 14 and 16 of the nucleotide sequence II in the antisense strand of the siRNA are fluoro modified nucleotides, and the nucleotides at the other positions in the antisense strand of the siRNA are methoxy modified nucleotides.

In some embodiments, the siRNA provided by the present disclosure is any one of siPNa1-M1, siPNa2-M1, siPNa1-M2, siPNa2-M2, siPNa1-M3, siPNa2-M3, siPNb1-M1, siPNb2-M1, siPNb1-M2, siPNb2-M2, siPNb1-M3, siPNb2-M3, siPNc1-M1, siPNc2-M1, siPNc1-M2, siPNc2-M2, siPNc1-M3, siPNc2-M3, siPNd1-M1, siPNd2-M1, siPNd1-M2, siPNd2-M2, siPNd1-M3, siPNd2-M3 listed in Tables 1a-1d.

The siRNA with the above modifications not only has a low cost, but also allow the ribonuclease in the blood to be less liable to cleaving the nucleic acid, thus increasing the stability of the nucleic acid and making the nucleic acid

more resistant to the hydrolysis using nuclease. Meanwhile, the modified siRNA has higher target mRNA inhibitory activity.

In some embodiments, at least a portion of the phosphate groups in phosphate-ribose backbone of at least one single strand in the sense strand and the antisense strand of the siRNA provided by the present disclosure is a phosphate group with modified group(s). In some embodiments, the phosphate group with modified group(s) is a phosphorothioate group formed by substituting at least one oxygen atom in a phosphodiester bond in the phosphate group with a sulfur atom. In some embodiments, the phosphate group with modified group(s) is a phosphorothioate group having a structure represented by Formula (1):

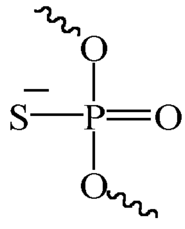
Formula (1).

This modification can stabilize the double-strand structure of the siRNA and maintains high specificity and high affinity for base pairing.

In some embodiments, in the siRNA provided by the present disclosure, a phosphorothioate linkage is located in at least one position selected from the group consisting of the following positions: the position between the first and the second nucleotides at either terminal of the sense or antisense strand, the position between the second and the third nucleotides at either terminal of the sense strand or antisense strand, or any combination thereof. In some embodiments, a phosphorothioate linkage is located in all the above positions except for 5' terminal of the sense strand. In some embodiments, a phosphorothioate linkage is located in all the above positions except for 3' terminal of the sense strand. In some embodiments, a phosphorothioate linkage is located in at least one of the group consisting of the following positions:

the position between the first and second nucleotides at 5' terminal of the sense strand;
the position between the second and third nucleotides at 5' terminal of the sense strand;
the position between the first and second nucleotides at 3' terminal of the sense strand;
the position between the second and third nucleotides at 3' terminal of the sense strand;
the position between the first and second nucleotides at 5' terminal of the antisense strand;
the position between the second and third nucleotides at 5' terminal of the antisense strand;
the position between the first and second nucleotides at 3' terminal of the antisense strand; and
the position between the second and third nucleotides at 3' terminal of the antisense strand.

In some embodiments, the siRNA provided by the present disclosure is any one of siPNa1-M1S, siPNa2-M1S, siPNa1-M2S, siPNa2-M2S, siPNa1-M3S, siPNa2-M3S, siPNb1-M1S, siPNb2-M1S, siPNb1-M2S, siPNb2-M2S, siPNb1-M3S, siPNb2-M3S, siPNc1-M1S, siPNc2-M1S, siPNc1-M2S, siPNc2-M2S, siPNc1-M3S, siPNc2-M3S, siPNd1-M1S, siPNd2-M1S, siPNd1-M2S, siPNd2-M2S, siPNd1-M3S, siPNd2-M3S listed in Tables 1a-1d.

In some embodiments, the nucleotide at 5' terminal of the antisense strand of the siRNA is a 5'-phosphate nucleotide or a 5'-phosphate analogue modified nucleotide.

The commonly used 5'-phosphate nucleotides or 5'-phosphate analogue modified nucleotides are well known to those skilled in the art, for example, 5'-phosphate nucleotide may have the following structure:

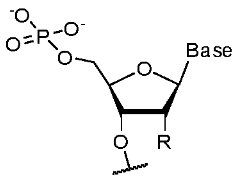
Formula (2);

as another example, the following four 5'-phosphate analogue modified nucleotides are disclosed in Anastasia Khvorova and Jonathan K. Watts, The chemical evolution of oligonucleotide therapies of clinical utility. Nature Biotechnology, 2017, 35(3): 238-48:

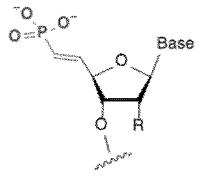
Formula (3)

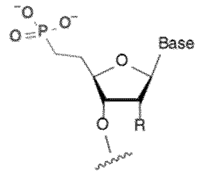
Formula (4)

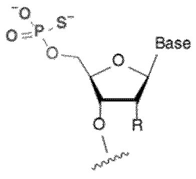
Formula (5)

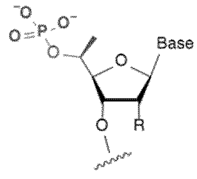
Formula (6)

wherein, R is selected from H, OH, methoxy, and F; Base represents a nucleic acid base selected from A, U, C, G, or T.

In some embodiments, the 5'-phosphate nucleotide is a nucleotide with 5'-phosphate modification represented by Formula (2), the 5'-phosphate analogue modified nucleotide is a nucleotide with 5'-(E)-vinylphosphonate (E-VP) modification represented by Formula (3), or phosphorothioate modified nucleotide represented by Formula (5).

In some embodiments, the siRNA provided by the present disclosure is any one of siPNal-MlP1, siPNa2-M1P1, siPNa1-M2P1, siPNa2-M2P1, siPNa1-M3P1, siPNa2-M3P1, siPNa1-M1SP1, siPNa2-M1SP1, siPNa1-M2SP1, siPNa2-M2SP1, siPNa1-M3SP1, siPNa2-M3SP1, siPNb1-M1P1, siPNb2-M1P1, siPNb1-M2P1, siPNb2-M2P1, siPNb1-M3P1, siPNb2-M3P1, siPNb1-M1SP1, siPNb2-M1SP1, siPNb1-M2SP1, siPNb2-M2SP1, siPNb1-M3SP1, siPNb2-M3SP1, siPNc1-M1P1, siPNc2-M1P1, siPNc1-M2P1, siPNc2-M2P1, siPNcl-M3P1, siPNc2-M3P1, siPNc1-M1SP1, siPNc2-M1SP1, siPNc1-M2SP1, siPNc2-M2SP1, siPNc1-M3SP1, siPNc2-M3SP1, siPNd1-M1P1, siPNd2-M1P1, siPNd1-M2P1, siPNd2-M2P1, siPNd1-M3P1, siPNd2-M3P1, siPNd1-M1SP1, siPNd2-M1SP1, siPNd1-M2SP1, siPNd2-M2SP1, siPNd1-M3SP1, siPNd2-M3SP1 listed in Tables 1a-1d.

The inventors of the present disclosure have unexpectedly discovered that the above siRNA provided by the present disclosure not only has significantly enhanced stability in plasma and lysosomal, but also shows high target mRNA inhibitory activity.

The siRNAs provided by the present disclosure may be obtained by conventional processes for preparing siRNA in the art, such as solid phase synthesis process and liquid phase synthesis process. Among them, commercial customized services have already been available for solid phase synthesis. A modified nucleotide group can be introduced into the siRNA of the present disclosure by using the nucleoside monomer with corresponding modification. The process for preparing the nucleotide monomer with corresponding modification and the process for introducing the modified nucleotide group into the siRNA are also well known to those skilled in the art.

Pharmaceutical composition

The present disclosure provides a pharmaceutical composition comprising the siRNA as described above as an active ingredient and a pharmaceutically acceptable carrier.

The pharmaceutically acceptable carrier may be a carrier conventionally used in the field of siRNA administration, for example, but not limited to, one or more of magnetic nanoparticles, such as $Fe_3O_4$ or $Fe_2O_3$-based nanoparticles, carbon nanotubes, mesoporous silicon, calcium phosphate nanoparticles, polyethylenimine (PEI), polyamidoamine (PAMAM) dendrimer, poly(L-lysine) (PLL), chitosan, 1,2-dioleoyl-3-trimethylammonium-propane (DOTAP), poly(D&L-lactic/glycolic acid)copolymer (PLGA), poly(2-aminoethyl ethylene phosphate) (PPEEA), and poly(2-dimethylaminoethyl methacrylate) (PDMAEMA), and derivatives thereof.

In the pharmaceutical composition, there are no special requirements for the contents of the siRNA and the pharmaceutically acceptable carrier, which may be the conventional content of each component. In some embodiments, the weight ratio of the siRNA and the pharmaceutically acceptable carrier may be 1: (1-500). In some embodiments, the above weight ratio is 1: (1-50).

In some embodiments, the pharmaceutical composition further contains other pharmaceutically acceptable excipients, which may be one or more of various Formulations or compounds conventionally employed in the art. For example, the other pharmaceutically acceptable excipients may include at least one of a pH buffer, a protective agent and an osmotic pressure regulator.

The pH buffer may be tris(hydroxymethyl) aminomethane hydrochloride buffer solution with a pH of 7.5-8.5, and/or phosphate buffer solution with a pH of 5.5-8.5, for example phosphate buffer solution with a pH of 5.5-8.5.

The protective agent may be at least one of inositol, sorbitol, sucrose, trehalose, mannose, maltose, lactose and glucose. The content of the protective agent may be from 0.01 wt % to 30 wt % on the basis of the total weight of the pharmaceutical composition.

The osmotic pressure regulator may be sodium chloride and/or potassium chloride. The content of the osmotic pressure regulator allows the osmotic pressure of the pharmaceutical composition to be 200-700 mOsm/kg. Depending on the desired osmotic pressure, those skilled in the art can readily determine the content of the osmotic pressure regulator.

In some embodiments, the pharmaceutical composition may be a liquid Formulation, for example, an injection solution; or a lyophilized powder for injection, which is mixed with a liquid excipient to form a liquid Formulation upon administration. The liquid Formulation may be administered by, but is not limited to, subcutaneous, intramuscular or intravenous injection, and also may be administrated to, but is not limited to, lung by spray, or to other organ tissues (such as liver) via lung by spray. In some embodiments, the pharmaceutical composition is administered by intravenous injection.

In some embodiments, the pharmaceutical composition may be in the form of a liposome formulation. In some embodiments, the pharmaceutically acceptable carrier used in the liposome formulation comprises an amine-containing transfection compound (hereinafter also referred to as an organic amine), a helper lipid and/or a PEGylated lipid. Among others, the organic amine, the helper lipid and the PEGylated lipid may be respectively selected from one or more of the amine-containing transfection compounds or pharmaceutically acceptable salts or derivatives thereof, the helper lipids and the PEGylated lipids described in CN103380113A, which is incorporated herein by reference in its entirety.

In some embodiments, the organic amine may be a compound represented by Formula (201) or a pharmaceutically acceptable salt thereof described in CN103380113A:

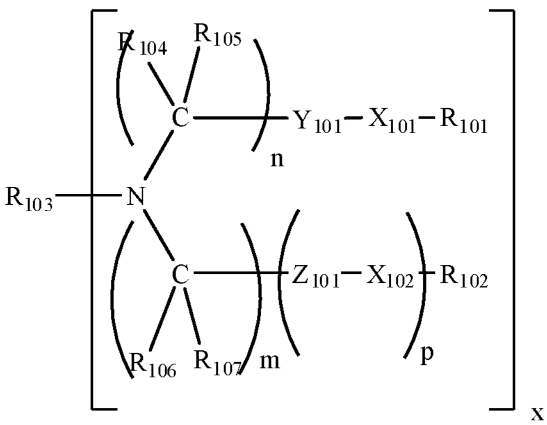

Formula (201),

wherein:

each of $X_{101}$ or $X_{102}$ is independently O, S, N-A or C-A, wherein A is hydrogen or a $C_1$-$C_{20}$ hydrocarbon chain;
each of $Y_{101}$ or $Z_{101}$ is independently C=O, C=S, S=O, CH-OH or $SO_2$;
each of $R_{101}$, $R_{102}$, $R_{103}$, $R_{104}$, $R_{105}$, $R_{106}$ or $R_{107}$ is independently hydrogen; a cyclic or an acyclic, substituted or unsubstituted, branched or linear aliphatic group; a cyclic or an acyclic, substituted or unsubstituted, branched or linear heteroaliphatic group; a substituted or unsubstituted, branched or linear acyl group; a substituted or unsubstituted, branched or linear aryl group; a substituted or unsubstituted, branched or linear heteroaryl group;
x is an integer of 1-10;
n is an integer of 1-3, m is an integer of 0-20, p is 0 or 1, wherein if m=p=0, then $R_{102}$ is hydrogen;
and if at least one of n or m is 2, then $R_{103}$ and nitrogen in Formula (201) form a structure as represented by Formula (202) or Formula (203):

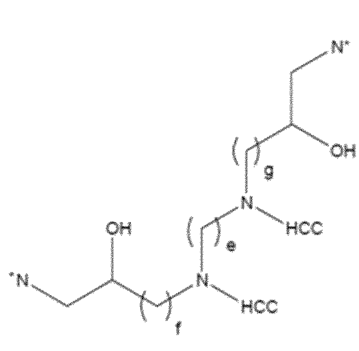

Formula (202),

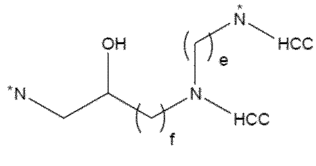

Formula (203);

wherein, each of g, e or f is independently an integer of 1-6, "HCC" represents a hydrocarbon chain, and each *N represents nitrogen atom in Formula (201).

In some embodiments, $R_{103}$ is polyamine. In other embodiments, $R_{103}$ is ketal. In some embodiments, each of $R_{101}$ and $R_{102}$ in Formula (201) is independently any substituted or unsubstituted, branched or linear alkyl or alkenyl, wherein the alkyl or alkenyl has 3 to about 20 carbon atoms, such as 8 to about 18 carbon atoms, and 0 to 4 double bonds, such as 0 to 2 double bonds.

In some embodiments, if each of n and m is independently a value of 1 or 3, $R_{103}$ may be any group of the following Formulae (204) to (213):

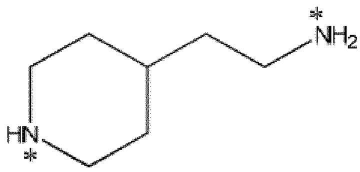

Formula (204),

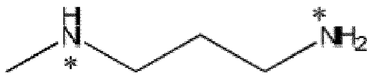

Formula (205),

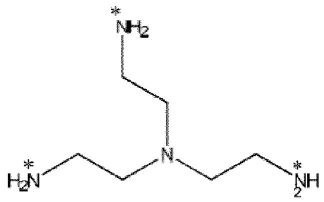

Formula (206),

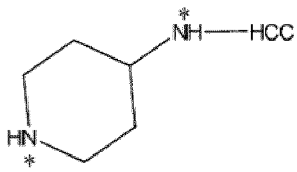

Formula (207),

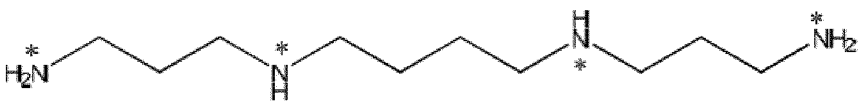

Formula (208),

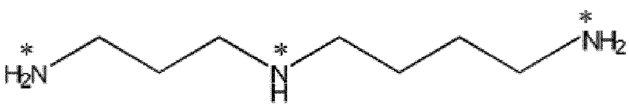

Formula (209),

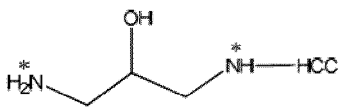

Formula (210),

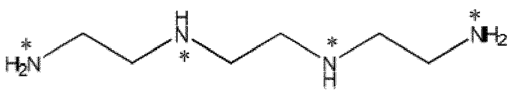

Formula (211),

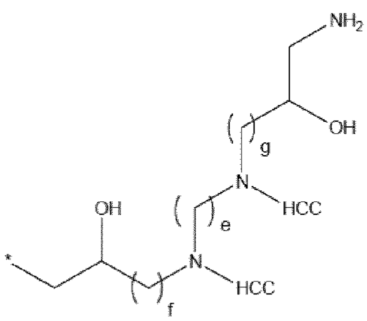

Formula (212) and

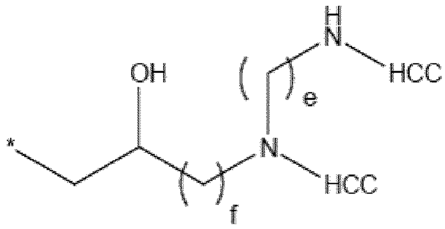

Formula (213);

wherein, in Formulae (204) to (213), each of g, e and f is independently an integer of 1-6, each "HCC" represents a hydrocarbon chain, and each * represents a possible attachment point of $R_{103}$ to the nitrogen atom in Formula (201), wherein each H at any * position may be replaced to achieve the attachment to the nitrogen atom in Formula (201).

The compound represented by Formula (201) may be prepared according to the description of CN103380113A.

In some embodiments, the organic amine is the organic amine represented by Formula (214) and/or the organic amine represented by Formula (215):

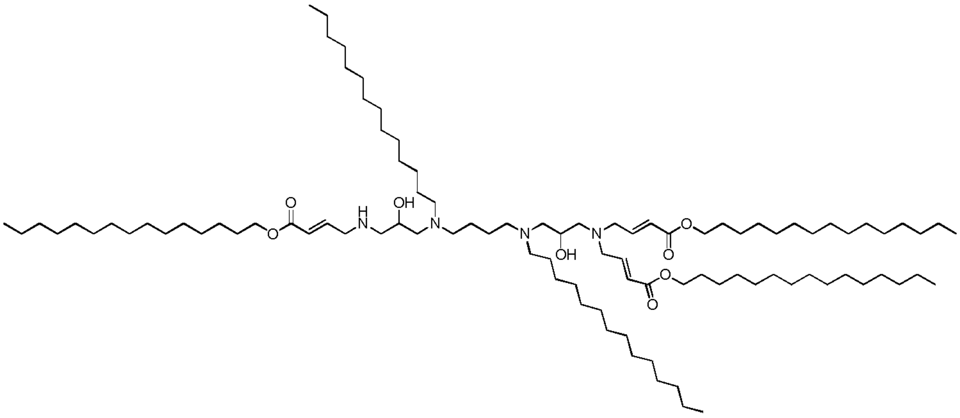

Formula (214),

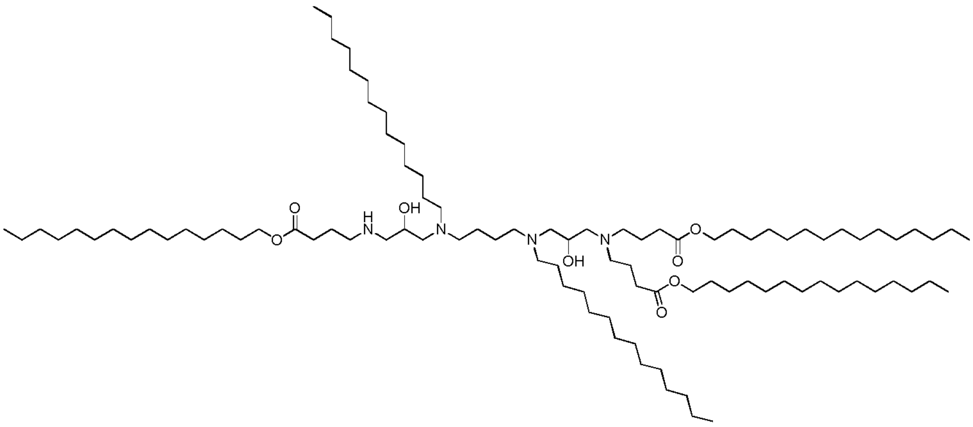

Formula (215);

the helper lipid is cholesterol, cholesterol analogues and/or cholesterol derivatives;

the PEGylated lipid is 1,2-dipalmitoylamine-sn-glycero-3-phosphatidylethanolamine-N-[methoxy(polyethylene glycol)]-2000.

In some embodiments, the molar ratio of the organic amine, the helper lipid and the PEGylated lipid in the pharmaceutical compositions is (19.7-80) : (19.7-80) : (0.3-50), for example (50-70) : (20-40) : (3-20).

In some embodiments, the particles of the pharmaceutical composition formed by the siRNA of the present disclosure and the above amine-containing transfection agent have an average diameter from about 30 nm to about 200 nm, typically from about 40 nm to about 135 nm, and more typically, the average diameter of the liposome particles is from about 50 nm to about 120 nm, from about 50 nm to about 100 nm, from about 60 nm to about 90 nm, or from about 70 nm to about 90 nm, for example, the average diameter of the liposome particles is about 30, 40, 50, 60, 70, 75, 80, 85, 90, 100, 110, 120, 130, 140, 150 or 160 nm.

In some embodiments, in the pharmaceutical composition formed by the siRNA of the present disclosure and

the above amine-containing transfection agent, the weight ratio (weight/weight ratio) of the siRNA to total lipids, e.g., the organic amines, the helper lipids and/or the PEGylated lipids, ranges from about 1:1 to about 1:50, from about 1:1 to about 1:30, from about 1:3 to about 1:20, from about 1:4 to about 1:18, from about 1:5 to about 1:17, from about 1:5 to about 1:15, from about 1:5 to about 1:12, from about 1:6 to about 1:12, or from about 1:6 to about 1:10, for example, the weight ratio of the siRNA of the present disclosure to total lipids is about 1:5, 1:6, 1:7, 1:8, 1:9, 1:10, 1:11, 1:12, 1:13, 1:14, 1:15, 1:16, 1:17 or 1:18.

In some embodiments, the pharmaceutical compositions may be marketed with each component being separate, and used in the form of liquid Formulation. In some embodiments, the pharmaceutical composition formed by the siRNA provided by the present disclosure and the above pharmaceutically acceptable carrier may be prepared according to various known processes, except replacing the existing siRNA with the siRNA of the present disclosure. In some embodiments, it may be prepared according to the following process:

The organic amines, the helper lipids and the PEGylated lipids in the molar ratio as described above are suspended in alchol and mixed uniformly to afford a lipid solution; the alcohol is used in such an amount that the afforded lipid solution has a total mass concentration of 2 to 25 mg/mL, for example, 8 to 18 mg/mL. The alcohol is selected from a pharmaceutically acceptable alcohol, for example an alcohol that is in liquid form at about room temperature, such as one or more of ethanol, propylene glycol, benzyl alcohol, glycerol, polyethylene glycol 200, polyethylene glycol 300, and polyethylene glycol 400, e.g. ethanol.

The siRNA provided by the present disclosure is dissolved in a buffer salt solution to produce an siRNA aqueous solution. The buffer salt solution is in a concentration of 0.05 to 0.5 M, for example 0.1 to 0.2 M. The pH of the buffer salt solution is adjusted to 4.0 to 5.5, for example 5.0 to 5.2. The buffer salt solution is used in an amount to make the concentration of siRNA no more than 0.6 mg/mL, for example 0.2 to 0.4 mg/mL. The buffer salt may be one or more selected from the group consisting of soluble acetate and soluble citrate, for example sodium acetate and/or potassium acetate.

The lipid solution and the siRNA aqueous solution are mixed. The product obtained after mixing is incubated at a temperature of 40 to 60°C for at least 2 minutes, for example 5 to 30 minutes, to produce a incubated liposome formulation. The volume ratio of the lipid solution to the aqueous solution of the siRNA is 1: (2-5).

The incubated liposome formulation is concetrated or diluted, and then subjected to impurity removal and sterilization to obtain the pharmaceutical composition provided by the present disclosure, of which the physical and chemical parameters are as follows: a pH of 6.5 to 8, an encapsulation percentage of not lower than 80%, a particle size of 40 to 200 nm, a polydispersity index of no greater than 0.30, and an osmotic pressure of 250 to 400 mOsm/kg; for example, the physical and chemical parameters may be as follows: a pH of 7.2 to 7.6, an encapsulation percentage of not lower than 90%, a particle size of 60 to 100 nm, a polydispersity index of no greater than 0.20, and an osmotic pressure of 300 to 400 mOsm/kg.

Therein, the concentration or dilution may be performed before, after or simultaneously with removing impurities. Methods for removing impurities may be various exisiting methods, for example, ultrafiltration under 100 K Da using a hollow fiber column, a phosphate buffer (PBS) at pH 7.4 as ultrafiltration exchange solution, and a cross flow system. Methods for sterilization may be various exisiting methods, such as filtration sterilization on a 0.22 μm filter.

siRNA conjugate

The present disclosure provides an siRNA conjugate, which comprises the above siRNA and a conjugating group linked to the siRNA by conjugation.

Generally speaking, the conjugating group comprises at least one pharmaceutically acceptable targeting group and an optional linker. Moreover, the siRNA, the linker and the targeting group are sequentialy linked. In some embodiments, the nubmer of the targeting group is 1 to 6. In some embodiments, the number of target groups is 2 to 4. The siRNA molecule may be non-covalently or covalently conjugated to the conjugating group, for example the siRNA molecule may be covalently conjugated to the conjugating group. The conjugating site between the siRNA and the conjugating group can be at the 3' terminal or 5' terminal of the sense strand of the siRNA, or at the 5' terminal of the antisense strand, and can be within the internal sequence of the siRNA. In some embodiments, the conjugating site between the siRNA and the conjugating group is at the 3' terminal of the sense strand of the siRNA.

In some embodiments, the conjugating group may be linked to a phosphate group, 2'-hydroxy or a base in the nucleotide. In some embodiments, the conjugating group may also be linkd to 3'-hydroxyl when the nucleotides are linkd via a 2'-5' phosphodiester bond. Where the cojugating group is linked to a terminal of the siRNA strand, the conjugating group is usually linked to a phosphate group of the nucleotide. Where the conjugating group is linked to an internal sequence of the siRNA, the conjugating group is usually linked to a ribose ring or a base. For various linking manners, reference shall be made to the document: Muthiah Manoharan et.al. siRNA conjugates carrying sequentially assembled trivalent N-acetylgalactosamine linked through nucleosides elicit robust gene silencing in vivo in hepatocytes. ACS Chemical biology, 2015, 10 (5): 1181-7.

In some embodiments, the siRNA and the conjugating group may be linked through acid-labile or reducible chemical bonds, and these chemical bonds may be degrated under the acidic environment of cell endosomes, thereby rendering the siRNA to be in free state. For non-degradable conjugating modes, the conjugating group may be linked to the sense strand of the siRNA, thereby minimizing the effect of conjugation on the siRNA activity.

In some embodiments, the pharmaceutically acceptable targeting group may be a ligand conventionally used in the field of siRNA administrion, for example, the various ligands described in WO2009082607A2, which is incorporated herein by reference in its entirety.

In some embodiments, the pharmaceutically acceptable targeting group may be selected from one or more of the ligands formed by the following targeting molecules or derivatives thereof: lipophilic molecules, such as cholesterol, bile acids, vitamins (such as vitamin E), lipid molecules of different chain lengths; polymers, such as polyethylene glycol; polypeptides, such as cell-penetrating peptide; aptamers; antibodies; quantum dots; saccharides, such as lactose, polylactose, mannose, galactose, N-acetylgalactosamine (GalNAc); folate; or receptor ligands expressed in hepatic parenchymal cells, such as asialoglycoprotein, asialo-sugar residue, lipoproteins (such as high density lipoprotein, low density lipoprotein and the like), glucagon, neurotransmitters (such as adrenaline), growth factors, transferrin and the like.

In some embodiments, each of the ligands is independently selected from a ligand capable of binding to a cell surface receptor. In some embodiments, at least one ligand is a lingand capable of binding to a hepatocyte surface receptor. In some embodiments, at least one ligand is a ligand capable of binding to a mammalian hepatocyte surface receptor. In some embodiments, at least one ligand is a ligand capable of binding to a human hepatocyte surface receptor. In some embodiments, at least one ligand is a ligand capable of binding to a hepatic surface asialoglycoprotein receptor (ASGPR). The kinds of these ligands are well-known to those skilled in the art, and they generally serve the function of binding to specific receptor on the surface of the target cells, thereby mediating the delivery of the siRNA linked to the ligands into target cells.

In some embodiments, the pharmaceutically acceptable targeting group may be any ligand that binds to the ASGPR on the surface of mammalian hepatocytes. In some embodiments, each ligand is independently an asialoglycoprotein, such as asialoorosomucoid (ASOR) or asialofetuin (ASF). In some embodiments, the ligand is a saccharide or derivatives thereof.

In some embodiments, at least one ligand is a saccharide. In some embodiments, each ligand is a saccharide. In some embodiments, at least one ligand is a monosaccharide, polysaccharide, modified saccharide, modified polysaccharide or saccharide derivatives. In some embodiments, at least one ligand may be a monosaccharide, disaccharide or trisaccharide. In some embodiments, at least one ligand is a modified saccharide. In some embodiments, each ligand is a modified saccharide. In some embodiments, each ligand is independently selected from polysaccharides, modified polysaccharides, monosaccharides, modified monosaccharides, polysaccharide derivatives or monosaccharide derivatives. In some embodiments, each or at least one ligand is selected from the group consisting of glucose and its derivatives, mannose and its derivatives, galactose and its derivatives, xylose and its derivatives, ribose and its derivatives, fucose and its derivatives, lactose and its derivatives, maltose and its derivatives, arabinose and its derivatives, fructose and its derivatives, and sialic acid.

In some embodiments, each ligand may be independently selected from the group consisting of D-mannopyranose, L-mannopyranose, D-arabinose, D-xylofuranose, L-xylofuranose, D-glucose, L-glucose, D-galactose, L-galactose, α-D-mannofuranose, β-D-mannofuranose, α-D-mannopyranose, β-D-mannopyranose, α-D-glucopyranose, β-D-glucopyranose, α-D-glucofuranose, β-D-glucoiuranose, α-D-fructofuranose, α-D-fructopyranose, α-D-galactopyranose, β-D-galactopyranose, α-D-galactofuranose, β-D-galactofuranose, glucosamine, sialic acid, galactosamine, N-acetylgalactosamine, N-trifluoroacetylgalactosamine, N-propionylgalactosamine, N-n-butyrylgalactosamine, N-isobutyrylgalactosamine, 2-amino-3-O-[(R)-1-carboxyethyl]-2-deoxy-β-D-glucopyranose, 2-deoxy-2-methylamino-L-glucopyranose, 4,6-dideoxy-4-formamido-2,3-di-O-methyl-D-mannopyranose, 2-deoxy-2-sulfoamino-D-glucopyranose, N-glycolyl-α-neuraminic acid, 5-thio-β-D-glucopyranose, methyl 2,3,4-tris-O-acetyl-1-thio-6-O-trityl-α-D-glucopyranoside, 4-thio-β-D-galactopyranose, ethyl 3,4,6,7-tetra-O-acetyl-2-deoxy-1,5-dithio-α-D-glucoheptopyranoside, 2,5-anhydro-D-allononitrile, ribose, D-ribose, D-4-thioribose, L-ribose, or L-4-thioribose. Other selections of the ligand may be found, for example, in the disclosure of CN105378082A, which is incorporated herein by reference in its entirety.

In some embodiments, the pharmaceutically acceptable targeting group in the siRNA conjugate may be galactose or N-acetylgalactosamine, wherein the galactose or N-acetylgalactosamine molecules may be of monovalence, divalence, trivalence or tetravalence. It should be understood that the monovalence, divalence, trivalence or tetravalence described herein respectively mean that the molar ratio of the siRNA molecule to the galactose or N-acetylgalactosamine molecule in the siRNA conjugate is 1:1, 1:2, 1:3 or 1:4, wherein the siRNA conjugate is formed from the siRNA molecule and the conjugating group containing galactose or N-acetylgalactosamine molecule as the targeting group. In some embodiments, the pharmaceutically acceptable targeting group is N-acetylgalactosamine. In some embodiments, when the siRNA of the present disclosure is conjugated to the conjugating group containing N-acetylgalactosamine, the N-acetylgalactosamine molecule is of trivalence or tetravalence. In some embodiments, when the siRNA of the present disclosure is conjugated to the conjugating group containing N-acetylgalactosamine, the N-acetylgalactosamine molecule

is of trivalence.

The targeting group may be linked to the siRNA molecule via an appropriate linker. Those skilled in the art could select appropriate linkers according to the specific kind of the targeting group. The kinds of these linkers and targeting groups and the linking modes with siRNA may be found in the disclosure of WO2015006740A2, which is incorporated herein by reference in its entirety.

In some embodiments, when the targeting group is N-acetylgalactosamine, the appropriate linker may have a structure represented by Formula (301):

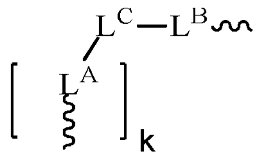

Formula (301)

wherein,

k is an integer of 1-3;

$L^A$ is an amide bond-comprising chain moiety having a structure represented by Formula (302), and each $L^A$ is respectively linked to a targeting group and the $L^C$ moiety through an ether bond at its two terminals:

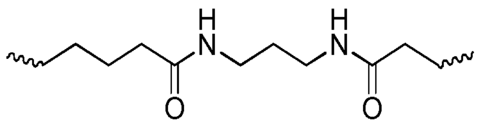

Formula (302);

$L^B$ is an N-acylpyrrolidine-comprising chain moiety having a structure represented by Formula (303), wherein the chain moiety has a carbonyl at its one terminal and is linked to the $L^C$ moiety through an amide bond, and has an oxy group at the other terminal and is linked to the siRNA via a phosphoester bond:

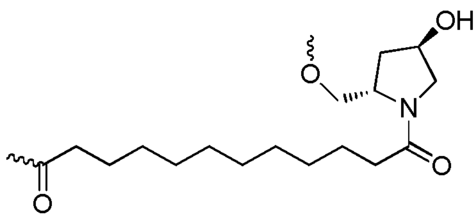

Formula (303);

$L^C$ is a bivalent to tetravalent linking group based on hydroxymethyl aminomethane, dihydroxymethyl aminomethane or trihydroxymethyl aminomethane, and is linked to each $L^A$ moiety through an ether bond via an oxygen atom, and is linked to the $L^B$ moiety through amide bond via a nitrogen atom.

In some embodiments, when n=3 and $L^C$ is a tetravalent linking group based on trihydroxymethyl aminomethane, the siRNA conjugate formed by linking N-acetylgalactosamine molecule(s) with an siRNA molecule via -$(L^A)_3$-trihydroxymethyl aminomethane-$L^B$- as a linker has a structure as represented by Formula (304):

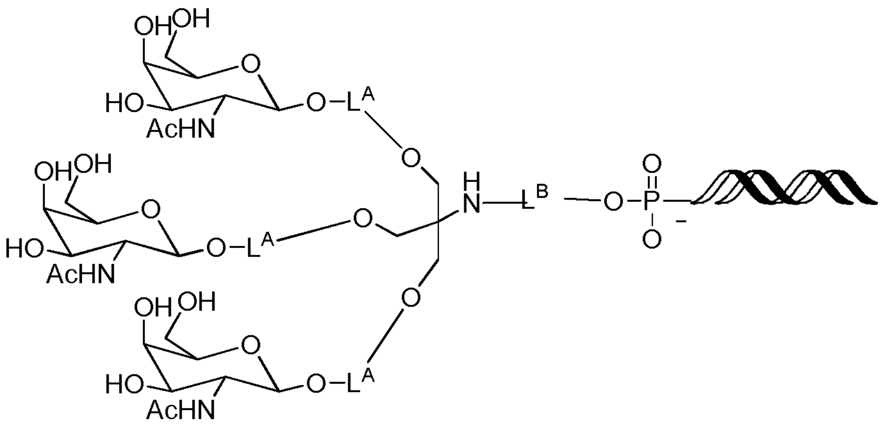

Formula (304),

wherein the double helix structure represents a siRNA.

Likewise, the conjugating site between the siRNA and the conjugating group can be at the 3' terminal or 5'-terminal of the sense strand of the siRNA, or at the 5' terminal of the antisense strand, or within the internal sequence of the siRNA.

In some embodiments, the 3' terminal of the sense strand of the siRNA of the present disclosure is covalently conjugated to three N-acetylgalactosamine (GalNAc) molecules via a linker $-(L^A)_3$-trihydroxymethyl aminomethane-$L^B$- to afford an siRNA conjugate in which the molar ratio of the siRNA molecule to the GalNAc molecule is 1:3 (hereinafter this siRNA conjugate may be referred to as $(GalNAc)_3$-siRNA), and this siRNA conjugate has a structure represented by Formula (305):

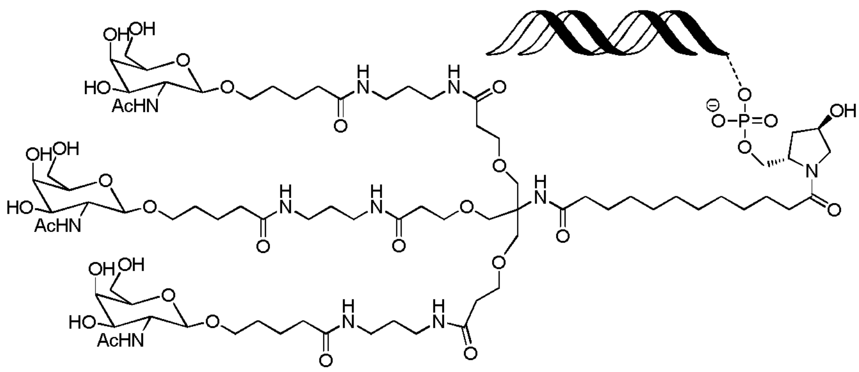

Formula (305),

wherein the double helix structure represents the siRNA, and the linker is linked to the 3' terminal of the sense strand of the siRNA.

In some embodiments, when the targeting group is N-acetylgalactosamine, the appropriate linker has a structure represented by Formula (306):

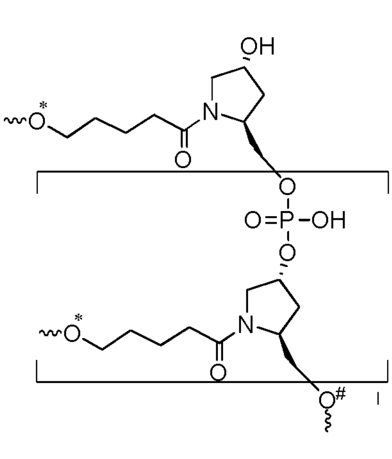

Formula (306),

wherein,

1 is an integer of 0-3;
* represents the site on the linker linked to the targeting group via an ether bond; and
# represents the site on the linker linked to the siRNA via a phosphoester bond.

In some embodiments, when 1=2, the siRNA conjugate has a structure represented by Formula (307):

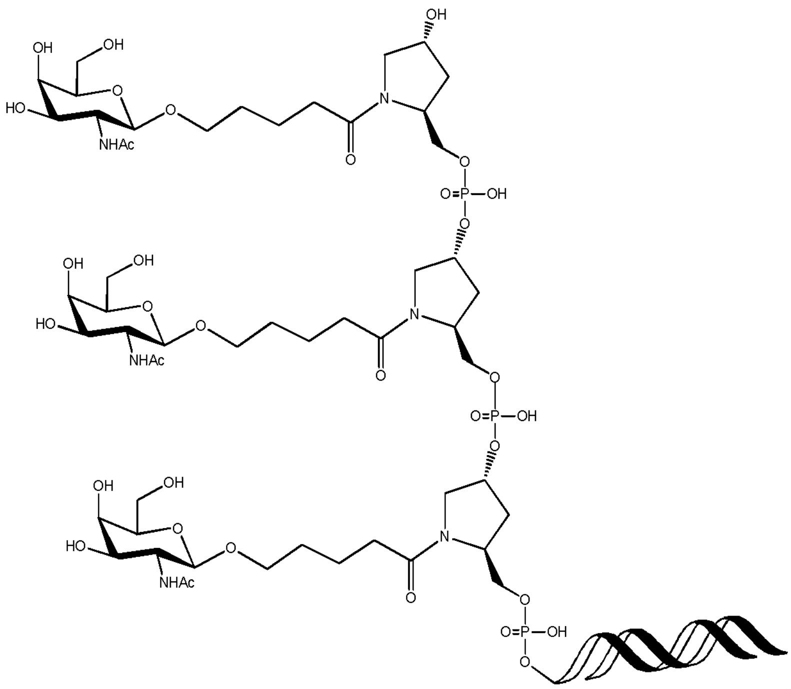

Formula (307),

wherein the double helix structure represents the siRNA, and the linker is linked to the 3' terminal of the sense strand of the siRNA.

The above conjugate may be synthesized by the method that has been described in detail in the prior art. For example, WO2015006740A2 describes in detail the preparation methods of various conjugates. The siRNA conjugate of the present disclosure may be obtained by the methods well-known to those skilled in the art. For example, WO2014025805A1 describes the preparation method of the structure represented by Formula (305). Rajeev et al. in ChemBioChem 2015, 16, 903-908 describes the preparation method of the structure represented by Formula (307).

In some embodiments, the siRNA conjugate has a structure represented by Formula (308):

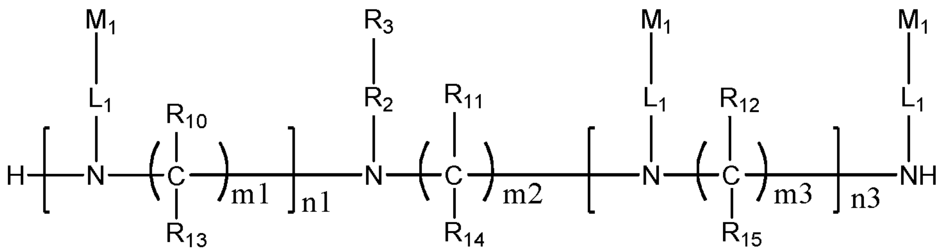

Formula (308),

wherein,

n1 is an integer of 1-3, and n3 is an integer of 0-4;

each of m1, m2, or m3 is independently an integer of 2-10;

each of $R_{10}$, $R_{11}$, $R_{12}$, $R_{13}$, $R_{14}$ or $R_{15}$ is independently H, or selected from the group consisting of $C_1$-$C_{10}$ alkyl,

$C_1$-$C_{10}$ haloalkyl, and $C_1$-$C_{10}$ alkoxy;

$R_3$ is a group having a structure represented by Formula (A59):

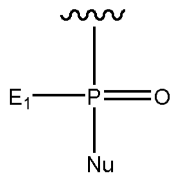
(A59)

wherein $E_1$ is OH, SH or $BH_2$, and Nu is the siRNA of the present disclosure;

$R_2$ is a linear alkylene of 1 to 20 carbon atoms in length, wherein one or more carbon atoms are optionally replaced with one or more groups selected from the group consisting of: C(O), NH, O, S, CH=N, $S(O)_2$, $C_2$-$C_{10}$ alkeylene, $C_2$-$C_{10}$ alkynylene, $C_6$-$C_{10}$ arylene, $C_3$-$C_{18}$ heterocyclylene, and $C_5$-$C_{10}$ heteroarylene, and wherein $R_2$ is optionally substituted by any one or more groups selected from the group consisting of: $C_1$-$C_{10}$ alkyl, $C_6$-$C_{10}$ aryl, $C_5$-$C_{10}$ heteroaryl, $C_1$-$C_{10}$ haloalkyl, -O$C_1$-$C_{10}$ alkyl, -O$C_1$-$C_{10}$ alkylphenyl, -$C_1$-$C_{10}$ alkyl-OH, -O$C_1$-$C_{10}$ haloalkyl, -S$C_1$-$C_{10}$ alkyl, -S$C_1$-$C_{10}$ alkylphenyl, -$C_1$-$C_{10}$ alkyl-SH, -S$C_1$-$C_{10}$ haloalkyl, halo, -OH, -SH, -$NH_2$, -$C_1$-$C_{10}$ alkyl-$NH_2$, -N($C_1$-$C_{10}$ alkyl)($C_1$-$C_{10}$ alkyl), -NH(Ci-Cio alkyl), -N($C_1$-$C_{10}$ alkyl)($C_1$-$C_{10}$ alkylphenyl), -NH(Ci-Cio alkylphenyl), cyano, nitro, -$CO_2$H, -C(O)O($C_1$-$C_{10}$ alkyl), -CON($C_1$-$C_{10}$ alkyl)($C_1$-$C_{10}$ alkyl), -CONH($C_1$-$C_{10}$ alkyl), -$CONH_2$, -NHC(O)($C_1$-$C_{10}$ alkyl), -NHC(O)(phenyl), -N($C_1$-$C_{10}$ alkyl)C(O)($C_1$-$C_{10}$ alkyl), -N($C_1$-$C_{10}$ alkyl)C(O)(phenyl), -C(O)$C_1$-$C_{10}$ alkyl, -C(O)$C_1$-$C_{10}$ alkylphenyl, -C(O)$C_1$-$C_{10}$ haloalkyl, -OC(O)$C_1$-$C_{10}$ alkyl, -$SO_2$($C_1$-$C_{10}$ alkyl), -$SO_2$(phenyl), -$SO_2$($C_1$-$C_{10}$ haloalkyl), - $SO_2NH_2$, -$SO_2$NH($C_1$-$C_{10}$ alkyl), -$SO_2$NH(phenyl), -NH$SO_2$($C_1$-$C_{10}$ alkyl), -NH$SO_2$(phenyl), and -NH$SO_2$($C_1$-$C_{10}$ haloalkyl);

each $L_1$ is a linear alkylene of 1 to 70 carbon atoms in length, wherein one or more carbon atoms are optionally replaced with one or more groups selected from the group consisting of: C(O), NH, O, S, CH=N, $S(O)_2$, $C_2$-$C_{10}$ alkeylene, $C_2$-$C_{10}$ alkynylene, $C_6$-$C_{10}$ arylene, $C_3$-$C_{18}$ heterocyclylene, and $C_5$-$C_{10}$ heteroarylene, and wherein $L_1$ is optionally substituted by any one or more groups selected from the group consisting of: $C_1$-$C_{10}$ alkyl, $C_6$-$C_{10}$ aryl, $C_5$-$C_{10}$ heteroaryl, $C_1$-$C_{10}$ haloalkyl, -O$C_1$-$C_{10}$ alkyl, -O$C_1$-$C_{10}$ alkylphenyl, -$C_1$-$C_{10}$ alkyl-OH, -O$C_1$-$C_{10}$ haloalkyl, -S$C_1$-$C_{10}$ alkyl, -S$C_1$-$C_{10}$ alkylphenyl, -$C_1$-$C_{10}$ alkyl-SH, -S$C_1$-$C_{10}$ haloalkyl, halo, -OH, -SH, -$NH_2$, -$C_1$-$C_{10}$ alkyl-$NH_2$, -N($C_1$-$C_{10}$ alkyl)($C_1$-$C_{10}$ alkyl), -NH(Ci-Cio alkyl), -N($C_1$-$C_{10}$ alkyl)($C_1$-$C_{10}$ alkylphenyl), -NH(Ci-Cio alkylphenyl), cyano, nitro, -$CO_2$H, -C(O)O($C_1$-$C_{10}$ alkyl), -CON($C_1$-$C_{10}$ alkyl)($C_1$-$C_{10}$ alkyl), -CONH($C_1$-$C_{10}$ alkyl), -$CONH_2$, -NHC(O)($C_1$-$C_{10}$ alkyl), -NHC(O)(phenyl), -N($C_1$-$C_{10}$ alkyl)C(O)($C_1$-$C_{10}$ alkyl), -N($C_1$-$C_{10}$ alkyl)C(O)(phenyl), -C(O)$C_1$-$C_{10}$ alkyl, -C(O)$C_1$-$C_{10}$ alkylphenyl, -C(O)$C_1$-$C_{10}$ haloalkyl, -OC(O)$C_1$-$C_{10}$ alkyl, -$SO_2$($C_1$-$C_{10}$ alkyl), -$SO_2$(phenyl), -$SO_2$($C_1$-$C_{10}$ haloalkyl), - $SO_2NH_2$, -$SO_2$NH($C_1$-$C_{10}$ alkyl), -$SO_2$NH(phenyl), -NH$SO_2$($C_1$-$C_{10}$ alkyl), -NH$SO_2$(phenyl), and -NH$SO_2$($C_1$-$C_{10}$ haloalkyl).

In some embodiments, $L_1$ may be selected from the group consisting of groups A1-A26 or any connection combinations thereof, wherein the structures and definitions of A1-A26 are as follows:

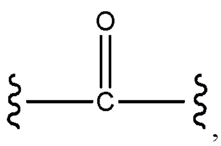,
(A1)

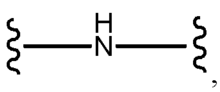,
(A2)

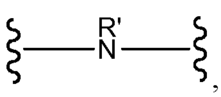,
(A3)

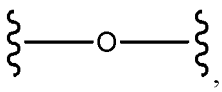,
(A4)

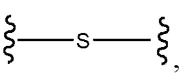,
(A5)

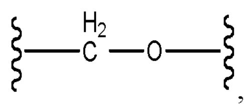,
(A6)

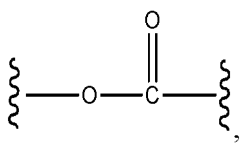,
(A7)

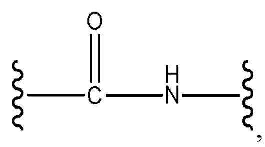,
(A8)

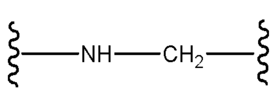,
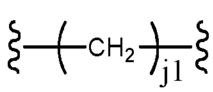,
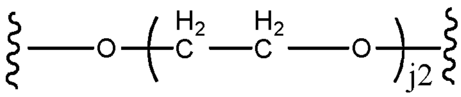,

(A9) (A10) (A11)

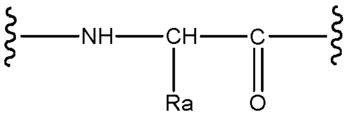,
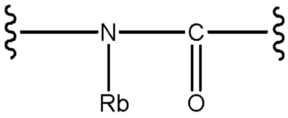,
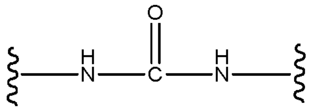,

(A12) (A13) (A14)

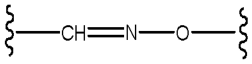,
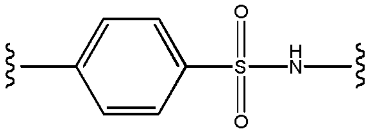,
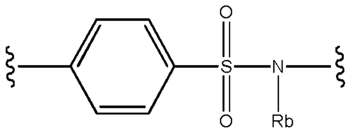,

(A15) (A16) (A17)

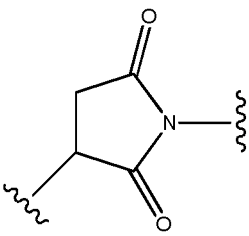,
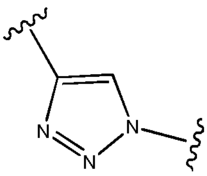,
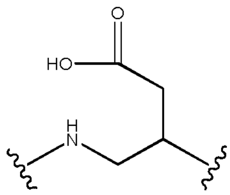,
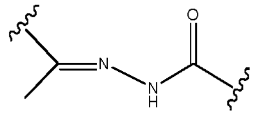,

(A18) (A19) (A20) (A21)

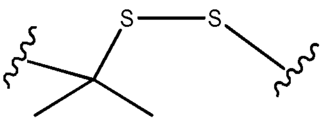,
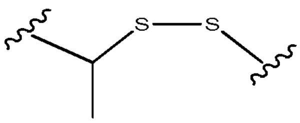,
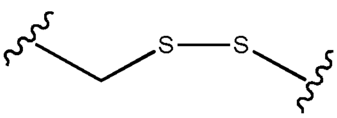,

(A22) (A23) (A24)

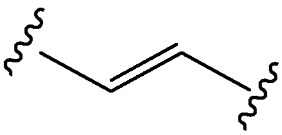, and
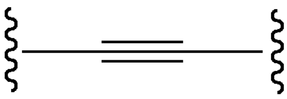;

(A25) (A26)

wherein, j 1 is an integer of 1-20; j2 is an integer of 1-20;

R' is a $C_1$-$C_{10}$ alkyl;

Ra is selected from the group consisting of the groups of Formulae (A27)-(A45) or any connection combinations thereof:

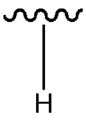 , 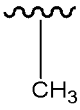 , 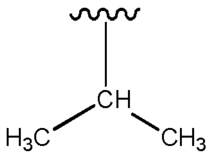 , 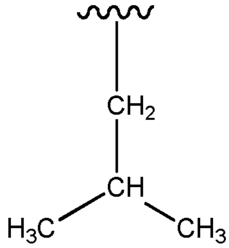 , 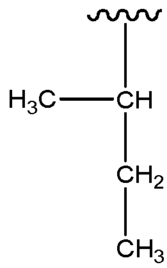 , 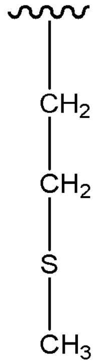 ,

(A27) (A28) (A29) (A30) (A31) (A32)

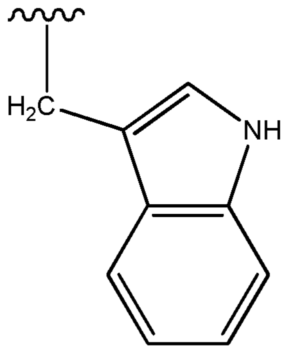 , 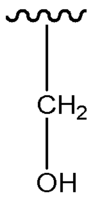 , 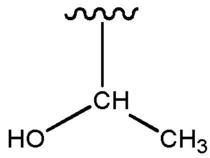 , 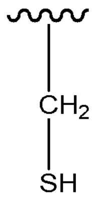 , 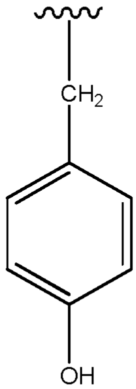 ,

(A33) (A34) (A35) (A36) (A37)

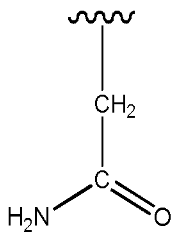 , 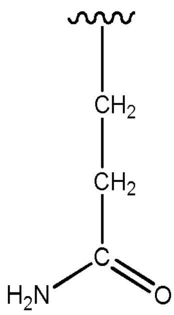 , 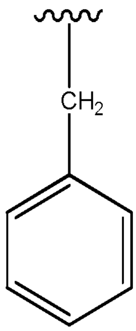 , 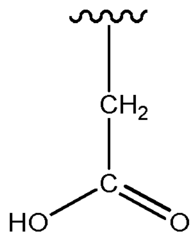 , 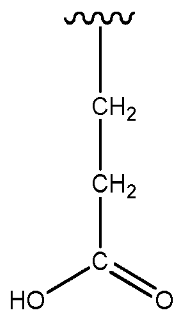 ,

(A38) (A39) (A40) (A41) (A42)

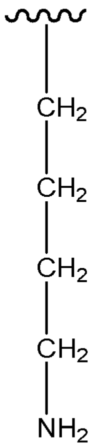, 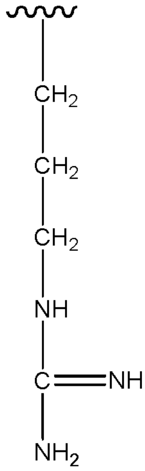 or 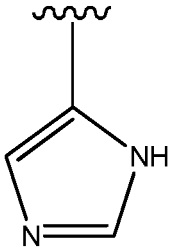 ;

(A43) (A44) (A45)

Rb is a $C_1$-$C_{10}$ alkyl;



represents the site at which a group is covalently linked.

Those skilled in the art would understand that, though $L_1$ is defined as a linear alkylene for convenience, it may not be a linear group or be named differently, such as an amine or alkenyl as a result of the above replacement and/or substitution. For the purpose of the present disclosure, the length of $L_1$ is the atom number in the chain connecting the two attachment points. For this purpose, a ring resulted from replacement of a carbon atom of the linear alkylene, such as a heterocyclylene or heteroarylene, is counted as one atom.

$M_1$ represents a targeting group, of which the definitions and options are the same as the above targeting groups. In some embodiments, each $M_1$ is independently selected from one of the ligands that have affinity to the asialoglycoprotein receptor on the surface of mammalian hepatocytes.

When $M_1$ is a ligand having affinity to the asialoglycoprotein receptor on the surface of mammalian hepatocytes, in some embodiments, n1 may be an integer of 1-3, and n3 may be an integer of 0-4 to ensure that the number of $M_1$ targeting groups in the conjugate may be at least 2. In some embodiments, n1+n3 ≥ 2, so that the number of the $M_1$ taregeting groups may be at least 3, thereby allowing the $M_1$ targeting group to more conveniently bind to the asialoglycoprotein receptor on the surface of liver, which may facilitate the endocytosis of the conjugate into cells. Experiments have shown that when the number of the $M_1$ targeting groups is greater than 3, the feasibility of binding the $M_1$ targeting groups to the asialoglycoprotein receptor on the surface of liver is not significantly increased. Therefore, in view of various aspects such as the synthesis convenience, structure/process costs and delivery efficiency, in some embodiments, n1 is an integer of 1-2, n3 is an integer of 0-1, and n1+n3 = 2-3.

In some embodiments, when each of m1, m2, or m3 are independently selected from an integer of 2-10, the steric position among a plurality of the $M_1$ targeting groups may be fit for binding the $M_1$ targeting groups to the asialoglycoprotein receptor on the surface of liver. In order to make the siRNA conjugate provided by the present disclosure have simpler structure, easier synthesis and/or reduced cost, in some embodiments, each of m1, m2 and m3 are independently an integer of 2-5, in some embodiments, m1 = m2 = m3.

It may be understood by those skilled in the art that when each of $R_{10}$, $R_{11}$, $R_{12}$, $R_{13}$, $R_{14}$, or $R_{15}$ is independently one selected from H, $C_1$-$C_{10}$ alkyl, $C_1$-$C_{10}$ haloalkyl, and $C_1$-$C_{10}$ alkoxy, the purpose of the present disclosure may be achieved without changing the properties of the siRNA conjugate of the present disclosure. In some embodiments, each of $R_{10}$, $R_{11}$, $R_{12}$, $R_{13}$, $R_{14}$, or $R_{15}$ is independently selected from H, methyl or ethyl. In some embodiments, each of $R_{10}$, $R_{11}$, $R_{12}$, $R_{13}$, $R_{14}$, and $R_{15}$ is H.

$R_3$ is a group having a structure represented by Formula (A59), wherein $E_1$ is OH, SH or $BH_2$. Considering the easy accessibility of the starting materials, in some embodiments, $E_1$ is OH or SH.

$R_2$ is selected to achieve the linkage of the N atom on a nitrogenous backbone to A59. In the context of the present disclosure, a "nitrogenous backbone" refers to a chain structure in which the carbon atom to which $R_{10}$, $R_{11}$,

$R_{12}$, $R_{13}$, $R_{14}$, and $R_{15}$ are linked and the N atom are linked to each other. Thus, $R_2$ may be any linking group capable of attaching group A59 to the N atom on a nitrogenous backbone in an appropriate manner. In some embodiments, in the case where the siRNA conjugate represented by Formula (308) is prepared by a solid phase synthesis process, it is required that $R_2$ group comprises both a site linking to the N atom on the nitrogenous backbone and a site linking to the P atom in $R_3$. In some embodiments, in $R_2$, the site linking to the N atom on the nitrogenous backbone forms an amide bond with the N atom, and the site linking to the P atom in $R_3$ forms a phosphoester bond with the P atom. In some embodiments, $R_2$ may be B5, B6, B5' or B6':

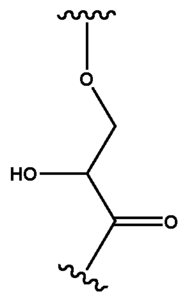 , 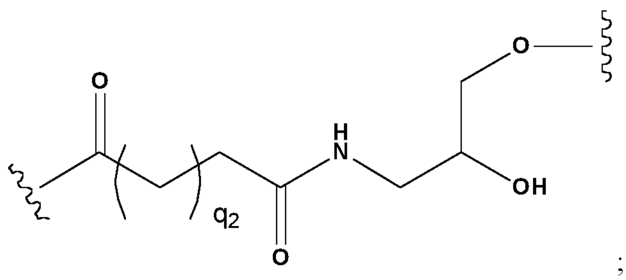 ;

(B5) (B6)

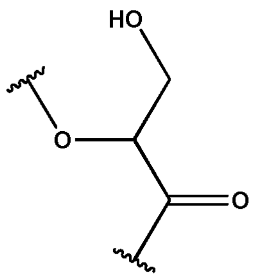 , 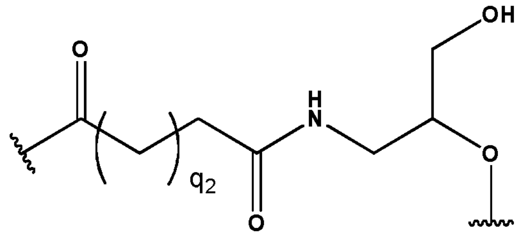 ;

(B5') (B6')

wherein,



represents the site where a group is covalently linked.

$q_2$ may be an integer of 1-10, and in some embodiments, $q_2$ is an integer of 1-5.

$L_1$ is used to link the $M_1$ targeting group to the N atom on the nitrogenous backbone, thereby providing liver targeting function for the siRNA conjugate represented by Formula (308). In some embodiments, $L_1$ is selected from connection combinations of one or more of the groups of Formulae (A1)-(A26). In some embodiments, $L_1$ is selected from connection combinations of one or more of A1, A4, A5, A6, A8, A10, A11, and A13. In some embodiments, $L_1$ is selected from connection combinations of at least two of A1, A4, A8, A10, and A11. In some embodiments, $L_1$ is selected from connection combinations of at least two of A1, A8, and A10.

In some embodiments, the length of $L_1$ may be 3 to 25, 3 to 20, 4 to 15, or 5 to 12 atoms. In some embodiments, the length of $L_1$ is 3, 4, 5, 6, 7, 8, 9, 10, 11, 12, 13, 14, 15, 16, 17, 18, 19, 20, 21, 22, 23, 24, 25, 30, 35, 40, 45, 50, 55, or 60 atoms.

In some embodiments, j1 is an integer of 2-10, and in some embodiments is an integer of 3-5. In some embodiments, j2 is an integer of 2-10, and in some embodiments is an integer of 3-5. R' is a $C_1$-$C_4$ alkyl, and in some embodiments is one of methyl, ethyl, and isopropyl. Ra is one of A27, A28, A29, A30, and A31, and in some embodiments is A27 or A28. Rb is a $C_1$-$C_5$ alkyl, and in some embodiments is one of methyl, ethyl, isopropyl, and butyl. In some embodiments, j1, j2, R', Ra, and Rb in Formulae (A1)-(A26) are respectively selected to achieve the linkage between the $M_1$ targeting group and the N atom on the nitrogenous backbone, and to make the steric position among the $M_1$ targeting group more suitable for binding the $M_1$ targeting group to the asialoglycoprotein receptor on the surface of liver.

In some embodiments, the conjugate has a structure represented by Formula (403), (404), (405), (406), (407), (408), (409), (410), (411), (412), (413), (414), (415), (416), (417), (418), (419), (420), (421) or (422):

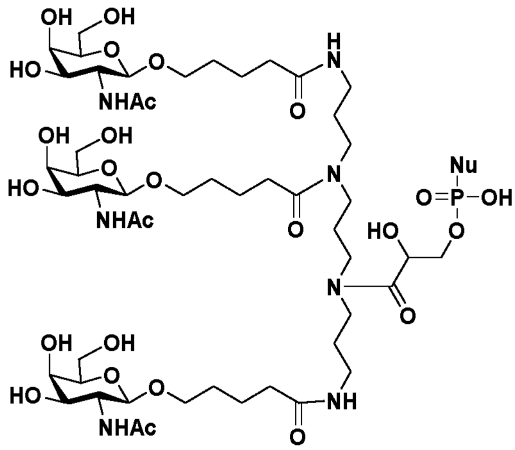

Formula (403)

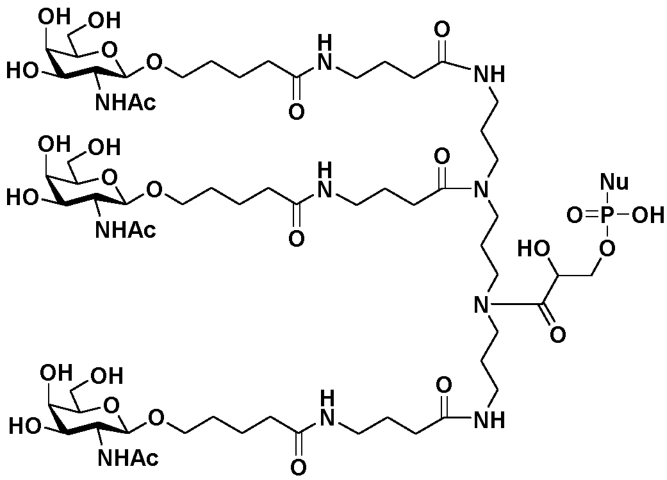

Formula (404)

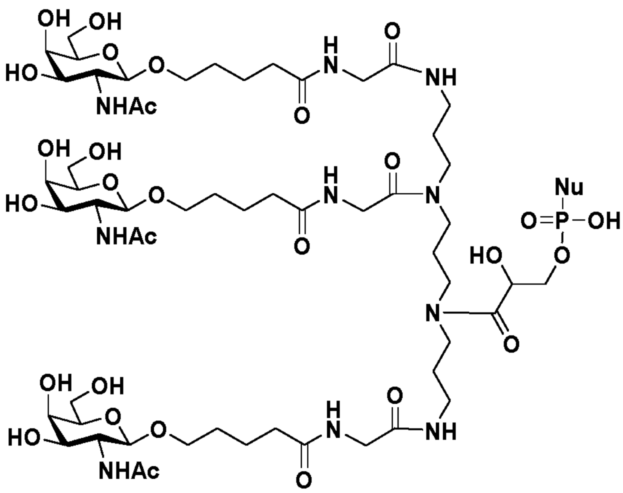

Formula (405)

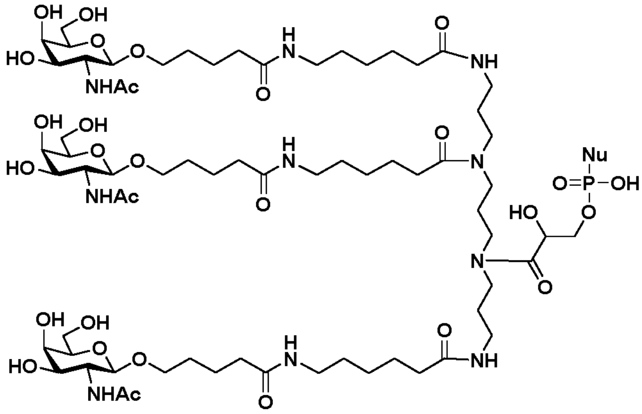

Formula (406)

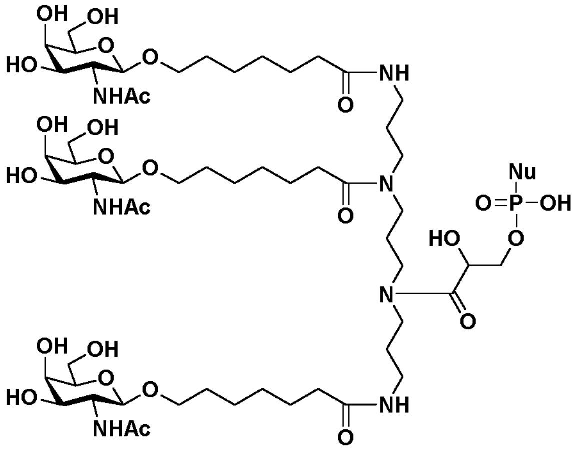

Formula (407)

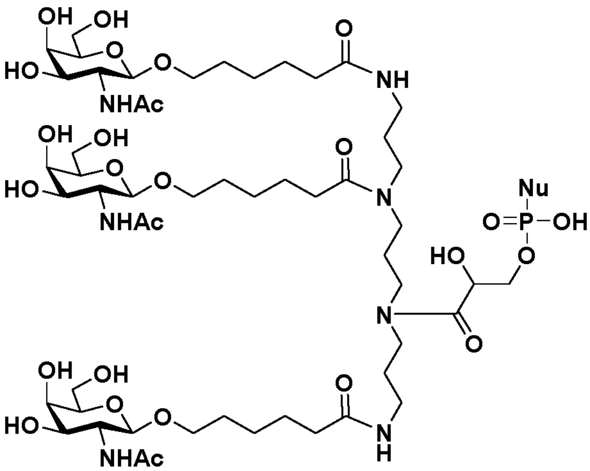

Formula (408)

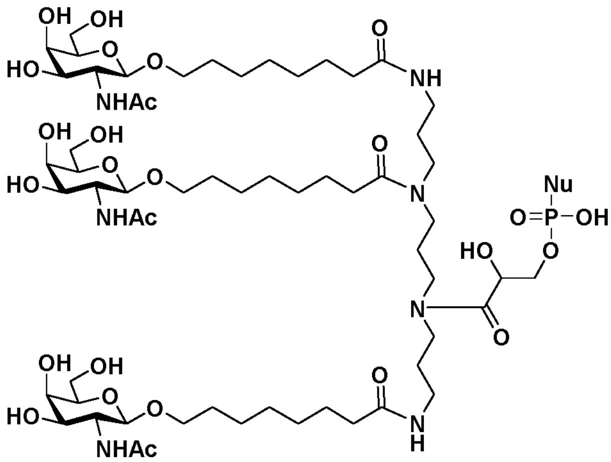

Formula (409)

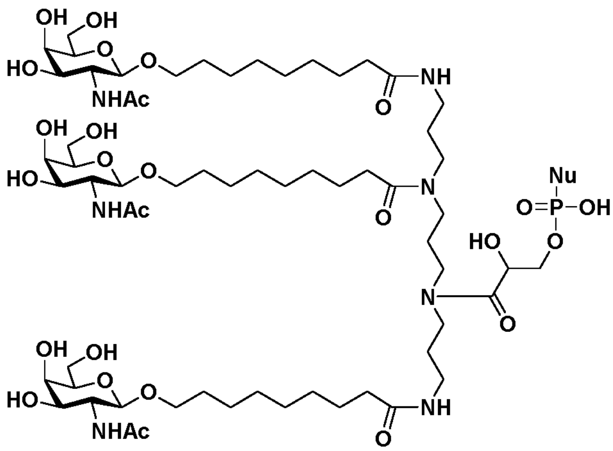

Formula (410)

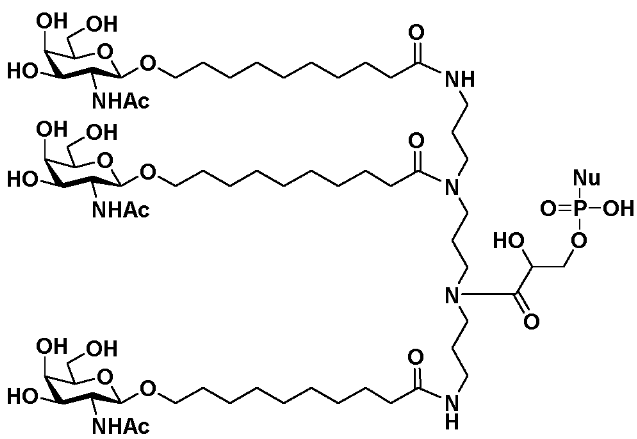

Formula (411)

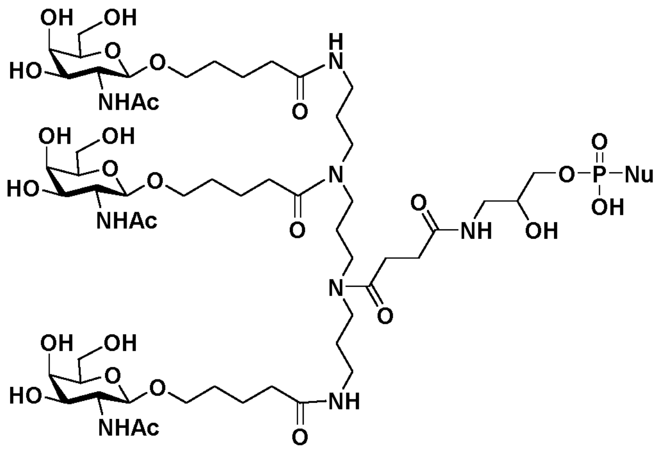

Formula (412)

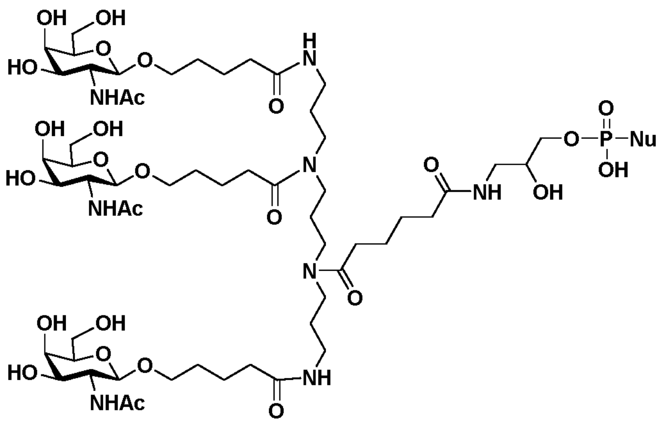

Formula (413)

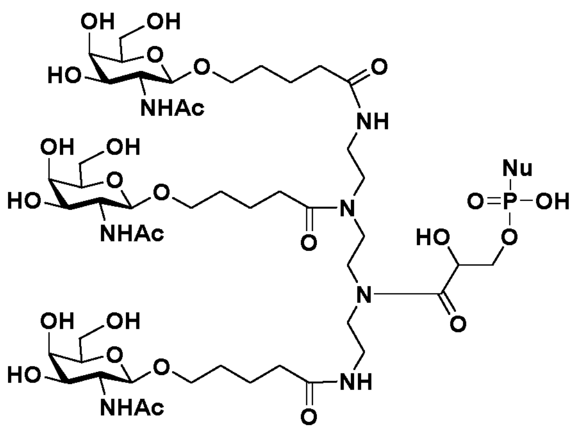

Formula (414)

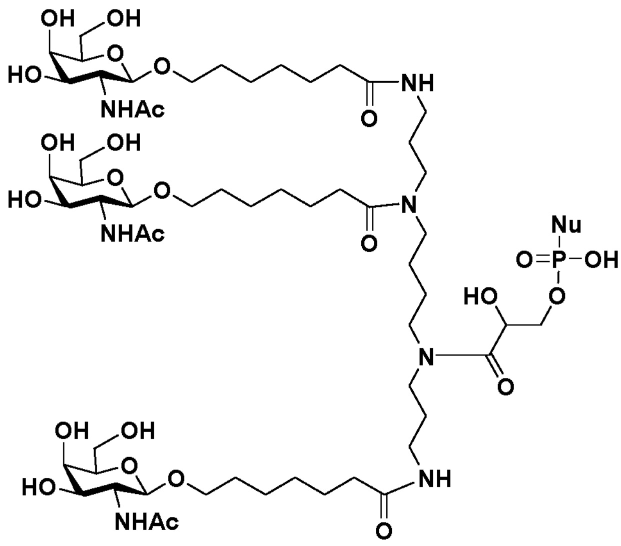

Formula (415)

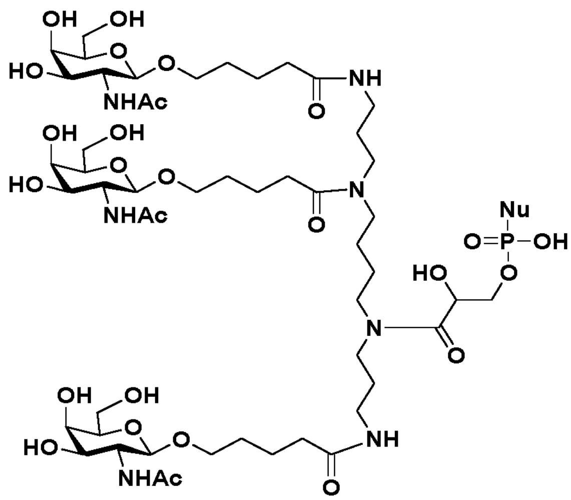

Formula (416)

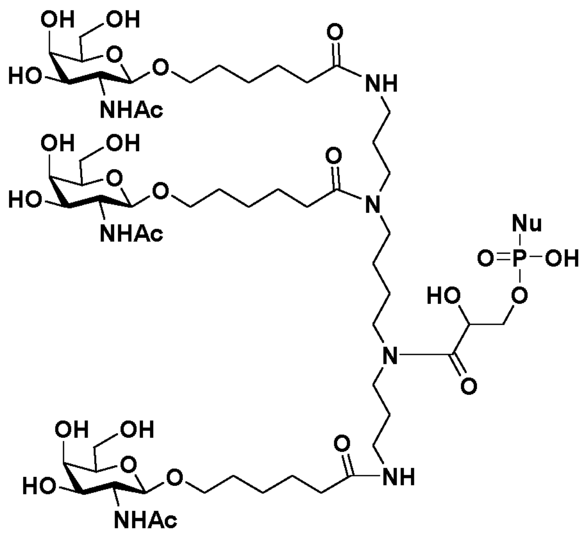

Formula (417)

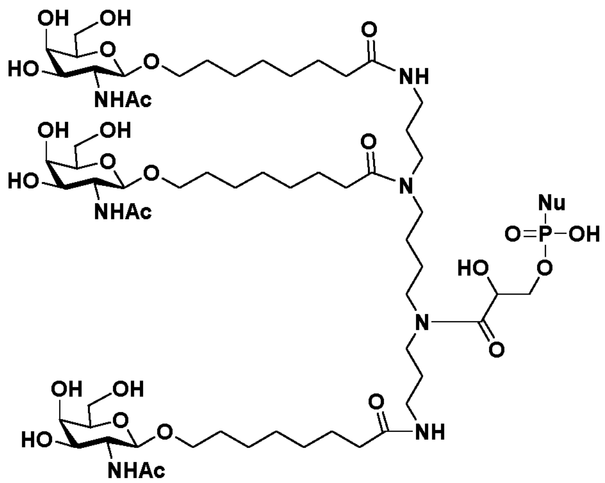

Formula (418)

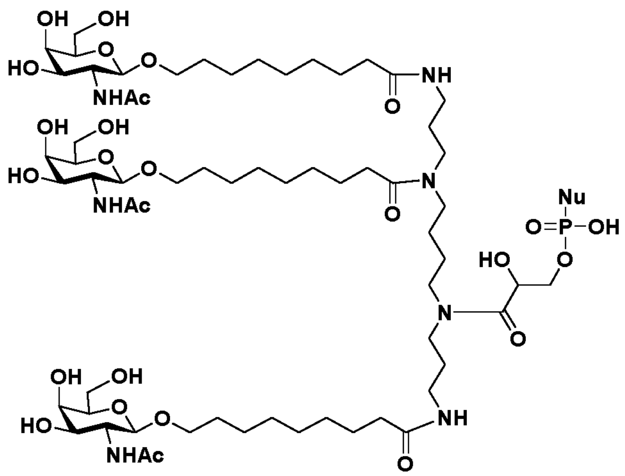

Formula (419)

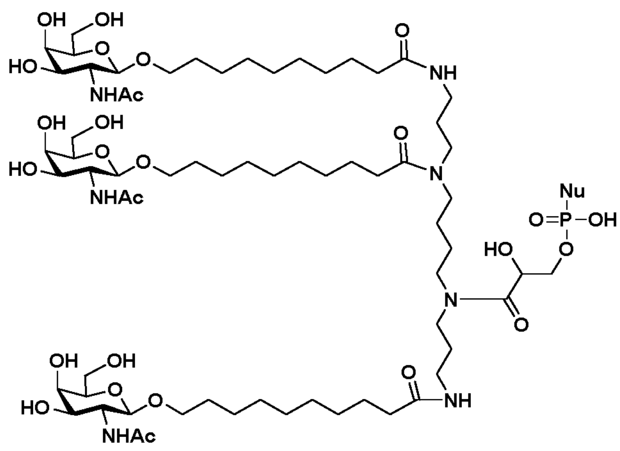

Formula (420)

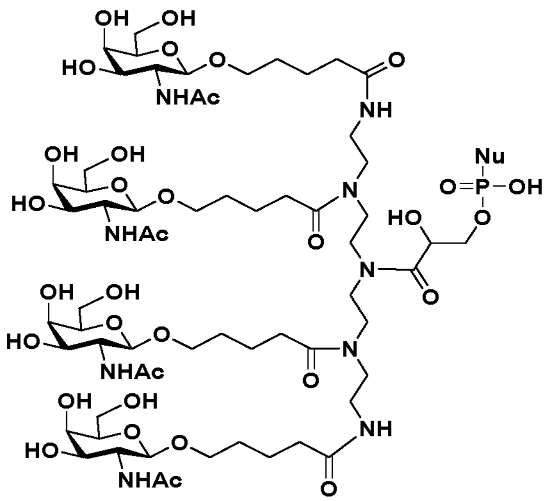

Formula (421)

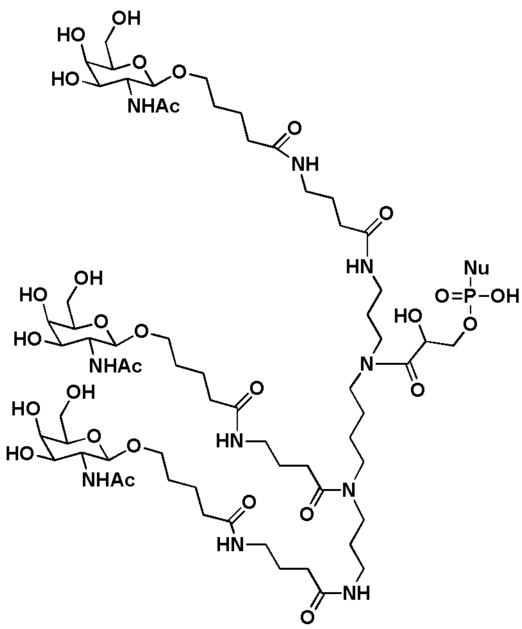

Formula (422)

In some embodiments, the P atom in Formula (A59) may be linked to any possible position in the siRNA sequence, for example, to any nucleotide in the sense strand or the antisense strand of the siRNA; in some embodiments, the P atom in the Formula (A59) is linked to any nucleotide in the sense strand of the siRNA. In some embodiments, the P atom in Formula (A59) is linked to a terminal region of the sense strand or the antisense strand of the siRNA; in some embodiments, the P atom in A59 is linked to a terminal region of the sense strand of the siRNA. The terminal region refers to the first 4 nucleotides counted from a terminal of the sense strand or the antisense strand. In some embodiments, the P atom in Formula (A59) is linked to a terminal of the sense strand or the antisense strand of the siRNA; in some embodiments, the P atom in Formula (A59) is linked to the 3' terminal of the sense strand of the siRNA. In the case where the P atom in Formula (A59) is linked to the above position in the sense strand of the siRNA, after entering into cells, the siRNA conjugate represented by Formula (308) can release a separate antisense strand of the siRNA during unwinding thereby blocking the translation of PNP mRNA into a protein and inhibiting the expression of PNP gene.

In some embodiments, the P atom in Formula (A59) may be linked to any possible position of the nucleotide in the siRNA, for example, to position 5', 2' or 3', or to the base of the nucleotide. In some embodiments, the P atom in Formula (A59) may be linked to position 2', 3' or 5' of the nucleotide in the siRNA by forming a phosphodiester bond. In some embodiments, the P atom in Formula (A59) is linked to an oxygen atom formed by dehydrogenation of 3'-hydroxy of the nucleotide at 3' terminal of the sense strand of the siRNA (in this case, the P atom in Formula (A59) may also be considered as a P atom in a phosphate group contained in the siRNA), or linked to a nucleotide by substituting a hydrogen atom in 2'-hydroxy of a nucleotide of the sense strand of the siRNA, or linked to a nucleotide by substituting a hydrogen atom in 5'-hydroxy of the nucleotide at 5' terminal of the sense strand of the siRNA.

The inventors of the present disclosure have unexpectedly found that the siRNA conjugate of the present disclosure not only has significantly improved stability in plasma and low off-target effect, but also exhibits higher silencing activity against PNP mRNA and also has higher effect in inhibiting blood uric acid concentration. In some embodiments, the siRNA of the present disclosure may be one of the siRNAs shown in Tables 1a-1d. The siRNA conjugates containing these siRNAs exhibit higher silencing activity against PNP mRNA.

Table 1a: The sequences of first siRNAs of the present disclosure

| siRNA No. | SEQ ID NO: | Sequence Direction 5' - 3' |
|---|---|---|
| siPNa1 | 9 | CCUAUGAAGAUUAUAAGAA |
| | 10 | UUCUUAUAAUCUUCAUAGGUG |

(continued)

| siRNA No. | SEQ ID NO: | Sequence Direction 5' - 3' |
|---|---|---|
| siPNa2 | 11 | CACCUAUGAAGAUUAUAAGAA |
| | 12 | UUCUUAUAAUCUUCAUAGGUGUA |
| siPNa1-M1 | 13 | CmCmUmAmUmGmAfAfGfAmUmUmAmUmAmAmGmAmAm |
| | 14 | UmUfCmUmUmAfUmAmAmUmCmUmUmCfAmUfAmGmGmUmGm |
| siPNa2-M1 | 15 | CmAmCmCmUmAmUmGmAfAfGfAmUmUmAmUmAmAmGmAmAm |
| | 16 | UmUfCmUmUmAfUmAmAmUmCmUmUmCfAmUfAmGmGmUmGmUmGm |
| siPNa1-M2 | 17 | CmCmUmAm UfGmAfAfGfAmUmUmAmUmAmAm GmAmAm |
| | 18 | UmUfCmUmUmAfUmAfAfUmCmUmUmCfAmUfAm Gm GmUmGm |
| siPNa2-M2 | 19 | CmAmCmCmUmAmUfGmAfAfGfAmUmUmAmUmAmAmGmAmAm |
| | 20 | UmUfCmUmUmAfUmAfAfUmCmUmUmCfAmUfAmGmGmUmGmUmGm |
| siPNa1-M3 | 21 | CmCmUmAm UfGmAfAfGfAmUmUmAmUmAmAm GmAmAm |
| | 22 | UmUfCmUmUmAfUmAmAmUmCmUmUmCfAmUfAmGmGmUmGm |
| siPNa2-M3 | 23 | CmAmCmCmUmAmUfGmAfAfGfAm UmUmAmUmAmAmGmAmAm |
| | 24 | UmUfCmUmUmAfUmAmAmUmCmUmUmCfAmUfAmGmGmUmGmUmGm |
| siPNa1-M1S | 25 | CmsCmsUmAmUmGmAfAfGfAmUmUmAmUmAmAmGmAmAm |
| | 26 | UmsUfsCmUmUmAfUmAmAmUmCmUmUmCfAmUfAmGmGmsUmsGm |
| siPNa2-M1S | 27 | CmsAmsCmCm UmAmUmGmAfAfGfAmUmUmAmUmAmAm GmAmAm |
| | 28 | UmsUfsCmUmUmAfUmAmAmUmCmUmUmCfAmUfAmGmGmUmGmsUmsGm |
| siPNa1-M2S | 29 | CmsCmsUmAmUtlimAtAtlitAmUmUmAmUmAmAmlimAmAm |
| | 30 | UmsUfsCmUmUmAfUmAfAfUmCmUmUmCfAmUfAmGmGmsUmsGm |
| siPNa2-M2S | 31 | CmsAmsCmCm UmAm UfGmAfAfGfAmUmUmAmUmAmAmGmAmAm |
| | 32 | UmsUfsCmUmUmAfUmAfAfUmCmUmUmCfAmUfAmGmGmUmGmsUmsGm |
| siPNa1-M3S | 33 | CmsCmsUmAmUfGmAfAfGfAmUmUmAmUmAmAmGmAmAm |
| | 34 | UmsUfsCmUmUmAfUmAmAmUmCmUmUmCfAmUfAmGmGmsUmsGm |
| siPNa2-M3S | 35 | CmsAmsCmCmUmAmUfGmAfAfGfAmUmUmAmUmAmAmGmAmAm |
| | 36 | UmsUfsCmUmUmAfUmAmAmUmCmUmUmCfAmUfAmGmGmUmGmsUmsGm |
| siPNa1-M1P1 | 37 | CmCmUmAmUmGmAfAfGfAmUmUmAmUmAmAmGmAmAm |
| | 38 | PlUmUfCmUmUmAfUmAmAmUmCmUmUmCfAmUfAmGmGmUmGm |
| siPNa2-M1P1 | 39 | CmAmCmCmUmAmUmGmAfAfGfAmUmUmAmUmAmAmGmAmAm |
| | 40 | P1UmUfCmUmUmAfUmAmAmUmCmUmUmCfAmUfAmGmGmUmGmUmGm |
| siPNa1-M2P1 | 41 | CmCmUmAmUfGmAfAfGfAmUmUmAmUmAmAmGmAmAm |
| | 42 | PlUmUfCmUmUmAfUmAfAfUmCmUmUmCfAmUfAmGmGmUmGm |

(continued)

| siRNA No. | SEQ ID NO: | Sequence Direction 5' - 3' |
|---|---|---|
| siPNa2-M2P1 | 43 | CmAmCmCmUmAmUfGmAfAfGfAm UmUmAmUmAmAmGmAmAm |
| | 44 | P1UmUfCmUmUmAfUmAfAfUmCmUmUmCfAmUfAmGmGmUmGmUmGm |
| siPNa1-M3P1 | 45 | CmCmUmAm UfGmAfAfGfAmUmUmAmUmAmAm GmAmAm |
| | 46 | PlUmUfCmUmUmAfUmAmAmUmCmUmUmCfAmUfAmGmGmUmGm |
| siPNa2-M3P1 | 47 | CmAmCmCmUmAmUfGmAfAfGfAm UmUmAmUmAmAmGmAmAm |
| | 48 | P1UmUfCmUmUmAfUmAmAmUmCmUmUmCfAmUfAmGmGmUmGmUmGm |
| siPNa1-M1SP1 | 49 | CmsCmsUmAmUmGmAfAfGfAmUmUmAmUmAmAmGmAmAm |
| | 50 | P1UmsUfsCmUmUmAfUmAmAmUmCmUmUmCfAmUfAmGmGmsUmsGm |
| siPNa2-M1SP1 | 51 | CmsAmsCmCm UmAmUmGmAfAfGfAmUmUmAmUmAmAm GmAmAm |
| | 52 | P1UmsUfsCmUmUmAfUmAmAmUmCmUmUmCfAmUfAmGmGmUmGms |
| | | UmsGm |
| siPNa1-M2SP1 | 53 | CmsCmsUmAmUfGmAfAfGfAmUmUmAmUmAmAm GmAmAm |
| | 54 | P1UmsUfsCmUmUmAfUmAfAfUmCmUmUmCfAmUfAmGmGmsUmsGm |
| siPNa2-M2SP1 | 55 | CmsAmsCmCm UmAm UfGmAfAfGfAmUmUmAmUmAmAmGmAmAm |
| | 56 | P1UmsUfsCmUmUmAfUmAfAfUmCmUmUmCfAmUfAmGmGmUmGmsUmsGm |
| siPNa1-M3SP1 | 57 | CmsCmsUmAmUfGmAfAfGfAmUmUmAmUmAmAm GmAmAm |
| | 58 | P1UmsUfsCmUmUmAfUmAmAmUmCmUmUmCfAmUfAmGmGmsUmsGm |
| siPNa2-M3SP1 | 59 | CmsAmsCmCm UmAm UfGmAfAfGfAmUmUmAmUmAmAmGmAmAm |
| | 60 | P1UmsUfsCmUmUmAfUmAmAmUmCmUmUmCfAmUfAmGmGmUmGmsUmsGm |

Table 1b: The sequences of second siRNAs of the present disclosure

| siRNA No. | SEQ ID NO: | Sequence Direction 5' - 3' |
|---|---|---|
| siPNb1 | 69 | GUACAGUACCAGAAGUUAU |
| | 70 | AUAACUUCUGGUACUGUACUC |
| siPNb2 | 71 | GAGUACAGUACCAGAAGUUAU |
| | 72 | AUAACUUCUGGUACUGUACUCAU |
| siPNb1-M1 | 73 | GmUmAmCmAmGmUfAfCfCmAmGmAmAmGmUmUmAmUm |
| | 74 | AmUfAmAmCmUfUmCmUmGmGmUmAmCfUmGfUmAmCmUmCm |

(continued)

| siRNA No. | SEQ ID NO: | Sequence Direction 5' - 3' |
|---|---|---|
| SiPNb2-M1 | 75 | GmAmGmUmAmCmAmGmUfAfCfCmAmGmAmAmGmUmUmAmUm |
| | 76 | AmUfAmAmCmUfUmCmUmGmGmUmAmCfUmGfUmAmCmUmCmAmUm |
| siPNb1-M2 | 77 | GmUmAmCmAfGmUfAfCfCmAmGmAmAmGmUmUmAmUm |
| | 78 | AmUfAmAmCmUfUmCfUfGmGmUmAmCfUm GfUmAmCmUmCm |
| siPNb2-M2 | 79 | GmAmGmUmAmCmAfGmUfAfCfCmAmGmAmAmGmUmUmAmUm |
| | 80 | AmUfAmAmCmUfUmCfUfGmGmUmAmCfUmGfUmAmCmUmCmAmUm |
| siPNb1-M3 | 81 | GmUmAmCmAfGmUfAfCfCmAm GmAmAmGmUmUmAmUm |
| | 82 | AmUfAmAmCmUfUmCmUmGmGmUmAmCfUmGfUmAmCmUmCm |
| siPNb2-M3 | 83 | GmAmGmUmAmCmAfGmUfAfCfCmAmGmAmAmGmUmUmAmUm |
| | 84 | AmUfAmAmCmUfUmCmUmGmGmUmAmCfUmGfUmAmCmUmCmAmUm |
| siPNb1-M1S | 85 | GmsUmsAmCmAmGmUfAfCfCmAmGmAmAmGmUmUmAmUm |
| | 86 | AmsUfsAmAmCmUfUmCmUmGmGmUmAmCfUmGfUmAmCmsUmsCm |
| SiPNb2-M1S | 87 | GmsAmsGmUmAmCmAmGmUfAfCfCmAmGmAmAmGmUmUmAmUm |
| | 88 | AmsUfsAmAmCmUfUmCmUmGmGmUmAmCfUmGfUmAmCmUmCmsAmsUm |
| siPNb1-M2S | 89 | GmsUmsAmCmAfGmUfAfCfCmAmGmAmAmGmUmUmAmUm |
| | 90 | AmsUfsAmAmCmUfUmCfUfGmGmUmAmCfUmGfUmAmCmsUmsCm |
| siPNb2-M2S | 91 | GmsAmsGmUmAmCmAfGmUfAfCfCmAmGmAmAmGmUmUmAmUm |
| | 92 | AmsUfsAmAmCmUfUmCfUfGmGmUmAmCfUmGfUmAmCmUmCmsAmsUm |
| siPNb1-M3S | 93 | Gms UmsAmCmAfGm UfAfCfCmAmGmAmAmGmUm UmAmUm |
| | 94 | AmsUfsAmAmCmUfUmCmUmGmGmUmAmCfUmGfUmAmCmsUmsCm |
| siPNb2-M3S | 95 | GmsAmsGmUmAmCmAfGmUfAfCfCmAmGmAmAmGmUmUmAmUm |
| | 96 | AmsUfsAmAmCmUfUmCmUmGmGmUmAmCfUmGfUmAmCmUmCmsAmsUm |
| siPNb1-M1P1 | 97 | GmUmAmCmAmGmUfAfCfCmAmGmAmAmGmUmUmAmUm |
| | 98 | PlAmUfAmAmCmUfUmCmUmGmGmUmAmCfUmGfUmAmCmUmCm |
| siPNb2-M1P1 | 99 | GmAmGmUmAmCmAmGmUfAfCfCmAmGmAmAmGmUmUmAmUm |
| | 100 | P1AmUfAmAmCmUfUmCmUmGmGmUmAmCfUmGfUmAmCmUmCmAmUm |
| siPNb1-M2P1 | 101 | GmUmAmCmAfGmUfAfCfCmAmGmAmAmGmUmUmAmUm |
| | 102 | PlAmUfAmAmCmUfUmCfUfGmGmUmAmCfUmGfUmAmCmUmCm |
| siPNb2-M2P1 | 103 | GmAmGmUmAmCmAfGmUfAfCfCmAmGmAmAmGmUmUmAmUm |
| | 104 | P1AmUfAmAmCmUfUmCfUfGmGmUmAmCfUmGfUmAmCmUmCmAmUm |

(continued)

| siRNA No. | SEQ ID NO: | Sequence Direction 5' - 3' |
|---|---|---|
| siPNb1-M3P1 | 105 | GmUmAmCmAfGmUtAfCfCmAmlimAmAmGmUmUmAmUm |
| | 106 | P1AmUfAmAmCmUfUmCmUmGmGmUmAmCfUmGfUmAmCmUmCm |
| siPNb2-M3P1 | 107 | GmAmGmUmAmCmAfGmUfAfCfCmAmGmAmAmGmUmUmAmUm |
| | 108 | P1AmUfAmAmCmUfUmCmUmGmGmUmAmCfUmGfUmAmCmUmCmAmUm |
| siPNb1-M1SP1 | 109 | GmsUmsAmCmAmGmUfAfCfCmAmGmAmAmGmUmUmAmUm |
| | 110 | PlAmsUfsAmAmCmUfUmCmUmGmGmUmAmCfUmGfUmAmCmsUmsCm |
| siPNb2-M1SP1 | 111 | GmsAmsGmUmAmCmAmGmUfAfCfCmAmGmAmAmGmUmUmAmUm |
| | 112 | P1AmsUfsAmAmCmUfUmCmUmGmGmUmAmCfUmGfUmAmCmUmCmsAmsUm |
| siPNb1-M2SP1 | 113 | GmsUmsAmCmAfGmUfAfCfCmAmGmAmAmGmUmUmAmUm |
| | 114 | PlAmsUfsAmAmCmUfUmCfUfGmGmUmAmCfUmGfUmAmCmsUmsCm |
| siPNb2-siPNb2-M2SP1 | 115 | GmsAmsGmUmAmCmAfGmUfAfCfCmAmGmAmAmGmUmUmAmUm |
| | 116 | P1AmsUfsAmAmCmUfUmCfUfGmGmUmAmCfUmGfUmAmCmUmCmsAmsUm |
| siPNb1-M3SP1 | 117 | GmsUmsAmCmAfGmUfAfCfCmAmGmAmAmGmUmUmAmUm |
| | 118 | PlAmsUfsAmAmCmUfUmCmUmGmGmUmAmCfUmGfUmAmCmsUmsCm |
| siPNb2-M3SP1 | 119 | GmsAmsGmUmAmCmAfGmUfAfCfCmAmGmAmAmGmUmUmAmUm |
| | 120 | P1AmsUfsAmAmCmUfUmCmUmGmGmUmAmCfUmGfUmAmCmUmCmsAmsUm |

Table 1c: The sequences of third siRNAs of the present disclosure

| siRNA No. | SEQ ID NO: | Sequence Direction 5' - 3' |
|---|---|---|
| siPNc1 | 129 | CAAACAAGCUGCACAGAAA |
| | 130 | UUUCUGUGCAGCUUGUUUGCC |
| siPNc2 | 131 | GGCAAACAAGCUGCACAGAAA |
| | 132 | UUUCUGUGCAGCUUGUUUGCCAG |
| siPNc1 - M1 | 133 | CmAmAmAmCmAmAfGfCfUmGmCmAmCmAmGmAmAmAm |
| | 134 | UmUfUmCmUmGfUmGmCmAmGmCmUmUfGmUfUmUmGmCmCm |
| siPNc2-M1 | 135 | GmGmCmAmAmAmCmAmAfGfCfUmGmCmAmCmAmGmAmAmAm |
| | 136 | UmUfUmCmUmGfUmGmCmAmGmCmUmUfGmUfUmUmGmCmCmAmGm |
| siPNc1-M2 | 137 | CmAmAmAmCfAmAfGfCfUmGmCmAmCmAmGmAmAmAm |
| | 138 | UmUfUmCmUmGfUmGfCfAmGmCmUmUfGmUfUmUmGmCmCm |
| siPNc2-M2 | 139 | GmGmCmAmAmAmCfAmAfGfCfUmGmCmAmCmAmGmAmAmAm |
| | 140 | UmUfUmCmUmGfUmGfCfAmGmCmUmUfGmUfUmUmGmCmCmAmGm |

(continued)

| siRNA No. | SEQ ID NO: | Sequence Direction 5' - 3' |
|---|---|---|
| siPNc1-M3 | 141 | CmAmAmAmCfAmAfGfCfUmGmCmAmCmAmGmAmAmAm |
| | 142 | UmUfUmCmUmGfUmGmCmAmGmCmUmUfGmUfUmUmGmCmCm |
| siPNc2-M3 | 143 | GmGmCmAmAmAmCfAmAfGfCfUmGmCmAmCmAmGmAmAmAm |
| | 144 | UmUfUmCmUmGfUmGmCmAmGmCmUmUfGmUfUmUmGmCmCmAmG m |
| siPNc1-M1S | 145 | CmsAmsAmAmCmAmAfGfCfUmGmCmAmCmAmGmAmAmAm |
| | 146 | UmsUfsUmCmUmGfUmGmCmAmGmCmUmUfGmUfUmUmGmsCmsCm |
| siPNc2-M1S | 147 | GmsGmsCmAmAmAmCmAmAfGfCfUmGmCmAmCmAmGmAmAmAm |
| | 148 | UmsUfsUmCmUmGfUmGmCmAmGmCmUmUfGmUfUmUmGmCmCmsA msGm |
| siPNc1-M2S | 149 | CmsAmsAmAmCfAmAfGfCfUmGmCmAmCmAmGmAmAmAm |
| | 150 | UmsUfsUmCmUmGfUmGfCfAmGmCmUmUfGmUfUmUmGmsCmsCm |
| siPNc2-M2S | 151 | GmsGmsCmAmAmAmCfAmAfGfCfUmGmCmAmCmAmGmAmAmAm |
| | 152 | UmsUfsUmCmUmGfUmGfCfAmGmCmUmUfGmUfUmUmGmCmCmsAms Gm |
| siPNc1-M3S | 153 | CmsAmsAmAmCfAmAfGfCfUmGmCmAmCmAmGmAmAmAm |
| | 154 | UmsUfsUmCmUmGfUmGmCmAmGmCmUmUfGmUfUmUmGmsCmsCm |
| siPNc2-M3S | 155 | GmsGmsCmAmAmAmCfAmAfGfCfUmGmCmAmCmAmGmAmAmAm |
| | 156 | UmsUfsUmCmUmGfUmGmCmAmGmCmUmUfGmUfUmUmGmCmCmsA msGm |
| siPNc1-M1P1 | 157 | CmAmAmAmCmAmAfGfCfUmGmCmAmCmAmGmAmAmAm |
| | 158 | P1UmUfUmCmUmGfUmGmCmAmGmCmUmUfGmUfUmUmGmCmCm |
| siPNc2-M1P1 | 159 | GmGmCmAmAmAmCmAmAfGfCfUmGmCmAmCmAmGmAmAmAm |
| | 160 | P1UmUfUmCmUmGfUmGmCmAmGmCmUmUfGmUfUmUmGmCmCmAm Gm |
| siPNc1-M2P1 | 161 | CmAmAmAmCfAmAfGfCfUmGmCmAmCmAmGmAmAmAm |
| | 162 | P1UmUfUmCmUmGfUmGfCfAmGmCmUmUfGmUfUmUmGmCmCm |
| siPNc2-M2P1 | 163 | GmGmCmAmAmAmCfAmAfGfCfUmGmCmAmCmAmGmAmAmAm |
| | 164 | P1UmUfUmCmUmGfUmGfCfAmGmCmUmUfGmUfUmUmGmCmCmAmG m |
| siPNc1-M3P1 | 165 | CmAmAmAmCfAmAfGfCfUmGmCmAmCmAmGmAmAmAm |
| | 166 | P1UmUfUmCmUmGfUmGmCmAmGmCmUmUfGmUfUmUmGmCmCm |
| siPNc2-M3P1 | 167 | GmGmCmAmAmAmCfAmAfGfCfUmGmCmAmCmAmGmAmAmAm |
| | 168 | P1UmUfUmCmUmGfUmGmCmAmGmCmUmUfGmUfUmUmGmCmCmAm Gm |
| siPNc1-M1SP1 | 169 | CmsAmsAmAmCmAmAfGfCfUmGmCmAmCmAmGmAmAmAm |
| | 170 | PlUmsUfsUmCmUmGfUmGmCmAmGmCmUmUfGmUfUmUmGmsCmsCm |

(continued)

| siRNA No. | SEQ ID NO: | Sequence Direction 5' - 3' |
|---|---|---|
| siPMc2-M1SP1 | 171 | GmsGmsCmAmAmAmCmAmAfGfCfUmGmCmAmCmAmGmAmAmAm |
| | 172 | P1UmsUfsUmCmUmGfUmGmCmAmGmCmUmUfGmUfUmUmGmCmCmsAmsGm |
| siPNc1-M2SP1 | 173 | CmsAmsAmAmCfAmAfGfCfUmGmCmAmCmAmGmAmAmAm |
| | 174 | P1UmsUfsUmCmUmGfUmGfCfAmGmCmUmUfGmUfUmUmGmsCmsCm |
| siPNc2-M2SP1 | 175 | GmsGmsCmAmAmAmCfAmAfGfCfUmGmCmAmCmAmGmAmAmAm |
| | 176 | P1UmsUfsUmCmUmGfUmGfCfAmGmCmUmUfGmUfUmUmGmCmCmsAmsGm |
| siPNc1-M3SP1 | 177 | CmsAmsAmAmCfAmAfGfCfUmGmCmAmCmAmGmAmAmAm |
| | 178 | P1UmsUfsUmCmUmGfUmGmCmAmGmCmUmUfGmUfUmUmGmsCmsCm |
| siPNc2-M3SP1 | 179 | GmsGmsCmAmAmAmCfAmAfGfCfUmGmCmAmCmAmGmAmAmAm |
| | 180 | P1UmsUfsUmCmUmGfUmGmCmAmGmCmUmUfGmUfUmUmGmCmCmsAmsGm |

Table Id: The sequences of fourth siRNAs of the present disclosure

| siRNA No. | SEQ ID NO: | Sequence Direction 5' - 3' |
|---|---|---|
| siPNd1 | 189 | CAAACAAGGACUAAUCCAA |
| | 190 | UUGGAUUAGUCCUUGUUUGGU |
| siPNd2 | 191 | ACCAAACAAGGACUAAUCCAA |
| | 192 | UUGGAUUAGUCCUUGUUUGGUCU |
| siPNd1-M1 | 193 | CmAmAmAmCmAmAfGfGfAmCmUmAmAmUmCmCmAmAm |
| | 194 | UmUfGmGmAmUfUmAmGmUmCmCmUmUfGmUfUmUmGmGmUm |
| siPNd2-M1 | 195 | AmCmCmAmAmAmCmAmAfGfGfAmCmUmAmAmUmCmCmAmAm |
| | 196 | UmUfGmGmAmUfUmAmGmUmCmCmUmUfGmUfUmUmGmGmUmCmUm |
| siPNd1-M2 | 197 | CmAmAmAmCfAmAfGfGfAmCmUmAmAmUmCmCmAmAm |
| | 198 | UmUfGmGmAmUfUmAfGfUmCmCmUmUfGmUfUmUmGmGmUm |
| siPNd2-M2 | 199 | AmCmCmAmAmAmCfAmAfGfGfAmCmUmAmAmUmCmCmAmAm |
| | 200 | UmUfGmGmAmUfUmAfGfUmCmCmUmUfGmUfUmUmGmGmUmCmUm |
| siPNd1-M3 | 201 | CmAmAmAmCfAmAfGfGfAmCmUmAmAmUmCmCmAmAm |
| | 202 | UmUfGmGmAmUfUmAmGmUmCmCmUmUfGmUfUmUmGmGmUm |
| siPNd2-M3 | 203 | AmCmCmAmAmAmCfAmAfGfGfAmCmUmAmAmUmCmCmAmAm |
| | 204 | UmUfGmGmAmUfUmAmGmUmCmCmUmUfGmUfUmUmGmGmUmCmUm |
| siPNd1-M1S | 205 | CmsAmsAmAmCmAmAfGfGfAmCmUmAmAmUmCmCmAmAm |
| | 206 | UmsUfsGmGmAmUfUmAmGmUmCmCmUmUfGmUfUmUmGmsGmsUm |

(continued)

| siRNA No. | SEQ ID NO: | Sequence Direction 5' - 3' |
|---|---|---|
| siPNd2-siPNd2-MIS | 207 | AmCmCmAmAmAmCmAmAfGfGfAmCmUmAmAmUmCmCmAmAm |
| | 208 | UmsUfsGmGmAmUfUmAmGmUmCmCmUmUfGmUfUmUmGmGmUmsCmsUm |
| siPNd1-M2S | 209 | CmsAmsAmAmCfAmAfGfGfAmCmUmAmAmUmCmCmAmAm |
| | 210 | UmsUfsGmGmAmUfUmAfGfUmCmCmUmUfGmUfUmUmGmsGmsUm |
| siPNd2-M2S | 211 | AmCmCmAmAmAmCfAmAfGfGfAmCmUmAmAmUmCmCmAmAm |
| | 212 | UmsUfsGmGmAmUfUmAfGfUmCmCmUmUfGmUfUmUmGmGmUmsCmsUm |
| siPNd1-M3S | 213 | CmsAmsAmAmCfAmAfGfGfAmCmUmAmAmUmCmCmAmAm |
| | 214 | UmsUfsGmGmAmUfUmAmGmUmCmCmUmUfGmUfUmUmGmsGmsUm |
| siPNd2-M3S | 215 | AmCmCmAmAmAmCfAmAfGfGfAmCmUmAmAmUmCmCmAmAm |
| | 216 | UmsUfsGmGmAmUfUmAmGmUmCmCmUmUfGmUfUmUmGmGmUmsCmsUm |
| siPNd1-M1P1 | 217 | CmAmAmAmCmAmAfGfGfAmCmUmAmAmUmCmCmAmAm |
| | 218 | PlUmUfGmGmAmUfUmAmGmUmCmCmUmUfGmUfUmUmGmGmUm |
| siPNd2-M1P1 | 219 | AmCmCmAmAmAmCmAmAfGfGfAmCmUmAmAmUmCmCmAmAm |
| | 220 | P1UmUfGmGmAmUfUmAmGmUmCmCmUmUfGmUfUmUmGmGmUmCmUm |
| siPNd1-M2P1 | 221 | CmAmAmAmCfAmAfGfGfAmCmUmAmAmUmCmCmAmAm |
| | 222 | PlUmUfGmGmAmUfUmAfGfUmCmCmUmUfGmUfUmUmGmGmUm |
| siPNd2-M2P1 | 223 | AmCmCmAmAmAmCfAmAfGfGfAmCmUmAmAmUmCmCmAmAm |
| | 224 | P1UmUfGmGmAmUfUmAfGfUmCmCmUmUfGmUfUmUmGmGmUmCmUm |
| siPNd1-M3P1 | 225 | CmAmAmAmCfAmAfGfGfAmCmUmAmAmUmCmCmAmAm |
| | 226 | PlUmUfGmGmAmUfUmAmGmUmCmCmUmUfGmUfUmUmGmGmUm |
| siPNd2-M3P1 | 227 | AmCmCmAmAmAmCfAmAfGfGfAmCmUmAmAmUmCmCmAmAm |
| | 228 | P1UmUfGmGmAmUfUmAmGmUmCmCmUmUfGmUfUmUmGmGmUmCmUm |
| siPNd1- | 229 | CmsAmsAmAmCmAmAfGfGfAmCmUmAmAmUmCmCmAmAm |
| M1SP1 | 230 | P1UmsUfsGmGmAmUfUmAmGmUmCmCmUmUfGmUfUmUmGmsGmsUm |
| siPNd2-M1SP1 | 231 | AmCmCmAmAmAmCmAmAfGfGfAmCmUmAmAmUmCmCmAmAm |
| | 232 | P1UmsUfsGmGmAmUfUmAmGmUmCmCmUmUfGmUfUmUmGmGmUmsCmsUm |
| siPNd1-M2SP1 | 233 | CmsAmsAmAmCfAmAfGfGfAmCmUmAmAmUmCmCmAmAm |
| | 234 | PlUmsUfsGmGmAmUfUmAfGfUmCmCmUmUfGmUfUmUmGmsGmsUm |

(continued)

| siRNA No. | SEQ ID NO: | Sequence Direction 5' - 3' |
|---|---|---|
| siPNd2-M2SP1 | 235 | AmCmCmAmAmAmCfAmAfGfGfAmCmUmAmAmUmCmCmAmAm |
| | 236 | P1UmsUfsGmGmAmUfUmAfGfUmCmCmUmUfGmUfUmUmGmGmUmsCmsUm |
| siPNd1-M3SP1 | 237 | CmsAmsAmAmCfAmAfGfGfAmCm UmAmAmUmCmCmAmAm |
| | 238 | P1UmsUfsGmGmAmUfUmAmGmUmCmCmUmUfGmUfUmUmGmsGmsUm |
| siPNd2-M3SP1 | 239 | AmCmCmAmAmAmCfAmAfGfGfAmCmUmAmAmUmCmCmAmAm |
| | 240 | P1UmsUfsGmGmAmUfUmAmGmUmCmCmUmUfGmUfUmUmGmGmUmsCmsUm |

Wherein, C, G, U, and A represent the base composition of the nucleotides; m represents that the nucleotide adjacent to the left side of the letter m is a methoxy modified nucleotide; f represents that the nucleotide adjacent to the left side of the letter f is a fluoro modified nucleotide; s represents that the two nucleotides adjacent to both sides of the letter are linked by a phosphorothioate linkage; PI represents that the nucleotide adjacent to the right side of PI is a 5'-phosphate nucleotide or a 5'-phosphate analogue modified nucleotide. In some embodiments, PI represents a specifically modified VP, Ps, or P, wherein the letter combination VP represents that the nucleotide adjacent to the right side of the letter combination VP is a vinyl phosphate modified nucleotide, the letter combination Ps represents that the nucleotide adjacent to the right side of the letter combination Ps is a phosphorothioate modified nucleotide, and P represents that the nucleotide adjacent to the right side of the letter P is a 5'- phosphate nucleotide.

In the siRNA or siRNA conjugate of the present disclosure, each pair of adjacent nucleotides is linked via a phosphodiester bond or a phosphorothioate diester bond. The non-bridging oxygen or sulfur atom in the phosphodiester bond or phosphorothioate diester bond has negative charges, and may be present in the form of hydroxy or sulfydryl. Moreover, the hydrogen ion in the hydroxy or sulfydryl may be partially or completely replaced with a cation. The cation may be any cation, such as one of a metal cation, an ammonium cation $NH_4^+$ or an organic ammonium cation. In order to increase solubility, in one embodiment, the cation is selected from one or more of alkali metal cation, an ammonium cation formed by a tertiary amine and a quaternary ammonium cation. The alkali metal ion may be $K^+$ and/or $Na^+$, and the cation formed by a tertiary amine may be ammonium cation formed by triethylamine and/or N,N-diisopropylethylamine. Thus, the siRNA or the siRNA conjugate of the present disclosure may be at least partially present in the form of salt. In one embodiment, the non-bridging oxygen or sulfur atom in the phosphodiester bond or phosphorothioate diester bond at least partly binds to sodium ion. The siRNA or the siRNA conjugate of the present disclosure is present or partially present in the form of sodium salt.

It is well-known to those skilled in the art that a modified nucleotide group may be introduced into the siRNA of the present disclosure by a nucleoside monomer with a corresponding modification. Methods for preparing a nucleoside monomer having a corresponding modification and for introducing a modified nucleotide group into an siRNA are also well-known to those skilled in the art. All modified nucleoside monomers may be either commercially available or prepared by known methods.

Preparation of the siRNA conjugate represented by Formula (308)

The siRNA conjugate represented by Formula (308) may be prepared by any appropriate synthetic routes.

In some embodiments, the siRNA conjugate represented by Formula (308) may be prepared by a method which comprises successively linking nucleoside monomers in 3' to 5' direction according to the nucleotide type and sequence of the sense strand and the antisense strand of the siRNA respectively, under a condition of phosphoramidite solid phase synthesis, wherein the linking of each nucleoside monomer includes a four-step reaction of deprotection, coupling, capping, and oxidation or sulfurization; isolating the sense strand and antisense strand of the siRNA; and annealing; wherein the siRNA is the siRNA of the present disclosure as described above;

moreover, wherein the method further comprises contacting the compound represented by Formula (321) with a nucleoside monomer or a nucleotide sequence linked to a solid phase support under a coupling reaction condition in the presence of a coupling reagent, thereby linking the compound represented by Formula (321) to the nucleotide sequence

via a coupling reaction. In the following text, the compound represented by Formula (321) is also referred to as a conjugating molecule.

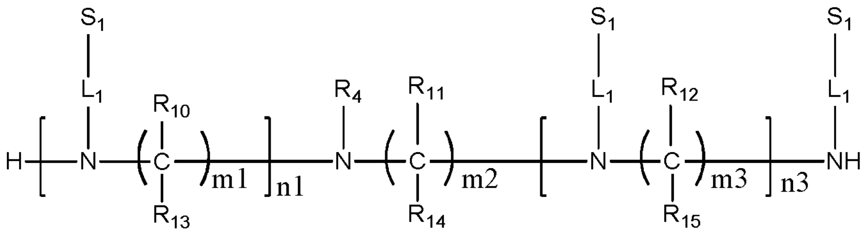

Formula (321)

wherein:

$R_4$ is a group capable of binding to the siRNA represented by Nu in the compound represented by formula (308). In some embodiments, $R_4$ is a group capable of binding to the siRNA represented by Nu via a covalent bond. In some embodiments, $R_4$ is a group capable of being conjugated to any functional group of siRNA represented by Nu via a phosphodiester bond by a reaction;

each $S_1$ is independently a group formed by substituting all the active hydroxyl in $M_1$ with YCOO-group, wherein each Y is independently one selected from the group consisting of methyl, trifluoromethyl, difluoromethyl, monofluoromethyl, trichloromethyl, dichloromethyl, monochloromethyl, ethyl, n-propyl, isopropyl, phenyl, halophenyl and alkylphenyl; in some embodiments, Y is methyl;

the definitions and options of nl, n3, m1, m2, m3, $R_{10}$, $R_{11}$, $R_{12}$, $R_{13}$, $R_{14}$, $R_{15}$, Li, and $M_1$ are respectively described as above.

$R_4$ is selected to achieve the linkage to the N atom on a nitrogenous backbone, and to provide an appropriate reaction site for the synthesis of the siRNA conjugate represented by Formula (308). In some embodiments, $R_4$ comprises a $R_2$ linking group or a protected $R_2$ linking group, and functional group(s) that can form the structure represented by Formula (A59) with an siRNA via reactions.

In some embodiments, $R_4$ comprises a first functional group that can react with a group on the siRNA represented by Nu or the nucleoside monomer to form a phosphite ester, and a second functional group that can react with hydroxy or amino to form a covalent bond, or comprises a solid phase support linked by a covalent bond. In some embodiments, the first functional group is a phosphoramidite, a hydroxy or a protected hydroxy. In some embodiments, the second functional group is a phosphoramidite, a carboxyl or a carboxylate salt. In some embodiments, the second functional group is a solid phase support linked to the rest of the molecule via a covalent bond, wherein the covalent bond is formed by hydroxy or amino. In some embodiments, the solid phase support is linked via a phosphoester bond, a carboxylate ester bond, or an amide bond. In some embodiments, the solid phase support is a resin.

In some embodiments, the first functional group comprises hydroxy, $-OR_k$ or a group represented by Formula (C3); and/or the second functional group has a structure represented by Formula (C1), (C2), (C3), (C1'), or (C3'):

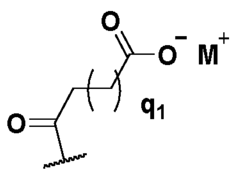,
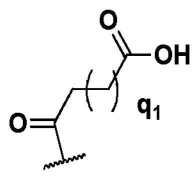,
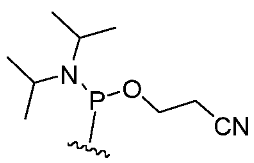,

(C1) (C2) (C3)

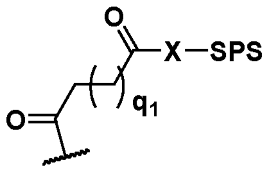, 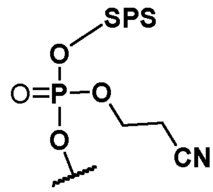,

(C1') (C3')

wherein $q_1$ is an integer of 1-4, X is O or NH, $M^+$ is a cation, $R_k$ is a hydroxyl protecting group, SPS represents a solid phase support, and represents the site where the group is covalently linked.

In some embodiments, the first functional group comprises a phosphoramidite group, such as the group represented by Formula (C3). The phosphoramidite group can form a phosphite ester with a hydroxy at any position (such as a 2'- or 3'- hydroxyl) on a nucleotide by a coupling reaction, and the phosphite ester can form a phosphodiester bond or a phosphorothioate bond represented by Formula (A59) via oxydation or sulfuration, so as to conjugate the conjugating molecule to siRNA. Here, even if the second functional group is not present, the compound represented by Formula (321) will still be able to be conjugated to the nucleotide, while not affecting the acquisition of the siRNA conjugate represented by Formula (308). Under such circumstances, after obtaining a sense strand or an antisense strand of the siRNA via a method such as phosphoramidite solid phase synthesis, the compound represented by Formula (321) reacts with the hydroxy on the nucleotide at the terminal of the nucleotide sequence, and a phosphodiester bond linkage or phosphorothioate linkage is formed in the following oxydization or sulfurization process, so as to conjugate the compound represented by Formula (321) to the siRNA.

In some embodiments, the first functional group comprises a protected hydroxy. In some embodiments, the second functional group comprises a group that can react with a solid phase support to provide a conjugating molecule comprising a solid phase support. In some embodiments, the second functional group comprises a carboxyl, a carboxylate salt or a phosphoramidite, such as represented by Formula (C1), (C2) or (C3). When the second functional group comprises a carboxyl or a carboxylate salt, the compound represented by Formula (321) can react via an esterification or an amidation with a hydroxy or an amino group on a solid phase support, such as a resin, to form a conjugating molecule comprising a solid phase support linked via a carboxylate ester bond. When the second functional group comprises a phosphoramidite functional group, the compound represented by Formula (321) can couple with a hydroxy group on a universal solid phase support, such as a resin, and form a conjugating molecule comprising a solid phase support linked via a phosphodiester bond by oxidation. Subsequently, starting from the above product linked to a solid phase support, the nucleoside monomers are successively linked according to the phosphoramidite solid phase synthesis method, so as to obtain a sense strand or an antisense strand of the siRNA linked to a conjugating group. In the process of phosphoramidite solid phase synthesis, the first functional group is deprotected, followed by coupled with a phosphoramidite group on a nucleoside monomer under a coupling reaction condition.

In some embodiments, the first functional group comprises a hydroxy or a protected hydroxy group; the second functional group comprises a solid phase support linked via a carboxylate ester bond, an amide bond, or a phosphodiester bond, as represented by Formula (C1') or (C3'). Here, starting from the compound represented by Formula (321) instead of a solid phase support, the nucleoside monomers are successively linked according to the phosphoramidite solid phase synthesis method, so as to obtain a sense strand or an antisense strand of the siRNA linked to a conjugating group.

In some embodiments, the carboxylate salt may be represented by $-COO^-M^+$, wherein $M^+$ is a cation such as one of a metal cation, an ammonium cation $NH4^+$ and an organic ammonium cation. In one embodiment, the metal cation may be an alkali metal cation, such as $K^+$ or $Na^+$. In order to increase solubility and facilitate the reaction, in some embodiments, the organic ammonium cation is an ammonium cation formed by a tertiary amine or a quaternary ammonium cation, such as an ammonium cation formed by triethylamine or N,N-diisopropylethylamine. In some embodiments, the carboxylate salt is a triethylamine carboxylate salt or an N,N-diisopropylethylamine carboxylate salt.

In some embodiments, $R_4$ has a structure represented by Formula (B9), (B10), (B9'), (B10'), (B11), (B12), (B11') or (B12'):

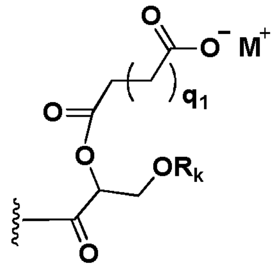 , 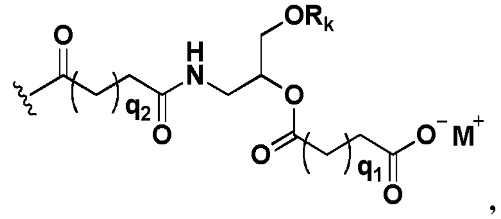 ,

(B9) (B10)

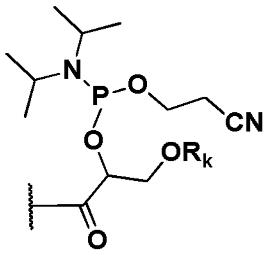 , 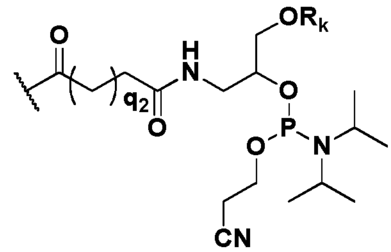 ,

(B9') (B10')

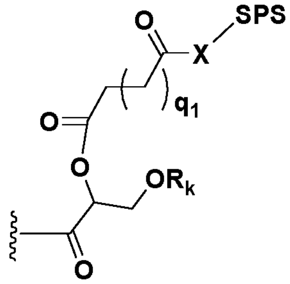 , 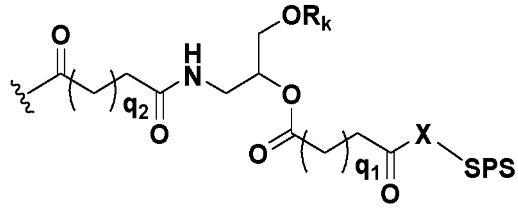 ,

(B11) (B12)

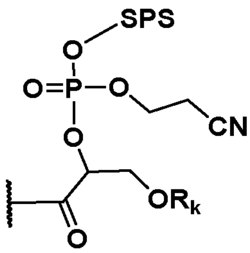 , 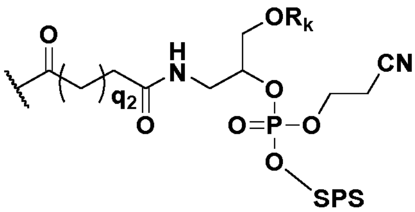 ,

(B11') (B12')

wherein $q_1$ is an integer of 1-4, $q_2$ is an integer of 1-10, X is O or NH, $M^+$ is a cation, $R_k$ is a hydroxy protecting group, SPS represents a solid phase support, and  represents the site where the group is covalently linked. In some embodiments, $q_1$ is 1 or 2. In some embodiments, $q_2$ is an integer of 1-5. In some embodiments, $R_4$ comprises a structure represented by Formula (B9) or (B10). In some embodiments, $R_4$ comprises a structure represented by Formula (B11) or (B12).

In some embodiments, $R_k$ is one or more of Tr (trityl), MMTr (4-methoxytrityl), DMTr (4,4'-dimethoxytrityl), and TMTr (4,4',4'-trimethoxytrityl). In some embodiments, $R_k$ may be DMTr, i.e. 4,4'-dimethoxytrityl.

The definition of $L_1$ is as described above.

In some embodiments, $L_1$ is used to link the $M_1$ targeting group to the N atom on the nitrogenous backbone, thereby providing liver targeting function for the siRNA conjugate represented by Formula (308). In some embodiments, $L_1$ comprises any one of A1-A26, or combination thereof.

According to the above description, those skilled in the art would readily understand that, compared with the phosphoramidite solid phase synthesis method well-known in the art, the siRNA conjugate represented by Formula (308) in which the conjugating molecule is linked to any possible position of the nucleotide sequence can be obtained through the above first functional group and an optional second functional group. For example, the conjugating molecule is linked to a terminal region of the nucleotide sequence, and the conjugating molecule is linked to a terminal of the nucleotide sequence. Correspondingly, unless otherwise indicated, in the following description regarding the preparation of the conjugate and/or conjugation molecule, when referring to the reactions such as "deprotection", "coupling", "capping", "oxidation" and "sulfurization", it should be understood that the reaction conditions and reagents involved in the phosphoramidite nucleic acid solid phase synthesis method well-known in the art would also apply to these reactions. Exemplary reaction conditions and reagents will be described in detail hereinafter.

In some embodiments, each $S_1$ is independently a $M_1$. In some embodiments, each $S_1$ is independently a group formed by protecting at least one active hydroxyl group in $M_1$ with a hydroxyl protecting group. In some embodiments, each $S_1$ is independently a group formed by protecting all active hydroxyl groups in $M_1$, if any, with hydroxyl protecting groups. In some embodiments, any hydroxyl protecting group known to those skilled in the art may be used to protect the active hydroxyl group in $M_1$. In some embodiments, the protected hydroxy can be represented by the formula YCOO-, wherein each Y is independently selected from the group consisting of $C_1$-$C_{10}$ alkyl and $C_6$-$C_{10}$ aryl, which is optionally substituted with one or more substituents selected from the group consisting of halo and $C_1$-$C_6$ alkyl. In some embodiments, each Y is independently selected from the group consisting of methyl, trifluoromethyl, difluoromethyl, monofluoromethyl, trichloromethyl, dichloromethyl, monochloromethyl, ethyl, n-propyl, isopropyl, phenyl, halophenyl, and $C_1$-$C_6$ alkylphenyl.

In some embodiments, each $S_1$ is independently selected from the group consisting of the groups of Formulae (A46)-(A54):

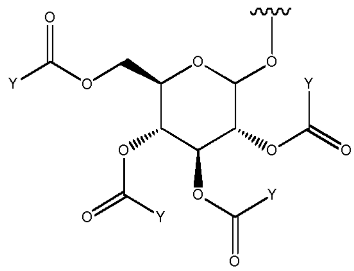

,

(A46)

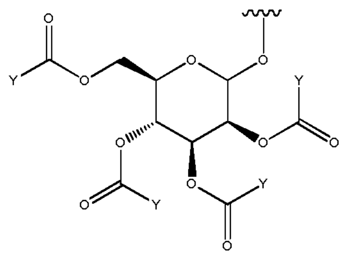

,

(A47)

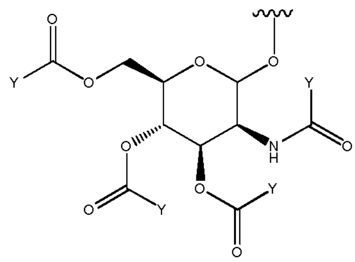

,

(A48)

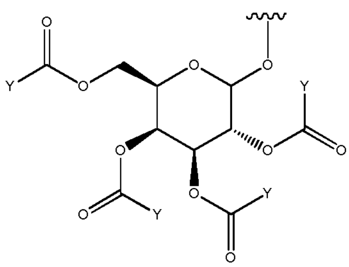

,

(A49)

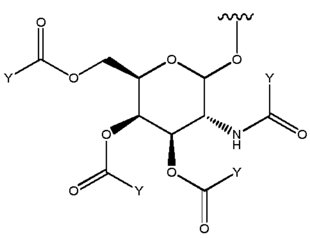

,

(A50)

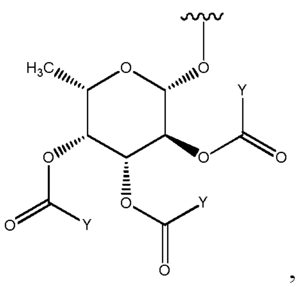

,

(A51)

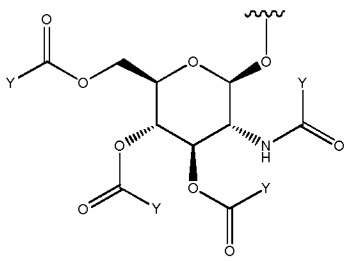, 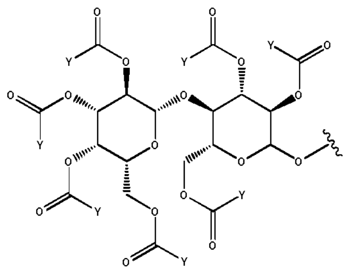, 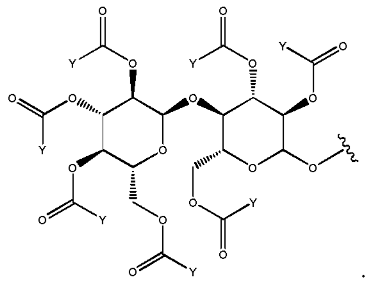.

(A52) (A53) (A54)

In some embodiments, Si is Formula (A49) or (A50).

In some embodiments, each Y is independently selected from one of methyl, trifluoromethyl, difluoromethyl, monofluoromethyl, trichloromethyl, dichloromethyl, monochloromethyl, ethyl, n-propyl, isopropyl, phenyl, halophenyl, and alkylphenyl. In some embodiments, Y is methyl.

As mentioned previously, the method for preparing the siRNA conjugate represented by Formula (308) further comprises the following steps: synthesizing the other strand of the siRNA (for example, when a sense strand of the siRNA linked to a conjugating molecule is synthesized in the above step, the method further comprises synthesizing an antisense strand of the siRNA according to the solid phase synthesis method, vice versa), isolating the sense strand and the antisense strand, and annealing. In particular, in the step of isolating, the solid phase support linked to the nucleotide sequence and/or a conjugating molecule is cleaved, and meanwhile the necessary protecting group is removed (in this case, each $S_1$ group in the compound represented by Formula (321) is converted to the corresponding $M_1$ targeting group), to afford a sense strand (or an antisense strand) of the siRNA linked to a conjugating molecule and the corresponding antisense strand (or sense strand), wherein the sense strand and the antisense strand are annealed to form a double-strand RNA structure, thereby affording the siRNA conjugate represented by Formula (308).

In some embodiments, the method for preparing the siRNA conjugate represented by Formula (308) comprises the following steps: contacting the compound represented by Formula (321) with the first nucleoside monomer at 3' terminal of the sense strand or the antisense strand under a coupling reaction condition in the presence of a coupling reagent, thereby linking the compound represented by Formula (321) to the first nucleotide in the sequence; successively linking nucleoside monomers in 3' to 5' direction to synthesize the sense strand or antisense strand of the siRNA under the phosphoramidite solid phase synthesis conditions according to the desired kind and sequence of nucleotides in the sense strand or antisense strand, wherein, the compound represented by Formula (321) is a compound in which $R_4$ comprises a first functional group comprising a protected hydroxy and a second functional group having a structure represented by Formula (C1') or (C3'), and the compound represented by (321) is deprotected before being linked to the first nucleoside monomer, and the linking of each nucleoside monomer comprising a four-step reaction of deprotection, coupling, capping, and oxidation or sulfurization, thus obtaining the sense strand or antisense strand of nucleic acid linked to a conjugating group; successively linking nucleoside monomers in 3' to 5' direction to synthesize the antisense strand or sense strand of the nucleic acid under the phosphoramidite solid phase synthesis conditions according to the kind and sequence of nucleotides in the antisense strand or sense strand, wherein the linking of each nucleoside monomer includes a four-step reaction of deprotection, coupling, capping, and oxidation or sulfurization; removing the protecting groups and cleaving the solid phase support; obtaining the sense strand and the antisense strand via isolation and purification; and annealing.

In some embodiments, the method for preparing the siRNA conjugate represented by Formula (308) comprises the following steps: successively linking nucleoside monomers in 3' to 5'direction to synthesize the sense strand and antisense strand according to the kind and sequence of nucleotides in the sense strand or antisense strand of the double-strand siRNA, wherein the linking of each nucleoside monomer includes a four-step reaction of deprotection, coupling, capping, and oxidation or sulfurization, to obtain the sense strand linked to the solid phase support and the antisense strand linked to the solid phase support; contacting the compound represented by Formula (321) with the sense strand linked to the solid phase support or the antisense strand linked to the solid phase support under a coupling reaction condition in the presence of a coupling reagent, so as to link the compound represented by Formula (321) to the sense strand or antisense strand, wherein the compound represented by Formula (321) is a compound in which $R_4$ comprises a first functional group that is a phosphoramidite group; removing the protecting group and cleaving the solid phase support; obtaining the sense strand or the antisense strand of the siRNA via isolation and purification; and annealing, wherein the sense strand or antisense strand of the siRNA is linked to a conjugating group.

In some embodiments, the P atom in the Formula (A59) is linked to the 3' terminal of the sense strand in the siRNA, and the method for preparing the siRNA conjugate as represented by Formula (308) comprises:

(1) removing the hydroxyl protecting group $R_k$ in the above compound represented by Formula (321) (wherein, the compound represented by Formula (321) is a compound wherein $R_4$ comprises a first functional group comprising a protected hydroxyl $OR_k$, and a second functional group having a structure represented by Formulas (C1') or (C3')); contacting the product resulting from deprotection with a nucleoside monomer to obtain a nucleoside monomer linked to a solid phase support via a conjugating molecule under the coupling reaction condition in the presence of a coupling reagent;

(2) synthesizing a sense strand of the siRNA in 3' to 5' direction by a phosphoramidite solid phase synthesis method, starting from the nucleoside monomer linked to a solid phase support via the conjugating molecule; and

(3) synthesizing an antisense strand of the siRNA by a phosphoramidite solid phase synthesis method; and

(4) isolating the sense strand and the antisense strand of the siRNA and annealing the same to obtain the siRNA conjugate represented by the Formula (308).

wherein in step (1), the method for removing the protecting group $R_k$ in the compound represented by Formula (321) comprises contacting the compound represented by Formula (321) with a deprotection agent under a deprotection condition. The deprotection condition comprises a temperature of 0-50°C, and in some embodiments of 15-35°C, and a reaction time of 30-300 seconds, and in some embodiments of 50-150 seconds. The deprotection agent may be selected from one or more of trifluoroacetic acid, trichloroacetic acid, dichloroacetic acid, and monochloroacetic acid, and in some embodiments is dichloroacetic acid. The molar ratio of the deprotection agent to the compound represented by Formula (321) is 10:1 to 1000:1, and in some embodiments is 50:1 to 500:1.

The coupling reaction condition and the coupling agent may be any conditions and agents appropriate for the above coupling reaction. In some embodiments, the same condition and reagent as the coupling reaction in the solid phase synthesis method employed can be used.

In some embodiments, the coupling reaction condition comprises a reaction temperature of 0-50°C, and in some embodiments of 15-35°C. The molar ratio of the compound represented by Formula (321) to the nucleoside monomer may be 1:1 to 1:50, and in some embodiments is 1:2 to 1:5. The molar ratio of the compound represented by Formula (321) to the coupling agent may be 1:1 to 1:50, and in some embodiments is 1:3 to 1:10. The reaction time may be 200-3000 seconds, and in some embodiments is 500-1500 seconds. The coupling agent may be selected from one or more of 1H-tetrazole, 5-ethylthio-1H-tetrazole and 5-benzylthio-lH-tetrazole, and in some embodiments is 5-ethylthio-lH-tetrazole. The coupling reaction may be performed in an organic solvent. The organic solvent may be selected from one or more of anhydrous acetonitrile, anhydrous DMF and anhydrous dichloromethane, and in some embodiments is anhydrous acetonitrile. The amount of the organic solvent is 3-50 L/mol, and in some embodiments, 5-20 L/mol with respect to the compound represented by Formula (321).

In step (2), a sense strand SS of the siRNA conjugate is synthesized in 3' to 5' direction by the phosphoramidite nucleic acid solid phase synthesis method, starting from the nucleoside monomer linked to a solid phase support via a conjugating molecule prepared in the above steps. In this case, the conjugating group is linked to the 3' terminal of the resulting sense strand.

Other conditions for solid phase synthesis described in steps (2) and (3), including the deprotection condition for the nucleoside monomer, type and amount of the deprotection agent, the coupling reaction condition, type and amount of the coupling agent, the capping reaction condition, type and amount of the capping agent, the oxidation reaction condition, type and amount of the oxidation agent, the sulfuration reaction condition, and type and amount of the sulfuration agent, are various agents, amounts, and conditions conventionally used in the art.

In some embodiments, for example, the solid phase synthesis described in steps (2) and (3) may be performed by using the following conditions:

The deprotection condition for the nucleoside monomer comprises a temperature of 0-50°C, and in some embodiments of 15-35°C, and a reaction time of 30-300 seconds, and in some embodiments of 50-150 seconds. The deprotection agent may be selected from one or more of trifluoroacetic acid, trichloroacetic acid, dichloroacetic acid, and monochloroacetic acid, and in some embodiments is dichloroacetic acid. The molar ratio of the deprotection agent to the protecting group 4,4'-dimethoxytrityl on the solid phase support may be 2:1 to 100:1, and in some embodiments is 3:1 to 50:1.

The coupling reaction condition comprises a temperature of 0-50°C, and in some embodiments of 15-35°C. The molar ratio of the nucleic acid sequence linked to the solid phase support to the nucleoside monomer may be 1:1 to 1:50, and in some embodiments is 1:5 to 1:15. The molar ratio of the nucleic acid sequence linked to the solid phase support to the coupling agent is 1:1 to 1:100, and in some embodiments is 1:50 to 1:80. The reaction time and the coupling agent are selected as above.

The capping reaction condition comprises a temperature of 0-50°C, and in some embodiments of 15-35°C, and a reaction time of 5-500 seconds, and in some embodiments of 10-100 seconds. The capping agent is selected as

above. The molar ratio of the total amount of the capping agent to the nucleic acid sequence linked to the solid phase support is 1:100 to 100:1, and in some embodiments is 1:10 to 10:1. In the case where equimolar acetic anhydride and N-methylimidazole are used as a capping agent, the molar ratio of acetic anhydride, N-methylimidazole, and the nucleic acid sequence linked to the solid phase support may be 1:1:10-10:10:1, and in some embodiments is 1:1:2-2:2:1.

The oxidation reaction condition comprises a temperature of 0-50°C, and in some embodiments of 15-35°C, and a reaction time of 1-100 seconds, and in some embodiments of 5-50 seconds. In some embodiments, the oxidation agent is iodine (and in some embodiments provided as iodine water). The molar ratio of the oxidation agent to the nucleic acid sequence linked to the solid phase support in the coupling step may be 1:1 to 100:1, and in some embodiments is 5:1 to 50:1. In some embodiments, the oxidation reaction is performed in a mixed solvent of tetrahydrofuran: water: pyridine = 3:1:1 - 1:1:3. The sulfuration reaction condition comprises a temperature of 0 - 50°C, and in some embodiments of 15 - 35°C, and a reaction time of 50-2000 seconds, and in some embodiments of 100-1000 seconds. In some embodiments, the sulfuration agent is xanthane hydride. The molar ratio of the sulfuration agent to the nucleic acid sequence linked to the solid phase support in the coupling step is 10:1 to 1000:1, and in some embodiments is 10:1 to 500:1. In some embodiments, the sulfuration reaction is performed in a mixed solvent of acetonitrile: pyridine = 1:3 - 3:1.

The method further comprises isolating the sense strand and the antisense strand of the siRNA after linking all nucleoside monomers and before the annealing. Methods for isolation are well known to those skilled in the art and generally comprise cleaving the synthesized nucleotide sequence from the solid phase support, removing the protecting groups on the bases, phosphate groups and ligands, purifying and desalting.

The synthesized nucleotide sequence may be cleaved from the solid phase support, and the protecting groups on the bases, phosphate groups and ligands are removed, according to conventional cleavage and deprotection methods in the synthesis of siRNA. For example, the resulting nucleotide sequence linked to the solid phase support is contacted with concentrated aqueous ammonia; during deprotection, the protecting group YCOO- in groups A46-A54 is converted to a hydroxyl group, thus the $S_1$ groups are converted to corresponding $M_1$ groups, providing the siRNA conjugate represented by Formula (308); wherein, the concentrated aqueous ammonia may be aqueous ammonia of a concentration of 25-30% by weight. The amount of the concentrated aqueous ammonia may be 0.2 ml/μmol-0.8 ml/(μmol with respect to the target siRNA sequence.

When there is at least one 2'-TBDMS protection on the synthesized nucleotide sequence, the method further comprises contacting the nucleotide sequence removed from the solid phase support with triethylamine trihydrofluoride to remove the 2'-TBDMS protection. Here, the corresponding nucleoside in the resulting target siRNA sequence has a free 2'-hydroxy. The amount of pure triethylamine trihydrofluoride with respect to the target siRNA sequence may be 0.4 ml/μmol-1.0 ml/μmol. As such, the siRNA conjugate represented by Formula (308) may be obtained.

Methods for purification and desalination are well known to those skilled in the art. For example, nucleic acid purification may be performed by using a preparative ion chromatography purification column with a gradient elution of NaBr or NaCl; after collection and combination of the product, a reversed phase chromatography purification column may be used for desalting.

In the resulting siRNA conjugate represented by Formula (308), the non-bridging oxygen atom or sulfur atom in the phosphodiester bond or phosphorothioate diester bond between the nucleotides substantially binds to a sodium ion, and the siRNA conjugate represented by Formula (308) is substantially present in the form of a sodium salt. Other forms of the siRNA conjugate represented by Formula (308) may be obtained by using a well-known ion exchange method in which said sodium ion is replaced with a hydrogen ion and/or another cation. The cation ion is described as above.

During synthesis, the purity and molecular weight of the nucleic acid sequence may be determined at any time, in order to better control the synthesis quality. Such determination methods are well known to those skilled in the art. For example, the purity of the nucleic acid may be determined by ion exchange chromatography, and the molecular weight may be determined by liquid chromatography-mass spectrometry (LC-MS).

Methods for annealing are also well known to those skilled in the art. For example, the synthesized sense strand (S strand) and antisense strand (AS strand) may be simply mixed in water for injection in an equimolar ratio, heated to 70-95 °C, and then cooled at room temperature to form a double stranded structure via a hydrogen bond. As such, the siRNA conjugate represented by formula (308) is thus obtained.

After obtaining the conjugate, in some embodiments, the siRNA conjugate represented by formula (308) thus synthesized may also be characterized by using a method such as LC-MS by the means such as molecular weight detection, to confirm that the synthesized siRNA conjugate is the siRNA conjugate represented by formula (308) as a designed target and the synthesized siRNA sequence is the desired siRNA sequence, for example, is one of the sequences listed in Tables 1a-1d.

The compound represented by Formula (321) may be prepared by the following method comprising: contacting a compound represented by Formula (313) with a cyclic anhydride under esterification reaction condition in the presence of a base and an esterification catalyst in an organic solvent; ion exchanging and isolating the compound represented by Formula (321):

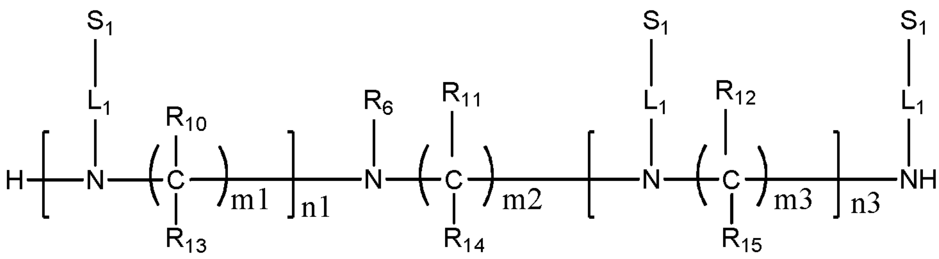

Formula (313)

wherein the definitions and options of n1, n3, m1, m2, m3, $R_{10}$, $R_{11}$, $R_{12}$, $R_{13}$, $R_{14}$, $R_{15}$, Li, $S_1$ are respectively described as above;

$R_6$ is a group for providing $R_4$ of Formula (321). In some embodiments, $R_6$ has a structure represented by Formula (A61):

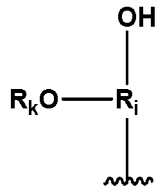
(A61),

wherein, $R_i$ is any group capable of linking to the N atom on the nitrogenous backbone, linking to $R_kO$ and linking to a free hydroxy group; $R_k$ is a hydroxy protecting group. In this case, a compound represented by Formula (321) is obtained, where $R_4$ comprises a first functional group as a hydroxy protecting group and a second functional group comprising a structure represented by Formula (C1) or (C2).

The esterification reaction condition includes a reaction temperature of 0-100°C and a reaction time of 8-48 hours. In some embodiments, the esterification reaction condition includes a reaction temperature of 10-40°C and a reaction time of 20-30 hours.

In some embodiments, the organic solvent comprises one or more of an epoxy solvent, an ether solvent, an haloalkane solvent, dimethyl sulfoxide, N,N-dimethylformamide, and N,N-diisopropylethylamine. In some embodiments, the epoxy solvent is dioxane and/or tetrahydrofuran, the ether solvent is diethyl ether and/or methyl tert-butyl ether, and the haloalkane solvent is one or more of dichloromethane, trichloromethane and 1,2-dichloroethane. In some embodiments, the organic solvent is dichloromethane. The amount of the organic solvent is 3-50 L/mol, and in some embodiments, 5-20 L/mol with respect to the compound represented by Formula (313).

In some embodiments, the cyclic anhydride is one of succinic anhydride, glutaric anhydride, adipic anhydride or pimelic anhydride, and in some embodiments is succinic anhydride. The molar ratio of the cyclic anhydride to the compound represented by Formula (313) is 1:1 to 10:1, and in some embodiments is 2:1 to 5:1.

The esterification catalyst may be any catalyst capable of catalyzing the esterification, such as 4-dimethylaminopyridine. The molar ratio of the catalyst to the compound represented by Formula (313) is 1:1 to 10:1, and in some embodiments is 2:1 to 5:1.

In some embodiments, the base may be any inorganic base, organic base or combination thereof. Considering the solubility as well as the product stability, the base may be, for example, a tertiary amine. In some embodiments, the tertiary amine is triethylamine or N,N-diisopropylethylamine. The molar ratio of the tertiary amine to the compound represented by Formula (313) is 1:1 to 20:1, and in some embodiments is 3:1 to 10:1.

The ion exchange serves to convert the compound represented by Formula (321) to a desired form of carboxylic acid or salt thereof. The method of ion exchange is well known to those skilled in the art, and a conjugating molecule with $M^+$ cation may be obtained by using suitable ion exchange solution and ion exchange condition, which is not described here in detail. In some embodiments, a triethylamine phosphate solution is employed in the ion exchange reaction. The concentration of the triethylamine phosphate solution is 0.2-0.8 M, and in some embodiments is 0.4-0.6 M. In some embodiments, the amount of the triethylamine phosphate solution is 3-6 L/mol, and in further embodiment is 4-5 L/mol with respect to the compound represented by Formula (313).

The compound represented by Formula (321) may be isolated from the reaction mixture using any suitable isolation method. In some embodiments, the compound represented by Formula (321) may be isolated by removal of solvent via evaporation followed by chromatography, for example, using the following two chromatographic conditions

for the isolation: (1) normal phase purification of silica gel: 200-300 mesh silica gel filler, with gradient elution of 1 wt%o triethylamine-containing dichloromethane: methanol = 100:18-100:20; or (2) reverse phase purification: C18 and C8 reverse phase filler, with gradient elution of methanol: acetonitrile = 0.1:1-1:0.1. In some embodiments, the solvent may be removed directly to obtain a crude product of the compound represented by Formula (321), which may be used directly in subsequent reactions.

In some embodiments, the method for preparing the compound represented by Formula (321) further comprises: further contacting the product obtained from the above ion exchange reaction with a solid phase support with an amino or hydroxy group under a condensation reaction condition in the presence of a condensing agent, a condensation catalyst and a tertiary amine in an organic solvent. In this case, a compound represented by Formula (321) is obtained, wherein $R_4$ comprises a first functional group containing a hydroxy protecting group and a second functional group having a strucuture represented by Formula (C1').

The solid phase support is one of the supports used in solid phase synthesis of siRNA, some of which are well known to those skilled in the art. For example, the solid phase support may be selected from solid phase supports having an active hydroxy or amino functional group. In some embodiments, the solid phase support is an amino or hydroxy resin. In some embodiments, the amino or hydroxy resin has a particle size of 100-400 mesh, and surface amino or hydroxy loading of 0.2-0.5 mmol/g. The amount ratio of the compound represented by Formula (321) to the solid phase support is 10-400 μmol compound per gram of the solid phase support (μmol/g). In some embodiments, the amount ratio of the compound represented by Formula (321) to the solid phase support is 50-200 μmol/g.

The organic solvent may be any suitable solvent or mixture of solvents known to those skilled in the art. In some embodiments, the organic solvent is one or more of acetonitrile, an epoxy solvent, an ether solvent, an haloalkane solvent, dimethyl sulfoxide, N,N-dimethylformamide, and N,N-diisopropylethylamine. In some embodiments, the epoxy solvent is dioxane and/or tetrahydrofuran, the ether solvent is diethyl ether and/or methyl tert-butyl ether, the haloalkane solvent is one or more of dichloromethane, trichloromethane and 1,2-dichloroethane. In some embodiments, the organic solvent is acetonitrile. The amount of the organic solvent is 20-200 L/mol, in some embodiments is 50-100 L/mol with respect to the compound represented by Formula (321).

In some embodiments, the condensing agent may be benzotriazol-1-yl- oxytripyrrolidinophosphonium hexafluorophosphate (PyBop), 3-diethoxyphosphoryl-1,2,3-benzotriazol-4(3H)-one (DEPBT) and/or O-benzotriazol-tetramethyluronium hexafluorophosphate. In some embodiments, the condensing agent is O-benzotriazol-tetramethyluronium hexafluorophosphate. The molar ratio of the condensing agent to the compound represented by Formula (321) is 1:1 to 20:1, and in further embodiments, 1:1 to 5:1.

In some embodiments, the tertiary amine is triethylamine and/or N,N-diisopropylethylamine, and in some embodiments is N,N-diisopropylethylamine. The molar ratio of the tertiary amine to the compound represented by Formula (321) is 1:1 to 20:1, and in some embodiments is 1:1 to 5:1.

In some embodiments, the method for preparing the compound represented by Formula (321) further comprises: contacting the obtained condensation product with a capping reagent and an acylation catalyst under a capping reaction condition in an organic solvent, and isolating the compound represented by Formula (321). The capping reaction serves to remove any active functional groups that are not completely reacted, so as to avoid unnecessary by-products in subsequent reactions. The capping reaction condition comprises a reaction temperature of 0-50°C, and in some embodiments of 15-35°C, and a reaction time of 1-10 hours, and in some embodiments of 3-6 hours. The capping reagent may be a capping reagent used in solid phase synthesis of siRNA, which are well known to those skilled in the art.

In some embodiments, the capping reagent is composed of capping reagent 1 (cap1) and capping reagent 2 (cap2). The capping reagent 1 is N-methylimidazole, and in some embodiments provided as a mixed solution of N-methylimidazole in pyridine/acetonitrile, wherein the volume ratio of pyridine to acetonitrile is 1:10 to 1:1, and in some embodiments is 1:3 to 1:1. The ratio of the total volume of pyridine and acetonitrile to the volume of N-methylimidazole is 1:1 to 10:1, and in some embodiments is 3:1 to 7:1. The capping reagent 2 is acetic anhydride. In some embodiments, the capping reagent 2 is provided as a solution of acetic anhydride in acetonitrile, wherein the volume ratio of acetic anhydride to acetonitrile is 1:1 to 1:10, and in further embodiments is 1:2 to 1:6.

In some embodiments, the ratio of the volume of the mixed solution of N-methylimidazole in pyridine/acetonitrile to the mass of the compound represented by Formula (321) is 5 ml/g-50 ml/g, and in some embodiments is 15ml/g-30ml/g. The ratio of the volume of the solution of acetic anhydride in acetonitrile to the mass of the compound represented by Formula (321) is 0.5 ml/g-10 ml/g, and in some embodiments is 1 ml/g-5 ml/g.

In some embodiments, the capping reagent is equimolar acetic anhydride and N-methylimidazole. In some embodiments, the organic solvent is one or more of acetonitrile, an epoxy solvent, an ether solvent, an haloalkane solvent, dimethyl sulfoxide, N,N-dimethylformamide, and N,N-diisopropylethylamine. In some embodiments, the organic solvent is acetonitrile. In some embodiments, the amount of the organic solvent is 10-50 L/mol, and in some embodiments is 5-30 L/mol with respect to the compound represented by Formula (321).

In some embodiments, the acylation catalyst may be selected from any catalyst that can be used for esterification condensation or amidation condensation, such as alkaline heterocyclic compounds. In some embodiments, the acylation

catalyst is 4-dimethylaminopyridine. The ratio of the mass of the catalyst to the mass of the compound represented by Formula (321) is 0.001:1 to 1:1, and in some embodiments is 0.01:1 to 0.1:1.

In some embodiments, the compound represented by Formula (321) may be isolated from the reaction mixture by any suitable isolation methods. In some embodiments, the compound represented by Formula (321) may be obtained by thoroughly washing with an organic solvent and filtering to remove unreacted reactants, excess capping reagent and other impurities, wherein the organic solvent is selected from acetonitrile, dichloromethane, or methanol, and in some embodiments is acetonitrile.

In some embodiments, the method for preparing the conjugating molecule represented by Formula (321) comprises contacting a compound represented by Formula (313) with a phosphorodiamidite under a coupling reaction condition in the presence of a coupling agent in an organic solvent, and isolating the compound represented by Formula (321). In this case, a compound represented by Formula (321) is obtained, wherein $R_4$ comprises a first functional group comprising a hydroxy protecting group and a second functional group comprising a structure represented by Formula (C3).

In some embodiments, the coupling reaction condition comprises a reaction temperature of 0-50°C, such as 15-35°C. The molar ratio of the compound represented by Formula (313) to the phosphorodiamidite may be 1:1 to 1:50, such as 1:5 to 1:15. The molar ratio of the compound represented by Formula (313) to the coupling agent may be 1:1 to 1:100, such as 1:50 to 1:80. The reaction time may be 200-3000 seconds, such as 500-1500 seconds. The phosphorodiamidite may be, such as bis(biisopropylamino)(2-cyanoethoxy)phosphine, which may be commercially available or synthesized according to methods well-known in the art. The coupling agent is selected from one or more of 1H-tetrazole, 5-ethylthio-lH-tetrazole and 5-benzylthio-1H-tetrazole, such as 5-ethylthio-1H-tetrazole. The coupling reaction may be performed in an organic solvent. The organic solvent is selected from one or more of anhydrous acetonitrile, anhydrous DMF and anhydrous dichloromethane, for example, anhydrous acetonitrile. In some embodiments, the amount of the organic solvent is 3-50 L/mol, such as 5-20 L/mol with respect to the compound represented by Formula (313). By the coupling reaction, the hydroxy group in the compound represented by Formula (313) reacts with the phosphorodiamidite to form a phosphoramidite group. In some embodiments, the solvent may be removed directly to obtain a crude product of the compound represented by Formula (321), which may be used directly in subsequent reactions.

In some embodiments, the method for preparing the compound represented by Formula (321) further comprises the following steps: further contacting the isolated product with a solid phase support with hydroxy group(s) under a coupling reaction condition in the presence of a coupling agent in an organic solvent, followed by capping, oxidation, and isolation, to obtain the compound represented by Formula (321), wherein $R_4$ comprises a first functional group comprising a hydroxy protecting group and a second functional group comprising a structure represented by Formula (C3').

In some embodiments, the solid phase support is a solid phase support well-known in the art used in solid phase synthesis of nucleic acid, such as a deprotected universal solid phase support, which is commercially available (such as NittoPhase®HL UnyLinker™ 300 Oligonucleotide Synthesis Support, Kinovate Life Sciences, represented by Formula B80):

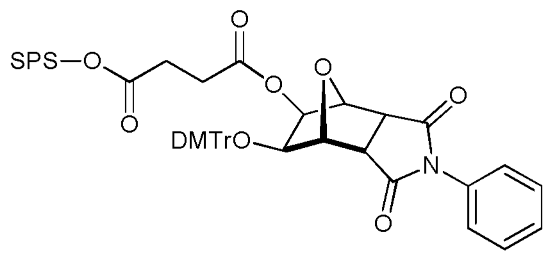

(B80).

A deprotection reaction is well known to those skilled in the art. In some embodiments, the deprotection condition comprises a temperature of 0-50°C, such as 15-35°C, and a reaction time of 30-300 seconds, such as 50-150 seconds. The deprotection agent may be selected from one or more of trifluoroacetic acid, trichloroacetic acid, dichloroacetic acid, and monochloroacetic acid, and in some embodiments is dichloroacetic acid. The molar ratio of the deprotection agent to the protecting group -DMTr (4,4'-dimethoxytrityl) on the solid phase support is 2:1 to 100:1, such as 3:1 to 50:1. By such deprotection, reactive free hydroxy groups are obtained on the surface of the solid phase support, for facilitating the subsequent coupling reaction.

The coupling reaction condition and the coupling agent may be selected as above. By such coupling reaction,

the free hydroxy group formed in the deprotection reaction reacts with the phosphoramidite group, so as to form a phosphite ester linkage.

In some embodiments, the capping reaction condition comprises a temperature of 0-50°C, such as 15-35°C, and a reaction time of 5-500 seconds, such as 10-100 seconds. The capping reaction is performed in the presence of the capping agent. The selection and the amount of the capping agent may be described above.

The oxidation reaction condition comprises a temperature of 0-50°C, such as 15-35°C, and a reaction time of 1-100 seconds, such as 5-50 seconds. The oxidation agent may be, for example, iodine (in some embodiments, provided as iodine water). In some embodiments, the molar ratio of the oxidation agent to the nucleic acid sequence linked to the solid phase support is 1:1 to 100:1, for example, 5:1 to 50:1. In some embodiments, the oxidation reaction is performed in a mixed solvent of tetrahydrofuran: water: pyridine = 3:1:1-1:1:3.

In some embodiments, $R_6$ is a group represented by Formula B7 or B8:

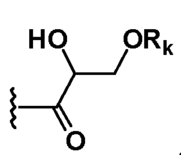
,
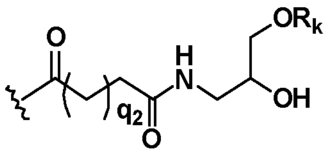
,

(B7) (B8)

wherein $q_2$ is defined as above.

In this case, the compound represented by Formula (313) may be obtained by the following preparation method comprising: contacting the compound represented by Formula (314) with a compound represented by Formula (A-1) or (A-2) under an amidation reaction condition in the presence of an condensing agent for amidation reaction and a tertiary amine, in an organic solvent, and followed by isolation:

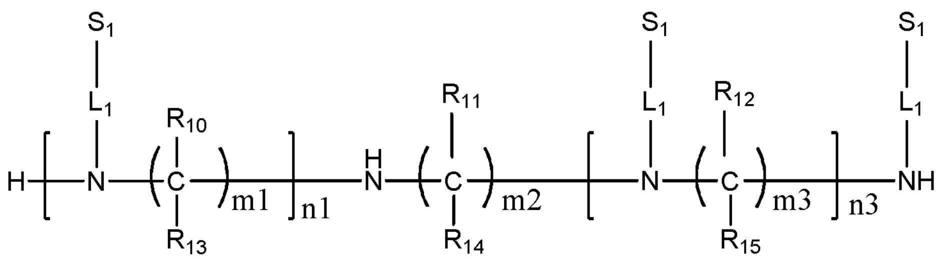

Formula (314)

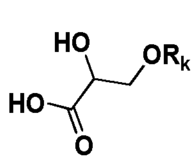
,
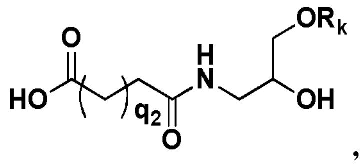
,

(A-1) (A-2)

wherein, the definitions and options of nl, n3, m1, m2, m3, $R_{10}$, $R_{11}$, $R_{12}$, $R_{13}$, $R_{14}$, $R_{15}$, Li, Si, $q_2$ and $R_k$ are respectively as described above.

The amidation reaction condition may comprise a reaction temperature of 0-100°C and a reaction time of 1-48 hours. In some embodiments, the amidation reaction condition is a reaction temperature of 10-40 °C and a reaction time of 2-16 hours.

In some embodiments, the organic solvent is one or more of an alcohol solvent, an epoxy solvent, an ether solvent, an haloalkane solvent, dimethyl sulfoxide, N,N-dimethylformamide, and N,N-diisopropylethylamine. In some embodiments, the alcohol solvent is one or more of methanol, ethanol and propanol, and in some embodiments is ethanol. In some embodiments, the epoxy solvent is dioxane and/or tetrahydrofuran. In some embodiments, the ether solvent is diethyl ether and/or methyl tert-butyl ether. In some embodiments, the haloalkane solvent is one or more of

dichloromethane, trichloromethane and 1,2-dichloroethane. In some embodiments, the organic solvent is dichloromethane. The amount of the organic solvent is 3-50 L/mol, and in further embodiments is 3-20 L/mol with respect to the compound represented by Formula (314).

In some embodiments, the condensing agent for amidation reaction is benzotriazol-1-yl-oxytripyrrolidinophosphonium hexafluorophosphate, 3-diethoxyphosphoiyl-1,2,3-benzotriazol-4(3H)-one, 4-(4,6-dimethoxytriazin-2-yl)-4-methylmorpholine hydrochloride, 2-ethoxy-1-ethoxycarbonyl-1,2-dihydroquinoline (EEDQ) or O-benzotriazol-tetramethyluronium hexafluorophosphate, and in further embodiments is 3-diethoxyphosphoryl-1,2,3-benzotriazol-4(3H)-one. The molar ratio of the condensing agent for amidation reaction to the compound represented by Formula (314) may be 1:1 to 10:1, and in some embodiments is 2.5:1 to 5:1.

In some embodiments, the tertiary amine is triethylamine or N,N-diisopropylethylamine, and in further embodiments is N,N-diisopropylethylamine. The molar ratio of the tertiary amine to the compound represented by Formula (314) is 3:1 to 20:1, and in some embodiments is 5:1 to 10:1.

In some embodiments, the compounds represented by Formulae (A-1) and (A-2) may be prepared by any suitable means. For example, when $R_k$ is a DMTr group, the compound represented by Formula (A-1) may be prepared by reacting calcium glycerate with DMTrCl. Similarly, the compound represented by Formula (A-2) may be prepared by firstly contacting 3-amino-1,2-propanediol with a cyclic anhydride which may have 4-13 carbon atoms, and in some embodiments 4-8 carbon atoms, followed by reacting with DMTrCl. It will be readily understood by those skilled in the art that the selection of different cyclic anhydride corresponds to different values for $q_2$ in the compound represented by Formula (A-2). For example, when the cyclic anhydride is succinic anhydride, $q_2$=1; when the cyclic anhydride is glutaric anhydride, $q_2$=2, and so on.

In some variations, the compound represented by Formula (313) can also be prepared by successively reacting the compound represented by Formula (314) with the cyclic anhydride, 3-amino-1,2-propanediol, and DMTrCl. It will be readily understood by those skilled in the art that these variations would not affect the structure and function(s) of the compound represented by Formula (313), and these variations are readily achieved by those skilled in the art on the basis of the above methods.

Similarly, the compound represented by Formula (313) may be isolated from the reaction mixture by any suitable isolation methods. In some embodiments, the compound represented by Formula (313) may be isolated by removal of solvent via evaporation followed by chromatography. For example, the following two sets of chromatographic conditions may be employed for isolation, (1) normal phase purification of silica gel: 200-300 mesh silica gel filler, with gradient elution of petroleum ether: ethyl acetate: dichloromethane: N,N-dimethylformamide = 1:1:1:0.5-1:1:1:0.6; and (2) reverse phase purification: C18 and C8 reverse phase fillers, with gradient elution of methanol: acetonitrile = 0.1:1-1:0.1. In some embodiments, the solvent may be removed directly to obtain a crude product of the compound represented by Formula (313), which may be directly used in subsequent reactions.

In some embodiment, the compound represented by Formula (314) may be obtained by a preparation method comprising contacting the compound represented by Formula (320) with the compound represented by Formula (316) under a condensation reaction condition in the presence of a condensing agent for amidation reaction and a tertiary amine in an organic solvent, and followed by isolation:

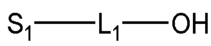

Formula (316)

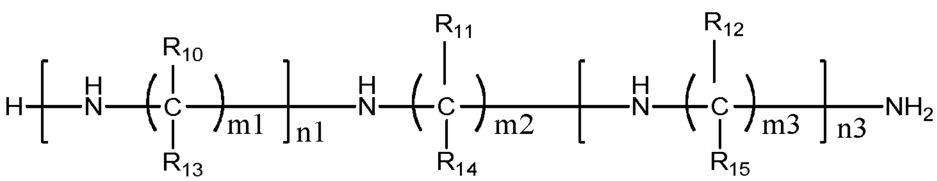

Formula (320)

wherein the definitions and options of n1, n3, m1, m2, m3, $R_{10}$, $R_{11}$, $R_{12}$, $R_{13}$, $R_{14}$, and $R_{15}$ are respectively as described above.

The compound represented by Formula (316) may be, such as, compounds disclosed in J. Am. Chem. Soc. 2014, 136, 16958-16961. Alternatively, the compound represented by Formula (316) may be prepared by those skilled in the art by various methods. For example, some compounds represented by Formula (316) may be prepared according to the method disclosed in Example 1 of US patent 8,106,022 B2, which is incorporated herein by reference in its entirety.

In some embodiments, the condensation reaction condition comprises a reaction temperature of 0-100 °C and a reaction time of 0.1-24 hours. In some embodiments, the reaction temperature is 10-40 °C and the reaction time is 0.5-16 hours.

Considering the structure of the desired product compound represented by Formula (314), the molar ratio of the compound represented by Formula (316) to the compound represented by Formula (320) should be determined based on the sum of n1 and n3 in Formula (320). In some embodiments, for example, when n1+n3=3, the molar ratio of the compound represented by Formula (316) to the compound represented by Formula (320) may be 3: 1 to 3.5: 1, and in some embodiments, 3.01: 1 to 3.15: 1, in order to ensure complete and not excessive reaction.

In some embodiments, the organic solvent is one or more of acetonitrile, an epoxy solvent, an ether solvent, an haloalkane solvent, dimethyl sulfoxide, N,N-dimethylformamide and N,N-diisopropylethylamine. In some embodiments, the epoxy solvent is dioxane and/or tetrahydrofuran. In some embodiments, the ether solvent is diethyl ether and/or methyl tert-butyl ether. In some embodiments, the haloalkane solvent is one or more of dichloromethane, trichloromethane and 1,2-dichloroethane. In some embodiments, the organic solvent is dichloromethane. The amount of the organic solvent is 3-50 L/mol, and in some embodiments is 5-20 L/mol with respect to the compound represented by Formula (320).

In some embodiments, the condensing agent for amidation reaction is one or more of benzotriazol-1-yl-oxytripyrrolidinophosphonium hexafluorophosphate, 3-diethoxyphosphoryl-1,2,3-benzotriazol-4(3H)-one (DEPBT), O-benzotriazol-tetramethyluronium hexafluorophosphate, 4-(4,6-dimethoxytriazin-2-yl)-4-methylmorpholine hydrochloride or 1-hydroxybenzotriazole, and in further embodiments is a mixture of benzotriazol-1-yl-oxytripyrrolidinophosphonium hexafluorophosphate and 1-hydroxybenzotriazole, wherein benzotriazol-1-yl-oxytripyrrolidinophosphonium hexafluorophosphate and 1-hydroxybenzotriazole are equimolar. The molar ratio of the total condensing agent for amidation reaction to the compound represented by Formula (316) may be 1:1 to 3:1, and in some embodiments is 1.05:1 to 1.5:1.

The tertiary amine may be N-methylmorpholine, triethylamine or N,N-diisopropylethylamine, and in some embodiments is N-methylmorpholine. The molar ratio of the tertiary amine to the compound represented by Formula (316) may be 2:1 to 10:1, and in some embodiments is 2:1 to 5:1.

Similarly, the compound represented by Formula (314) may be isolated from the reaction mixture by any suitable isolation method. In some embodiments, the compound represented by Formula (314) may be isolated by removal of solvent via evaporation followed by chromatography, for example, using the following two sets of chromatographic conditions for isolation: (1) normal phase purification of silica gel: 200-300 mesh silica gel filler, with gradient elution of dichloromethane: methanol = 100:5-100:7; and (2) reverse phase purification: C18 and C8 reverse phase fillers, with gradient elution of methanol: acetonitrile = 0.1:1-1:0.1. In some embodiments, the solvent may be removed directly to obtain a crude product of the compound represented by Formula (314), which may be used directly in subsequent reactions.

The compound represented by Formula (320) may be commercially available, or obtained by those skilled in the art via known methods. For example, in the case that m1=m2=m3=3, n1=1, n3=2, and each of Rio, $R_{11}$, $R_{12}$, $R_{13}$, $R_{14}$ and $R_{15}$ is H, the compound represented by Formula (320) is commercially available from Alfa Aesar Inc.

The siRNA conjugate of the present disclosure may also be used in combination with other pharmaceutically acceptable excipients, which may be one or more of various formulations or compounds conventionally employed in the art. For details, please refer to the above description of the pharmaceutical composition of the present disclosure.

## Use of the siRNA, the pharmaceutical composition comprising the siRNA and the siRNA conjugate of the present disclosure

In some embodiments, the present disclosure provides the use of the siRNA and/or pharmaceutical composition and/or siRNA conjugate of the present disclosure in the manufacture of a medicament for treating and/or preventing abnormal uric acid metabolism or diseases or physiological conditions caused by abnormal uric acid metabolism.

In some embodiments, the present disclosure provides a method for preventing and/or treating abnormal uric acid metabolism or diseases or physiological conditions caused by abnormal uric acid metabolism, comprising administering an effective amount of the siRNA and/or pharmaceutical composition and/or siRNA conjugate of the present disclosure to a subject in need thereof.

By administering the siRNA active ingredient of the present disclosure to a subject in need thereof, the purpose of preventing and/or treating abnormal uric acid metabolism or diseases or physiological conditions caused by abnormal uric acid metabolism may be achieved by a mechanism of RNA interference. Therefore, the siRNA and/or pharmaceutical composition and/or siRNA conjugate of the present disclosure may be used to prevent and/or treat abnormal uric acid metabolism or diseases or physiological conditions caused by abnormal uric acid metabolism, or to prepare a medicament for preventing and/or treating abnormal uric acid metabolism or diseases or physiological conditions caused by abnormal uric acid metabolism.

In some embodiments, the disease or physiological condition caused by the abnormal uric acid metabolism

refers to hyperuricemia or gout, which is generally manifested as increased uric acid level in the blood and severe joint pain, inconvenience in movement and other symptoms as a direct result of the increased uric acid level in the blood.

As used herein, the term "administration/administrate" refers to placing the siRNA, pharmaceutical composition and/or siRNA conjugate of the present disclosure into a subject' body by a method or route where at least, in part, the siRNA or pharmaceutical composition and/or the siRNA conjugate of the present disclosure is located at a desired site to achieve a desired effect. The administration routes suitable for the method of the present disclosure include topical administration and systemic administration. In general, topical administration results in the delivery of more siRNA conjugate to a particular site as compared to the systemic circulation of the subject; while systemic administration results in the delivery of the siRNA, the pharmaceutical composition and/or the siRNA conjugate of the present disclosure to the systemic circulation of the subject. Considering that the present disclosure is intended to provide means for the prevention and/or treatment of gout, in some embodiments, an administration mode that can deliver drugs to the liver is employed.

Any suitable route known in the art may be used for administration to the subject, including, but is not limited to, oral or parenteral route, including intravenous administration, intramuscular administration, subcutaneous administration, transdermal administration, endotracheal administration (aerosol), pulmonary administration, nasal administration, rectal administration and topical administration (including buccal administration and sublingual administration). The frequency of administration may be once or more times daily, weekly, biweekly, triweekly, monthly, bimonthly, trimonthly, half-yearly, or yearly.

The used dosage of the siRNA, the pharmaceutical composition or the siRNA conjugate of the present disclosure may be a conventional dose in the art, which may be determined according to various parameters, especially the age, weight and gender of the subject. Toxicity and therapeutic efficacy can be determined by standard pharmaceutical procedures in cell cultures or experimental animals, e.g., by determining $LD_{50}$ (the dose that is lethal to 50% of the population) and $ED_{50}$ (the dose that can cause 50% of the maximum response intensity in the quantitative response, or the dose that can cause a positive response in 50% of the experimental subjects in the qualitative response). The dose range for human use may be derived based on the data obtained from cell culture assays and animal studies.

When administrating the siRNA, the pharmaceutical composition and/or the siRNA conjugate of the present disclosure is administered, for example, to C57BL/6J mice having a weight of 18 to 25 g, either male or female, with an age of 6 to 12 weeks, on the basis of the amount of the siRNA: (i) for an siRNA conjugate, the dosage of siRNA thereof may be from 0.001 to 100 mg/kg body weight, in some embodiments from 0.01 to 50 mg/kg body weight, in some embodiments from 0.05 to 20 mg/kg body weight, in further embodiments from 0.1 to 15 mg/kg body weight, and in further embodiments from 0.1 to 10 mg/kg body weight; (ii) for a pharmaceutical composition formed by an siRNA and a pharmaceutically acceptable support, the dosage of siRNA thereof may be from 0.001 to 50 mg/kg body weight, in some embodiments from 0.01 to 10 mg/kg body weight, in some embodiments from 0.05 to 5 mg/kg body weight, and in some embodiments from 0.1 to 3 mg/kg body weight.

In some embodiments, the present disclosure provides a method of inhibiting the expression of PNP gene in hepatocytes, which comprises contacting an effective amount of the siRNA and/or the pharmaceutical composition and/or the siRNA conjugate of the present disclosure with the hepatocytes, and introducing the siRNA and/or the pharmaceutical composition and/or the siRNA conjugate of the present disclosure into the hepatocytes, so as to achieve the purpose of inhibiting the expression of PNP gene in hepatocytes through the mechanism of RNA interference. The hepatocytes may be selected from hepatoma cell lines such as SMMC-7721, HepG2 or Huh7, or isolated liver primary cells. In some embodiments, the hepatocytes are SMMC-7721 hepatoma cells.

In the case where the expression of PNP gene in a cell is inhibited by using the method provided by the present disclosure, the amount of siRNA in the provided modified siRNA, the pharmaceutical composition and/or the siRNA conjugate is generally an amount sufficient to reduce the expression of the target gene and result in an extracellular concentration of 1 pM to 1 μM, or 0.01 nM to 100 nM, or 0.05 nM to 50 nM, or 0.05 nM to about 5 nM on the surface of the target cells. The amount required to achieve this local concentration will vary with various factors, including the delivery method, the delivery site, the number of cell layers between the delivery site and the target cells or tissue, the delivery route (topical or systemic), etc. The concentration at the delivery site may be significantly higher than that on the surface of the target cells or tissue.

## Kit

The present disclosure provides a kit, which comprises an effective amount of at least one of the modified siRNA, the pharmaceutical composition and the siRNA conjugate of the present disclosure.

In some embodiments, the kit of the present disclosure may provide a modified siRNA in a container. In some embodiments, the kit of the present disclosure may comprise a container comprising a pharmaceutically acceptable excipient. In some embodiments, the kit further comprises other ingredients, such as stabilizers or preservatives. In some embodiments, the kit of the present disclosure may comprise at least one additional therapeutic agent in a container

different than the container comprising the modified siRNA of the present disclosure. In some embodiments, the kit may comprise an instruction for mixing the modified siRNA with a pharmaceutically acceptable carrier and/or excipient or other ingredients (if present).

In the kit of the present disclosure, the modified siRNA and the pharmaceutically acceptable carrier and/or excipient as well as the modified siRNA, the pharmaceutical composition and/or the siRNA conjugate and/or the conjugate, and/or the pharmaceutically acceptable exceipient may be provided in any form, such as in a liquid form, a dried form or a lyophilized form. In some embodiments, the modified siRNA and the pharmaceutically acceptable carrier and/or excipient and the pharmaceutical composition and/or conjugate and optional pharmaceutically acceptable excipient(s) are substantially pure and/or sterilized. In some embodiments, sterilized water may be provided in the kit of the present disclosure.

Hereinafter, the present disclosure will be further illustrated by way of examples, but will not be limited to them.

## Examples

Unless otherwise specified, the reagents and culture media used in following examples are all commercially available, and operations used such as nucleic acid electrophoresis and real-time PCR are all performed according to methods described in Molecular Cloning (Cold Spring Harbor Laboratory Press (1989)).

SMMC-7721 cells (Guangzhou Ginio Biotechnology Co., Ltd.) were cultured in H-DMEM complete medium (HyClone company) containing 10% fetal bovine serum (FBS, HyClone company) and 0.2% by volume of Penicillin-Streptomycin (HyClone) at 37°C in an incubator containing 5% $CO_2$/95% air.

HepG2 cells (Guangzhou Ginio Biotechnology Co., Ltd.) were cultured in H-DMEM complete medium (HyClone company) containing 20% fetal bovine serum (FBS, HyClone company) and 0.2% by volume of Penicillin-Streptomycin (HyClone) at 37°C in an incubator containing 5% $CO_2$/95% air.

Huh7 cells (Guangzhou Ginio Biotechnology Co., Ltd.) were cultured in H-DMEM complete medium (HyClone company) containing 10% fetal bovine serum (FBS, Hyclone company) and 0.2% by volume of Penicillin-Streptomycin (Hyclone) at 37°C in an incubator containing 5% $CO_2$/95% air.

When the siRNA or the siRNA conjugate against PNP gene synthesized in the present disclosure or the siRNA or the siRNA conjugate as negative control was used to transfect cells, Lipofectamine™ 2000 (Invitrogen) or INTERFERin (Polyplus) was used as a transfection reagent. The specific procedures thereof may refer to the instruction provided by the manufacturer.

Unless otherwise specified, ratios of reagents provided below are all calculated by volume ratio (v/v).

All the experimental data are expressed as X±SEM, and the data are analyzed by using Graphpad prism 5.0 statistical analysis software.

### Preparation Example 1: Preparation of conjugate 1

Conjugate 1 was synthesized in this preparation example. Conjugate 1 was an siRNA conjugate formed by conjugation of the L-9 conjugating molecule with the siRNA numbered as siPNalMIS in Table 3.

#### (1-1) Synthesis of compound L-10

Compound L-10 was synthesized according to the following method:

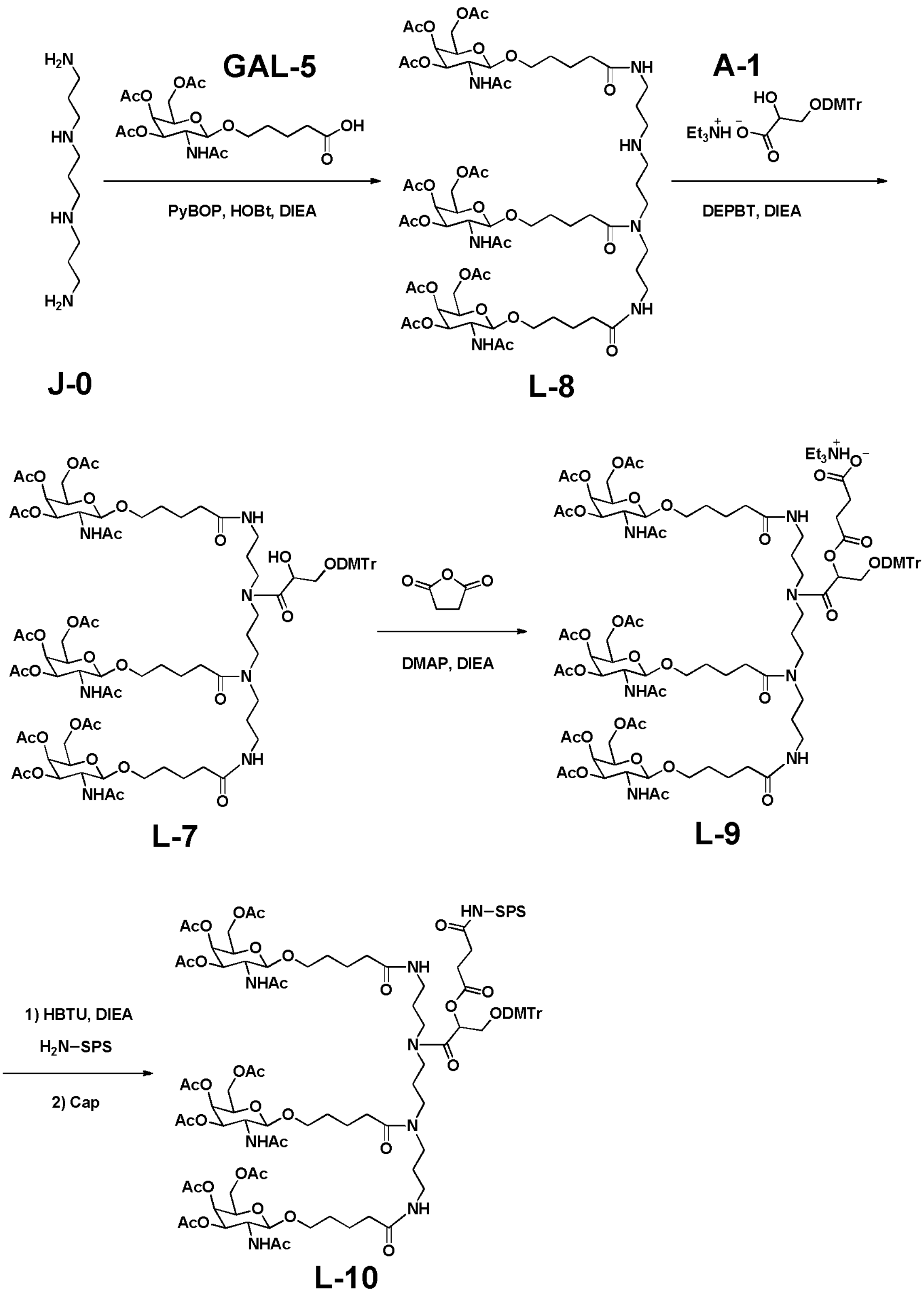


(1-1-1) Synthesis of the conjugating terminal segment GAL-5

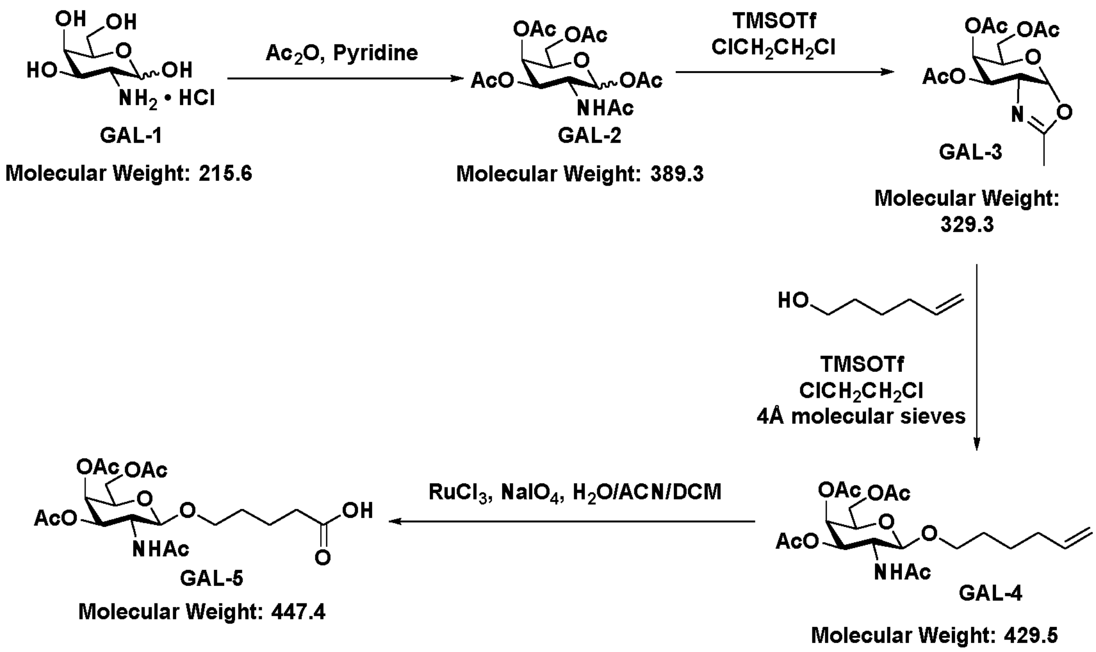


(1-1-1a) Synthesis of GAL-2

100.0 g of GAL-1 (N-acetyl-D-galactosamine hydrochloride, CAS No.: 1772-03-8, purchased from Ningbo hongxiang bio-chem Co., Ltd., 463.8 mmol) was dissolved in 1000 ml of anhydrous pyridine, to which 540 ml of acetic anhydride (purchased from Enox Inc., 5565.6 mmol) was added in an ice water bath to react for 1.5 hours under stirring at room temperature. The resulting reaction solution was poured into 10L of ice water and was subjected to suction filtration under reduced pressure. The residue was washed with 2L of ice water, and then added with a mixed acetonitrile/toluene solvent (v/v ratio of acetonitrile: toluene = 1:1) until complete dissolution. The solvent was evaporated to give 130.0 g of product GAL-2 as a white solid.

(1-1-1b) Synthesis of GAL-3

GAL-2 (35.1 g, 90.0 mmol) obtained in step (1-1-1a) was dissolved in 213 ml of anhydrous 1,2-dichloroethane, to which 24.0 g of TMSOTf (CAS No.: 27607-77-8, purchased from Macklin Inc., 108.0 mmol) was added in an ice water bath and under nitrogen atmosphere to react overnight at room temperature.

400 ml dichloromethane was added to the reaction solution for dilution, filtered with diatomite, and then 1L saturated aqueous sodium bicarbonate solution was added to the resulting reaction solution and stirred evenly. The organic phase was isolated. The aqueous phase was extracted twice, each with 300 ml of dichloroethane, and the organic phases were combined and washed with 300 ml of saturated aqueous sodium bicarbonate solution and 300 ml of saturated brine, respectively. The organic phase was isolated and dried with anhydrous sodium sulfate. The solvent was evaporated under reduced pressure to give 26.9 g of product GAL-3 as a light yellow viscous syrup.

(1-1-1c) Synthesis of GAL-4

GAL-3 (26.9 g, 81.7 mmol) obtained in step (1-1-1b) was dissolved in 136 ml of anhydrous 1,2-dichloroethane, added with 30 g of 4Å molecular sieve as a dry powder followed by 9.0 g of 5-hexen-1-ol (CAS No.: 821-41-0, purchased from Adamas-beta Inc., 89.9 mmol), and stirred for 30 minutes at room temperature. 9.08 g of TMSOTf (40.9 mmol) was added in an ice bath and nitrogen atmosphere to react overnight under stirring at room temperature. The 4Å molecular sieve powder was removed by filtration. 300 ml dichloroethane was added to the filtrate for dilution, filtered with diatomite, and then 500 ml of saturated aqueous sodium bicarbonate solution was added to the resulting reaction solution and stirred for 10 minutes for washing. The organic phase was isolated. The aqueous phase was extracted once with 300 ml of dichloroethane. The organic phases were combined and washed with 300 ml of saturated aqueous sodium bicarbonate solution and 300 ml of saturated brine respectively. The organic phase was isolated and dried with anhydrous sodium sulfate. The solvent was evaporated under reduced pressure to give 41.3g of product GAL-4 as a yellow syrup, which was directly used in the next oxidation reaction without purification.

(1-1-1d) Synthesis of GAL-5

GAL-4 (14.9 g, 34.7 mmol) obtained according to the method described in step (1-1-1c) was dissolved in a mixed solvent of 77 ml of dichloromethane and 77 ml of acetonitrile, added with 103 ml of deionized water and 29.7 g of sodium periodate (CAS No.: 7790-28-5, purchased from Aladdin Inc., 138.8 mmol) respectively, and stirred in an ice bath for 10 minutes. Ruthenium trichloride (CAS No.: 14898-67-0, available from Energy Chemical, 238 mg, 1.145 mmol) was added to react overnight at room temperature. The resulting reaction solution was diluted by adding 300 ml of water, stirred, and adjusted to a pH of about 7.5 by adding saturated sodium bicarbonate. The organic phase was isolated and discarded. The aqueous phase was extracted three times, each with 200 ml of dichloromethane, and the organic phase resulted from the extraction was discarded. The aqueous phase resulted from the extraction was adjusted to a pH of about 3 with citric acid solid and extracted three times, each with 200 ml of dichloromethane, and the organic phases were combined and dried with anhydrous sodium sulfate. The solvent was evaporated under reduced pressure to give 6.85 g of product GAL-5 as a white foamy solid. $^1$H NMR (400 MHz, DMSO) δ 12.01 (br, 1H), 7.83 (d, *J* = 9.2 Hz, 1H), 5.21 (d, *J* = 3.2 Hz, 1H), 4.96 (dd, *J* = 11.2, 3.2 Hz, 1H), 4.49 (d, *J* = 8.4 Hz, 1H), 4.07 - 3.95 (m, 3H), 3.92 - 3.85 (m, 1H), 3.74 - 3.67 (m, 1H), 3.48 - 3.39 (m, 1H), 2.20 (t, *J* = 6.8 Hz, 2H), 2.11 (s, 3H), 2.00 (s, 3H), 1.90 (s, 3H), 1.77 (s, 3H), 1.55 - 1.45 (m, 4H).

(1-1-2) Synthesis of L-8

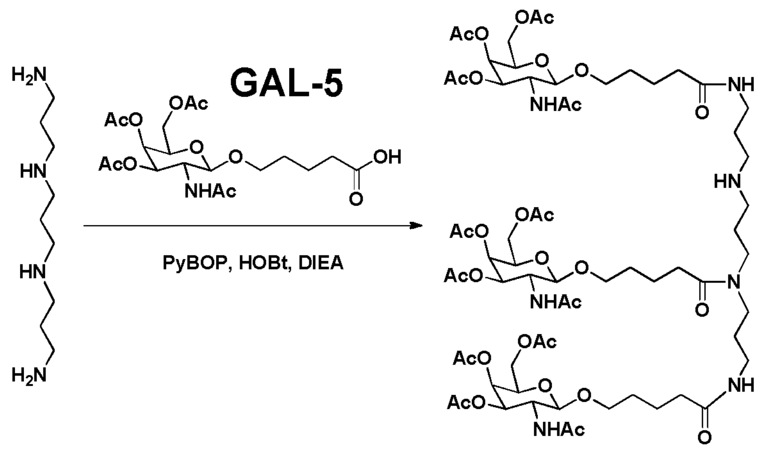


J-0 L-8

J-0 (9.886 g, 52.5 mmol, commercially available from Alfa Aesar Inc.) and GAL-5 (72.819 g, 162.75 mmol, obtained by combining multiple batches of products) obtained in step (1-1-1) were dissolved in 525ml of dichloromethane, and added with diisopropylethylamine (DIEA, 44.782 g, 346.50 mmol), benzotriazol-1-yl-oxytripyrrolidinophosphonium hexafluorophosphate (PyBOP, 90.158 g, 173.25 mmol) and hydroxybenzotriazole (HOBt, 23.410 g, 173.25 mmol) to react for 4 hours at room temperature. The resulting reaction solution was washed with 20 ml of saturated sodium bicarbonate and 200 ml of saturated brine. The aqueous phase was extracted twice, each with 100 ml of dichloromethane. The organic phases were combined, dried with anhydrous sodium sulfate and filtered. Then the solvent was evaporated under reduced pressure to give a crude product. The crude product was purified by using a normal phase silica gel column having 200-300 mesh. The column was added with 10 wt% triethylamine for neutralizing the acidity of silica gel, equilibrated with 1wt‰ triethylamine, and eluted with a gradient elution of dichloromethane: methanol = 100:25-100:40. The eluate of product was collected, and the solvent was evaporated under reduced pressure to give 38.8 g of pure product L-8. $^1$H NMR (400 MHz, DMSO) δ 7.84 (d, *J* = 9.0 Hz, 3H), 7.27 - 7.23 (m, 1H), 7.13 - 7.18 (m, 1H), 5.22 (d, *J* = 3.1 Hz, 3H), 4.97 (dd, *J* = 11.3, 3.1 Hz, 3H), 4.48 (d, *J* = 8.4 Hz, 3H), 4.09 - 3.98 (m, 9H), 3.88 (dd, *J* = 19.3, 9.3 Hz, 3H), 3.75 - 3.66 (m, 3H), 3.44 - 3.38 (m, 3H), 3.17 - 3.30 (m, 4H), 3.10 - 2.97 (m, 4H), 2.35 - 2.20 (m, 6H), 2.15 - 2.08 (m, 9H), 2.07 - 1.98 (m, 13H), 1.94 - 1.87 (m, 9H), 1.81 - 1.74 (m, 9H), 1.65 - 1.42 (m, 18H). MS m/z: $C_{85}H_{119}N_7O_{30}$ $[M+H]^+$, calculated: 1477.59, measured: 1477.23.

(1-1-3) Synthesis of L-7

(1-1-3a) Synthesis of A-1

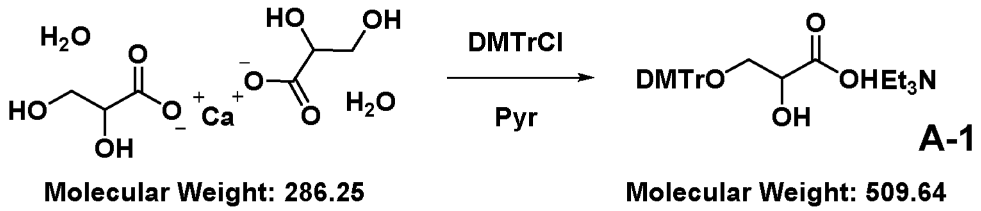


DMTrCl (4,4'-dimethoxytrityl chloride, 101.65 g, 300 mmol) was dissolved in 1000 ml of anhydrous pyridine, and added with calcium DL-glycerate hydrate (28.63 g, 100 mmol) to react for 20 hours at 45°C. The resulting reaction solution was filtered. The residue was rinsed with 200 ml of DCM, and the filtrate was concentrated to dryness under reduced pressure. The residue was redissolved in 500 ml of dichloromethane and washed twice, each with 200 ml of 0.5 M triethylamine phosphate (pH = 7-8). The aqueous phase was extracted twice, each with 200 ml of dichloromethane. The organic phases were combined, dried with anhydrous sodium sulfate, and filtered. The solvent was evaporated under reduced pressure, and the residue was purified by using a normal phase silica gel column having 200-300 mesh, eluted with a gradient elution of petroleum ether: ethyl acetate: dichloromethane: methanol = 1:1:1:0.35-1:1:1:0.55. The eluate of product was collected, and the solvent was evaporated under reduced pressure. The residue was redissolved in 600 ml of dichloromethane, and washed once with 200 ml of 0.5 M triethylamine phosphate. The aqueous phase was extracted once with 200 ml of dichloromethane. The organic phases were combined, dried with anhydrous sodium sulfate, and filtered. The solvent was evaporated under reduced pressure, and the residue was subject to a reduced pressure with a vacuum oil pump overnight to give 50.7 g of product A-1 as a white solid. $^1$H NMR (400 MHz, DMSO-d6) δ 7.46 (ddd, J = 6.5, 2.3, 1.1 Hz, 1H), 7.40 - 7.28 (m, 7H), 6.89 - 6.81 (m, 4H), 4.84 (d, J = 5.0 Hz, 1H), 4.36 - 4.24 (m, 1H), 4.29 (s, 6H), 3.92 (dd, J = 12.4, 7.0 Hz, 1H), 3.67 (dd, J = 12.3, 7.0 Hz, 1H), 2.52 (q, J = 6.3 Hz, 6H), 1.03 (t, J = 6.3 Hz, 9H). MS m/z: $C_{24}H_{23}O_6$, $[M-H]^-$, calculated: 407.15, measured: 406.92.

(1-1-3b) Synthesis of L-7

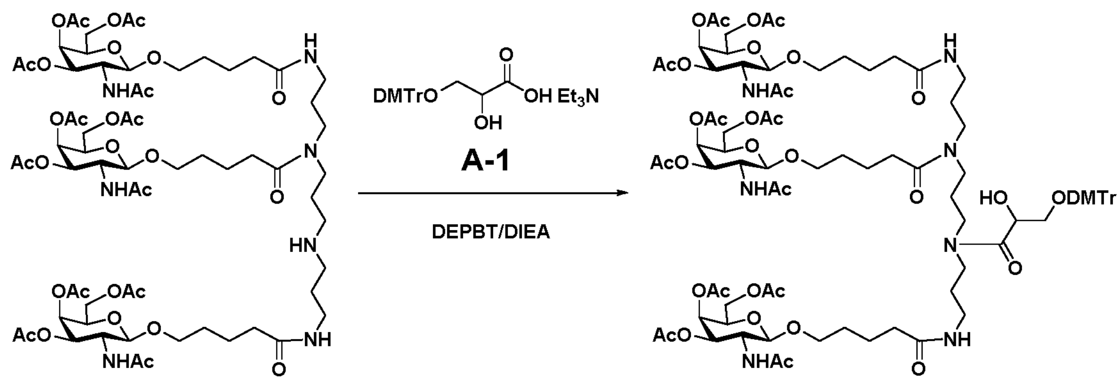


L-8 L-7

L-8 (40 g, 27.09 mmol, obtained by combining multiple batches of products) obtained in step (1-1-2) and A-1 (41.418 g, 81.27 mmol) obtained in step (1-1-3a) were mixed and dissolved in 271 ml of dichloromethane, added with 3-diethoxyphosphoryl-1,2,3-benzotriazol-4(3H)-one (DEPBT, 24.318 g, 81.37 mmol), and further added with diisopropylethylamine (21.007 g, 162.54 mmol) to react for 1.5 hours under stirring at 25°C. The organic phase was washed with 800 ml of saturated sodium bicarbonate. The aqueous phase was extracted three times, each with 50 ml of dichloromethane. The organic phase was washed with 150 ml of saturated brine, and the aqueous phase isolated was extracted once with 50 ml of dichloromethane, and the obtained organic phases were combined, dried with anhydrous sodium sulfate and filtered. The solvent was evaporated under reduced pressure, and the residue was foam-dried overnight

with a vacuum oil pump to give a crude product. The crude product was subjected to a column purification. The column was filled with 2 kg of normal phase silica gel having 200-300 mesh, and added with 200 ml triethylamine for neutralizing the acidity of silica gel. The column was equilibrated with petroleum ether containing 1 wt% triethylamine and eluted with a gradient elution of petroleum ether: ethyl acetate: dichloromethane: N,N-dimethylformamide = 1:1:1:0.5-1:1:1:0.6. The eluate of product was collected, and the solvent was evaporated under reduced pressure to give 40.4 g of pure product L-7. $^1$H NMR (400 MHz, DMSO) δ7.90 - 7.78 (m, 4H), 7.75 - 7.64 (m, 1H), 7.38 - 7.18 (m, 9H), 6.91 - 6.83 (m, 4H), 5.25 - 5.10 (m, 4H), 4.97 *(dd, J* = 11.2, 3.2 Hz, 3H), 4.48 - 4.30 (m, 4H), 4.02 (s, 9H), 3.93 - 3.84 (m, 3H), 3.76 - 3.66 (m, 9H), 3.45 - 3.35 (m, 3H), 3.24 - 2.98 (m, 10H), 2.30 - 2.20 (m, 2H), 2.11 - 1.88 (m, 31H), 1.80 - 1.40 (m, 28H). MS m/z: $C_{90}H_{128}N_7O_{35}$, [M-DMTr]$^+$, calced: 1564.65, measured: 1564.88.

(1-1-4) Synthesis of L-9

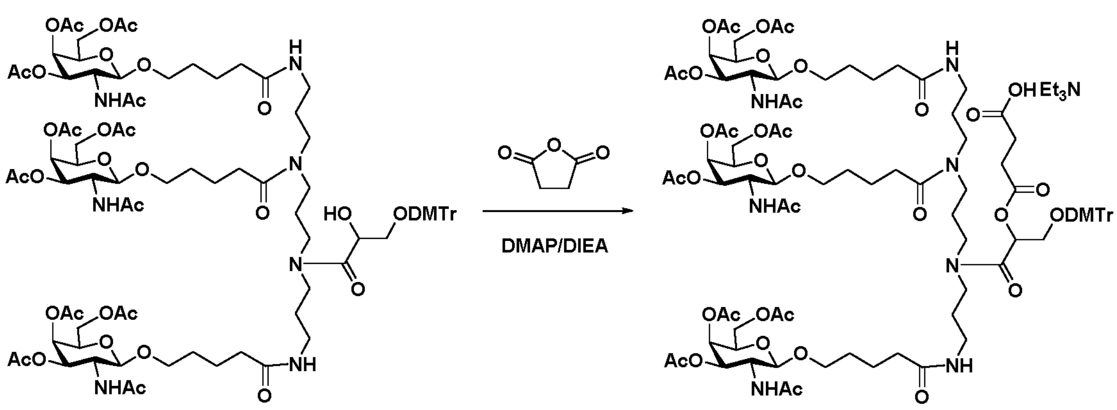


L-7 L-9

L-7 (40 g, 21.4247 mmol) obtained in step (1-1-3b), succinic anhydride (4.288 g, 42.8494 mmol) and 4-dimethylaminopyridine (DMAP, 5.235 g, 42.8494 mmol) were mixed and dissolved in 215 ml of dichloromethane, then added with diisopropylethylamine (DIEA, 13.845 g, 107.1235 mmol), and stirred for 24 hours at 25°C. The resulting reaction solution was washed with 800 ml of 0.5 M triethylamine phosphate. The aqueous phase was extracted three times, each with 5 ml of dichloromethane. The organic phases were combined, and evaporated under reduced pressure to give a crude product. The crude product was subjected to a column purification. The column was filled with 1 kg of normal phase silica gel having 200-300 mesh, and added with 1 wt% triethylamine for neutralizing the acidity of silica gel. The column was equilibrated with dichloromethane and eluted with a gradient elution of 1wt‰ triethylamine-containing dichloromethane: methanol = 100:18-100:20. The eluate of product was collected, and the solvent was evaporated under reduced pressure to give 31.0 g of pure product L-9 conjugating molecule. $^1$H NMR (400 MHz, DMSO) δ 8.58 (d, J = 4.2 Hz, 1H), 7.94 - 7.82 (m, 3H), 7.41 - 7.29 (m, 5H), 7.22 (d, J = 8.1 Hz, 5H), 6.89 *(d, J* = 8.3 Hz, 4H), 5.49 - 5.37 (m, 1H), 5.21 (d, *J* = 3.0 Hz, 3H), 4.97 (d, J = 11.1 Hz, 3H), 4.49 (d, *J* = 8.2 Hz, 3H), 4.02 (s, 9H), 3.88 (dd, *J* = 19.4, 9.4 Hz, 3H), 3.77 - 3.65 (m, 9H), 3.50 - 3.39 (m, 6H), 3.11 - 2.90 (m, 5H), 2.61 - 2.54 (m, 4H), 2.47 - 2.41 (m, 2H), 2.26 - 2.17 (m, 2H), 2.15 - 1.95 (m, 22H), 1.92 - 1.84 (m, 9H), 1.80 - 1.70 (m, 10H), 1.65 - 1.35 (m, 17H), 1.31 - 1.19 (m, 4H), 0.96 (t, *J* = 7.1 Hz, 9H). MS m/z: $C_{94}H_{132}N_7O_{38}$, [M-DMTr]$^+$, calculated: 1664.72, measured: 1665.03.

(1-1-5) Synthesis of compound L-10

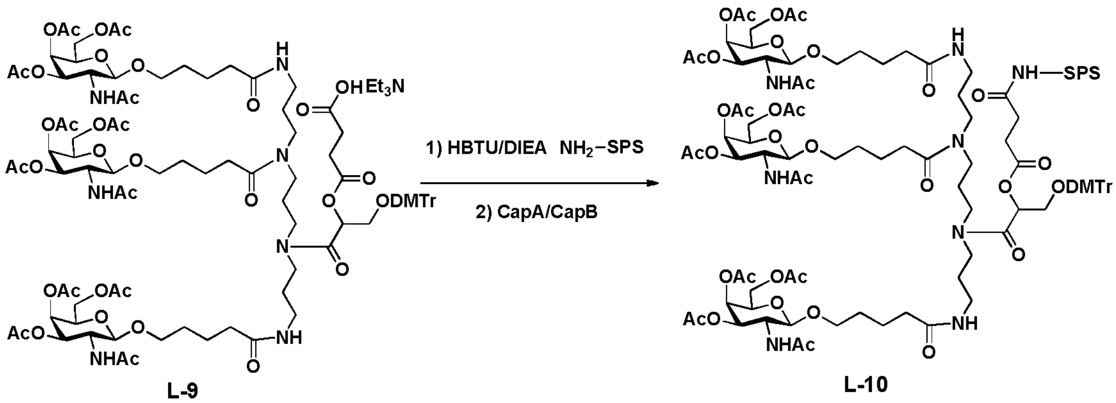


L-9 L-10

In this step, compound L-10 was prepared by linking the L-9 conjugating molecule to a solid phase support.

The L-9 conjugating molecule (22.751 g, 11 mmol) obtained in step (1-1-4), O-benzotriazol-tetramethyluronium hexafluorophosphate (HBTU, 6.257 g, 16.5 mmol) and diisopropylethylamine (DIEA, 2.843 g, 22 mmol) were mixed and dissolved in 900 ml of acetonitrile, and stirred at room temperature for 5 minutes. Aminomethyl resin (88 g, 100-200 mesh, amino loading: 400 μmol/g, purchased from Tianjin Nankai HECHENG S&T Co., Ltd.) was added into the reaction solution. The reaction was performed on a shaker at 25°C and 150 rpm/min for 18 hours, followed by filtration. The residue was rinsed twice, each with 300 ml of DCM; three times, each with 300 ml of acetonitrile; and then dried for 18 hours with a vacuum oil pump. Then a capping reaction was performed by adding starting-materials (CapA, CapB, 4-dimethylaminopyridine (DMAP) and acetonitrile) according to the feeding ratio shown in Table 2. The reaction was placed on a shaker at 25°C and 150 rpm/min for 5 hours. The reaction solution was filtered. The residue was rinsed three times, each with 300 ml of acetonitrile. The solvent was evaporated under reduced pressure, and the residue was dried overnight under a reduced pressure with a vacuum oil pump to give 102 g of compound L-10 (i.e., L-9 conjugating molecule linked to a solid phase support), with a loading of 90.8 μmol/g.

Table 2: The feeding ratio of capping reaction

| Starting materials | Amount | grade | Lot No. | Manufacturer |
|---|---|---|---|---|
| CapA | 1980ml | -- | - | - |
| CapB | 220ml | - | - | - |
| DMAP | 1.100g | analytical pure | 11422139 | Aladdin |
| acetonitrile | 220ml | spectroscopic pure | 015161001 | CINC (Shanghai) Co., Ltd |

wherein CapA and CapB are solutions of capping reagents. CapA is a solution of 20% by volume of N-methylimidazole in a mixture of pyridine/acetonitrile, wherein the volume ratio of pyridine to acetonitrile is 3:5. CapB is a solution of 20% by volume of acetic anhydride in acetonitrile.

(1-2) Synthesis of sense strand of conjugate 1

Nucleoside monomers were linked one by one in 3' to 5' direction according to the sequence of nucleotides in the sense strand of the siRNA corresponding to the siRNA conjugate 1 listed in Table 3 by a solid phase phosphoramidite method, starting the cycles from the compound L-10 prepared in the above step. The linking of each nucleoside monomer included a four-step reaction of deprotection, coupling, capping, and oxidation or sulfuration. Therein, when a phosphoester linkage was used between two nucleotides, a four-step reaction of deprotection, coupling, capping, and oxidation was included during linking of the later nucleoside monomer; and when a phosphorothioate linkage was used between two nucleotides, a four-step reaction of deprotection, coupling, capping, and sulfurization was included during linking of the later nucleoside monomer. The synthesis condition was given as follows:

The nucleoside monomers were provided in a 0.1 M solution in acetonitrile. The conditions were the same for each deprotection reaction, i.e., a temperature of 25 °C, a reaction time of 70 seconds, a solution of dichloroacetic acid in dichloromethane (3% v/v) as a deprotection agent, and a molar ratio of dichloroacetic acid to the protecting group 4,4'-dimethoxytrityl on the solid phase support of 5:1.

The conditions were the same for each coupling reaction, including a temperature of 25°C, a molar ratio of the nucleic acid sequence linked to the solid phase support to nucleoside monomers of 1:10, a molar ratio of the nucleic

acid sequence linked to the solid phase support to a coupling reagent of 1:65, a reaction time of 600 seconds, and a coupling reagent of 0.5 M solution of 5-ethylthio-IH-tetrazole (ETT) in acetonitrile.

The conditions were the same for each capping reaction, including a temperature of 25°C and a reaction time of 15 seconds. The capping reagent was a mixed solution of Cap A and Cap B in a molar ratio of 1:1; and a molar ratio of the capping reagent to the nucleic acid sequence linked to the solid phase support was acetic anhydride: N-methylimidazole: the nucleic acid sequence linked to the solid phase support = 1:1:1.

The conditions were the same for each oxidation reaction, including a temperature of 25°C, a reaction time of 15 seconds, and 0.05 M iodine water as an oxidation reagent; and a molar ratio of iodine to the nucleic acid sequence linked to the solid phase support in the coupling step was 30:1. The reaction was carried out in a mixed solvent of tetrahydrofuran: water: pyridine = 3:1:1.

The conditions were the same for each sulfuration reaction, including a temperature of 25°C, a reaction time of 300 seconds, and xanthane hydride as a sulfurization reagent. The molar ratio of the sulfurization reagent to the nucleic acid sequence linked to the solid phase support in the coupling step was 120:1. The reaction was carried out in a mixed solvent of acetonitrile: pyridine = 1:1.

After the last nucleoside monomer was linked, the nucleic acid sequence linked to the solid phase support was subjected to cleaved, deprotected, purified and desalted in turn, and then lyophilized to obtain the sense strand. Therein, the conditions for cleavage and deprotection were as follows: The synthesized nucleotide sequence linked to the support was added to 25 wt% aqueous ammonia to react for 16 hours at 55°C, and the aqueous ammonia was used in an amount of 0.5 ml/μmol. The remaining support was removed by filtration, and the supernatant was concentrated in vacuum to dryness.

The conditions for purification and desalination were as follows: purification of the nucleic acid was achieved by using a preparative ion chromatography column (Source 15Q) with a gradient elution of NaCl. Specifically, eluent A: 20 mM sodium phosphate (pH 8.1), solvent: water/acetonitrile = 9:1 (v/v); eluent B: 1.5 M sodium chloride, 20 mM sodium phosphate (pH 8.1), solvent: water/acetonitrile = 9:1 (v/v); elution gradient: the ratio of eluent A: eluent B = 100:0-50:50. The eluate of product was collected, combined and desalted by using a reversed phase chromatography column. The specific condition included that a Sephadex column was used for desalination, with Sephadex-G25 as the filler and deionized water for eluting.

The detection method was as follows: The purity of the above sense strand was detected by ion exchange chromatography (IEX-HPLC), and the molecular weight was analyzed by Liquid Chromatography-Mass Spectrometry (LC-MS). The measured value was in conformity with the calculated value, indicating that a sense strand SS conjugated with L-9 conjugating molecule at 3' terminal was synthesized.

(1-3) Synthesis of antisense strand of conjugate 1

The antisense strand AS of the conjugate 1 was synthesized by starting the cycles using a universal solid phase support (UnyLinker™ loaded NittoPhase®HL Solid Supports, Kinovate Life Sciences Inc.) according to a solid phase phosphoramidite method, which has the sequence of the antisense strand of the siRNA corresponding to conjugate 1 listed in Table 3. Deprotection, coupling, capping, and oxidization or sulfuration reaction in the solid phase synthesis method, cleavage and deprotection, and purification and desalination were conducted under the same conditions as those in the synthesis of the sense strand. Subsequently, the antisense strand was obtained by lyophilization. The purity of the antisense strand was detected by ion exchange chromatography (IEX-HPLC), and the molecular weight was analyzed by Liquid Chromatography-Mass Spectrometry (LC-MS). As a result, the measured value was in conformity with the calculated value, indicating that an antisense strand AS conjugated with the target sequence was synthesized.

(1-4) Synthesis of conjugate 1

For conjugate 1, the sense strand and the antisense strand were dissolved in water for injection to give a solution of 40 mg/mL, respectively. They are mixed in an equimolar ratio, heated for 15 minutes at 50°C, and then cooled to room temperature to obtain the annealed product, and lyophilized to obtain lyophilized powder. The conjugate was diluted to a concentration of 0.2 mg/mL by using ultra-pure water (homemade by Milli-Q ultra-pure water instrument, with resistivity of 18.2M Ω *cm (25°C)). The molecular weight was measured by LC-MS (Liquid Chromatography-Mass Spectrometry) (purchased from Waters Corp., model: LCT Premier). The measured value was in conformity with the calculated value, indicating that the synthesized siRNA conjugate 1 was the designed target double stranded nucleic acid sequence with the L-9 conjugating molecule. Its structure is shown in Formula (403), and the siRNA contained in the conjugate has the sequence of the siRNA corresponding to conjugate 1 in Table 3.

Preparation Example 2: Preparation of conjugates 2-13

Conjugates 2-13 were synthesized by using the same method as that in Preparation Example 1 and subjected to molecular weight detection. Except that: 1) the siRNAs were the sequences corresponding to conjugates 2-13 shown in Table 3, respectively; 2) when the target sequence comprises 5-phosphate at the first nucleotide at the 5'-terminal of the antisense strand, the CPR-I monomer (purchased from Suzhou GenePharma Inc. as Cat#13-2601-XX) was linked to the 5' terminal of the antisense strand by a four-step reaction of deprotection, coupling, capping, and oxidation after linking the last nucleoside monomer of the antisense strand in the process of preparing the antisense strand according to a solid phase phosphoramidite method to form a 5'-phosphate modification.

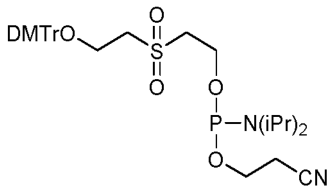

(CPR-I)

In the linkage, the deprotection, coupling, capping, oxidation reaction, cleavage and deprotection, purification and desalination used were conducted under the same conditions as those in the synthesis of the sense strand. The conjugate number and siRNA sequence composition were listed in Table 3.

Table 3: siRNA conjugates

| Conjugate | No. | Sequence Direction 5'-3' | | SEQ ID NO |
|---|---|---|---|---|
| Conjugate1 | L10-siPNa1M IS | Sense strand | CmsCmsUmAmUmGmAfAfGfAmUmUmAmUmAmAmGmAmAm | 241 |
| | | Antisense strand | UmsUfsCmUmUmAfUmAmAmUmCmUmUmCfAmUfAmGmGmsUmsGm | 242 |
| Conjugate2 | L10-siPNb1M IS | Sense strand | GmsUmsAmCmAmGmUfAfCfCmAmGmAmAmGmUmUmAmUm | 243 |
| | | Antisense strand | AmsUfsAmAmCmUfUmCmUmGmGmUmAmCfUmGfUmAmCmsUmsCm | 244 |
| Conjugate3 | L10-siPNc1M IS | Sense strand | CmsAmsAmAmCmAmAfGfCfUmGmCmAmCmAmGmAmAmAm | 245 |
| | | Antisense strand | UmsUfsUmCmUmGfUmGmCmAmGmCmUmUfGmUfUmUmGmsCmsCm | 246 |
| Conjugate4 | L10-siPNa1M 1SP | Sense strand | CmsCmsUmAmUmGmAfAfGfAmUmUmAmUmAmAmGmAmAm | 247 |
| | | Antisense strand | PUmsUfsCmUmUmAfUmAmAmUmCmUmUmCfAmUfAmGmGmsUmsGm | 248 |
| Conjugate5 | L10-siPNb1M 1SP | Sense strand | GmsUmsAmCmAmGmUfAfCfCmAmGmAmAmGmUmUmAmUm | 249 |
| | | Antisense strand | PAmsUfsAmAmCmUfUmCmUmGmGmUmAmCfUmGfUmAmCmsUmsCm | 250 |
| Conjugate6 | L10-siPNc1M 1SP | Sense strand | CmsAmsAmAmCmAmAfGfCfUmGmCmAmCmAmGmAmAmAm | 251 |
| | | Antisense strand | PUmsUfsUmCmUmGfUmGmCmAmGmCmUmUfGmUfUmUmGmsCmsCm | 252 |

(continued)

| Conjugate | No. | Sequence Direction 5'-3' | | SEQ ID NO |
|---|---|---|---|---|
| Conjugate7 | L10-siPNdlM 1SP | Sense strand | CmsAmsAmAmCmAmAfGfGfAmCmUmAmAm UmCmCmAmAm | 253 |
| | | Antisense strand | PUmsUfsGmGmAmUfUmAmGmUmCmCmUmU fGmUfUmUmGmsGmsUm | 254 |
| Conjugate8 | L10-siPNdlM IS | Sense strand | CmsAmsAmAmCmAmAfGfGfAmCmUmAmAm UmCmCmAmAm | 255 |
| | | Antisense strand | UmsUfsGmGmAmUfUmAmGmUmCmCmUmUf GmUfUmUmGmsGmsUm | 256 |
| Conjugate9 | L10-siPNb1M 1SU | Sense strand | GmsUmsAmCmAmGmUfAfCfCmAmGmAmAm GmUmUmAmAm | 257 |
| | | Antisense strand | UmsUfsAmAmCmUfUmCmUmGmGmUmAmCf UmGfUmAmCmsUmsCm | 258 |
| Conjugate1 0 | L10-siPNb1M1SUU | Sense strand | GmsUmsAmCmAmGmUfAfCfCmAmGmAmAm GmUmUmAmAm | 259 |
| | | Antisense strand | UmsUfsAmAmCmUfUmCmUmGmGmUmAmCf UmGfUmAmCmsUmsUm | 260 |
| Conjugate1 1 | L10-siPNc1M 1SU | Sense strand | CmsAmsAmAmCmAmAfGfCfUmGmCmAmCm AmGmAmAmAm | 261 |
| | | Antisense strand | UmsUfsUmCmUmGfUmGmCmAmGmCmUmUf GmUfUmUmGmsUmsUm | 262 |
| Conjugate1 2 | L10-siPNd1M1SU | Sense strand | CmsAmsAmAmCmAmAfGfGfAmCmUmAmAm UmCmCmAmAm | 263 |
| | | Antisense strand | UmsUfsGmGmAmUfUmAmGmUmCmCmUmUf GmUfUmUmGmsUmsUm | 264 |

(continued)

| Conjugate | No. | Sequence Direction 5'-3' | | SEQ ID NO |
|---|---|---|---|---|
| Conjugate1 3 | L10-siPNelM 1SP | Sense strand | GmsCmsAmCmAmGmUfCfCfCmAmGmAmAm GmUmUmAmUm | 265 |
| | | Antisense strand | PAmsUfsAmAmCmUfUmCmUmGmGmGmAmC fUmGfUmGmCmsUmsCm | 266 |

Wherein, C, G, U, and A represent the base composition of the nucleotides; m represents that the nucleotide adjacent to the left side of the letter m is a methoxy modified nucleotide; f represents that the nucleotide adjacent to the left side of the letter f is a fluoro modified nucleotide; s represents that the two nucleotides adjacent to both sides of the letter s are linked by a phosphorothioate linkage; and P represents that the nucleotide adjacent to the right side of the letter P is a 5'- phosphate nucleotide.

Preparation Example 3: Synthesis of siRNA sequence

The siRNAs listed in Table 4 were respectively synthesized by solid phase synthesis method. Equimolar sense strand and antisense strand complementary to each other in Table 4 were respectively dissolved with DEPC-treated water followed by annealing to obtain siRNAs 1-4 and reference siRNA NC provided by the present disclosure.

Table 4: siRNA sequences

| siRNA | No. | Sequence Direction 5'-3' | | SEQ ID NO |
|---|---|---|---|---|
| siRNA 1 | siPNa 1M1S | Sense strand | CmsCmsUmAmUmGmAfAfGfAmUmUmAmUm AmAmGmAmAm | 267 |
| | | Antisense strand | UmsUfsCmUmUmAfUmAmAmUmCmUmUmCf AmUfAmGmGmsUmsGm | 268 |
| siRNA 2 | siPNb 1M1S | Sense strand | GmsUmsAmCmAmGmUfAfCfCmAmGmAmAm GmUmUmAmUm | 269 |
| | | Antisense strand | AmsUfsAmAmCmUfUmCmUmGmGmUmAmCf UmGfUmAmCmsUmsCm | 270 |
| siRNA 3 | siPNc 1M1S | Sense strand | CmsAmsAmAmCmAmAfGfCfUmGmCmAmCm AmGmAmAmAm | 271 |
| | | Antisense strand | UmsUfsUmCmUmGfUmGmCmAmGmCmUmUf GmUfUmUmGmsCmsCm | 272 |
| siRNA 4 | siPNd 1M1S | Sense strand | CmsAmsAmAmCmAmAfGfGfAmCmUmAmAm UmCmCmAmAm | 273 |
| | | Antisense strand | UmsUfsGmGmAmUfUmAmGmUmCmCmUmUf GmUfUmUmGmsGmsUm | 274 |
| NC | - | Sense strand | UUCUCCGAACGUGUCACGUdTdT | 275 |
| | | Antisense strand | ACGUGACACGUUCGGAGAAdTdT | 276 |

Wherein, C, G, U, and A represent the base composition of the nucleotides; m represents that the nucleotide adjacent to the left side of the letter m is a methoxy modified nucleotide; f represents that the nucleotide adjacent to the left side of the letter f is a fluoro modified nucleotide; s represents that the two nucleotides adjacent to both sides of the letter s are linked by a phosphorothioate linkage; and dT represents thymine deoxynucleotide.

In the preparation of the above sequences, when the target sequence comprises unmodified nucleotides, in the cleavage and deprotection conditions, after treatment with aqueous ammonia, the product was dissolved in N-methylpyrrolidone in an amount of 0.4 ml/$\mu$mol, followed by addition of 0.3 ml/$\mu$mol of triethylamine and 0.6 ml/$\mu$mol of triethylamine trihydrofluoride, with respect to the amount of the single strand nucleic acid, thereby removing the 2'-TBDMS protection on ribose.

After the preparation of the siRNAs or the conjugates of the present disclosure, they were lyophilized to solid powder via standard processes and stored until used. When used, they can be resolved with such as water for injection, normal saline (NS), phosphate buffer (PB) or phosphate salt buffer (PBS) to a solution at a desired concentration.

Experimental Example 1: Detection of the inhibitory efficiency of siRNA against the expression amount of PNP mRNA in SMMC-7721 cells.

The SMMC-7721 cells were seeded into a 24-well plate at 7.5 $\times$ $10^4$ cells/well. When a cell growth density reached 70-80% after 16 hours, the H-DMEM complete medium in the culture wells was sucked out, and 500 μl Opti-MEM medium (GIBCO) was added into each well for continuous culture for 1.5 hours.

Each of the following siRNAs was prepared into a 10 μM of the siRNA working solution with DEPC-treated water, respectively. The siRNA was siPNa1M1S, siPNb1M1S, siPNclMIS or siPNdlMIS and negative control NC, respectively.

1A solution was prepared. For each siRNA, 1A solution was prepared, respectively. Each portion of the 1A solution in turn contains 3 μl of the above siRNA working solution with a concentration of 10 μM and 50 μl of Opti-MEM medium.

1B solution was prepared. Each portion of the 1B solution contains 1 μl of LipofectamineTM 2000 and 50 μl of Opti-MEM medium.

One portion of 1B solution was mixed with the obtained 1A solution of each siRNA, respectively, and incubated at room temperature for 20 minutes, respectively, to obtain a transfection complex IX of each siRNA.

One portion of 1B solution was mixed with 50 μl of Opti-MEM medium and incubated for 20 minutes at room temperature to obtain a transfection complex IX'.

In the culture wells, transfection complex IX of each siRNA was respectively added and mixed evenly, with an amount of 100 μl/well to obtain the transfection complex with the final concentration of each siRNA of about 50 nM respectively. The transfection complex IX of each siRNA was respectively transfected into two culture wells to obtain a transfection mixture comprising siRNA, which was labeled as a test group.

In the other two culture wells, the transfection complex IX' was added respectively with an amount of 100 μl/well to obtain a transfection mixture without siRNA, which was labeled as a blank control group.

After transfection of the transfection mixture comprising siRNA and the transfection mixture without siRNA in culture wells for 4 hours, 1ml of H-DMEM complete medium containing 20% FBS was added to each well. The 24-well plate was placed in a $CO_2$ incubator for continuous culture for 24 hours.

Subsequently, the total RNA of the cells in each well was extracted according to the method described in the instruction using RNAVzol (purchased from Vigolous Biotechnology (Beijing) Co., Ltd., as N002).

For the cells in each well, 1 μg of the total RNA was taken out respectively with the reagents provided by the reverse transcription kit of Goldenstar™ RT6 cDNA Synthesis Kit (purchased from Beijing Qingke Xinye Biotechnology Co., Ltd., as TSK301M), among which Goldenstar™ Oligo $(dT)_{17}$ was selected as a primer. 20 μl of the reverse transcription reaction system was prepared according to the reverse transcription operation steps in the instruction of the kit to reverse transcribe the total RNA of the cells in each well. The conditions of the reverse transcription were as follows: For each reverse transcription reaction system, the reverse transcription reaction system was incubated at 50°C for 50 minutes, then at 85°C for 5 minutes, and finally at 4°C for 30 seconds. After the reaction was completed, 80 μl of DEPC-treated water was added into the reverse transcription reaction system to obtain a solution containing cDNA.

For each reverse transcription reaction system, 5 μl of the above cDNA-containing solution was used as a template. 20 μl of qPCR reaction system was prepared using the reagents provided by NovoStart® SYBR qPCR SuperMix Plus kit (purchased from Novoprotein Technology Co., Ltd., as E096-01B). The PCR primer sequences for amplifying the target gene PNP and the internal reference gene GAPDH were shown in Table 7, and the final concentration of each primer was 0.25 μM. Each qPCR reaction system was placed on an ABI StepOnePlus Real-Time PCR instrument for amplification using a three-step method. The amplification procedure was pre-denaturation at 95°C for 10 minutes, followed by denaturation at 95°C for 30 seconds, annealing at 60°C for 30 seconds, and extension at 72°C for 30 seconds. After the above processes of denaturation, annealing, and extension were repeated a total of 40 times, a product W containing the amplified target gene PNP and internal reference gene GAPDH was obtained. The product W was incubated at 95°C for 15 seconds, 60°C for 1 minutes and 95°C for 15 seconds in sequence, and the dissolution curves of the target gene PNP and the internal reference gene GAPDH in the product W were collected by a Quantitative Real-Time PCR instrument, respectively, to obtain the Ct values of the target gene PNP and the internal reference gene GAPDH.

Table 5: Information of primers

| Gene name | Primer type | Nucleotide sequence ( 5'→3' ) | SEQ ID NO |
|---|---|---|---|
| Human PNP | Upstream primer | ACTGGTGTTTGGGTTCCTGA | 277 |
| | Downstream primer | TCCTGCTGCATTGGTGACTA | 278 |

(continued)

| Gene name | Primer type | Nucleotide sequence ( 5'→3' ) | SEQ ID NO |
|---|---|---|---|
| Human GAPDH | Upstream primer | GGTCGGAGTCAACGGATTT | 279 |
| | Downstream primer | CCAGCATCGCCCCACTTGA | 280 |

The relative quantitative calculation of the target gene PNP in each test group was carried out using the comparative Ct (ΔΔCt) method, and the calculation method was as follows:

$$\Delta Ct\ (\text{test group}) = Ct\ (\text{target gene of test group}) - Ct\ (\text{internal reference gene of test group})$$

$$\Delta Ct\ (\text{control group}) = Ct\ (\text{target gene of control group}) - Ct\ (\text{internal reference gene of control group})$$

$$\Delta\Delta Ct\ (\text{test group}) = \Delta Ct\ (\text{test group}) - \Delta Ct\ (\text{average of control group})$$

$$\Delta\Delta Ct\ (\text{control group}) = \Delta Ct\ (\text{control group}) - \Delta Ct\ (\text{average of control group})$$

wherein ΔCt (average of control group) was the arithmetic average of ΔCt (control group) for each of the three culture wells of the control group. Thus, each well in the test and control groups corresponded to a ΔΔCt value.

The expression level of PNP mRNA in the test group was normalized based on the control group, and the expression level of PNP mRNA in the blank control group was defined as 100%,

$$\text{Relative expression level of PNP mRNA in the test group} = 2^{-\Delta\Delta Ct\ (\text{test group})} \times 100\%$$

$$\text{PNP mRNA inhibition rate in the test group} = (1 - \text{relative expression level of PNP mRNA in the test group}) \times 100\%$$

The inhibition rate of each siRNA against PNP mRNA was summarized in Table 6. For the siRNA of the same test group, the mRNA inhibition rate was the arithmetic average of PNP mRNA inhibition rate of the test group measured in two culture wells.

Table 6: Inhibition of PNP mRNA in SMMC-7721 cells

| siRNA | No. | mRNA inhibition rate% |
|---|---|---|
| siRNA 1 | siPNalMIS | 74.83 |
| siRNA 2 | siPNb1M1S | 64.37 |
| siRNA 3 | siPNclMIS | 61.43 |
| siRNA 4 | siPNd1M1S | 66.15 |
| NC | - | -0.18 |

As can be seen from the results of Table 6, the modified siRNA provided by the present disclosure had higher inhibitory activity in SMMC-7721 cell lines, and PNP mRNA inhibition rate of was at least 61.43% and even up to 74.83% at an siRNA concentration of 50 nM.

Experimental Example 2: Detection of the inhibitory efficiency of siRNA against the expression amount of PNP mRNA in Huh7 cells.

In this Experimental Example, the inhibitory efficiency of the siRNA of the present disclosure against the expression amount of PNP mRNA in Huh7 cells was detected.

The inhibition rate of siRNA was detected by the same method as that in Experimental Example 1, except that SMMC-7721 cells were replaced with Huh7 cells. The inhibitory activity of each siRNA in vitro in Huh7 cells was shown in Table 7.

Table 7: Inhibition of PNP mRNA in Huh7 cells

| siRNA | No. | mRNA inhibition rate% |
|---|---|---|
| siRNA 1 | siPNalMIS | 45.17 |
| siRNA 2 | siPNb1M1S | 53.56 |
| siRNA 3 | siPNclMIS | 66.62 |
| siRNA 4 | siPNd1M1S | 89.15 |
| NC | - | -0.93 |

As can be seen from the results of Table 7, the siRNA provided by the present disclosure had higher inhibitory activity in Huh7 cell lines, and in particular, the inhibition rate of 50 nM of siRNA4 against the expression amout of PNP mRNA can be as high as 89.15%.

Experimental Example 3: Detection of the inhibitory efficiency of siRNA against the expression amount of PNP mRNA in HepG2 cells.

The inhibition rate of siRNA was detected by the same method as that in Experimental Example 1, except that SMMC-7721 cells were replaced with HepG2 cells. The inhibitory activity of each siRNA in vitro in HepG2 cells was shown in Table 8.

Table 8: Inhibition of PNP mRNA in HepG2 cells

| siRNA | No. | mRNA inhibition rate% |
|---|---|---|
| siRNA 1 | siPNalMIS | 65.04 |
| siRNA 2 | siPNb1M1S | 57.35 |
| siRNA 3 | siPNclMIS | 65.37 |
| siRNA 4 | siPNd1M1S | 63.63 |
| NC | - | -9.50 |

As can be seen from the results of Table 8, the siRNA provided by the present disclosure also had higher inhibitory activity in HepG2 cell lines, with the inhibition rate ranging from 57.35% to 65.37%.

Experimental Example 4: Detection of the inhibitory efficiency of different doses of siRNA against the expression amount of PNP mRNA in SMMC-7721 cells.

The inhibition rate of siRNA was detected by the same method as that in Experimental Example 1, except that for each siRNA used, working solutions at concentrations of 10 μM, 1μM, and 0.1μM were prepared respectively, i.e., detected at final siRNA concentrations of 50 nM, 5 nM, and 0.5 nM, respectively. The inhibitory activity of each siRNA measured in vitro in SMMC-7721 cells was shown in Table 9.

Table 9: Inhibition of different concentrations of siRNA against PNP mRNA in SMMC-7721 cells

| siRNA | No. | mRNA inhibition rate% | | |
|---|---|---|---|---|
| | | 50 nM | 5 nM | 0.5 nM |
| siRNA 1 | siPNalMIS | 68.37 | 67.55 | 43.75 |
| siRNA 2 | siPNb1M1S | 80.25 | 76.24 | 64.08 |
| siRNA 3 | siPNclMIS | 74.89 | 72.90 | 70.14 |
| siRNA 4 | siPNd1M1S | 81.23 | 70.22 | 52.15 |

(continued)

| siRNA | No. | mRNA inhibition rate% | | |
|---|---|---|---|---|
| | | 50 nM | 5 nM | 0.5 nM |
| NC | - | -0.06 | - | - |

As can be seen from the results of Table 9, the siRNA provided by the present disclosure showed higher inhibitory activity at various concentrations in SMMC-7721 cell lines. Specifically, the siRNA of the present disclosure showed a target mRNA inhibition rate of at least 43.75% to 70.14% at a concentration of 0.5 nM and a PNP mRNA inhibition rate of up to 81.23% at a concentration of 50 nM.

Experimental Example 5: Detection of inhibitory efficiency of different concentrations of siRNA against the expression amount of PNP mRNA in HepG2 cells.

The inhibition rate of siRNA was detected by the same method as that in Experimental Example 1, except that SMMC-7721 cells were replaced with HepG2 cells. For each siRNA used, working solutions at concentrations of 10 μM and 1 μM were prepared respectively, i.e., detected at final siRNA concentrations of 50 nM and 5 nM, respectively. The inhibitory activity of each siRNA measured in vitro in HepG2 cells was shown in Table 10.

Table 10: Inhibition of different concentrations of siRNA against PNP mRNA in HepG2 Cells

| siRNA | No. | mRNA inhibition rate% | |
|---|---|---|---|
| | | 50 nM | 5 nM |
| siRNA 1 | siPNalMIS | 59.5 | 34.0 |
| siRNA 2 | siPNb1M1S | 64.5 | 46.5 |
| siRNA 3 | siPNclMIS | 64.5 | 29.0 |
| siRNA 4 | siPNd1M1S | 68.0 | 36.0 |
| NC | - | 6.5 | - |

As can be seen from the results of Table 10, the siRNA provided by the present disclosure also showed higher inhibitory activity at various concentrations in HepG2 cell lines.

Experimental Example 6: Determination of $IC_{50}$ of siRNA conjugates against PNP mRNA in SMMC-7721 cells.

In this experimental example, the relative expression level of PNP mRNA in SMMC-7721 cells after transfected with different concentrations of siRNA conjugate 4, conjugate 5, conjugate 6 or conjugate 7 was detected by Quantitative Real-Time PCR, and the $IC_{50}$ value of each siRNA conjugate against PNP mRNA was determined.

The SMMC-7721 cells were seeded into a 24-well plate at $5 \times 10^4$ cells/well. When a cell growth density reached 70-80% after 16 hours, the H-DMEM complete medium in the culture wells was sucked out, and 500 μl Opti-MEM medium (GIBCO company) was added into each well for continuous culture for 1.5 hours.

Each conjugate was prepared with DEPC-treated water as a working solution with 8 different concentrations of siRNA conjugates at 50 μM, 12.5 μM, 3.125 μM, 0.7813 μM, 0.1953 μM, 0.0488 μM, 0.0122 μM, and 0.0031 μM (calculated as siRNA), respectively, using the above conjugates 4-7.

For each conjugate, 6A1-6A8 solutions were prepared respectively, and each 6A1-6A8 solution contains, in turn, 3 μL of the above siRNA conjugate working solution at the 8 concentrations and 50 μl of Opti-MEM medium.

A portion of 1B solution was mixed with a portion of 6A1-6A8 solution of each conjugate obtained, and incubated at room temperature for 20 minutes, respectively, to obtain transfection complexes 6X1-6X8 of each conjugate.

A portion of 1B solution was mixed with 50 μL of Opti-MEM medium and incubated for 20 minutes at room temperature to obtain transfection complex 6X'.

One of the above transfection complexes 6×1 to 6X8 was added into the above culture wells for culturing SMMC-7721 cells, respectively, and mixed evenly with an amount of 100 μL/well to obtain a transfection mixture with a final conjugate concentration (calculated by siRNA) of 250 nM, 60 nM, 15.625 nM, 3.9065 nM, 0.9765 nM, 0.244 nM, 0.061 nM, and 0.155 nM, respectively. The transfection complex of each conjugate was transfected into two culture wells, respectively. Transfection mixtures containing the siRNA conjugates of the present disclosure were obtained and

labeled as the test groups.

In the other two culture wells, transfection complex 6X' was added with an amount of 100 μL/well to obtain a transfection mixture without conjugate, which was labeled as the control group.

After the transfection mixtures of the above test group and control group were transfected into culture wells for 4 hours, 1 ml of H-DMEM complete medium containing 20% FBS was added to each well. The 24-well plate was placed in a $CO_2$ incubator for continuous culture for 24 hours.

Subsequently, the total RNA of the cells in each well was extracted according to the method described in the instruction using RNAVzol (purchased from Vigolous Biotechnology (Beijing) Co., Ltd., as N002).

For the cells in each well, 1μg of the total RNA was taken out respectively with the reagents provided by the reverse transcription kit of Goldenstar™ RT6 cDNA Synthesis Kit (purchased from Beijing Qingke Xinye Biotechnology Co., Ltd., as TSK301M), among which Goldenstar™ Oligo $(dT)_{17}$ was selected as a primer. 20 μl of the reverse transcription reaction system was prepared according to the reverse transcription operation steps in the instruction of the kit to reverse transcribe the total RNA of the cells in each well. The conditions of the reverse transcription were as follows: The reverse transcription reaction system was incubated at 50°C for 50 minutes, then at 85°C for 5 minutes, and finally at 4°C for 30 seconds. After the reaction was completed, 80 μl of DEPC-treated water was added into the reverse transcription reaction system to obtain a solution containing cDNA.

For each reverse transcription reaction system, 5 μl of the above cDNA-containing solution was used as a template. 20 μl of qPCR reaction system was prepared using the reagents provided by NovoStart® SYBR qPCR SuperMix Plus kit (purchased from Novoprotein Technology Co., Ltd., as E096-01B). The PCR primer sequences for amplifying the target gene PNP and the internal reference gene GAPDH were shown in Table 5, and the final concentration of each primer was 0.25 μM. Each qPCR reaction system was placed on an ABI StepOnePlus Real-Time PCR instrument for amplification using a three-step method. The amplification procedure was pre-denaturation at 95°C for 10 minutes, followed by denaturation at 95°C for 30 seconds, annealing at 60°C for 30 seconds, and extension at 72°C for 30 seconds. After the above processes of denaturation, annealing, and extension were repeated a total of 40 times, a product W containing the amplified target gene PNP and internal reference gene GAPDH was obtained. The product W was incubated at 95°C for 15 seconds, 60°C for 1 minutes and 95°C for 15 seconds in turn, and the dissolution curves of the target gene PNP and the internal reference gene GAPDH in the product W were collected by a Quantitative Real-Time PCR instrument, respectively, to obtain the Ct values of the target gene PNP and the internal reference gene GAPDH.

The relative quantitative calculation of the target gene PNP in each test group was carried out using the comparative Ct (ΔΔCt) method, and the calculation method was as follows:

$$\Delta Ct\text{ (test group)} = Ct\text{ (target gene of test group)} - Ct\text{ (internal reference gene of test group)}$$

$$\Delta Ct\text{ (control group)} = Ct\text{ (target gene of control group)} - Ct\text{ (internal reference gene of control group)}$$

$$\Delta\Delta Ct\text{ (test group)} = \Delta Ct\text{ (test group)} - \Delta Ct\text{ (average of control group)}$$

$$\Delta\Delta Ct\text{ (control group)} = \Delta Ct\text{ (control group)} - \Delta Ct\text{ (average of control group)}$$

wherein ΔCt (average of control group) was the arithmetic average of ΔCt (control group) for each of the three culture wells of the control group. Thus, each well in the test and control groups corresponded to a ΔΔCt value.

The expression level of PNP mRNA in the test group was normalized based on the control group, and the expression level of PNP mRNA in the control group was defined as 100%,

$$\text{Relative expression level of PNP mRNA in the test group} = 2^{-\Delta\Delta Ct\text{ (test group)}} \times 100\%.$$

The dose-effect curves were fitted by using Function log (inhibitor) vs. response-Variable slope of Graphpad 5.0 software. The $IC_{50}$ value of each conjugate against PNP mRNA was calculated on the basis of the dose-effect curve. Specifically, the dose-effect curve obtained by fitting conformed to the following calculation equation:

$$Y = Bot + \frac{Top - Bot}{1 + 10^{\left(X' - X\right) \times HillSlope}}$$

wherein:

Y is the relative expression level of mRNA in each test group,
X is the logarithm corresponding to the final concentration of siRNA used in the test group,
Bot is the Y value at the bottom of the steady-state phase,
Top is the Y value at the top of the steady-state phase,
X' is the X value obtained by fitting when Y is median value between the bottom and the top, and HillSlope is the slope of the curve obtained by fitting at X'.

Based on the dose-effect curve and the corresponding calculation equation, the corresponding $X_{50}$ value was determined when Y=50%, and the $IC_{50}$ value of each siRNA was calculated to be $10^\wedge X_{50}$ (nM).

Figs.1A-1D are, in sequence, the dose-effect curves fitted based on the relative expression levels of PNP mRNA in SMMC-7721 cells after transfected with different concentrations of siRNA conjugates 4-7. Wherein the logarithmic value of siRNA concentration (lg nM) was taken as the abscissa and the relative expression level of PNP mRNA (%) was taken as the ordinate, and each dot represented the average value of the relative expression levels of PNP mRNA in 2 replicate wells.

The $IC_{50}$ values of each conjugate against PNP mRNA were summarized in Table 11.

Table 11: $IC_{50}$ of siRNA conjugates

| Conjugate | No. | $IC_{50}$ |
|---|---|---|
| Conjugate 4 | L10-siPNa1M1SP | 0.017 nM |
| Conjugate 5 | L10-siPNb1M1SP | 0.045 nM |
| Conjugate 6 | L10-siPNc1M1SP | 0.113 nM |
| Conjugate 7 | L10-siPNd1M1SP | 0.062 nM |

As can be seen from the results of Figs. 1A-1D and Table 11, the siRNA conjugates provided by the present disclosure had very high activity of inhibiting PNP mRNA in vitro in SMMC-7721 cells with an $IC_{50}$ of 0.017-0.113 nM.

Experimental Example 7: Detection of inhibitory efficiency of different doses of siRNA against the expression amount of PNP mRNA in HepG2 cells.

The inhibition rate of siRNA was detected by the same method as that in Experimental Example 1, except that: (1) For each siRNA used, the detection was performd at the final concentrations of siRNA of 50 nM, 5nM and 0.5 nM, respectively; (2) INTERFERin was used as the transfection reagent; (3) The detected siRNAs were siRNAs 2-4 in Table 4, and a NC negative control group was added; (4) SMMC-7721 cells were replaced with HepG2 cells. The inhibitory activity of each siRNA measured *in vitro* in HepG2 cells was shown in Table 12.

Table 12: Inhibition of different concentrations of siRNA against PNP mRNA in HepG2 Cells

| siRNA | No. | mRNA inhibition rate% | | |
|---|---|---|---|---|
| | | 50 nM | 5 nM | 0.5 nM |
| siRNA 2 | siPNblMIS | 64.5 | 46.5 | 25.5 |
| siRNA 3 | siPNclMIS | 64.5 | 29.0 | 15.5 |
| siRNA 4 | siPNdlMIS | 68.0 | 36.0 | 18.5 |
| NC | - | - | - | - |

As can be seen from the results of Table 12, the siRNA provided by the present disclosure had higher inhibitory activity in HepG2 cell lines, and in particular, the inhibition rate of 50 nM of siRNA4 against the expression amount of PNP mRNA can be as high as 68.0%.

Experimental Example 8: Detection of inhibitory efficiency of the siRNA conjugate against the expression amount of PNP mRNA in SMMC-7721 cells.

The inhibition rate of the siRNA conjugate was detected by the same method as that in Experimental Example 1, except that the siRNA to be detected was replaced with conjugates 2, 3, and 8, and a NC negative control group was added, and the concentration used was calculated based on the siRNA. The inhibitory activity of each siRNA conjugate measured *in vitro* in SMMC-7721 cells is shown in Table 13.

Table 13: Inhibition of the siRNA conjugates against PNP mRNA in SMMC-7721 Cells

| siRNA | No. | mRNA inhibition rate% |
|---|---|---|
| Conjugate 2 | L10-siPNb1M1S | 80.46 |
| Conjugate 3 | L10-siPNc1M1S | 80.88 |
| Conjugate 8 | L10-siPNd1M1S | 88.93 |
| NC | - | -4.96 |

As can be seen from the results of Table 13, the siRNA conjugates provided by the present disclosure had higher inhibitory activity in SMMC-7721 cell lines, and in particular, the inhibition rate of conjugate 8 against the expression amount of PNP mRNA can be up to 88.93%.

Experimental Example 9: Detection of inhibitory efficiency of the siRNA conjugate against the expression amount of PNP mRNA in Huh7 cells.

The inhibition rate of the siRNA conjugate was detected by the same method as that in Experimental Example 1, except that the siRNA was replaced with conjugates 2, 3, and 8, and the concentration used was calculated based on the siRNA, and SMMC-7721 cells were replaced with HepG2 cells. The inhibitory activity of each siRNA conjugate measured *in vitro* in Huh7 cells is shown in Table 14.

Table 14: Inhibition of the siRNA conjugates against PNP mRNA in Huh7 cells

| siRNA | No. | mRNA inhibition rate% |
|---|---|---|
| Conjugate 2 | L10-siPNb1M1S | 60.22 |
| Conjugate 3 | L10-siPNc1M1S | 62.58 |
| Conjugate 8 | L10-siPNd1M1S | 77.89 |
| NC | - | 2.00 |

As can be seen from the results of Table 14, the siRNA conjugates provided by the present disclosure had higher inhibitory activity in the Huh7 cell lines, and in particular, the inhibition rate of conjugate 8 against the expression amount of PNP mRNA can be up to 77.89%.

Experimental Example 10: Detection of inhibitory efficiency of the siRNA conjugate against the expression amount of PNP mRNA in SMMC-7721 cells.

The inhibition rate of the siRNA conjugate was detected by the same method as that in Experimental Example 1, except that the siRNA was replaced with conjugates 2, 9, 10, 3, 11, 8 and 12, and a NC negative control group was added, and the concentration used was calculated based on the final concentration of siRNA. The inhibitory activity of each siRNA conjugate measured *in vitro* in SMMC-7721 cells is shown in Table 15.

Table 15: Inhibition of the siRNA conjugates against PNP mRNA in SMMC-7721 Cells

| siRNA | No. | mRNA inhibition rate% |
|---|---|---|
| Conjugate 2 | L10-siPNb1M1S | 78.28 |
| Conjugate 9 | L10-siPNb1M1SU | 81.4 |

(continued)

| siRNA | No. | mRNA inhibition rate% |
|---|---|---|
| Conjugate 10 | L10-siPNb1M1SUU | 75.93 |
| Conjugate 3 | L10-siPNc1M1S | 75.96 |
| Conjugate 11 | L10-siPNc1M1SU | 82.21 |
| Conjugate 8 | L10-siPNa1M1SP | 86.96 |
| Conjugate 12 | L10-siPNd1M1SU | 82.07 |
| NC | - | -5.04 |

As can be seen from the results of Table 15, the siRNA conjugates provided by the present disclosure showed higher inhibitory activity in SMMC-7721 cells, with the inhibition rate against PNP mRNA of at least 75.96% and even as high as 86.96%.

Experimental Example 11: Detection of inhibitory efficiency of the siRNA conjugate against the expression amount of PNP mRNA in Huh7 Cells.

The inhibition rate of the siRNA conjugate was detected by the same method as that in Experimental Example 1, except that: the siRNA was replaced with conjugates 2, 9, 10, 3, 11, 8 and 12, and the concentration used was calculated based on the siRNA, and a NC negative control group was added, and SMMC-7721 cells were replaced with Huh7 cells. The inhibitory activity of each siRNA conjugate measured *in vitro* in Huh7 cells is shown in Table 16.

Table 16: Inhibition of the siRNA conjugates against PNP mRNA in Huh7 cells

| siRNA | No. | mRNA inhibition rate% |
|---|---|---|
| Conjugate 2 | L10-siPNb1M1S | 69.31 |
| Conjugate 9 | L10-siPNb1M1SU | 69.46 |
| Conjugate 10 | L10-siPNb1M1SUU | 62.80 |
| Conjugate 3 | L10-siPNc1M1S | 65.12 |
| Conjugate 11 | L10-siPNc1M1SU | 61.46 |
| Conjugate 8 | L10-siPNa1M1S | 74.46 |
| Conjugate 12 | L10-siPNd1M1SU | 64.45 |
| NC | - | -4.30 |

As can be seen from the results of Table 16, the siRNA conjugates provided by the present disclosure showed higher inhibitory activity in Huh7 cells, with the inhibition rate against PNP mRNA ranging from about 61.46% to 74.46%.

Experimental Example 12: Determination of $IC_{50}$ of siRNA conjugates against PNP mRNA in SMMC-7721 cells.

The $IC_{50}$ values of conjugate 2 and conjugate 8 against PNP mRNA in SMMC-7721 cells were determined by the same method as that in Experimental Example 6, except that the siRNA conjugate tested was conjugate 2 or conjugate 8. The inhibition of conjugate 2 or conjugate 8 against the expression of PNP mRNA in SMMC-7721 cells and the corresponding fitted dose-effect curves are shown in Figs.2A-2B. Meanwhile, the $IC_{50}$ values of the siRNA to be detected targeting PNP mRNA were calculated according to the dose-effect curve. The $IC_{50}$ values of conjugate 2 and conjugate 8 were 0.491 nM and 0.400 nM, respectively, showing an excellent effect of inhibiting PNP mRNA.

Experimental Example 13: Inhibitory activity of the siRNA conjugate of the present disclosure in an acute hyperuricemia mouse model

Adenine was dissolved in 0.5wt% of sodium carboxymethyl cellulose solution at a concentration of 8 mg/ml to obtain an adenine solution.

Oteracil potassium was dissolved in a 0.5wt% of sodium carboxymethyl cellulose solution at a concentration of 40 mg/ml to obtain an oteracil potassium solution.

Normal female mice C57BL/6J of 6-8 week old were randomly divided into groups (5 mice in each group). The five mice of the first group were not injected with any substance as a blank control. The five mice of the second group were first intragastrically administered with the above adenine solution at a dose of 12.5 ml/kg of mouse body weight, that is, the adenine dose was 100 mg/kg, and each mouse was subcutaneously injected with 200 μl of the above oteracil potassium solution after intragastric administration for 30 minutes. The five mice of the third group were first subcutaneously injected with conjugate 13 (5 mg/kg mouse body weight, based on siRNA), and then intragastrically administered with the above adenine solution at a dose of 12.5 ml/kg mouse body weight, that is, the adenine dose was 100 mg/kg, and each mouse was subcutaneously injected with 200 μl of the above oteracil potassium solution after intragastric administration for 30 minutes. 200 μL blood was collected from the orbit at 0 hour, 1 hour, 2 hous and 4 hours respectively, and 100 μL serum was collected after centrifugation. The uric acid content was detected at each time point (detected by Dean Detection Company). The blood collection point at 0 hour was to complete the blood collection within 2 hours before administration. The detection results are shown in Fig.3, wherein the uric acid content values in serum of each group were normalized based on the data of the average uric acid content values in serum of the blank control group at 0 hour and each time point, and the normalized relative uric acid content in serum was taken as the ordinate.

As can be seen from Fig.3, relative to the mice of the second group injected with only adenine and oteracil potassium, the uric acid content in serum of the mice of the third group injected with conjugate 13, adenine and oteracil potassium showed a significant reduction, indicating that the siRNA conjugate of the present disclosure can significantly reduce the blood uric acid content in high uric acid model mice.

Some embodiments of the present disclosure are described in detail above, but the present disclosure is not limited to the specific details of the above-described embodiments. Various simple variations to the technical solution of the present disclosure can be made within the scope of the technical concept of the present disclosure, and these simple variations are within the scope of the present disclosure.

It is to be noted that each of the specific technical features described in the above embodiments can be combined in any suitable manner as long as no contradiction is caused. In order to avoid unnecessary repetition, the various possible combination manners are no longer described in the present disclosure.

In addition, the various different embodiments of the present disclosure may also be carried out in any combination as long as it does not contravene the idea of the present disclosure, which should also be regarded as the disclosure of the present disclosure.

Incorporated by reference

All publications, patents, and patent applications mentioned in this specification are incorporated herein by reference to the same extent as each individual publication, patent, or patent application is specifically and individually incorporated herein by reference.

## Claims

1. An siRNA, comprising a sense strand and an antisense strand, each nucleotide in the siRNA being independently a modified or unmodified nucleotide; wherein the sense strand comprises a nucleotide sequence I, and the antisense strand comprises a nucleotide sequence II; the nucleotide sequence I and the nucleotide sequence II are at least partly reverse complementary to form a double-stranded region; wherein the nucleotide sequence I has the same length as the nucleotide sequence represented by SEQ ID NO: 1 with no more than 3 nucleotide differences; and

the nucleotide sequence II has the same length as the nucleotide sequence represented by SEQ ID NO: 2 with no more than 3 nucleotide differences:

5'-CCUAUGAAGAUUAUAAGA$Z_1$-3' (SEQ ID NO: 1);
5'-$Z_2$UCUUAUAAUCUUCAUAGG-3' (SEQ ID NO: 2),

wherein $Z_1$ is A, and $Z_2$ is U; the nucleotide sequence I comprises a nucleotide $Z_3$ at the position corresponding to $Z_1$; the nucleotide sequence II comprises a nucleotide $Z_4$ at the position corresponding to $Z_2$; the $Z_4$ is the first nucleotide at 5' terminal of the antisense strand; or
the nucleotide sequence I has the same length as the nucleotide sequence represented by SEQ ID NO: 61 with no more than 3 nucleotide differences; and the nucleotide sequence II has the same length as the nucleotide sequence represented by SEQ ID NO: 62 with no more than 3 nucleotide differences:

5'-GUACAGUACCAGAAGUUA$Z_5$-3' (SEQ ID NO: 61);
5'-$Z_6$UAACUUCUGGUACUGUAC-3' (SEQ ID NO: 62),

wherein $Z_5$ is U, and $Z_6$ is A; the nucleotide sequence I comprises a nucleotide $Z_7$ at the position corresponding to $Z_5$; the nucleotide sequence II comprises a nucleotide $Z_8$ at the position corresponding to $Z_6$; the $Z_8$ is the first nucleotide at 5' terminal of the antisense strand; or
the nucleotide sequence I has the same length as the nucleotide sequence represented by SEQ ID NO: 121 with no more than 3 nucleotide differences; and the nucleotide sequence II has the same length as the nucleotide sequence represented by SEQ ID NO: 122 with no more than 3 nucleotide differences:

5'-CAAACAAGCUGCACAGAA$Z_9$-3' (SEQ ID NO: 121);
5'-$Z_{10}$UUCUGUGCAGCUUGUUUG-3' (SEQ ID NO: 122),

wherein $Z_9$ is A, and $Z_{10}$ is U;

the nucleotide sequence I comprises a nucleotide $Z_{11}$ at the position corresponding to $Z_9$; the nucleotide sequence II comprises a nucleotide $Z_{12}$ at the position corresponding to $Z_{10}$; the $Z_{12}$ is the first nucleotide at 5' terminal of the antisense strand; or
the nucleotide sequence I has the same length as the nucleotide sequence represented by SEQ ID NO: 181 with no more than 3 nucleotide differences; and the nucleotide sequence II has the same length as the nucleotide sequence represented by SEQ ID NO: 182 with no more than 3 nucleotide differences:

5'- CAAACAAGGACUAAUCCA$Z_{13}$-3' (SEQ ID NO: 181);
5'-$Z_{14}$UGGAUUAGUCCUUGUUUG-3' (SEQ ID NO: 182),

wherein $Z_{13}$ is A, and $Z_{14}$ is U; the nucleotide sequence I comprises a nucleotide $Z_{15}$ at the position corresponding to $Z_{13}$; the nucleotide sequence II comprises a nucleotide $Z_{16}$ at the position corresponding to $Z_{14}$; the $Z_{16}$ is the first nucleotide at 5' terminal of the antisense strand.

**2.** The siRNA according to claim 1, wherein there is no more than 1 nucleotide difference between the nucleotide sequence I and the nucleotide sequence represented by SEQ ID NO: 1, and/or there is no more than 1 nucleotide difference between the nucleotide sequence II and the nucleotide sequence represented by SEQ ID NO: 2;

or there is no more than 1 nucleotide difference between the nucleotide sequence I and the nucleotide sequence represented by SEQ ID NO: 61, and/or there is no more than 1 nucleotide difference between the nucleotide sequence II and the nucleotide sequence represented by SEQ ID NO: 62;
or there is no more than 1 nucleotide difference between the nucleotide sequence I and the nucleotide sequence represented by SEQ ID NO: 121, and/or there is no more than 1 nucleotide difference between the nucleotide sequence II and the nucleotide sequence represented by SEQ ID NO: 122;
or there is no more than 1 nucleotide difference between the nucleotide sequence I and the nucleotide sequence represented by SEQ ID NO: 181, and/or there is no more than 1 nucleotide difference between the nucleotide sequence II and the nucleotide sequence represented by SEQ ID NO: 182.

**3.** The siRNA according to claim 1 or 2, wherein the nucleotide difference between the nucleotide sequence II and the nucleotide sequence represented by SEQ ID NO: 2 includes a difference at the position of $Z_4$, wherein $Z_4$ is selected

from A, C or G;

or the nucleotide difference between the nucleotide sequence II and the nucleotide sequence represented by SEQ ID NO: 62 includes a difference at the position of $Z_8$, wherein $Z_8$ is selected from U, C or G;
or the nucleotide difference between the nucleotide sequence II and the nucleotide sequence represented by SEQ ID NO: 122 includes a difference at the position of $Z_{12}$, wherein $Z_{12}$ is selected from A, C or G;
or the nucleotide difference between the nucleotide sequence II and the nucleotide sequence represented by SEQ ID NO: 182 includes a difference at the position of $Z_{16}$, wherein $Z_{16}$ is selected from A, C or G.

**4.** The siRNA according to any one of claims 1 to 3, wherein $Z_3$ is a nucleotide complementary to $Z_4$; or $Z_7$ is a nucleotide complementary to $Z_8$; or $Z_{11}$ is a nucleotide complementary to $Z_{12}$; or $Z_{15}$ is a nucleotide complementary to $Z_{16}$.

**5.** The siRNA according to any one of claims 1 to 4, wherein the nucleotide sequence I and the nucleotide sequence II are basically reverse complementary, substantially reverse complementary, or completely reverse complementary to each other; the "basically reverse complementary" means that there is no more than 3 base mispairings between two nucleotide sequences; the "substantially reverse complementary" means that there is no more than 1 base mispairing between two nucleotide sequences; the "completely reverse complementary" means that there is no base mispairing between two nucleotide sequences.

**6.** The siRNA according to any one of claims 1 to 5, wherein the nucleotide sequence I is a nucleotide sequence represented by SEQ ID NO: 3, and the nucleotide sequence II is a nucleotide sequence represented by SEQ ID NO: 4:

5'-CCUAUGAAGAUUAUAAGA$Z_3$-3' (SEQ ID NO: 3);
5'- $Z_4$UCUUAUAAUCUUCAUAGG-3' (SEQ ID NO: 4),

wherein $Z_3$ is selected from A, U, G or C, and $Z_4$ is a nucleotide complementary to $Z_3$;
or the nucleotide sequence I is a nucleotide sequence represented by SEQ ID NO: 63, and the nucleotide sequence II is a nucleotide sequence represented by SEQ ID NO: 64:

5'-GUACAGUACCAGAAGUUA$Z_7$-3' (SEQ ID NO: 63);
5'-$Z_8$UAACUUCUGGUACUGUAC-3' (SEQ ID NO: 64),

wherein $Z_7$ is selected from A, U, G or C, and $Z_8$ is a nucleotide complementary to $Z_7$;
or the nucleotide sequence I is a nucleotide sequence represented by SEQ ID NO: 123, and the nucleotide sequence II is a nucleotide sequence represented by SEQ ID NO: 124:

5'- CAAACAAGCUGCACAGAA$Z_{11}$-3' (SEQ ID NO: 123);
5'-$Z_{12}$UUCUGUGCAGCUUGUUUG-3' (SEQ ID NO: 124),

wherein $Z_{11}$ is selected from A, U, G or C, and $Z_{12}$ is a nucleotide complementary to $Z_{11}$;
or the nucleotide sequence I is a nucleotide sequence represented by SEQ ID NO: 183, and the nucleotide sequence II is a nucleotide sequence represented by SEQ ID NO: 184:

5'- CAAACAAGGACUAAUCCA$Z_{15}$-3' (SEQ ID NO: 183);
5'-$Z_{16}$UGGAUUAGUCCUUGUUUG-3' (SEQ ID NO: 184),

wherein $Z_{15}$ is selected from A, U, G or C, and $Z_{16}$ is a nucleotide complementary to $Z_{15}$.

**7.** The siRNA according to claim 6, wherein $Z_3$ is A, and $Z_4$ is U; or $Z_7$ is U, and $Z_8$ is A; or $Z_{11}$ is A, and $Z_{12}$ is U; or $Z_{15}$ is A, and $Z_{16}$ is U.

**8.** The siRNA according to any one of claims 1 to 7, wherein the sense strand further comprises a nucleotide sequence III, the antisense strand further comprise a nucleotide sequence IV, and the nucleotide sequence III and the nucleotide sequence IV each independently have a length of 1 to 4 nucleotides; the nucleotide sequence III is linked to the 5' terminal of the nucleotide sequence I, and the nucleotide sequence IV is linked to the 3' terminal of the nucleotide sequence II; and the nucleotide sequence III and the nucleotide sequence IV have the same length and are substantially reverse complementary or completely reverse complementary; the "substantially reverse complementary" means that there is no more than 1 base mispairing between two nucleotide sequences; the "completely reverse

complementary" means that there is no mispairing between two nucleotide sequences.

**9.** The siRNA according to claim 8, wherein the nucleotide sequence I has the same length as the nucleotide sequence represented by SEQ ID NO: 1 with no more than 3 nucleotide differences; and the nucleotide sequence III and the nucleotide sequence IV both have a length of one nucleotide, and the base of the nucleotide sequence III is A; or the nucleotide sequence III and the nucleotide sequence IV both have a length of 2 nucleotides, and in the direction from 5' terminal to 3' terminal, the base composition of the nucleotide sequence III is CA; or the nucleotide sequence III and the nucleotide sequence IV both have a length of 3 nucleotides, and in the direction from 5' terminal to 3' terminal, the base composition of the nucleotide sequence III is ACA; or the nucleotide sequence III and the nucleotide sequence IV both have a length of 4 nucleotides, and in the direction from 5' terminal to 3' terminal, the base composition of the nucleotide sequence III is UACA; or

the nucleotide sequence I has the same length as the nucleotide sequence represented by SEQ ID NO: 61 with no more than 3 nucleotide differences; and the nucleotide sequence III and the nucleotide sequence IV both have a length of one nucleotide, and the base of the nucleotide sequence III is A; or the nucleotide sequence III and the nucleotide sequence IV both have a length of 2 nucleotides, and in the direction from 5' terminal to 3' terminal, the base composition of the nucleotide sequence III is GA; or the nucleotide sequence III and the nucleotide sequence IV both have a length of 3 nucleotides, and in the direction from 5' terminal to 3' terminal, the base composition of the nucleotide sequence III is UGA; or the nucleotide sequence III and the nucleotide sequence IV both have a length of 4 nucleotides, and in the direction from 5' terminal to 3' terminal, the base composition of the nucleotide sequence III is AUGA; or

the nucleotide sequence I has the same length as the nucleotide sequence represented by SEQ ID NO: 121 with no more than 3 nucleotide differences; and the nucleotide sequence III and the nucleotide sequence IV both have a length of one nucleotide, and the base of the nucleotide sequence III is G; or the nucleotide sequence III and the nucleotide sequence IV both have a length of 2 nucleotides, and in the direction from 5' terminal to 3' terminal, the base composition of the nucleotide sequence III is GG; or the nucleotide sequence III and the nucleotide sequence IV both have a length of 3 nucleotides, and in the direction from 5' terminal to 3' terminal, the base composition of the nucleotide sequence III is UGG; or the nucleotide sequence III and the nucleotide sequence IV both have a length of 4 nucleotides, and in the direction from 5' terminal to 3' terminal, the base composition of the nucleotide sequence III is CUGG;

or, the nucleotide sequence I has the same length as the nucleotide sequence represented by SEQ ID NO: 181 with no more than 3 nucleotide differences; and the nucleotide sequence III and the nucleotide sequence IV both have a length of one nucleotide, and the base of the nucleotide sequence III is C; or the nucleotide sequence III and the nucleotide sequence IV both have a length of 2 nucleotides, and in the direction from 5' terminal to 3' terminal, the base composition of the nucleotide sequence III is AC; or the nucleotide sequence III and the nucleotide sequence IV both have a length of 3 nucleotides, and in the direction from 5' terminal to 3' terminal, the base composition of the nucleotide sequence III is GAC; or the nucleotide sequence III and the nucleotide sequence IV both have a length of 4 nucleotides, and in the direction from 5' terminal to 3' terminal, the base composition of the nucleotide sequence III is AGAC.

**10.** The siRNA according to any one of claims 1 to 9, wherein the antisense strand futher comprises nucleotide sequence V, and the nucleotide sequence V has a length of 1 to 3 nucleotides and is linked to the 3' terminal of the antisense strand, thereby forming a 3' overhang terminal of the antisense strand;

or, the nucleotide sequence V has a length of two nucleotides;
or, the nucleotide sequence V is 2 continuous thymine deoxyribonucleotides or 2 continuous uridine ribonucleotides;
or, the nucleotide sequence V is complementary to the nucleotide(s) at the corresponding position(s) of the target mRNA.

**11.** The siRNA according to any one of claims 1 to 10, wherein the sense strand of the siRNA comprises the nucleotide sequence represented by SEQ ID NO: 5, and the antisense strand of the siRNA comprises the nucleotide sequence represented by SEQ ID NO: 6:

5'- CCUAUGAAGAUUAUAAGA$Z_3$-3' (SEQ ID NO: 5);
5'- $Z_4$UCUUAUAAUCUUCAUAGGUG-3' (SEQ ID NO: 6);

or, the sense strand of the siRNA comprises the nucleotide sequence represented by SEQ ID NO: 7, and the

antisense strand of the siRNA comprises the nucleotide sequence represented by SEQ ID NO: 8:

5'-CACCUAUGAAGAUUAUAAGA$Z_3$-3' (SEQ ID NO: 7);
5'- $Z_4$UCUUAUAAUCUUCAUAGGUGUA-3' (SEQ ID NO: 8);

wherein, the $Z_4$ is the first nucleotide at the 5' terminal of the antisense strand, $Z_3$ is selected from of A, U, G or C, and $Z_4$ is a nucleotide complementary to $Z_3$;
or, the sense strand of the siRNA comprises the nucleotide sequence represented by SEQ ID NO: 65, and the antisense strand of the siRNA comprises the nucleotide sequence represented by SEQ ID NO: 66:

5'- GUACAGUACCAGAAGUUA$Z_7$-3' (SEQ ID NO: 65);
5'- $Z_8$UAACUUCUGGUACUGUACUC-3' (SEQ ID NO: 66),

or, the sense strand of the siRNA comprises the nucleotide sequence represented by SEQ ID NO: 67, and the antisense strand of the siRNA comprises the nucleotide sequence represented by SEQ ID NO: 68:

5'- GAGUACAGUACCAGAAGUUA$Z_7$-3' (SEQ ID NO: 67);
5'- $Z_8$UAACUUCUGGUACUGUACUCAU-3' (SEQ ID NO: 68),

wherein, the $Z_8$ is the first nucleotide at the 5' terminal of the antisense strand, $Z_7$ is selected from of A, U, G or C, and $Z_8$ is a nucleotide complementary to $Z_7$;
or, the sense strand of the siRNA comprises the nucleotide sequence represented by SEQ ID NO: 125, and the antisense strand of the siRNA comprises the nucleotide sequence represented by SEQ ID NO: 126:

5'- CAAACAAGCUGCACAGAA$Z_{11}$-3' (SEQ ID NO: 125);
5'-$Z_{12}$UUCUGUGCAGCUUGUUUGCC-3' (SEQ ID NO: 126),

or, the sense strand of the siRNA comprises the nucleotide sequence represented by SEQ ID NO: 127, and the antisense strand of the siRNA comprises the nucleotide sequence represented by SEQ ID NO: 128:

5'- GGCAAACAAGCUGCACAGAA$Z_{11}$-3' (SEQ ID NO: 127);
5'-$Z_{12}$UUCUGUGCAGCUUGUUUGCCAG-3' (SEQ ID NO: 128),

wherein, the $Z_{12}$ is the first nucleotide at the 5' terminal of the antisense strand, $Z_{11}$ is selected from of A, U, G or C, and $Z_{12}$ is a nucleotide complementary to $Z_{11}$.
or, the sense strand of the siRNA comprises the nucleotide sequence represented by SEQ ID NO: 185, and the antisense strand of the siRNA comprises the nucleotide sequence represented by SEQ ID NO: 186:

5'- CAAACAAGGACUAAUCCA$Z_{15}$-3' (SEQ ID NO: 185);
5'-$Z_{16}$UGGAUUAGUCCUUGUUUGGU-3' (SEQ ID NO: 186),

or, the sense strand of the siRNA comprises the nucleotide sequence represented by SEQ ID NO: 187, and the antisense strand of the siRNA comprises the nucleotide sequence represented by SEQ ID NO: 188:

5'- ACCAAACAAGGACUAAUCCA$Z_{15}$-3' (SEQ ID NO: 187);
5'-$Z_{16}$UGGAUUAGUCCUUGUUUGGUCU-3' (SEQ ID NO: 188),

wherein, the $Z_{16}$ is the first nucleotide at the 5' terminal of the antisense strand, $Z_{15}$ is selected from of A, U, G or C, and $Z_{16}$ is a nucleotide complementary to $Z_{15}$.

**12.** The siRNA according to any one of claims 1 to 11, wherein the siRNA is any one of siPNa1, siPNa2, siPNb1, siPNb2, siPNc1, siPNc2, siPNd1 or siPNd2.

**13.** The siRNA according to any one of claims 1 to 12, wherein at least one nucleotide in the sense strand or the antisense strand is a modified nucleotide, and/or at least one phosphate group is a phosphate group with modified group(s).

**14.** The siRNA according to any one of claims 1 to 13, wherein each nucleotide in the sense strand and the antisense strand is independently a fluoro modified nucleotide or a non-fluoro modified nucleotide.

**15.** The siRNA according to claim 14, wherein the fluoro modified nucleotide is present in the nucleotide sequence I and nucleotide sequence II, and in the direction from 5' terminal to 3' terminal, at least the nucleotides at positions 7, 8, 9 of the nucleotide sequence I are fluoro modified nucleotides; in the direction from 5' terminal to 3' terminal, at least the nucleotides at positions 2, 6, 14 and 16 of the nucleotide sequence II are fluoro modified nucleotides; preferably, in the direction from 5' terminal to 3' terminal, the nucleotides at positions 7, 8 and 9 or at positions 5, 7, 8 and 9 of the nucleotide sequence I in the sense strand are fluoro modified nucleotides, and the nucleotides at the other positions in the sense strand are non-fluoro modified nucleotides; in the direction from 5' terminal to 3' terminal, the nucleotides at positions 2, 6, 14 and 16 or at positions 2, 6, 8, 9, 14 and 16 of the nucleotide sequence II in the antisense strand are fluoro modified nucleotides, and the nucleotides at the other positions in the antisense strand are non-fluoro modified nucleotides.

**16.** The siRNA according to any one of claims 14 to 15, wherein each non-fluoro modified nucleotide is independently selected from a nucleotide formed by substituting the 2'-hydroxy of the ribose group thereof with a non-fluoro group, or a nucleotide analogue.

**17.** The siRNA according to claim 16, wherein the nucleotide formed by substituting the 2'-hydroxy of the ribose group thereof with a non-fluoro group is one selected from the group consisting of 2'-alkoxy modified nucleotides, 2'-substituted alkoxy modified nucleotides, 2'-alkyl modified nucleotides, 2'-substituted alkyl modified nucleotides, 2'-amino modified nucleotides, 2'-substituted amino modified nucleotides and 2'-deoxy nucleotides; and the nucleotide analogue is one selected from isonucleotide, LNA, ENA, cET, UNA and GNA.

**18.** The siRNA according to any one of claims 14 to 15, wherein each non-fluoro modified nucleotide is a methoxy modified nucleotide, and the methoxy modified nucleotide refers to a nucleotide formed by substituting the 2'-hydroxy of the ribose group with a methoxy group.

**19.** The siRNA according to claim 18, wherein in the direction from 5' terminal to 3' terminal, the nucleotides at positions 5, 7, 8 and 9 of the nucleotide sequence I in the sense strand of the siRNA are fluoro modified nucleotides, and the nucleotides at the other positions in the sense strand of the siRNA are methoxy modified nucleotides; and in the direction from 5' terminal to 3' terminal, the nucleotides at positions 2, 6, 8, 9, 14 and 16 of the nucleotide sequence II in the antisense strand of the siRNA are fluoro modified nucleotides, and the nucleotides at the other positions in the antisense strand of the siRNA are methoxy modified nucleotides;

or, in the direction from 5' terminal to 3' terminal, the nucleotides at positions 5, 7, 8 and 9 of the nucleotide sequence I in the sense strand of the siRNA are fluoro modified nucleotides, and the nucleotides at the other positions of the sense strand of the siRNA are methoxy modified nucleotides; and in the direction from 5' terminal to 3' terminal, the nucleotides at positions 2, 6, 14 and 16 of the nucleotide sequence II in the antisense strand of the siRNA are fluoro modified nucleotides, and the nucleotides at the other positions in the antisense strand of the siRNA are methoxy modified nucleotides;

or, in the direction from 5' terminal to 3' terminal, the nucleotides at positions 7, 8 and 9 of the nucleotide sequence I in the sense strand of the siRNA are fluoro modified nucleotides, and the nucleotides at the other positions in the sense strand of the siRNA are methoxy modified nucleotides; and in the direction from 5' terminal to 3' terminal, the nucleotides at positions 2, 6, 14 and 16 of the nucleotide sequence II in the antisense strand of the siRNA are fluoro modified nucleotides, and the nucleotides at the other positions in the antisense strand of the siRNA are methoxy modified nucleotides.

**20.** The siRNA according to any one of claims 1 to 19, wherein the siRNA is any one of siPNa1-M1, siPNa2-M1, siPNa1-M2, siPNa2-M2, siPNa1-M3, siPNa2-M3, siPNb1-M1, siPNb2-M1, siPNb1-M2, siPNb2-M2, siPNb1-M3, siPNb2-M3, siPNc1-M1, siPNc2-M1, siPNc1-M2, siPNc2-M2, siPNc1-M3, siPNc2-M3, siPNd1-M1, siPNd2-M1, siPNd1-M2, siPNd2-M2, siPNd1-M3, siPNd2-M3.

**21.** The siRNA according to claim 13, wherein the phosphate group with modified group(s) is a phosphorothioate group formed by substituting at least one oxygen atom in a phosphodiester bond in the phosphate group with a sulfur atom.

**22.** The siRNA according to claim 13 or 21, wherein the phosphate group with modified group(s) is a phosphorothioate group having a structure represented by Formula (1):

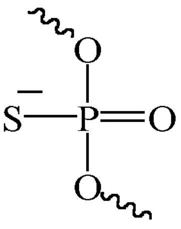
Formula (1).

**23.** The siRNA according to claim 21 or 22, wherein in the siRNA, a phosphorothioate linkage is located in at least one of the group consisting of the following positions:

the position between the first and second nucleotides at 5' terminal of the sense strand;
the position between the second and third nucleotides at 5' terminal of the sense strand;
the position between the first and second nucleotides at 3' terminal of the sense strand;
the position between the second and third nucleotides at 3' terminal of the sense strand;
the position between the first and second nucleotides at 5' terminal of the antisense strand;
the position between the second and third nucleotides at 5' terminal of the antisense strand;
the position between the first and second nucleotides at 3' terminal of the antisense strand; and
the position between the second and third nucleotides at 3' terminal of the antisense strand.

**24.** The siRNA according to any one of claims 1 to 23, wherein the siRNA is any one of siPNa1-M1S, siPNa2-M1S, siPNa1-M2S, siPNa2-M2S, siPNa1-M3S, siPNa2-M3S, siPNb1-M1S, siPNb2-M1S, siPNb1-M2S, siPNb2-M2S, siPNb1-M3S, siPNb2-M3S, siPNc1-M1S, siPNc2-M1S, siPNc1-M2S, siPNc2-M2S, siPNc1-M3S, siPNc2-M3S, siPNd1-M1S, siPNd2-M1S, siPNd1-M2S, siPNd2-M2S, siPNd1-M3S, siPNd2-M3S.

**25.** The siRNA according to any one of claims 1 to 24, wherein the nucleotide at 5' terminal of the antisense strand is a 5'-phosphate nucleotide or a 5'-phosphate analogue modified nucleotide; preferably, the 5'-phosphate nucleotide is a nucleotide having a structure represented by Formula (2), and the 5'-phosphate analogue modified nucleotide is a nucleotide having a structure represented by any of Formulae (3)-(6):

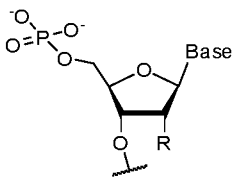
Formula (2)

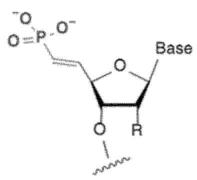
Formula (3)

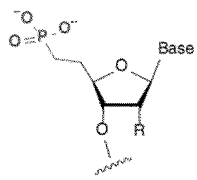
Formula (4)

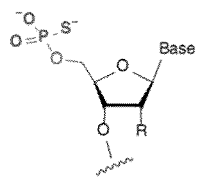
Formula (5)

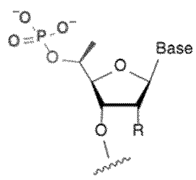
Formula (6)

wherein R is selected from H, OH, methoxy, or F; Base represents a nucleic acid base selected from A, U, C, G, or T.

**26.** The siRNA according to any one of claims 1 to 25, wherein the siRNA is any one of siPNa1-M1P1, siPNa2-M1P1, siPNa1-M2P1, siPNa2-M2P1, siPNa1-M3P1, siPNa2-M3P1, siPNa1-M1SP1, siPNa2-M1SP1, siPNa1-M2SP1, siPNa2-M2SP1, siPNa1-M3SP1, siPNa2-M3SP1, siPNb1-M1P1, siPNb2-M1P1, siPNb1-M2P1, siPNb2-M2P1, siPNb1-M3P1, siPNb2-M3P1, siPNb1-M1SP1, siPNb2-M1SP1, siPNb1-M2SP1, siPNb2-M2SP1, siPNb1-M3SP1, siPNb2-M3SP1, siPNc1-M1P1, siPNc2-M1P1, siPNc1-M2P1, siPNc2-M2P1, siPNc1-M3P1, siPNc2-M3P1, siPNc1-M1SP1, siPNc2-M1SP1, siPNc1-M2SP1, siPNc2-M2SP1, siPNc1-M3SP1, siPNc2-M3SP1, siPNd1-M1P1, siPNd2-M1P1, siPNd1-M2P1, siPNd2-M2P1, siPNd1-M3P1, siPNd2-M3P1, siPNd1-M1SP1, siPNd2-M1SP1, siPNd1-M2SP1, siPNd2-M2SP1, siPNd1-M3SP1, siPNd2-M3SP1.

**27.** A pharmaceutical composition, **characterized in that** the pharmaceutical composition comprises the siRNA according to any one of claims 1 to 26 and a pharmaceutically acceptable carrier.

**28.** The pharmaceutical composition according to claim 27, wherein the weight ratio of the siRNA and the pharmaceutically acceptable carrier is 1: (1-500); preferably, the weight ratio of the siRNA and the pharmaceutically acceptable carrier is 1: (1-50).

**29.** The pharmaceutical composition according to any one of claims 27 to 28, wherein the pharmaceutically acceptable carrier comprises an organic amine, a helper lipid and a PEGylated lipid; wherein the organic amine is a compound represented by Formula (201) and/or a pharmaceutically acceptable salt thereof:

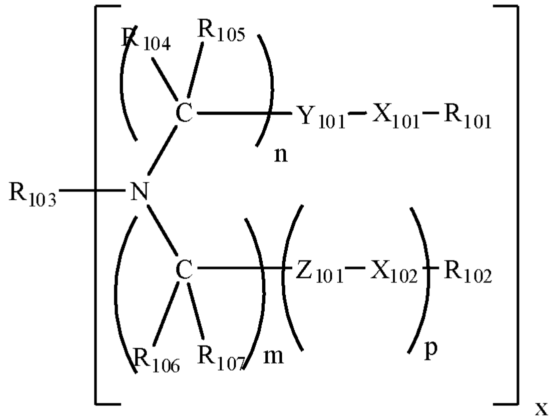

Formula (201),

wherein:

each of $X_{101}$ or $X_{102}$ is independently O, S, N-A or C-A, wherein A is hydrogen or a $C_1$-$C_{20}$ hydrocarbon chain;
each of $Y_{101}$ or $Z_{101}$ is independently C=O, C=S, S=O, CH-OH or $SO_2$;
each of $R_{101}$, $R_{102}$, $R_{103}$, $R_{104}$, $R_{105}$, $R_{106}$ or $R_{107}$ is independently hydrogen; a cyclic or an acyclic, substituted or unsubstituted, branched or linear aliphatic group; a cyclic or an acyclic, substituted or unsubstituted, branched or linear heteroaliphatic group; a substituted or unsubstituted, branched or linear acyl group; a substituted or unsubstituted, branched or linear aryl group; a substituted or unsubstituted, branched or linear heteroaryl group;
x is an integer of 1-10;
n is an integer of 1-3, m is an integer of 0-20, p is 0 or 1, wherein if m=p=0, then $R_{102}$ is hydrogen;
and if at least one of n or m is 2, then $R_{103}$ and nitrogen in Formula (201) form a structure as represented by Formula (202) or Formula (203):

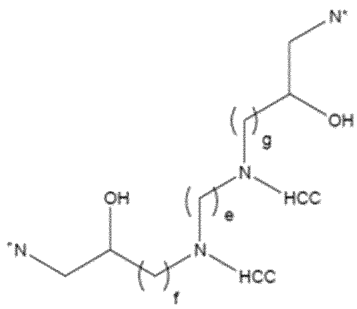

Formula (202),

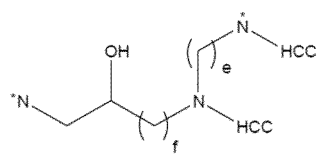

Formula (203);

wherein, each of g, e or f is independently an integer of 1-6, "HCC" represents a hydrocarbon chain, and each *N represents nitrogen atom in Formula (201); preferably, the organic amine is the organic amine represented by Formula (214) and/or the organic amine represented by Formula (215):

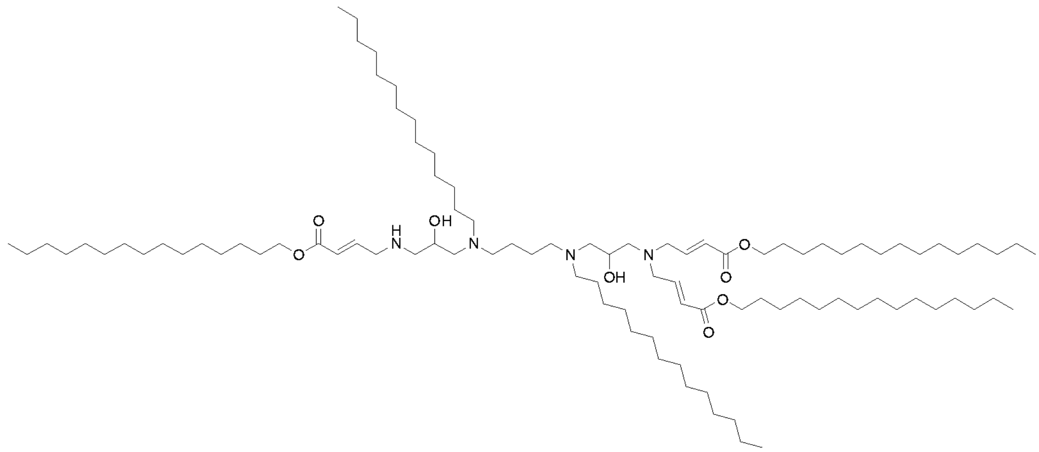

Formula (214),

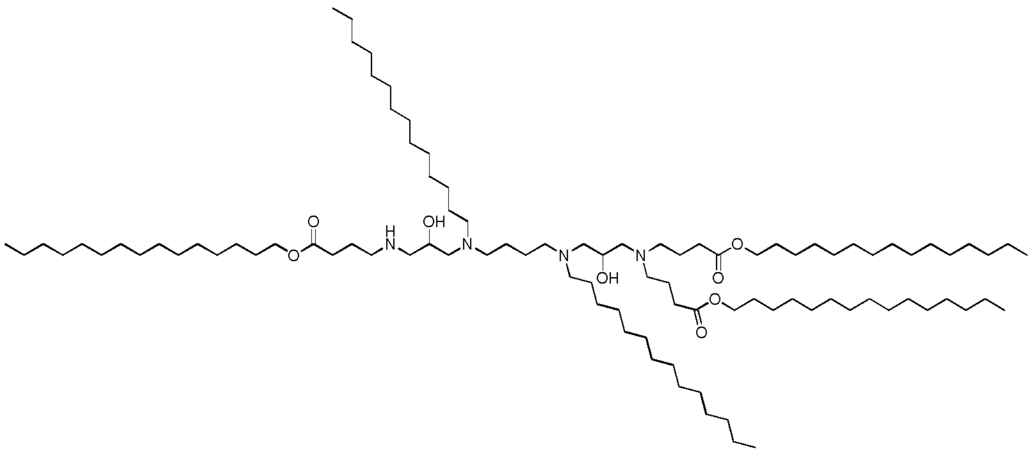

Formula (215);

the helper lipid is cholesterol, cholesterol analogues and/or cholesterol derivatives; and
the PEGylated lipid is 1,2-dipalmitoyl-sn-glycero-3-phosphatidylethanolamine-N-[methoxy(polyethylene glycol)]-2000.

**30.** The pharmaceutical composition according to claim 28 or 29, wherein the molar ratio of the organic amine, the helper lipid and the PEGylated lipid is (19.7-80) : (19.7-80) : (0.3-50); preferably, the molar ratio of the organic amine, the helper lipid and the PEGylated lipid is (50-70) : (20-40) : (3-20).

**31.** An siRNA conjugate comprising the siRNA according to any one of claims 1 to 26 and a conjugating group conjugated to the siRNA.

**32.** The siRNA conjugate according to claim 31, wherein the conjugating group comprises a pharmaceutically acceptable targeting group and a linker, and the siRNA, the linker and the targeting group are sequentially linked covalently or non-covalently; preferably, the linker has a structure represented by Formula (301):

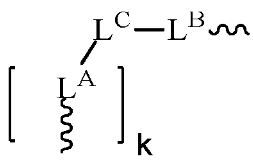

Formula (301),

wherein,

k is an integer of 1-3;

$L^A$ is an amide bond-comprising chain moiety having a structure represented by Formula (302), and each $L^A$ is respectively linked to the targeting group and the $L^C$ moiety through an ether bond at its two terminals:

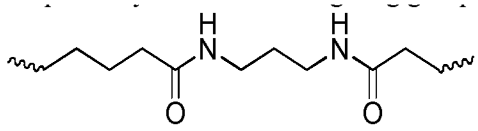

Formula (302);

$L^B$ is an N-acylpyrrolidine-comprising chain moiety having a structure represented by Formula (303), wherein the chain moiety has a carbonyl at its one terminal and is linked to the $L^C$ moiety through an amide bond, and has an oxygen atom at the other terminal and is linked to the siRNA via a phosphoester bond:

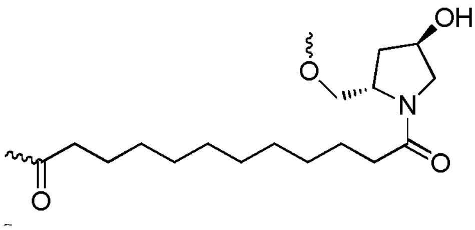

Formula (303);

$L^C$ is a bivalent to tetravalent linking group based on hydroxymethyl aminomethane, dihydroxymethyl aminomethane or trihydroxymethyl aminomethane, and is linked to each $L^A$ moiety through ether bond via an oxygen atom, and is linked to the $L^B$ moiety through amide bond via a nitrogen atom; preferably, the siRNA conjugate has a structure represented by Formula (305):

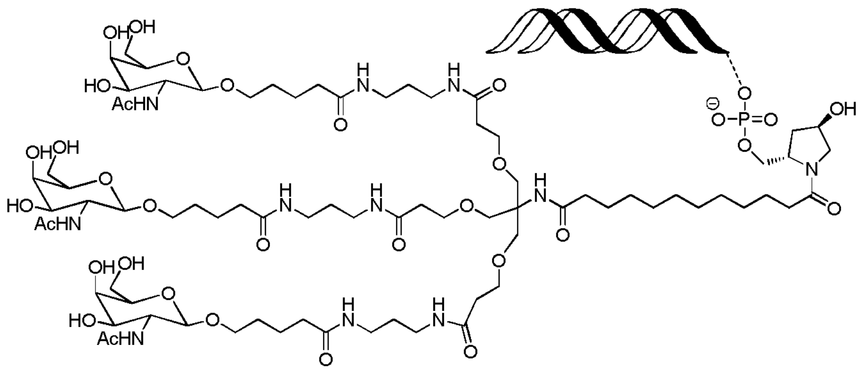

Formula (305),

wherein the double helix structure represents the siRNA.

**33.** The siRNA conjugate according to claim 32, wherein the linker has a structure represented by Formula (306):

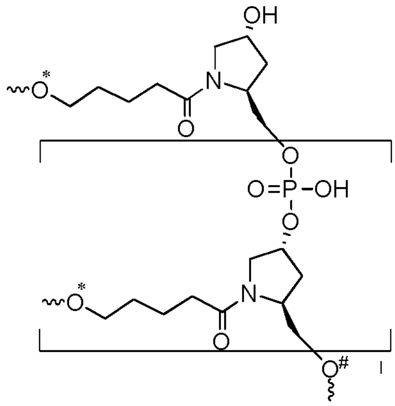

Formula (306),

wherein,

1 is an integer of 0-3;
* represents the site on the linker linked to the targeting group via an ether bond; and
# represents the site on the linker linked to the siRNA via a phosphoester bond.

**34.** The siRNA conjugate according to any one of claims 31 to 33, wherein the siRNA conjugate has a structure represented by Formula (307):

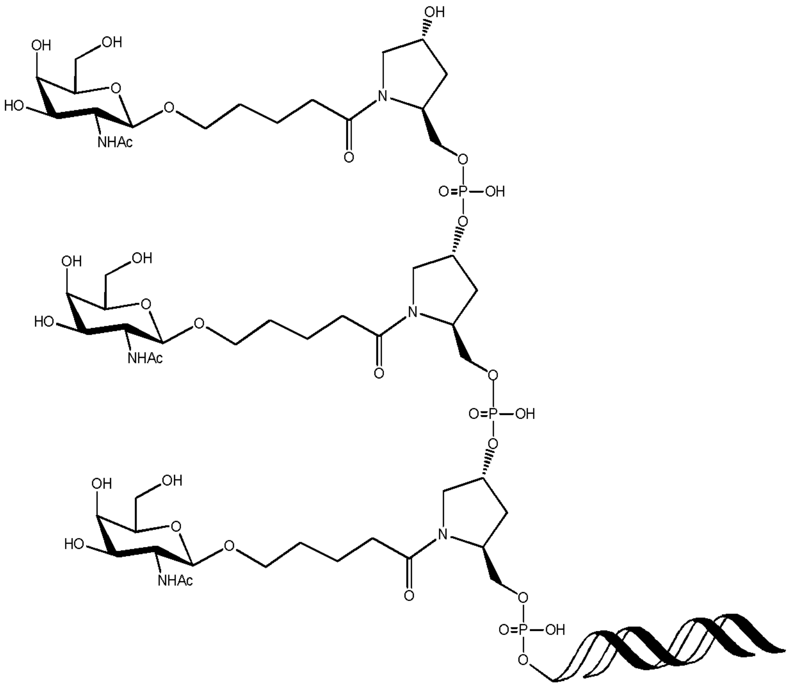

Formula (307),

wherein the double helix structure represents the siRNA.

**35.** The siRNA conjugate according to any one of claims 31 to 34, wherein the linker is linked to the 3' terminal of the sense strand of the siRNA.

**36.** The siRNA conjugate according to claim 35, wherein the conjugate has a structure represented by Formula (308):

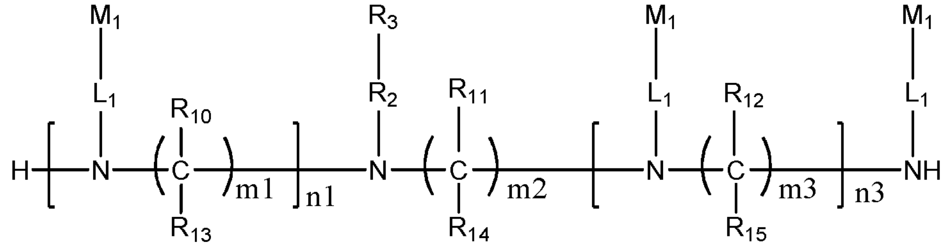

Formula (308),

wherein,

n1 is an integer of 1-3, and n3 is an integer of 0-4;
each of m1, m2, or m3 is independently an integer of 2-10;
each of $R_{10}$, $R_{11}$, $R_{12}$, $R_{13}$, $R_{14}$ or $R_{15}$ is independently H, or selected from the group consisting of $C_1$-$C_{10}$ alkyl, $C_1$-$C_{10}$ haloalkyl, and $C_1$-$C_{10}$ alkoxy;
$R_3$ is a group having a structure represented by Formula (A59):

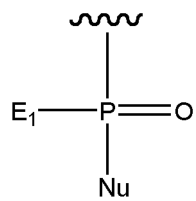

(A59)

wherein $E_1$ is OH, SH or $BH_2$, and Nu is the siRNA according to any one of claims 1 to 26;

$R_2$ is a linear alkylene of 1 to 20 carbon atoms in length, wherein one or more carbon atoms are optionally replaced with any one or more groups selected from the group consisting of: C(O), NH, O, S, CH=N, $S(O)_2$, $C_2$-$C_{10}$ alkeylene, $C_2$-$C_{10}$ alkynylene, $C_6$-$C_{10}$ arylene, $C_3$-$C_{18}$ heterocyclylene, and $C_5$-$C_{10}$ heteroarylene, and wherein $R_2$ is optionally substituted by any one or more groups selected from the group consisting of: $C_1$-$C_{10}$ alkyl, $C_6$-$C_{10}$ aryl, $C_5$-$C_{10}$ heteroaryl, $C_1$-$C_{10}$ haloalkyl, -$OC_1$-$C_{10}$ alkyl, -$OC_1$-$C_{10}$ alkylphenyl, -$C_1$-$C_{10}$ alkyl-OH, -$OC_1$-$C_{10}$ haloalkyl, -$SC_1$-$C_{10}$ alkyl, -$SC_1$-$C_{10}$ alkylphenyl, -$C_1$-$C_{10}$ alkyl-SH, -$SC_1$-$C_{10}$ haloalkyl, halo, -OH, -SH, -$NH_2$, -$C_1$-$C_{10}$ alkyl-$NH_2$, -N($C_1$-$C_{10}$ alkyl)($C_1$-$C_{10}$ alkyl), -NH($C_1$-$C_{10}$ alkyl), -N($C_1$-$C_{10}$ alkyl)($C_1$-$C_{10}$ alkylphenyl), -NH($C_1$-$C_{10}$ alkylphenyl), cyano, nitro, -$CO_2$H, -C(O)O($C_1$-$C_{10}$ alkyl), -CON($C_1$-$C_{10}$ alkyl)($C_1$-$C_{10}$ alkyl), -CONH($C_1$-$C_{10}$ alkyl), -$CONH_2$, -NHC(O)($C_1$-$C_{10}$ alkyl), -NHC(O)(phenyl), -N($C_1$-$C_{10}$ alkyl)C(O)($C_1$-$C_{10}$ alkyl), -N($C_1$-$C_{10}$ alkyl)C(O)(phenyl), -C(O)$C_1$-$C_{10}$ alkyl, -C(O)$C_1$-$C_{10}$ alkylphenyl, -C(O)$C_1$-$C_{10}$ haloalkyl, -OC(O)$C_1$-$C_{10}$ alkyl, -$SO_2$($C_1$-$C_{10}$ alkyl), -$SO_2$(phenyl), -$SO_2$($C_1$-$C_{10}$ haloalkyl), - $SO_2NH_2$, -$SO_2$NH($C_1$-$C_{10}$ alkyl), -$SO_2$NH(phenyl), -$NHSO_2$($C_1$-$C_{10}$ alkyl), -$NHSO_2$(phenyl), and -$NHSO_2$($C_1$-$C_{10}$ haloalkyl);
each $L_1$ is independently a linear alkylene of 1 to 70 carbon atoms in length, wherein one or more carbon atoms are optionally replaced with any one or more groups selected from the group consisting of: C(O), NH, O, S, CH=N, $S(O)_2$, $C_2$-$C_{10}$ alkeylene, $C_2$-$C_{10}$ alkynylene, $C_6$-$C_{10}$ arylene, $C_3$-$C_{18}$ heterocyclylene, and $C_5$-$C_{10}$ heteroarylene, and wherein $L_1$ is optionally substituted by any one or more groups selected from the group consisting of: $C_1$-$C_{10}$ alkyl, $C_6$-$C_{10}$ aryl, $C_5$-$C_{10}$ heteroaryl, $C_1$-$C_{10}$ haloalkyl, -$OC_1$-$C_{10}$ alkyl, -$OC_1$-$C_{10}$ alkylphenyl, -$C_1$-$C_{10}$ alkyl-OH, -$OC_1$-$C_{10}$ haloalkyl, -$SC_1$-$C_{10}$ alkyl, -$SC_1$-$C_{10}$ alkylphenyl, -$C_1$-$C_{10}$ alkyl-SH, -$SC_1$-$C_{10}$ haloalkyl, halo, -OH, -SH, -$NH_2$, -$C_1$-$C_{10}$ alkyl-$NH_2$, -N($C_1$-$C_{10}$ alkyl)($C_1$-$C_{10}$ alkyl), -NH($C_1$-$C_{10}$ alkyl), -N($C_1$-$C_{10}$ alkyl)($C_1$-$C_{10}$ alkylphenyl), -NH($C_1$-$C_{10}$ alkylphenyl), cyano, nitro, -$CO_2$H, -C(O)O($C_1$-$C_{10}$ alkyl), -CON($C_1$-$C_{10}$ alkyl)($C_1$-$C_{10}$ alkyl), -CONH($C_1$-$C_{10}$ alkyl), -$CONH_2$, -NHC(O)($C_1$-$C_{10}$ alkyl), -NHC(O)(phenyl), -N($C_1$-$C_{10}$ alkyl)C(O)($C_1$-$C_{10}$ alkyl), -N($C_1$-$C_{10}$ alkyl)C(O)(phenyl), -C(O)$C_1$-$C_{10}$ alkyl, -C(O)$C_1$-$C_{10}$ alkylphenyl, -C(O)$C_1$-$C_{10}$ haloalkyl, -OC(O)$C_1$-$C_{10}$ alkyl, -$SO_2$($C_1$-$C_{10}$ alkyl), -$SO_2$(phenyl), - $SO_2$($C_1$-$C_{10}$ haloalkyl), -$SO_2NH_2$, -$SO_2$NH($C_1$-$C_{10}$ alkyl), -$SO_2$NH(phenyl), -$NHSO_2$($C_1$-$C_{10}$ alkyl), - $NHSO_2$(phenyl), and -$NHSO_2$($C_1$-$C_{10}$ haloalkyl);

 represents the site at which a group is covalently linked; and
$M_1$ represent a targeting group.

**37.** The siRNA conjugate according to claim 36, wherein each $L_1$ is independently selected from the group consisting of groups A1-A26 and any combinations thereof:

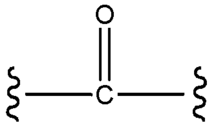,
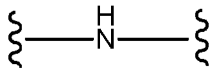,
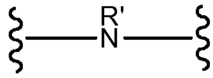,
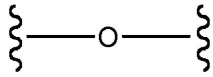,

(A1) (A2) (A3) (A4)

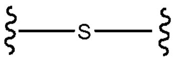,
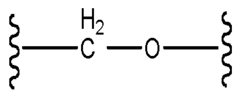,
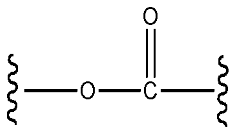,
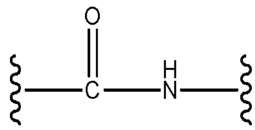,

(A5) (A6) (A7) (A8)

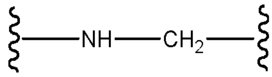,
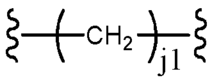,
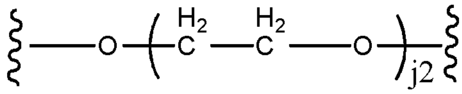,

(A9) (A10) (A11)

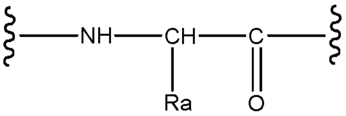,
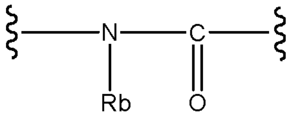,
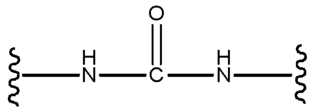,

(A12) (A13) (A14)

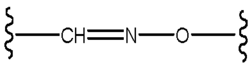,
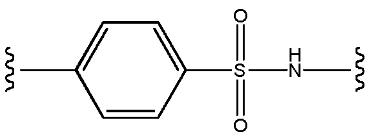,
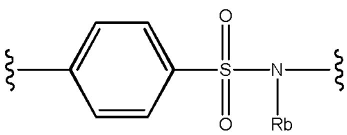,

(A15) (A16) (A17)

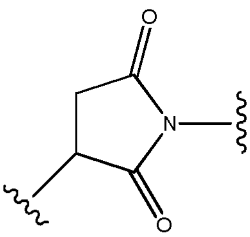,
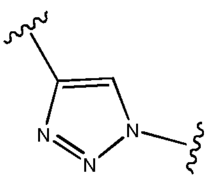,
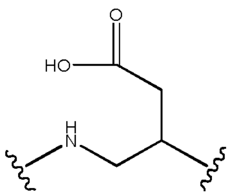,
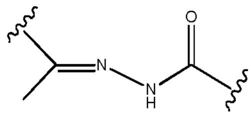,

(A18) (A19) (A20) (A21)

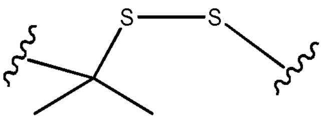, 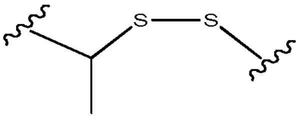, 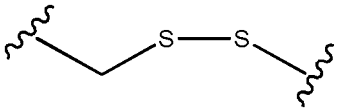,

(A22) (A23) (A24)

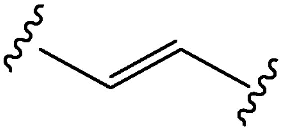, and 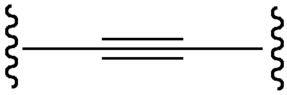;

(A25) (A26)

wherein,

each j1 is independently an integer of 1-20;
each j2 is independently an integer of 1-20;
each R' is independently a $C_1$-$C_{10}$ alkyl;
each Ra is selected from the group consisting of A27-A45 and any combinations thereof:

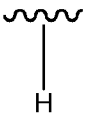, 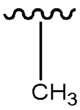, 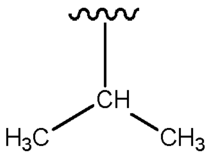, 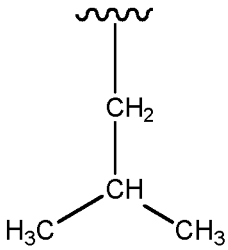, 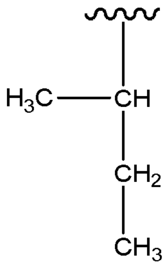, 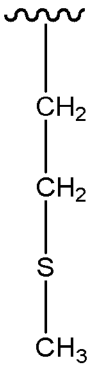,

(A27) (A28) (A29) (A30) (A31) (A32)

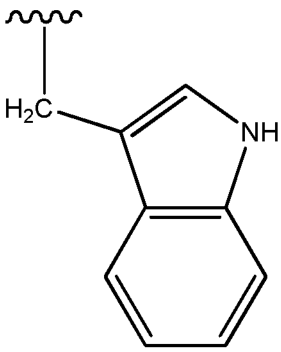, 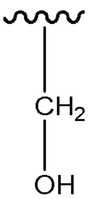, 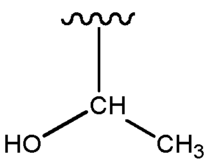, 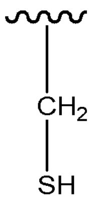, 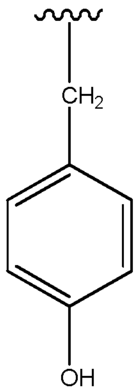,

(A33) (A34) (A35) (A36) (A37)

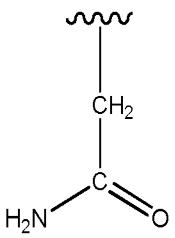, 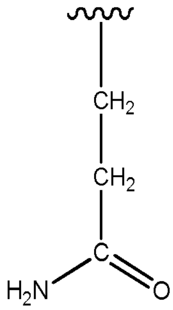, 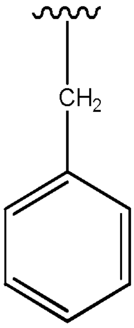, 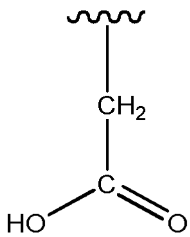, 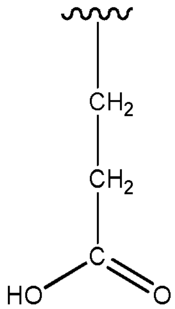,

(A38) (A39) (A40) (A41) (A42)

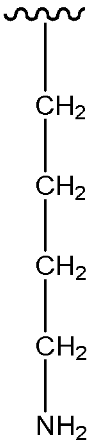, 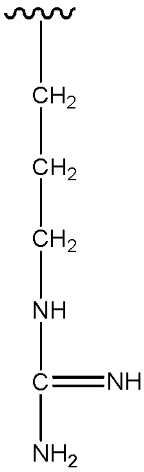 or 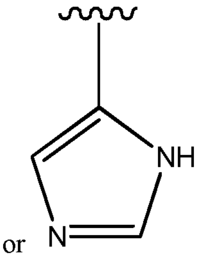;

(A43) (A44) (A45)

each Rb is independently a $C_1$-$C_{10}$ alkyl;

 represents the site at which a group is covalently linked.

**38.** The siRNA conjugate according to claim 39, wherein $L_1$ is selected from the group consisting of groups A1, A4, A5, A6, A8, A10, A11, A13 and connection combinations thereof; preferably, $L_1$ is a connection combination of at least two of groups A1, A4, A8, A10, and A11; preferably, $L_1$ is a connection combination of at least two of groups A1, A8, and A10.

**39.** The siRNA conjugate according to any one of claims 31 to 38, wherein $L_1$ is 3 to 25 atoms in length; preferably, $L_1$ is 4 to 15 atoms in length.

**40.** The siRNA conjugate according to any one of claims 37 to 39, wherein j1 is an integer of 2-10, j2 is an integer of 2-10, R' is a $C_1$-$C_4$ alkyl, Ra is one of A27, A28, A29, A30, and A31, and Rb is a $C_1$-$C_5$ alkyl; preferably, j1 is an integer of 3-5, j2 is an integer of 3-5, R' is one of methyl, ethyl, and isopropyl, Ra is A27 or A28, and Rb is one of methyl, ethyl, isopropyl, and butyl.

**41.** The siRNA conjugate according to any one of claims 38 to 40, wherein n1 is an integer of 1-2, n3 is an integer of 0-1, and n1+n3 = 2-3.

**42.** The siRNA conjugate according to any one of claims 36 to 41, wherein each m1, m2 and m3 are independently an integer of 2-5; preferably, m1=m2=m3.

**43.** The siRNA conjugate according to any one of claims 31 to 42, wherein each of the targeting group is independently a ligand having affinity to the asialoglycoprotein receptor on the surface of mammalian hepatocytes; preferably, each of the targeting groups is independently an asialoglycoprotein or a saccharide; preferably, each of the targeting

group is independently one selected from the group consisting of D-mannopyranose, L-mannopyranose, D-arabinose, D-xylofuranose, L-xylofuranose, D-glucose, L-glucose, D-galactose, L-galactose, α-D-mannofuranose, β-D-mannofuranose, α-D-mannopyranose, β-D-mannopyranose, α-D-glucopyranose, β-D-glucopyranose, α-D-glucofuranose, β-D-glucoiuranose, α-D-fructofuranose, α-D-fructopyranose, α-D-galactopyranose, β-D-galactopyranose, α-D-galactofuranose, β-D-galactofuranose, glucosamine, sialic acid, galactosamine, N-acetylgalactosamine, N-trifluoroacetylgalactosamine, N-propionylgalactosamine, N-n-butyrylgalactosamine, N-isobutyrylgalactosamine, 2-amino-3-O-[(R)-1-carboxyethyl]-2-deoxy-β-D-glucopyranose, 2-deoxy-2-methylamino-L-glucopyranose, 4,6-dideoxy-4-formamido-2,3-di-O-methyl-D-mannopyranose, 2-deoxy-2-sulfoamino-D-glucopyranose, N-glycolyl-α-neuraminic acid, 5-thio-β-D-glucopyranose, methyl 2,3,4-tris-O-acetyl-1-thio-6-O-trityl-α-D-glucopyranoside, 4-thio-β-D-galactopyranose, ethyl 3,4,6,7-tetra-O-acetyl-2-deoxy-1,5-dithio-α-D-glucoheptopyranoside, 2,5-anhydro-D-allononitrile, ribose, D-ribose, D-4-thioribose, L-ribose, or L-4-thioribose; preferably, at least one or each of the targeting groups is galactose or N-acetylgalactosamine.

**44.** The siRNA conjugate according to any one of claims 31 to 43, wherein $R_{10}$, $R_{11}$, $R_{12}$, $R_{13}$, $R_{14}$ or $R_{15}$ is independently H, methyl or ethyl.

**45.** The siRNA conjugate according to any one of claims 31 to 44, wherein $R_2$ comprises both a site linking to the N atom on the nitrogenous backbone and a site linking to the P atom in $R_3$; preferably, in $R_2$, the site linking to the N atom on the nitrogenous backbone forms an amide bond with the N atom, and the site linking to the P atom in $R_3$ forms a phosphoester bond with the P atom; preferably $R_2$ is selected from B5, B6, B5' or B6':

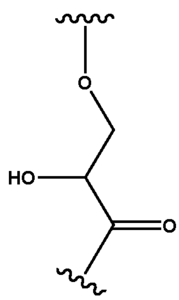
,
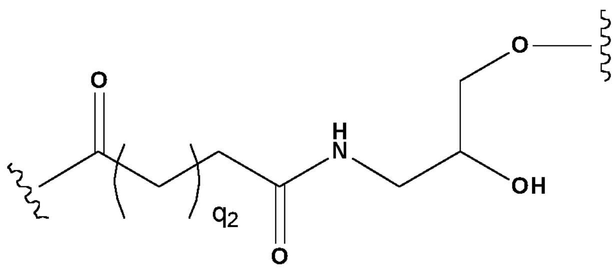
;

(B5) (B6)

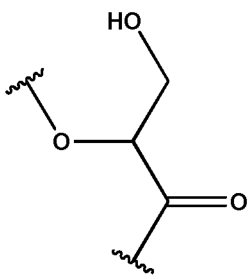
,
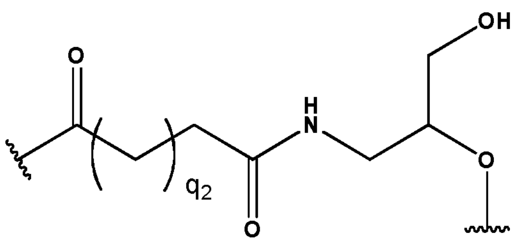
;

(B5') (B6')

wherein,



represents the site where a group is covalently linked, $q_2$ is an integer of 1-10; preferably, $q_2$ is an integer of 1-5.

**46.** The siRNA conjugate according to any one of claims 31 to 45, wherein the conjugate has a structure represented by Formula (403), (404), (405), (406), (407), (408), (409), (410), (411), (412), (413), (414), (415), (416), (417), (418), (419), (420), (421) or (422).

**47.** The siRNA conjugate according to any one of claims 31 to 46, wherein the P atom in Formula (A59) is linked to a terminal region of the sense strand or the antisense strand of the siRNA, wherein the terminal region refers to the first 4 nucleotides counting from either terminal of the sense strand or the antisense strand; preferably, the P atom in Formula (A59) is linked to a terminal of the sense strand or the antisense strand of the siRNA; preferably, the P atom in the Formula (A59) is linked to 3' terminal of the sense strand of the siRNA; preferably, the P atom in Formula (A59) is linked to position 2', 3', or 5' of a nucleotide in the siRNA by forming a phosphodiester bond.

**48.** Use of the siRNA according to any one of claims 1 to 26, the pharmaceutical composition according to any one of claims 27 to 30 and/or the siRNA conjugate according to any one of claims 31 to 47 in the manufacture of a medicament for treating and/or preventing abnormal uric acid metabolism or diseases or physiological conditions caused by it.

**49.** The use according to claim 48, wherein the disease or physiological condition caused by abnormal uric acid metabolism is hyperuricemia or gout.

**50.** A method for treating and/or preventing abnormal uric acid metabolism or diseases or physiological conditions caused by it, wherein the method comprises administering an effective amount of the siRNA according to any one of claims 1 to 26, the pharmaceutical composition according to any one of claims 27 to 30 and/or the siRNA conjugate according to any one of claims 31 to 47 to a subject suffering from abnormal uric acid metabolism.

**51.** The method according to claim 50, wherein the disease or physiological condition caused by abnormal uric acid metabolism is hyperuricemia or gout.

**52.** A method for inhibiting the expression of PNP gene in hepatocytes, comprising contacting an effective amount of the siRNA according to any one of claims 1 to 26, the pharmaceutical composition according to any one of claims 27 to 30 and/or the siRNA conjugate according to any one of claims 31 to 47 with the hepatocytes.

**53.** A kit, comprising the siRNA according to any one of claims 1 to 26, the pharmaceutical composition according to any one of claims 27 to 30 and/or the siRNA conjugate according to any one of claims 31 to 47.

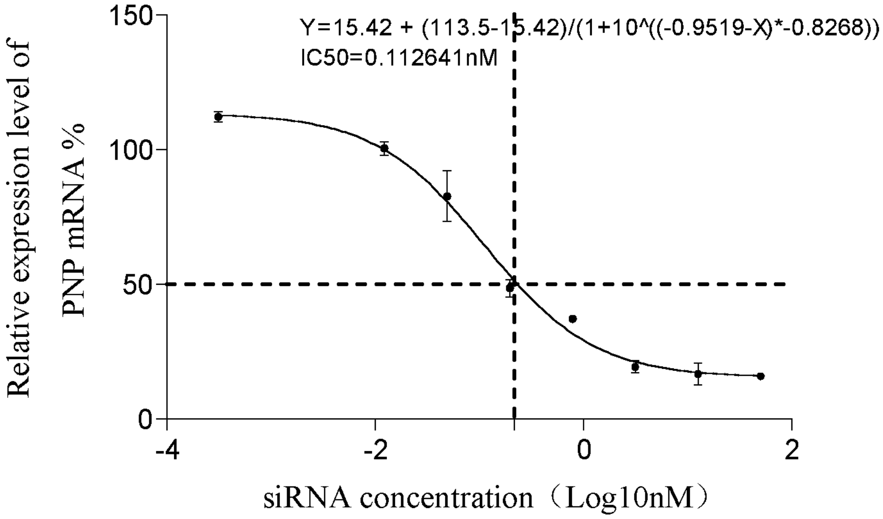


Figure 1A

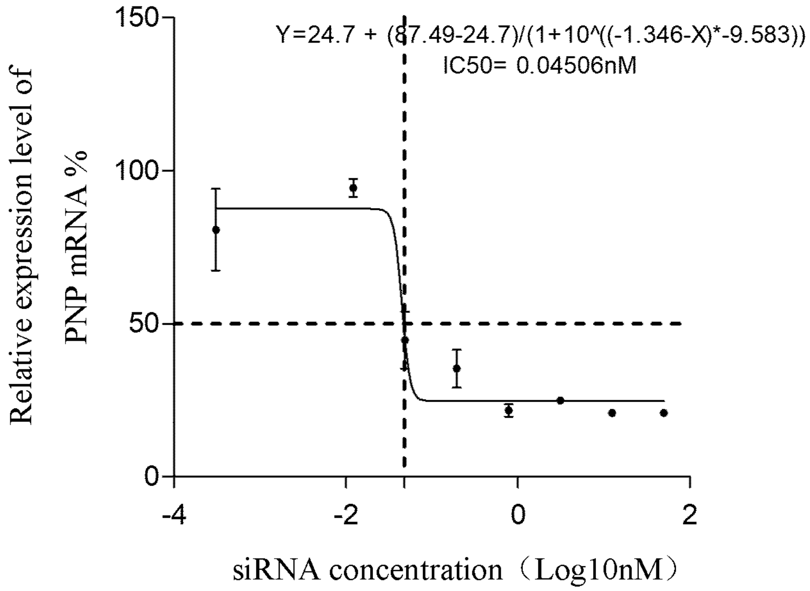


Figure 1B

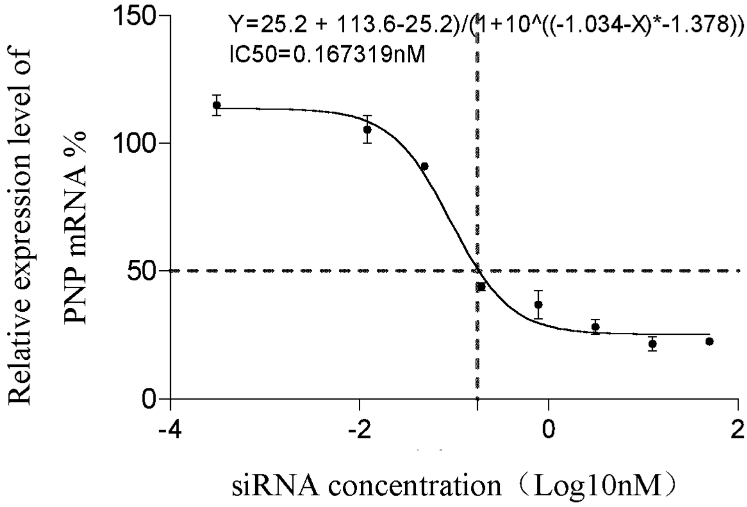


Figure 1C

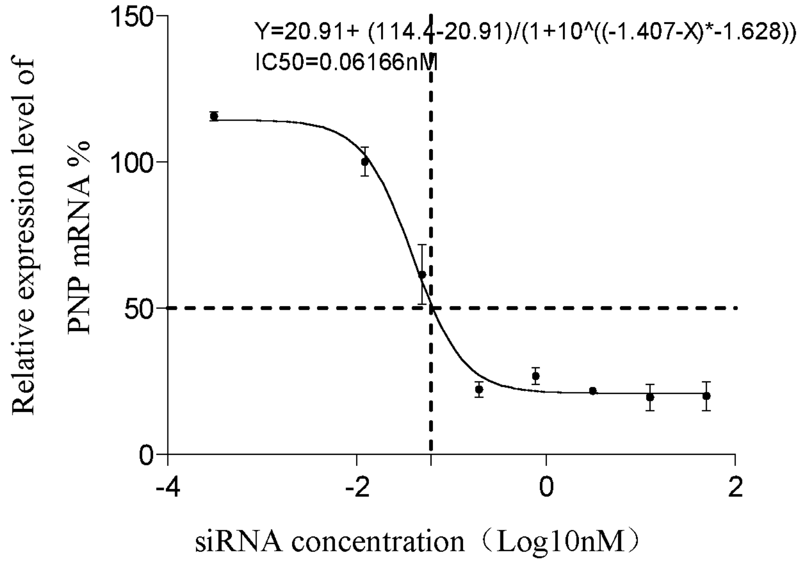


Figure 1D

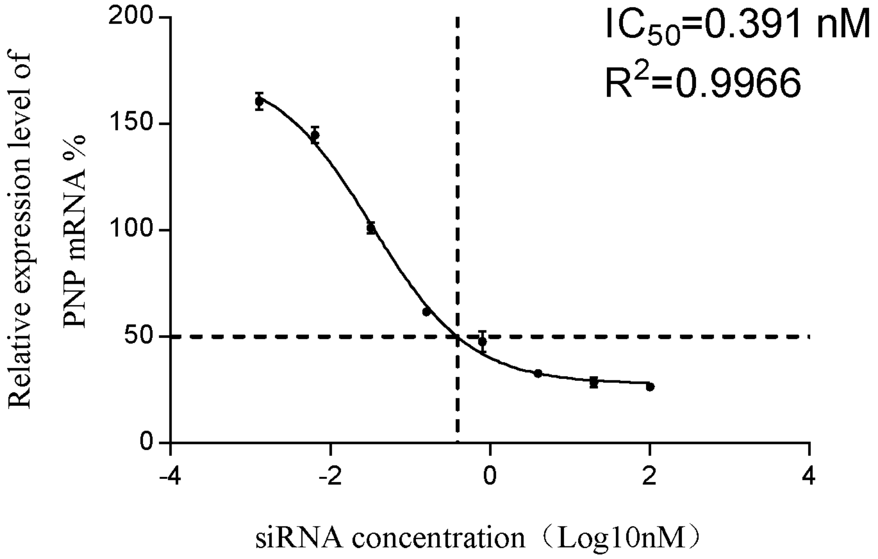


Figure 2A

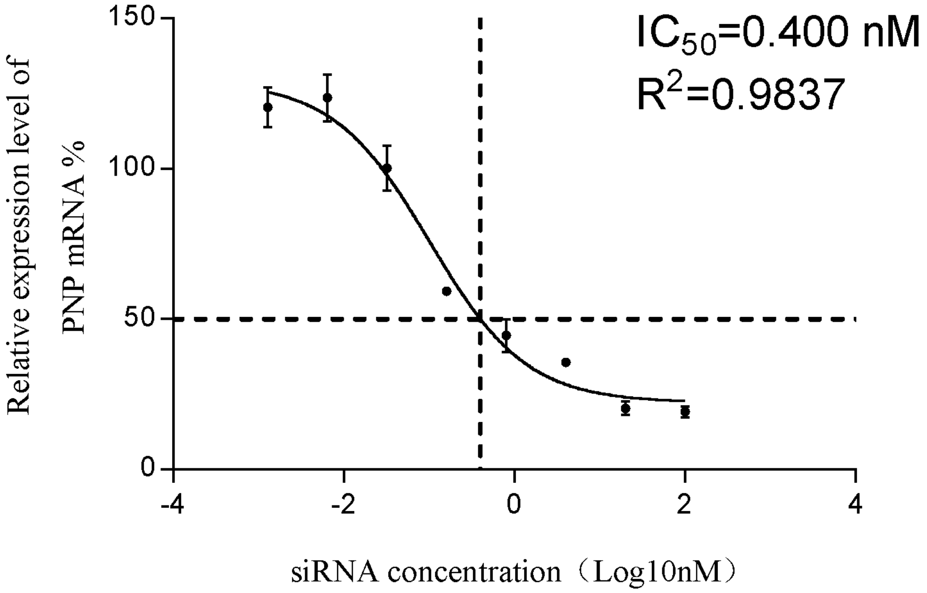


Figure 2B

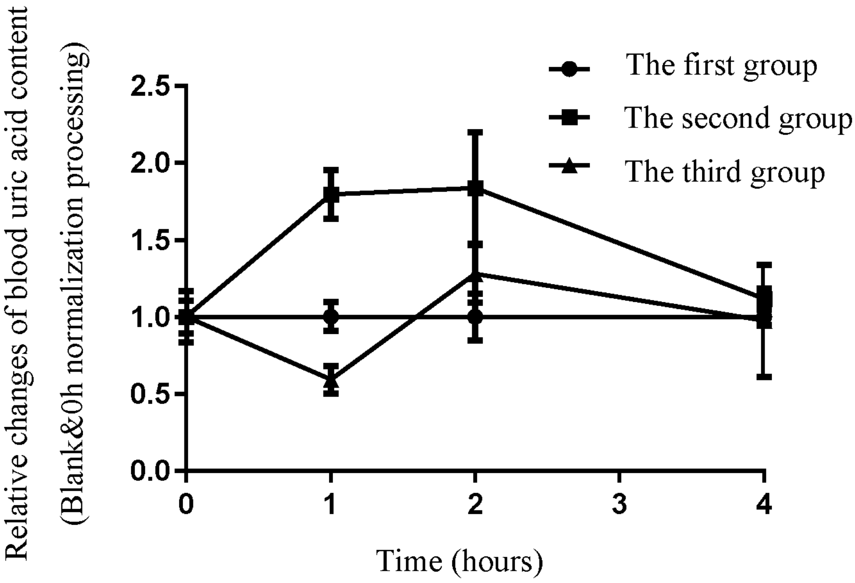
Relative changes of blood uric acid content
(Blank&0h normalization processing)
2.5
2.0
1.5
1.0
0.5
0.0
0
1
2
3
4
Time (hours)
The first group
The second group
The third group

Figure 3

## INTERNATIONAL SEARCH REPORT

International application No.
**PCT/CN2020/091485**

**A. CLASSIFICATION OF SUBJECT MATTER**

C12N 15/113(2010.01)i； C07D 211/58(2006.01)i； C07C 229/16(2006.01)i； C07D 211/28(2006.01)i； C07C 229/26(2006.01)i； C07C 237/06(2006.01)i； C07C 229/24(2006.01)i； A61K 31/7088(2006.01)i； A61K 47/54(2017.01)i； A61P 19/06(2006.01)i

According to International Patent Classification (IPC) or to both national classification and IPC

**B. FIELDS SEARCHED**

Minimum documentation searched (classification system followed by classification symbols)

C12N, C07D, C07C, A61K, A61P

Documentation searched other than minimum documentation to the extent that such documents are included in the fields searched

Electronic data base consulted during the international search (name of data base and, where practicable, search terms used)

CNABS, CNTXT, CSCD, CJFD, SIPONPL, DWPI, SIPOABS, CATXT, USTXT, EPTXT, WOTXT, KRTXT, JPTXT, CPEA, CNKI, WEB OF SCIENCE, PUBMED: 嘌呤核苷磷酸化酶, 痛风, Purine Nucleoside Phosphorylase, pnpase, pnp, gout, sirna, shrna, dsrna, "ccuaugaagauuauaaga", "cctatgaagattataaga", "guacaguaccagaaguua", "gtacagtaccagaagtta"

**C. DOCUMENTS CONSIDERED TO BE RELEVANT**

| Category* | Citation of document, with indication, where appropriate, of the relevant passages | Relevant to claim No. |
|---|---|---|
| A | WO 2015006498 A2 (HARVARD COLLEGE et al.) 15 January 2015 (2015-01-15) see claims 93-95, SEQ ID NO: 2581, figure 11 | 1-49 and 53 (all in part) |
| A | CN 106232831 A (THE UNITED STATES OF AMERICA, AS REPRESENTED BY THE SECRETARY, DEPARTMENT OF HEALTH & HUMAN SERVICES et al.) 14 December 2016 (2016-12-14) see table 1, SEQ ID NO: 1549 | 1-49 and 53 (all in part) |
| A | CN 104717982 A (ALNYLAM PHARMACEUTICALS, INC.) 17 June 2015 (2015-06-17) see claims 1-26 | 1-49 and 53 (all in part) |
| A | WO 2015006740 A2 (ALNYLAM PHARMACEUTICALS INC) 15 January 2015 (2015-01-15) see entire document | 1-49 and 53 (all in part) |
| A | CN 103380113 A (LIFE TECHNOLOGIES CORP) 30 October 2013 (2013-10-30) see claims 1-42 | 1-49 and 53 (all in part) |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **11 August 2020** | **25 August 2020** |
| Name and mailing address of the ISA/CN<br>**China National Intellectual Property Administration (ISA/CN)**<br>**No. 6, Xitucheng Road, Jimenqiao Haidian District, Beijing 100088**<br>**China** | Authorized officer |
| Facsimile No. **(86-10)62019451** | Telephone No. |

Form PCT/ISA/210 (second sheet) (January 2015)

INTERNATIONAL SEARCH REPORT

| International application No. |
|---|
| PCT/CN2020/091485 |

C. DOCUMENTS CONSIDERED TO BE RELEVANT

| Category* | Citation of document, with indication, where appropriate, of the relevant passages | Relevant to claim No. |
|---|---|---|
| A | Jonathan K Watts et al. "Chemically modified siRNA: tools and applications" *Drug Discov Today,* Vol. 13, No. 19-20, 07 July 2008 (2008-07-07), ISSN: 1359-6446, pages 842-855, see figure 2 | 1-49 and 53 (all in part) |
| A | Luis EmilianoPeña-Altamira et al. "Release of soluble and vesicular purine nucleoside phosphorylase from rat astrocytes and microglia induced by pro-inflammatory stimulation with extracellular ATP via P2X7 receptors" *Neurochemistry International,* Vol. 115, 31 May 2018 (2018-05-31), ISSN: 0197-0186, pages 37-49, see page 40 right-hand column paragraph 3 | 1-49 and 53 (all in part) |
| A | Kallanthottathil G Rajeev et al. "Hepatocyte-specific delivery of siRNAs conjugated to novel non-nucleosidic trivalent N-acetylgalactosamine elicits robust gene silencing in vivo" *Chembiochem,* Vol. 16, No. 6, 13 April 2015 (2015-04-13), ISSN: 1439-4227, pages 903-908, see abstract, figure 1, page 905 | 1-49 and 53 (all in part) |

Form PCT/ISA/210 (second sheet) (January 2015)

INTERNATIONAL SEARCH REPORT

International application No.
**PCT/CN2020/091485**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **50-52**
because they relate to subject matter not required to be searched by this Authority, namely:

[1] Claims 50-52 relate to a method for treatment of the human or animal body.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

[1] Invention one: claims 1-49 and 53 (all in part) relate to subject matter related to SEQ ID NOs: 1-8, siPNa1 and siPNa2, and modified nucleotides thereof.

[2] Invention two: claims 1-49 and 53 (all in part) relate to subject matter related to SEQ ID NOs: 61-68, siPNb1 and siPNb2, and modified nucleotides thereof.

[3] Invention three: claims 1-49 and 53 (all in part) relate to subject matter related to SEQ ID NOs: 121-128, siPNc1 and siPNc2, and modified nucleotides thereof.

[4] Invention four: claims 1-49 and 53 (all in part) relate to subject matter related to SEQ ID NOs: 181-188, siPNd1 and siPNd2, and modified nucleotides thereof.

[5] The technical feature shared by inventions one to four described above is siRNAs having general structures. However, the general siRNA structures are well-known siRNA frameworks in the present field. Therefore, inventions one to four described above do not share a same or corresponding special technical feature, and thus do not satisfy the criteria of PCT Rule 13.1, 13.2 and 13.3.

Form PCT/ISA/210 (continuation of first sheet) (January 2015)

**INTERNATIONAL SEARCH REPORT**

International application No.
**PCT/CN2020/091485**

**Box No. III Observations where unity of invention is lacking (Continuation of item 3 of first sheet)**

1. ☐ As all required additional search fees were timely paid by the applicant, this international search report covers all searchable claims.

2. ☐ As all searchable claims could be searched without effort justifying additional fees, this Authority did not invite payment of additional fees.

3. ☑ As only some of the required additional search fees were timely paid by the applicant, this international search report covers only those claims for which fees were paid, specifically claims Nos.: **The subject matter in claims 1-49 and 53 (all in part) related to SEQ ID NOs: 1-8, siPNa1 and siPNa2, and SEQ ID NOs: 61-68, siPNb1 and siPNb2, and modified nucleotides thereof.**

4. ☐ No required additional search fees were timely paid by the applicant. Consequently, this international search report is restricted to the invention first mentioned in the claims; it is covered by claims Nos.:

**Remark on Protest**

☑ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

**INTERNATIONAL SEARCH REPORT**
**Information on patent family members**

International application No.
**PCT/CN2020/091485**

| Patent document cited in search report | Publication date (day/month/year) | Patent family member(s) | Publication date (day/month/year) |
|---|---|---|---|
| WO 2015006498 A2 | 15 January 2015 | WO 2016057835 A2 | 14 April 2016 |
| | | WO 2015006498 A3 | 02 April 2015 |
| | | US 2015152436 A1 | 04 June 2015 |
| | | EP 3019595 A2 | 18 May 2016 |
| | | EP 3019595 A4 | 30 November 2016 |
| | | US 2020048659 A1 | 13 February 2020 |
| | | US 10208319 B2 | 19 February 2019 |
| | | JP 2016528890 A | 23 September 2016 |
| | | WO 2016057835 A3 | 25 August 2016 |
| CN 106232831 A | 14 December 2016 | US 2016283653 A1 | 29 September 2016 |
| | | KR 20160127713 A | 04 November 2016 |
| | | WO 2015069790 A9 | 02 June 2016 |
| | | AU 2014346788 A1 | 26 May 2016 |
| | | EP 3066215 B1 | 24 April 2019 |
| | | US 10607717 B2 | 31 March 2020 |
| | | JP 2020078323 A | 28 May 2020 |
| | | WO 2015069790 A1 | 14 May 2015 |
| | | US 2020143906 A1 | 07 May 2020 |
| | | EP 3066215 A1 | 14 September 2016 |
| | | AU 2014346788 A8 | 16 June 2016 |
| | | JP 2017502686 A | 26 January 2017 |
| | | CA 2929826 A1 | 14 May 2015 |
| | | EP 3594359 A1 | 15 January 2020 |
| | | ES 2738289 T3 | 21 January 2020 |
| | | JP 6657105 B2 | 04 March 2020 |
| CN 104717982 A | 17 June 2015 | AU 2013299717 B2 | 28 June 2018 |
| | | JP 2015525797 A | 07 September 2015 |
| | | US 10086081 B2 | 02 October 2018 |
| | | EP 2879718 A1 | 10 June 2015 |
| | | JP 2018062520 A | 19 April 2018 |
| | | US 2015196655 A1 | 16 July 2015 |
| | | WO 2014025805 A1 | 13 February 2014 |
| | | HK 1210952 A1 | 13 May 2016 |
| | | AU 2013299717 A1 | 26 February 2015 |
| | | US 2019134206 A1 | 09 May 2019 |
| | | CN 104717982 B | 28 August 2018 |
| | | CA 2879693 A1 | 13 February 2014 |
| WO 2015006740 A2 | 15 January 2015 | US 2016376585 A1 | 29 December 2016 |
| | | EP 3019200 A2 | 18 May 2016 |
| | | AU 2014287002 A1 | 11 February 2016 |
| | | JP 2016529230 A | 23 September 2016 |
| | | JP 6702862 B2 | 03 June 2020 |
| | | CA 2917161 A1 | 15 January 2015 |
| | | WO 2015006740 A3 | 07 May 2015 |
| | | AU 2020202093 A1 | 09 April 2020 |
| CN 103380113 A | 30 October 2013 | US 2018200374 A1 | 19 July 2018 |
| | | JP 2018080187 A | 24 May 2018 |
| | | JP 6383480 B2 | 29 August 2018 |
| | | JP 2017031186 A | 09 February 2017 |
| | | WO 2012068176 A1 | 24 May 2012 |

Form PCT/ISA/210 (patent family annex) (January 2015)

**INTERNATIONAL SEARCH REPORT**
**Information on patent family members**

International application No.
**PCT/CN2020/091485**

| Patent document cited in search report | Publication date (day/month/year) | Patent family member(s) | | | Publication date (day/month/year) |
|---|---|---|---|---|---|
| | | EP | 2640700 | A1 | 25 September 2013 |
| | | JP | 2014508102 | A | 03 April 2014 |
| | | US | 2012136073 | A1 | 31 May 2012 |
| | | EP | 3470395 | A1 | 17 April 2019 |
| | | US | 2020061197 | A1 | 27 February 2020 |
| | | CN | 103380113 | B | 30 March 2018 |
| | | ES | 2702428 | T3 | 28 February 2019 |
| | | JP | 2018197255 | A | 13 December 2018 |
| | | US | 2015174260 | A1 | 25 June 2015 |
| | | DK | 2640700 | T3 | 14 January 2019 |
| | | EP | 2640700 | B1 | 31 October 2018 |
| | | US | 10406237 | B2 | 10 September 2019 |
| | | CN | 108358812 | A | 03 August 2018 |
| | | US | 9901642 | B2 | 27 February 2018 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 103380113 A
- WO 2009082607 A2
- CN 105378082 A
- WO 2015006740 A2
- WO 2014025805 A1
- US 8106022 B2


### Non-patent literature cited in the description


- **BEAUCAGE et al.** *Tetrahedron,* 1992, vol. 48, 2223-2311
- **GREENE ; WUTS.** Protective Groups in Organic Synthesis. John Wiley & Sons, 1991
- *Nature Biotechnology,* 2017, vol. 35 (3), 238-48
- **MUTHIAH MANOHARAN.** siRNA conjugates carrying sequentially assembled trivalent N-acetylgalactosamine linked through nucleosides elicit robust gene silencing in vivo in hepatocytes. *ACS Chemical biology,* 2015, vol. 10 (5), 1181-7
- **RAJEEV et al.** *ChemBioChem,* 2015, vol. 16, 903-908
- *J. Am. Chem. Soc.,* 2014, vol. 136, 16958-16961
- Molecular Cloning. Cold Spring Harbor Laboratory Press, 1989
- *CHEMICAL ABSTRACTS,* 1772-03-8
- *CHEMICAL ABSTRACTS,* 27607-77-8
- *CHEMICAL ABSTRACTS,* 821-41-0
- *CHEMICAL ABSTRACTS,* 7790-28-5
- *CHEMICAL ABSTRACTS,* 14898-67-0